(19) Europäisches Patentamt / European Patent Office / Office européen des brevets

(11) EP 4 624 468 A1

(12) **EUROPEAN PATENT APPLICATION**
published in accordance with Art. 153(4) EPC

(43) Date of publication:
**01.10.2025 Bulletin 2025/40**

(21) Application number: **23894237.9**

(22) Date of filing: **12.09.2023**

(51) International Patent Classification (IPC):
***C07D 403/14*** *(2006.01)* ***C07D 487/04*** *(2006.01)*
***H10K 85/60*** *(2023.01)* ***H10K 101/20*** *(2023.01)*
***H10K 101/30*** *(2023.01)* ***H10K 101/40*** *(2023.01)*
***H10K 50/12*** *(2023.01)* ***H10K 59/10*** *(2023.01)*

(52) Cooperative Patent Classification (CPC):
**C07D 403/14; C07B 59/002; C07D 487/04; C07D 491/048; C07D 495/04; C07D 519/00; H10K 50/12; H10K 59/10; H10K 85/60;**
C07B 2200/05; H10K 2101/20; H10K 2101/30; H10K 2101/40

(86) International application number:
**PCT/JP2023/033219**

(87) International publication number:
**WO 2024/111223 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.11.2022 JP 2022186514**

(71) Applicant: **Kyulux, Inc.**
**Fukuoka-shi, Fukuoka 819-0388 (JP)**

(72) Inventors:
- **CHO, Yong Joo**
**Fukuoka-shi, Fukuoka 819-0388 (JP)**
- **YAMANE, Yu**
**Fukuoka-shi, Fukuoka 819-0388 (JP)**
- **SHIMAMURA, Naomi**
**Fukuoka-shi, Fukuoka 819-0388 (JP)**
- **HAMASAKI, Taro**
**Fukuoka-shi, Fukuoka 819-0388 (JP)**
- **MORIMOTO, Kei**
**Fukuoka-shi, Fukuoka 819-0388 (JP)**
- **KODAMA, Yuka**
**Fukuoka-shi, Fukuoka 819-0388 (JP)**
- **KANAHARA, Kousei**
**Fukuoka-shi, Fukuoka 819-0388 (JP)**

(74) Representative: **Zimmermann & Partner Patentanwälte mbB**
**P.O. Box 330 920**
**80069 München (DE)**

# (54) COMPOUND, LIGHT-EMITTING MATERIAL AND LIGHT-EMITTING ELEMENT

(57) An organic light emitting device using a compound of the following general formula has excellent characteristics. $R^1$ to $R^5$ each are H, D, a cyano group, an alkyl group, an aryl group or a donor group, one or more are cyano groups, one or more are donor groups; $X^1$ to $X^3$ each are N or C(R); R is H, D or a substituent; one or more of $Ar^1$ and $Ar^2$ each are a heteroaryl group bonding via N; $L^1$ is a single bond or a linked group.

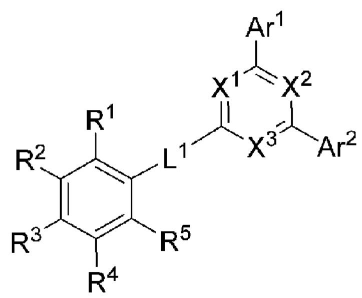

EP 4 624 468 A1

## Description

### Technical Field

The present invention relates to a compound useful as a light emitting material, and a light emitting device using the compound.

### Background Art

Studies for enhancing the light emission efficiency of light emitting devices such as organic electroluminescent devices (organic EL devices) are being made actively. In particular, various kinds of efforts have been made for increasing light emission efficiency by newly developing and combining an electron transporting material, a hole transporting material, and a light emitting material to constitute an organic electroluminescent device. Among them, there are seen some reports relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher light emission efficiency.

Since such a principle has been clarified, various studies have led to the discovery of various delayed fluorescent materials. Among these, many compounds in which a benzene ring is substituted with a donor group and an acceptor group are included. For example, a compound having the following skeleton, in which the benzene ring is substituted with a carbazol-9-yl group of a donor group and with a cyano group and a substituted triazinyl group of acceptor groups, has been proposed (see PTL 1).

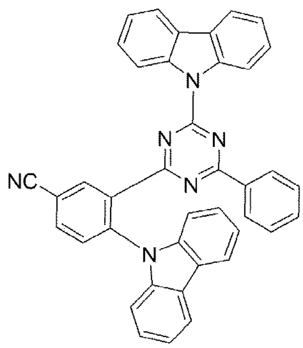

### Citation List

### Patent Literature

PTL 1:WO2022/074122A1

### Summary of Invention

### Technical Problem

Even if a material emits delayed fluorescence, one having extremely good characteristics and having no problem in practical use has not been provided. For example, PTL 1 describes that use of the above-mentioned delayed fluorescent material can improve the lifetime of an organic electroluminescent device, but the effect of improving the device lifetime is not sufficient. Accordingly, it would be even more useful if a delayed fluorescent material with further more superior properties can be provided. However, the improvement of delayed fluorescent materials is in the stage of trial and error,

and it is not easy to generalize the chemical structure of useful light emitting materials.

Under such circumstances, the present inventors have conducted research for the purpose of providing a compound more useful as a delayed fluorescent material for a light emitting device. Then, the present inventors have conducted intensive studies for the purpose of deriving and generalizing a general formula of a compound more useful as a delayed fluorescent material.

Solution to Problem

As a result of intensive studies for achieving the above object, the present inventors have found that a compound having a structure that satisfies a specific requirement is useful as a light emitting material. The present invention has been proposed based on these findings, and specifically has the following configuration.

[1] A compound represented by the following general formula (1).

General Formula (1)

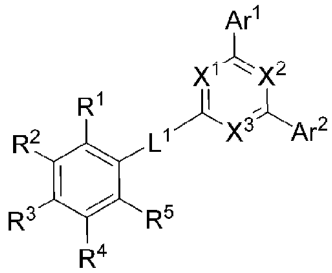

In the general formula (1), $R^1$ to $R^5$ each independently represent a hydrogen atom, a deuterium atom, a cyano group, a substituted or unsubstituted alkyl group, a substituted or unsubstituted aryl group, or a donor group. One or more of $R^1$ to $R^5$ is a cyano group, one or more of $R^1$ to $R^5$ is a donor group, 0 to 2 of $R^1$ to $R^5$ is a hydrogen atom or a deuterium atom, and 0 to 1 of $R^1$ to $R^5$ is a substituted or unsubstituted aryl group. $X^1$ to $X^3$ each independently represent N or C(R), and at least one of $X^1$ to $X^3$ is N; R represents a hydrogen atom, a deuterium atom or a substituent; $Ar^1$ and $Ar^2$ each independently represent a substituted or unsubstituted aryl group, or a substituted or unsubstituted heteroaryl group containing a nitrogen atom as a ring skeleton-constituting atom, and at least one of $Ar^1$ and $Ar^2$ is a substituted or unsubstituted heteroaryl group bonding via the nitrogen atom; $L^1$ represents a single bond or a divalent linking group.
[2] The compound according to [1], wherein only one of $R^1$ to $R^5$ is a cyano group.
[3] The compound according to [2], wherein $R^2$ is a cyano group.
[4] The compound according to any one of [1] to [3], wherein only one of $R^1$ to $R^5$ is a substituted or unsubstituted aryl group.
[5] The compound according to [4], wherein $R^4$ is a substituted or unsubstituted aryl group.
[6] The compound according to any one of [1] to [5], wherein two of $R^1$ to $R^5$ are donor groups.
[7] The compound according to any one of [1] to [5], wherein three of $R^1$ to $R^5$ are donor groups.
[8] The compound according to any one of [1] to [7], wherein the donor group is a substituted or unsubstituted carbazol-9-yl group.
[9] The compound according to any one of [1] to [8], wherein $R^3$ to $R^5$ each are independently a substituted or unsubstituted aryl group, or a donor group.
[10] The compound according to any one of [1] to [9], wherein $X^1$ to $X^3$ are N.
[11] The compound according to any one of [1] to [10], wherein $Ar^1$ is a substituted or unsubstituted carbazol-9-yl group, and $Ar^2$ is a substituted or unsubstituted aryl group.
[12] The compound according to any one of [1] to [10], wherein $Ar^1$ and $Ar^2$ each are independently a substituted or unsubstituted carbazol-9-yl group.
[13] The compound according to any one of [1] to [12], wherein $L^1$ is a single bond.
[14] The compound according to any one of [1] to [13], wherein $R^1$ is a hydrogen atom.
[15] The compound according to any one of [1] to [14], wherein the compound has at least one deuterium atom.
[16] A light emitting material including the compound according to any one of [1] to [15].
[17] A delayed fluorescent material including the compound according to any one of [1] to [15].
[18] A film including the compound according to any one of [1] to [15].
[19] An organic semiconductor device including the compound according to any one of [1] to [15].
[20] An organic light emitting device including the compound according to any one of [1] to [15].
[21] The organic light emitting device according to [20], wherein the device has a layer containing the compound, and

the layer also contains a host material.
[22] The organic light emitting device according to [21],wherein the layer containing the compound further contains a delayed fluorescent material in addition to the compound and the host material, and the delayed fluorescent material has a lowest excited singlet energy lower than that of the host material and higher than that of the compound.
[23] The organic light emitting device according to [21] or [22], wherein the device has a layer containing the compound, and the layer also contains a light emitting material having a structure different from that of the compound.
[24] The organic light emitting device according to [21] or [22], wherein the amount of light emitted from the compound is the largest among materials contained in the device.
[25] The organic light emitting device according to [23], wherein the amount of light emitted from the light emitting material is larger than the amount of light emitted from the compound.
[26] The organic light emitting device according to any one of [20] to [25], which is an organic electroluminescent device.
[27] The organic light emitting device according to any one of [20] to [26], which emits delayed fluorescence.

## Advantageous Effects of Invention

The compound of the present invention shows excellent light emission characteristics. The compound of the present invention is useful as a material for an organic light emitting device.

## Description of Embodiments

The contents of the present invention will be described in detail below. The constitutional elements may be described below with reference to representative embodiments and specific examples of the present invention, but the invention is not limited to the embodiments and the specific examples. In the description herein, a numerical range expressed as "to" means a range that includes the numerical values described before and after "to" as the lower limit and the upper limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the present description, the term "substituent" means an atom or an atomic group except a hydrogen atom and a deuterium atom. On the other hand, the term "substituted or unsubstituted" means that a hydrogen atom can be substituted with a deuterium atom or a substituent.

## [Compound Represented by General Formula (1)]

The compound represented by the following general formula (1) is described.

General Formula (1)

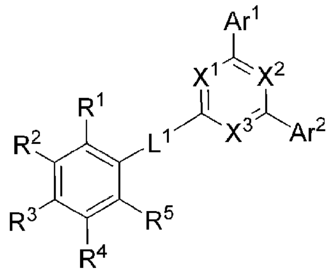

In the general formula (1), $R^1$ to $R^5$ each independently represent a hydrogen atom, a deuterium atom, a cyano group, a substituted or unsubstituted alkyl group, a substituted or unsubstituted aryl group, or a donor group. $Ar^1$ and $Ar^2$ each independently represent a substituted or unsubstituted aryl group, or a substituted or unsubstituted heteroaryl group containing a nitrogen atom as a ring skeleton-constituting atom.
The alkyl group that $R^1$ to $R^5$ can take can be linear, branched or cyclic. Two or more of a linear moiety, a cyclic moiety and a branched moiety can be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent can be further substituted with, for example, a deuterium atom, an aryl group, an alkoxy group, an aryloxy group, and a halogen atom. In one aspect of the present invention, the substituent for the alkyl group is one or more selected from the group consisting of an aryl group and a deuterium atom. In one preferred aspect of the present invention, the alkyl group is unsubstituted, and, for example, can be selected from the group consisting of a methyl group, an ethyl group, an isopropyl group and a tert-butyl group.

The aryl group that $R^1$ to $R^5$ and $Ar^1$ and $Ar^2$ can take each can be a monocyclic ring or can be a fused ring of two or more kinds of rings. In the case of a fused ring, the number of fused rings is preferably 2 to 6, and can be selected from, for example, 2 to 4. Specific examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group can be selected from Substituent Group A, can be selected from Substituent Group B, can be selected from Substituent Group C, can be selected from Substituent Group D, or can be selected from Substituent Group E. In one aspect of the present invention, the substituent for the aryl group is one or more selected from the group consisting of an alkyl group, an aryl group and a deuterium atom. In one preferred aspect of the present invention, the aryl group is substituted with at least one deuterium atom. In one aspect of the present invention, the aryl group is unsubstituted.

Specific examples of the substituted or unsubstituted aryl group that $R^1$ to $R^5$ and $Ar^1$ and $Ar^2$ can take are shown below. However, the aryl group which can be employed in the present invention should not be limitatively interpreted by the following specific examples. In the following specific examples, * indicates a bonding site. A methyl group is not shown. Consequently, Ar2 to Ar7 represent structures substituted with a methyl group.

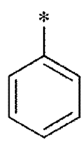
Ar1
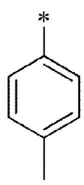
Ar2
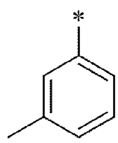
Ar3
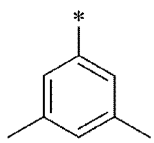
Ar4
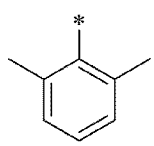
Ar5
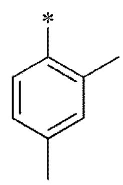
Ar6

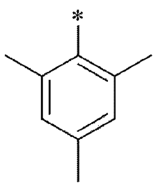
Ar7
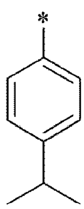
Ar8
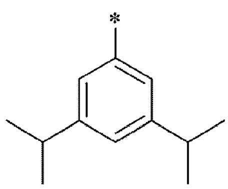
Ar9
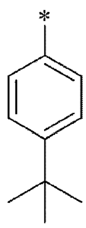
Ar10
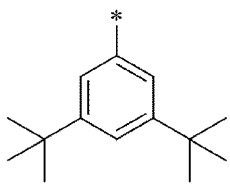
Ar11

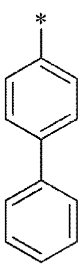
Ar12
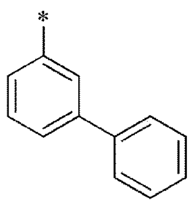
Ar13
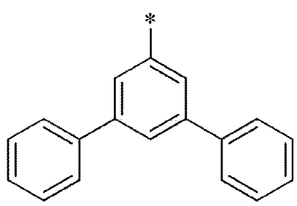
Ar14
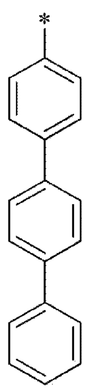
Ar15
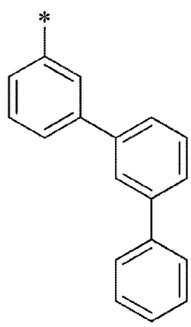
Ar16

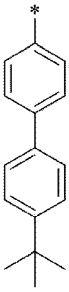
Ar17

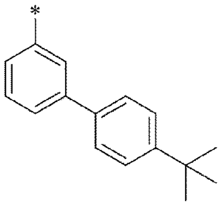
Ar18

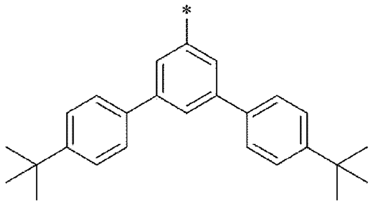
Ar19

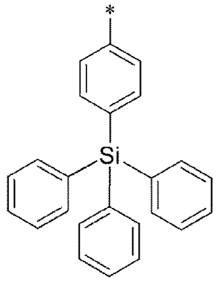
Ar20

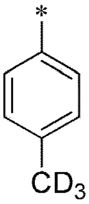
Ar21

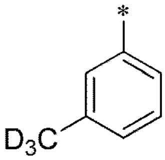
Ar22

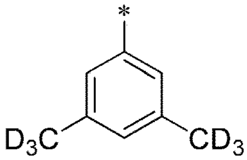
Ar23

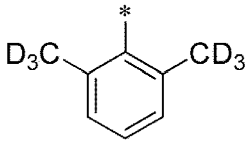
Ar24

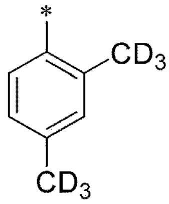
Ar25

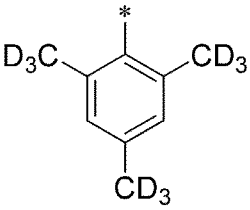
Ar26

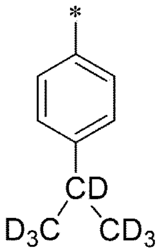
Ar27

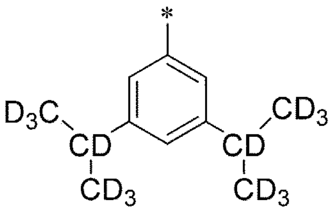
Ar28

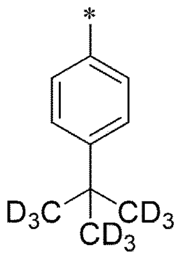
Ar29

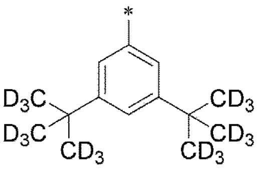
Ar30

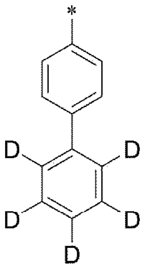
Ar31

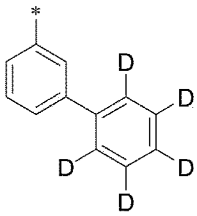
Ar32

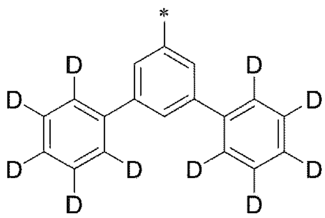
Ar33

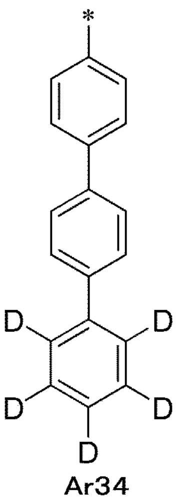
Ar34

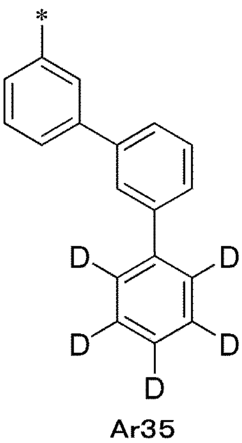
Ar35

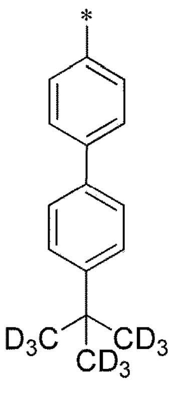
Ar36

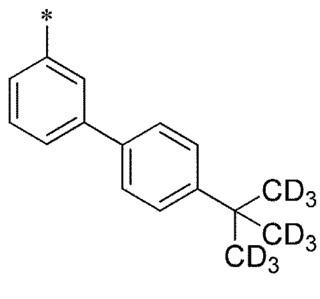
Ar37

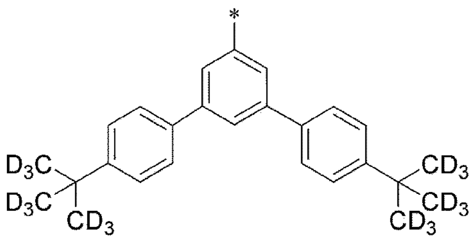

Ar38

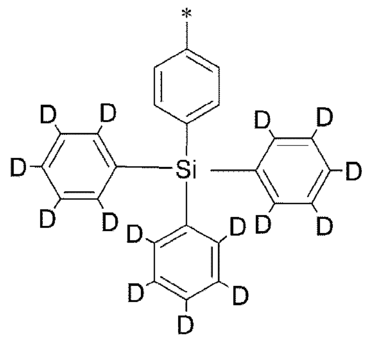

Ar39

In addition to the above-mentioned specific examples, groups obtained by substituting all hydrogen atoms present in Ar1 to Ar20 with deuterium atoms are disclosed as Ar40 to Ar59, respectively.

In one aspect of the present invention, the aryl group that $R^1$ to $R^5$ can take is Ar1 or Ar40. In one aspect of the present invention, the aryl group that $R^1$ to $R^5$ can take is selected from the group consisting of Ar2 to Ar11, Ar21 to Ar30 and Ar41 to Ar50. In one aspect of the present invention, the aryl group that $R^1$ to $R^5$ can take is selected from the group consisting of Ar12 to Ar16, Ar31 to Ar35 and Ar51 to Ar55. In one aspect of the present invention, the aryl group that $R^1$ to $R^5$ can take is selected from the group consisting of Ar1, Ar12 to Ar16, Ar40, Ar31 to Ar35 and Ar51 to Ar55. In one aspect of the present invention, the aryl group that $R^1$ to $R^5$ can take is selected from the group consisting of Ar21 to Ar59.

In one aspect of the present invention, the aryl group that $Ar^1$ and $Ar^2$ can take is Ar1 or Ar40. In one aspect of the present invention, the aryl group that $Ar^1$ and $Ar^2$ can take is selected from the group consisting of Ar2 to Ar11, Ar21 to Ar30 and Ar41 to Ar50. In one aspect of the present invention, the aryl group that $Ar^1$ and $Ar^2$ can take is selected from the group consisting of Ar12 to Ar16, Ar31 to Ar35 and Ar51 to Ar55. In one aspect of the present invention, the aryl group that $Ar^1$ and $Ar^2$ can take is selected from the group consisting of Ar1, Ar12 to Ar16, Ar40, Ar31 to Ar35 and Ar51 to Ar55. In one aspect of the present invention, the aryl group that $Ar^1$ and $Ar^2$ can take is selected from the group consisting of Ar21 to Ar59.

At least one of $R^1$ to $R^5$ in the general formula (1) is a donor group. The donor group that $R^1$ to $R^5$ can take does not include a substituted or unsubstituted aryl group.

The "donor group" can be selected from groups having a negative Hammett's σp value. The Hammett's σp value is proposed by L. P. Hammett and quantifies the influence of a substituent on the reaction rate or equilibrium of a para-substituted benzene derivative. Specifically, the value is a constant (σp) specific to the substituent in the following formula that is established between substituents and reaction rate constants or equilibrium constants in para-substituted benzene derivatives:

$$\log(k/k_0) = \rho\sigma p$$

or

$$\log(K/K_0) = \rho\sigma p$$

In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and ρ represents a reaction constant to be determined by the kind and the condition of reaction. Regarding the description relating to the "Hammett's σp value" and the numerical value of each substituent in the present invention, reference can be made to the description relating to σp value in Hansch, C. et. al., Chem. Rev., 91, 165-195 (1991).

The donor group that $R^1$ to $R^5$ can take preferably has σp of -0.3 or less, more preferably -0.5 or less, and even more preferably -0.7 or less. For example, the value can be selected from a range of -0.9 or less, or from a range of -1.1 or less.

The donor group in the present invention is preferably a group containing a substituted amino group. The donor group can be a substituted amino group, or can be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a

substituted or unsubstituted heteroaryl group, more preferably a substituted or unsubstituted aryl group or a substituted or unsubstituted heteroaryl group. Especially, the substituted amino group is preferably a substituted or unsubstituted diarylamino group, or a substituted or unsubstituted diheteroarylamino group. As referred to herein, the two aryl groups constituting the diarylamino group can bond to each other, and the two heteroaryl groups constituting the diheteroarylamino group can bond to each other.

The donor group that $R^1$ to $R^5$ can take is preferably a group represented by the following general formula (a).

General Formula (a)

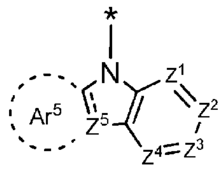

In the general formula (a), $Z^1$ represents C-$R^{14}$ or N, $Z^2$ represents C-$R^{15}$ or N, $Z^3$ represents C-$R^{16}$ or N, and $Z^4$ represents C-$R^{17}$ or N. $Z^5$ represents C or N, $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each can bond to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent can be selected from Substituent Group A, can be selected from Substituent Group B, can be selected from Substituent Group C, can be selected from Substituent Group D, or can be selected from Substituent Group E. When two or more of $R^{14}$ to $R^{17}$ represent substituents, the two or more substituents can be the same or different. Zero to two of $R^{14}$ to $R^{17}$ are preferably a substituent, and for example, one can be a substituent, or zero can be a substituent ($R^{14}$ to $R^{17}$ are a hydrogen atom or a deuterium atom).

$R^{14}$ and $R^{15}$, $R^{15}$ and $R^{16}$, and $R^{16}$ and $R^{17}$ each can bond to each other to form a cyclic structure. The cyclic structure can be any of an aromatic ring, an heteroaromatic ring, an aliphatic hydrocarbon ring, and an aliphatic heterocyclic ring, and can be a ring obtained by fusing these rings. The structure is preferably an aromatic ring or a heteroaromatic ring. Examples of the aromatic ring include a substituted or unsubstituted benzene ring. Another benzene ring can be further fused to the benzene ring, and a heterocyclic ring such as a pyridine ring can be fused to the benzene ring. The heteroaromatic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein can be unsubstituted, can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. It is preferable that a substituted or unsubstituted aryl group bonds to the nitrogen atom constituting the pyrrole ring of indole, and examples of the substituent include a substituent selected from any of Substituent Group A to Substituent Group E. The cyclic structure can be a substituted or unsubstituted cyclopentadiene ring. In one aspect of the present invention, a pair of $R^{14}$ and $R^{15}$, $R^{15}$ and $R^{16}$, and $R^{16}$ and $R^{17}$ bonds to each other to form a cyclic structure. In one aspect of the present invention, none of $R^{14}$ and $R^{15}$, $R^{15}$ and $R^{16}$, and $R^{16}$ and $R^{17}$ bonds to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ is C, and $Ar^5$ is a substituted or unsubstituted aromatic ring or a substituted or unsubstituted heteroaromatic ring. In one aspect of the present invention, $Z^5$ is N, and $Ar^5$ is a substituted or unsubstituted heteroaromatic ring.

Examples of the aromatic ring that $Ar^5$ can take include a benzene ring. Another benzene ring can be further fused to the benzene ring, and a heterocyclic ring such as a pyridine ring can be fused to the benzene ring. The heteroaromatic ring which is employable by $Ar^5$ is preferably a 5- to 7-membered ring, and for example, a 5-membered ring or a 6-membered ring can be employed. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ is C, and the heteroaromatic ring is a furan ring of a substituted or

unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ is N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole and benzimidazole referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E.

When $Z^5$ in the general formula (a) is C, a group represented by the following general formula (b) is preferred.

General Formula (b)

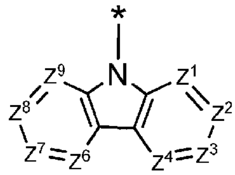

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{18}$ and $R^{19}$, $R^{19}$ and $R^{20}$, and $R^{20}$ and $R^{21}$ each can bond to each other to form a cyclic structure.

For $Z^1$ to $Z^4$ and $R^{14}$ to $R^{17}$ in the general formula (b), the corresponding description of the general formula (a) can be referred to. $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and for the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

Preferably, the donor group that $R^1$ to $R^5$ can take is a substituted or unsubstituted carbazol-9-yl group. The carbazol-9-yl group referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Further one or more rings can be fused to the two benzene rings constituting the carbazol-9-yl group. In one preferred aspect of the present invention, the donor group that $R^1$ to $R^5$ can take is a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2 to 7-positions, more preferably at least one of 3 to 6-positions, even more preferably a 3-position and a 6-position.

In one aspect of the present invention, the donor group that $R^1$ to $R^5$ can take is a carbazol-9-yl group fused with one or more rings, and hereinunder this is referred to as "ring-fused carbazol-9-yl group". The ring-fused carbazol-9-yl group that $R^1$ to $R^5$ can take can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group and an aryl group or with a group formed by combining two or more thereof.

The number of rings constituting the fused ring in the ring-fused carbazol-9-yl group is 4 or more, preferably 5 or more, more preferably 5 to 9, even more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group that bonds via the nitrogen atom constituting the ring skeleton of carbazole, and has a structure in which a ring is fused to at least one of the two benzene rings constituting carbazole. The fused ring can be any of an aromatic hydrocarbon ring, an aromatic heterocyclic ring, an aliphatic hydrocarbon ring, and an aliphatic heterocyclic ring, and can be a ring obtained by further fusing these rings. An aromatic hydrocarbon ring and an

aromatic heterocyclic ring are preferable. Examples of the aromatic hydrocarbon ring include a substituted or unsubstituted benzene ring. Another benzene ring can be further fused to the benzene ring, and a heterocyclic ring such as a pyridine ring can be fused to the benzene ring. The aromatic heterocyclic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) bonds to the nitrogen atom of the pyrrole ring, and it is more preferable that an aryl group which can be substituted with an alkyl group or an aryl group is bonded. In the present invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indolo structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

The ring-fused carbazol-9-yl group employable herein includes a benzofuro[2,3-a]carbazol-9-yl group, a benzofuro[3,2-a]carbazol-9-yl group, a benzofuro[2,3-b]carbazol-9-yl group, a benzofuro[3,2-b]carbazol-9-yl group, a benzofuro[2,3-c]carbazol-9-yl group, and a benzofuro[3,2-c]carbazol-9-yl group. In addition, the ring-fused carbazol-9-yl group also employable herein includes a benzothieno[2,3-a]carbazol-9-yl group, a benzothieno[3,2-a]carbazol-9-yl group, a benzothieno[2,3-b]carbazol-9-yl group, a benzothieno[3,2-b]carbazol-9-yl group, a benzothieno[2,3-c]carbazol-9-yl group, and a benzothieno[3,2-c]carbazol-9-yl group. The ring-fused carbazol-9-yl group also employable herein includes an indolo[2,3-a]carbazol-9-yl group, an indolo[3,2-a]carbazol-9-yl group, an indolo[2,3-b]carbazol-9-yl group, an indolo[3,2-b]carbazol-9-yl group, an indolo[2,3-c]carbazol-9-yl group, and an indolo[3,2-c]carbazol-9-yl group.

The number of the substituents substituted on the ring-fused carbazol-9-yl group is preferably 1 to 10, more preferably 1 to 6, even more preferably 1 to 4, and can be, for example 1, or can be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the heteroatom present in the ring-fused carbazol-9-yl group. In one preferred aspect of the present invention, the compound has at least one substituent only on the para-position of the benzene ring viewed from the heteroatom present in the ring-fused carbazol-9-yl group. In one preferred aspect of the present invention, the compound has substituents on all the substitutable para-positions of the benzene ring viewed from the heteroatom present in the ring-fused carbazol-9-yl group.

Specific examples of the donor group that $R^1$ to $R^5$ in the general formula (1) can take are shown below. However, the donor group which can be employed in the present invention shall not be construed as being limited by the following specific examples. In the following specific examples, Ph represents a phenyl group ($C_6H_5$), and * indicates a bonding site. A methyl group is not shown, and for example, D2 has one methyl group. However, a deuterated methyl group is expressed as $CD_3$. $C_6D_5$ represents a phenyl group in which all hydrogen atoms are deuterated. D represents a deuterium atom.

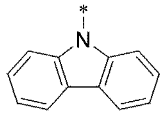
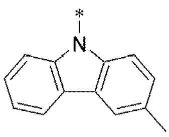
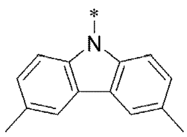
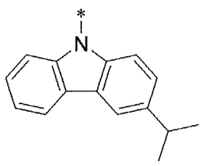
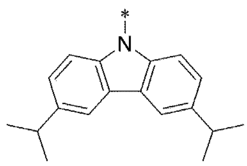

D1 D2 D3 D4 D5

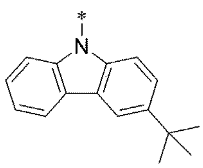
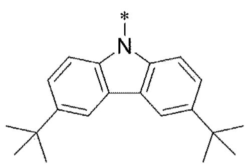
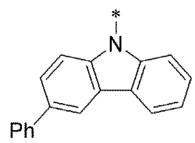
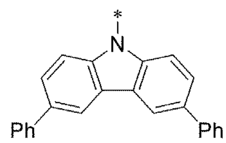

D6 D7 D8 D9

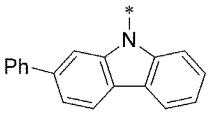

D10

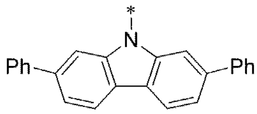

D11

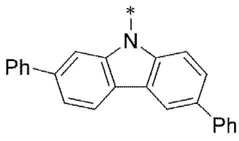

D12

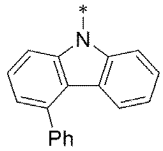

D13

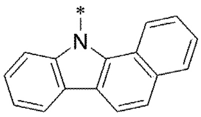

D14

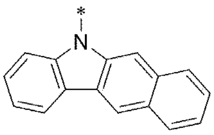

D15

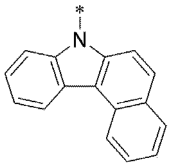

D16

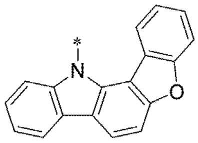

D17

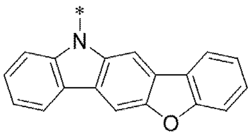

D18

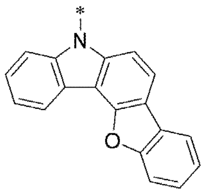

D19

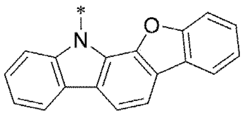

D20

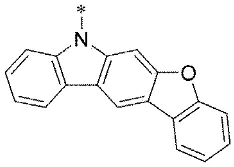

D21

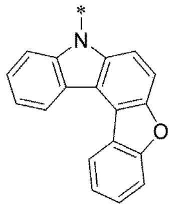

D22

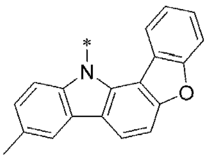

D23

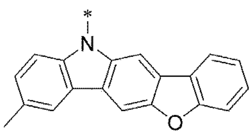

D24

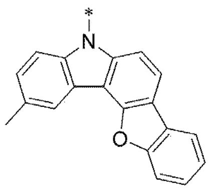

D25

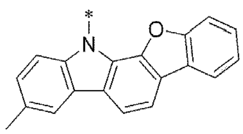

D26

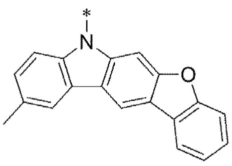

D27

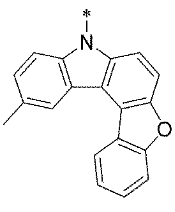

D28

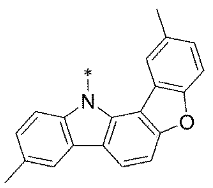

D29

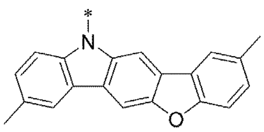

D30

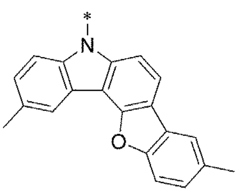

D31

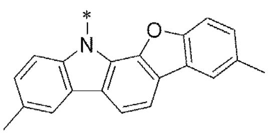

D32

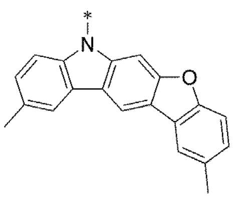

D33

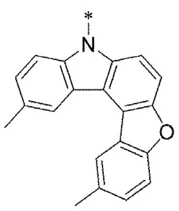

D34

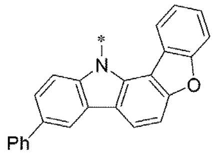

D35

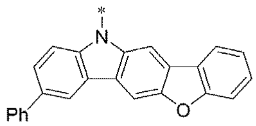

D36

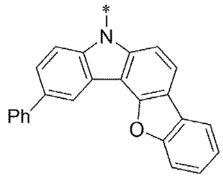

D37

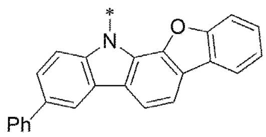

D38

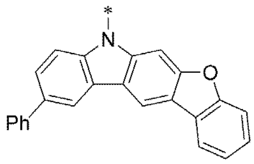

D39

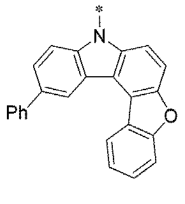

D40

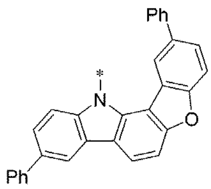

D41

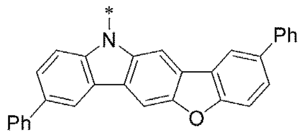

D42

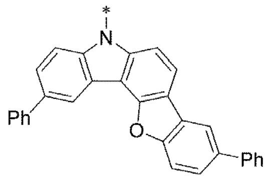

D43

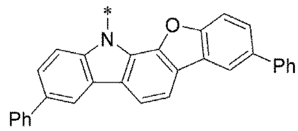

D44

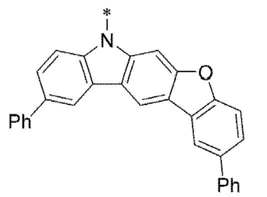

D45

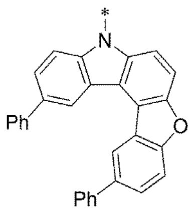

D46

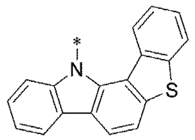

D47

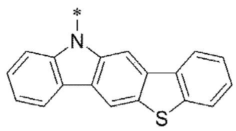

D48

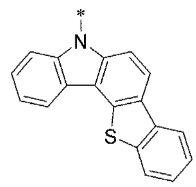

D49

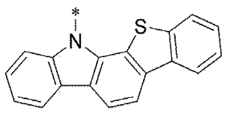

D50

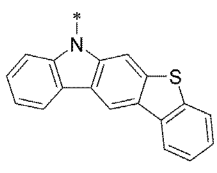

D51

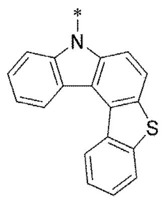

D52

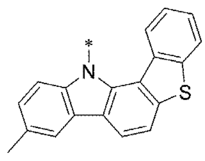

D53

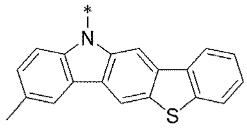

D54

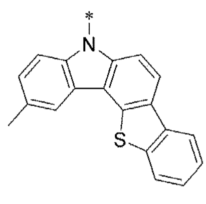

D55

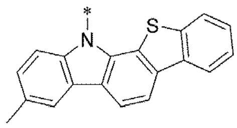

D56

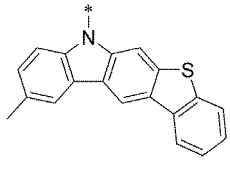

D57

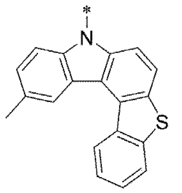
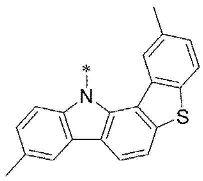
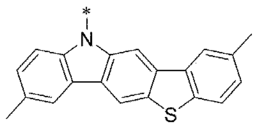
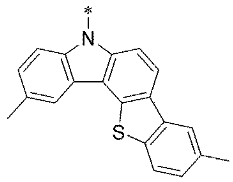

D58 D59 D60 D61

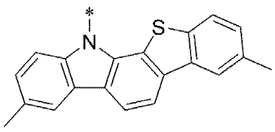
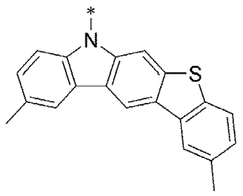
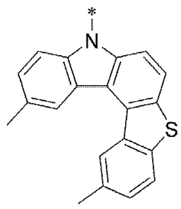
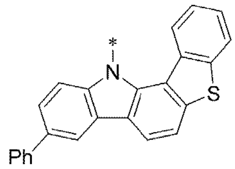

D62 D63 D64 D65

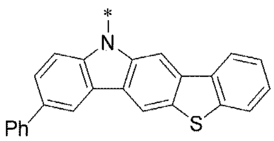
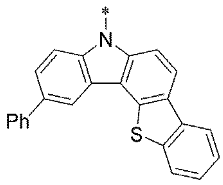
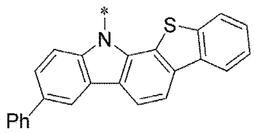
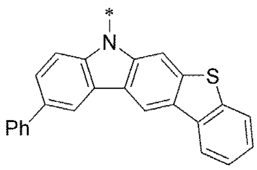

D66 D67 D68 D69

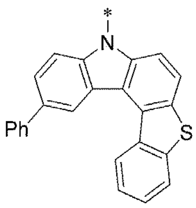
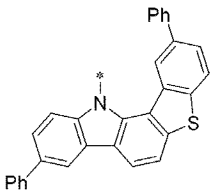
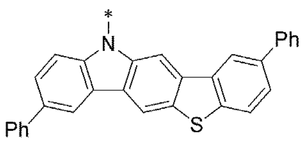
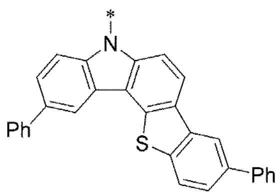

D70 D71 D72 D73

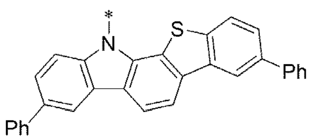
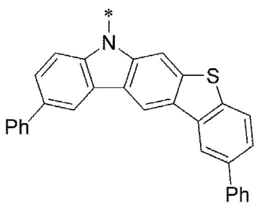
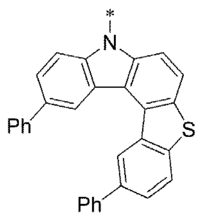
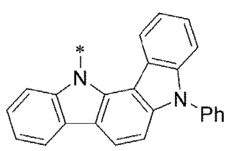

D74 D75 D76 D77

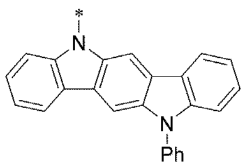
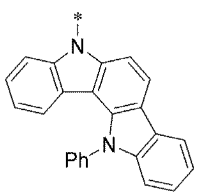
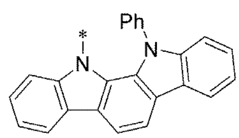
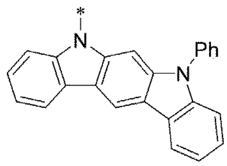

D78 D79 D80 D81

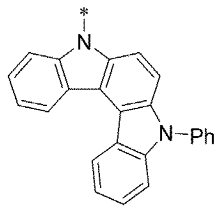

D82

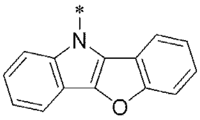

D83

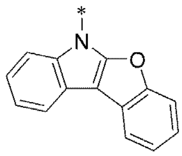

D84

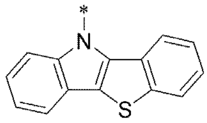

D85

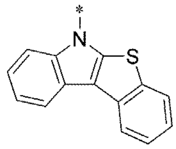

D86

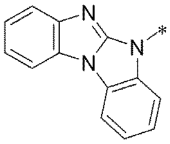

D87

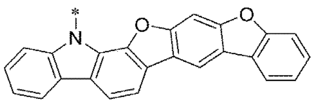

D88

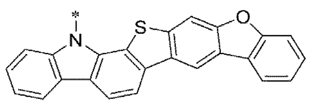

D89

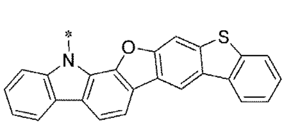

D90

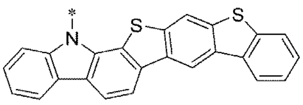

D91

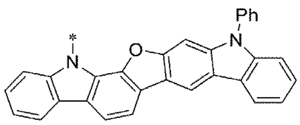

D92

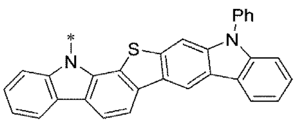

D93

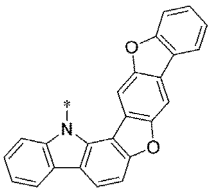

D94

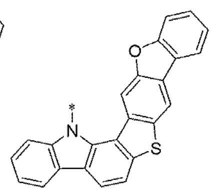

D95

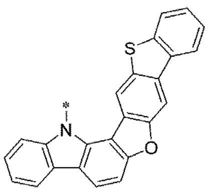

D96

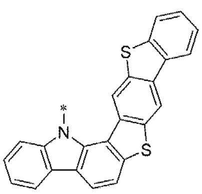

D97

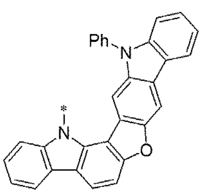

D98

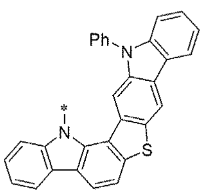

D99

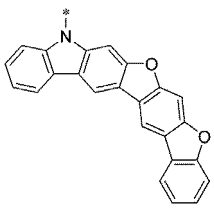

D100

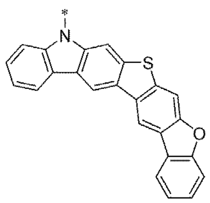

D101

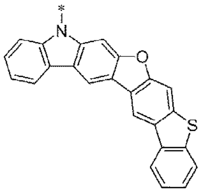

D102

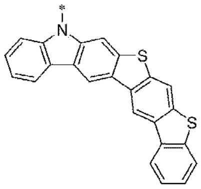

D103

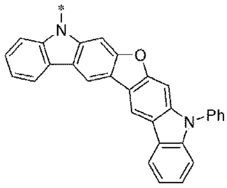

D104

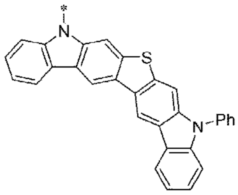

D105

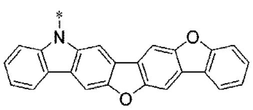

D106

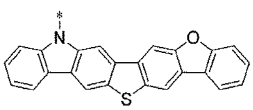

D107

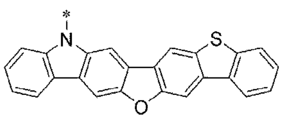

D108

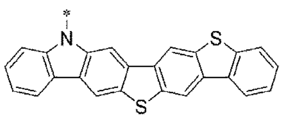
D109

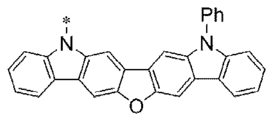
D110

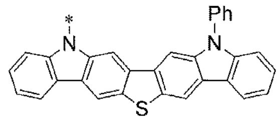
D111

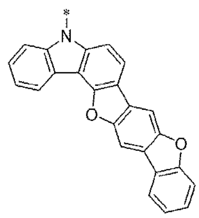
D112

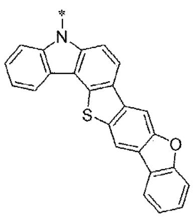
D113

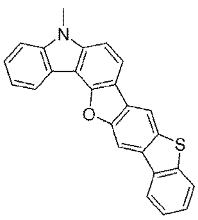
D114

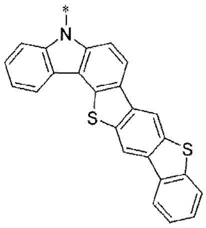
D115

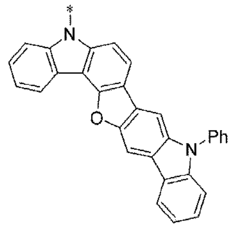
D116

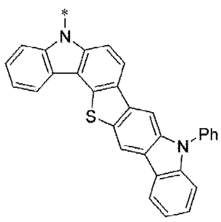
D117

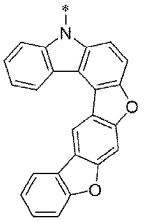
D118

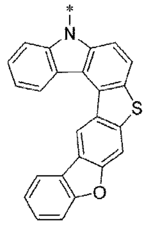
D119

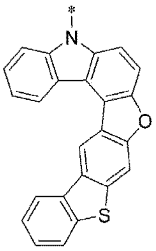
D120

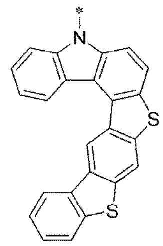
D121

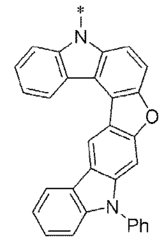
D122

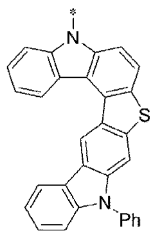
D123

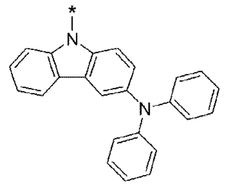
D124

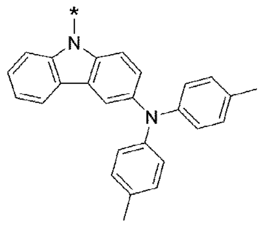
D125

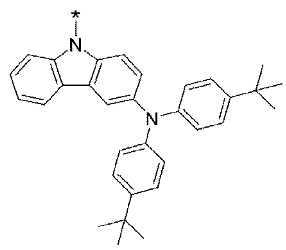
D126

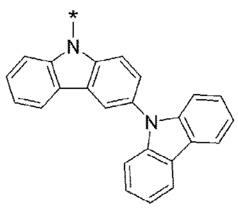
D127

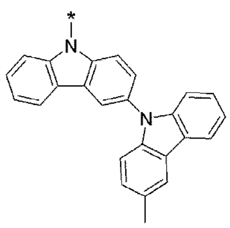
D128

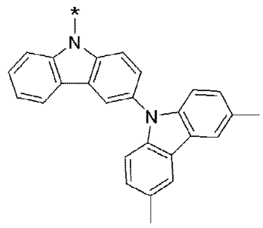
D129

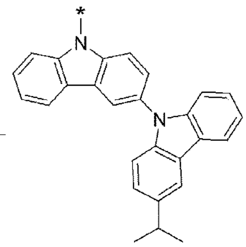
D130

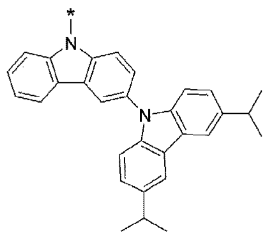

D131

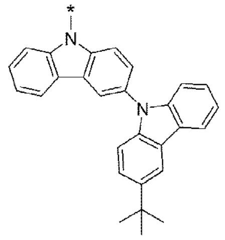

D132

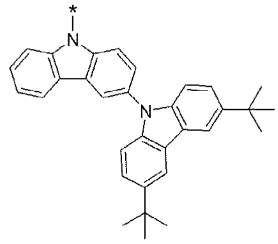

D133

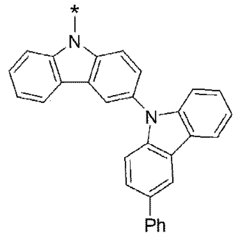

D134

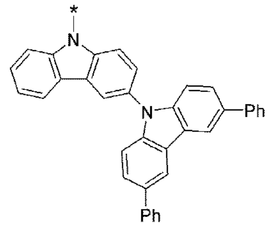

D135

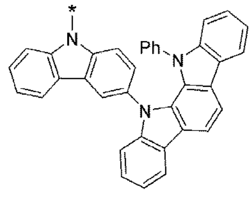

D136

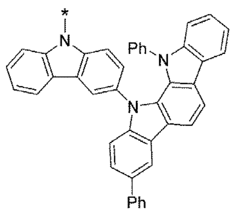

D137

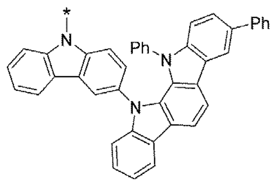

D138

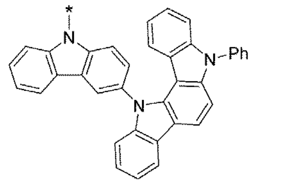

D139

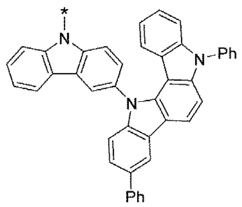

D140

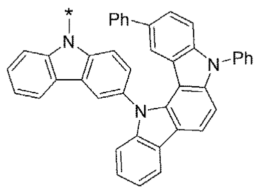

D141

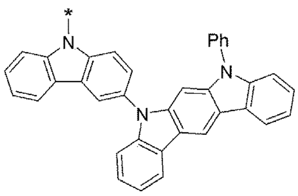

D142

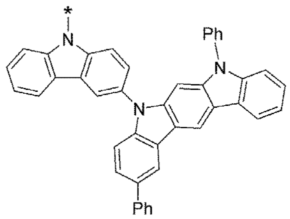

D143

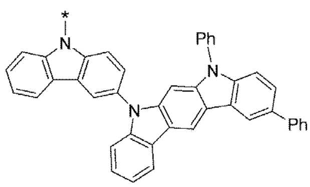

D144

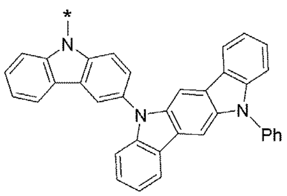

D145

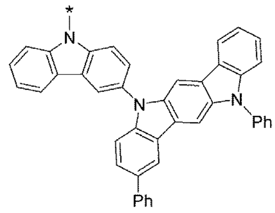 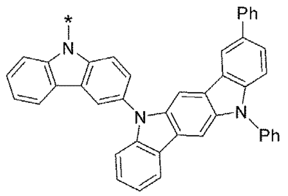 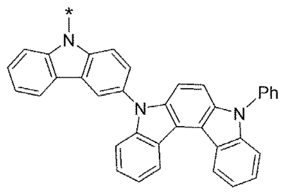

D146 D147 D148

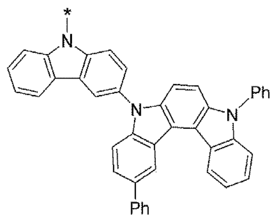 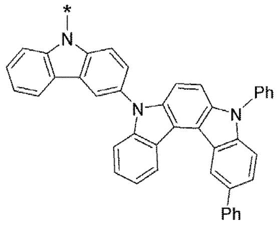 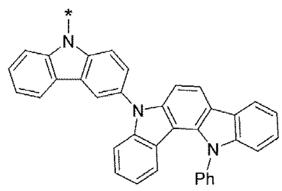

D149 D150 D151

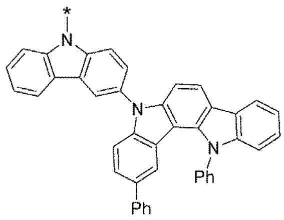 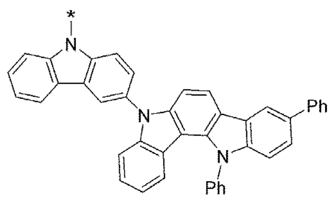 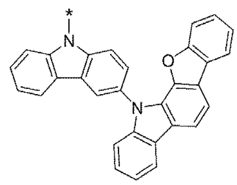

D152 D153 D154

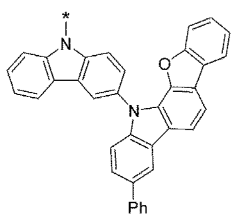 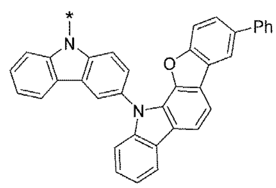 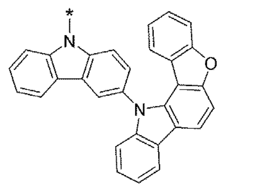

D155 D156 D157

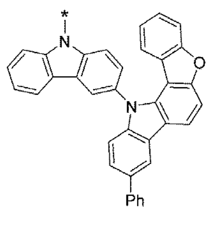 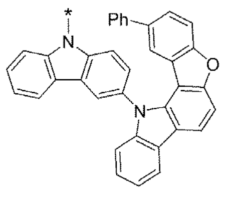 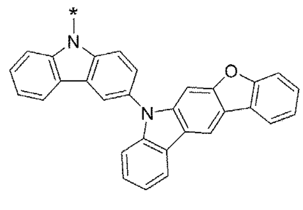

D158 D159 D160

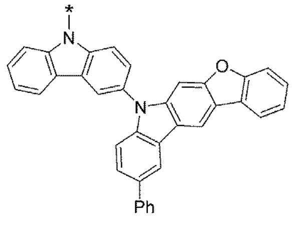 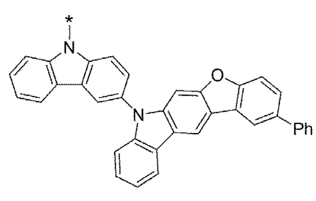 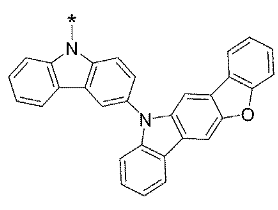

D161 D162 D163

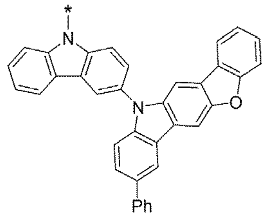
D164

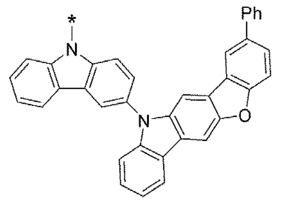
D165

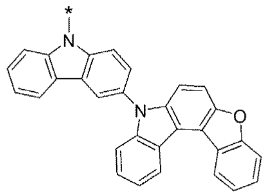
D166

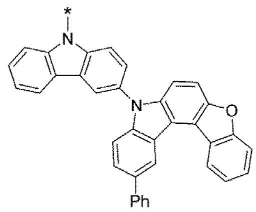
D167

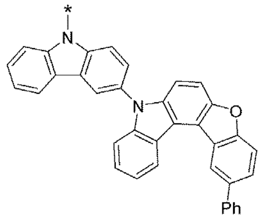
D168

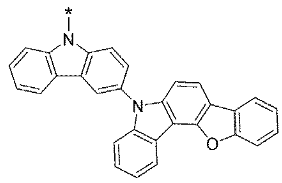
D169

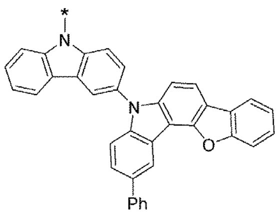
D170

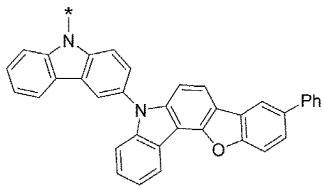
D171

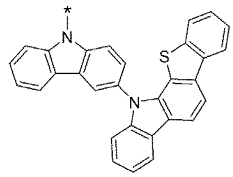
D172

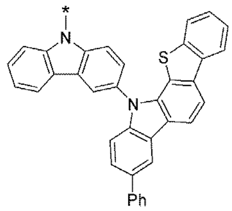
D173

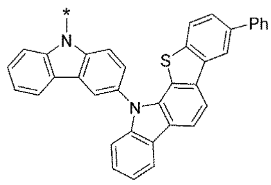
D174

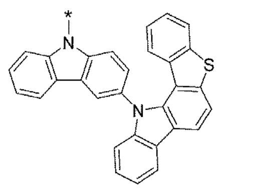
D175

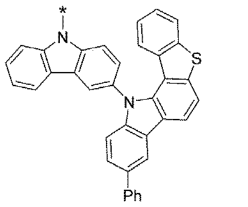
D176

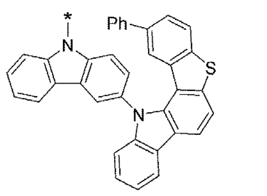
D177

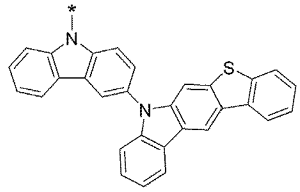
D178

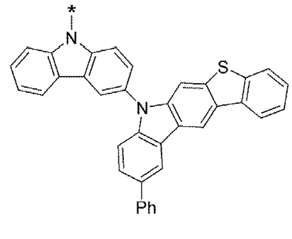
D179

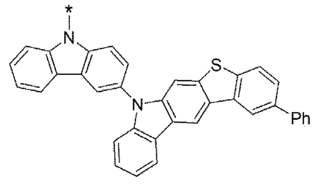
D180

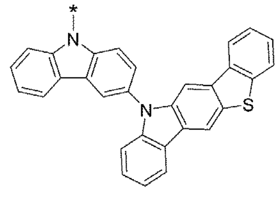
D181

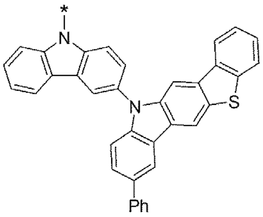

D182

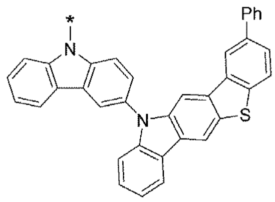

D183

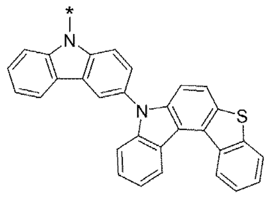

D184

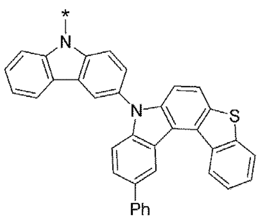

D185

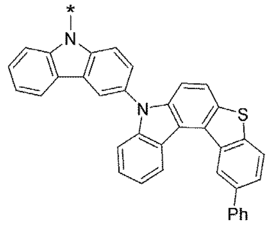

D186

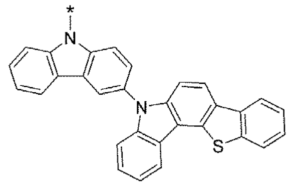

D187

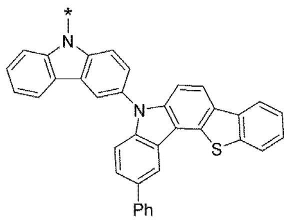

D188

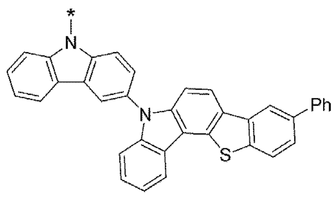

D189

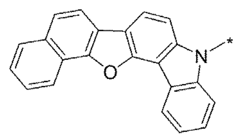

D190

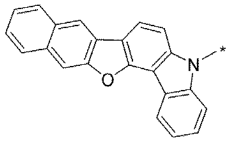

D191

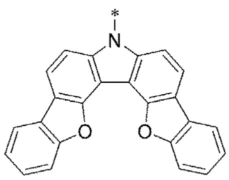

D192

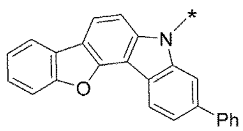

D193

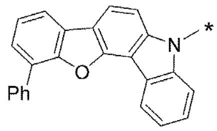

D194

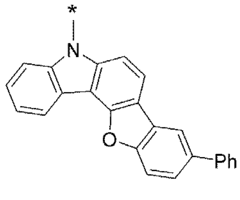

D195

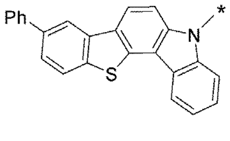

D196

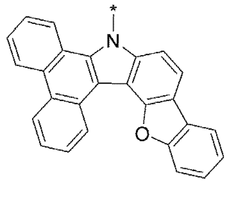

D197

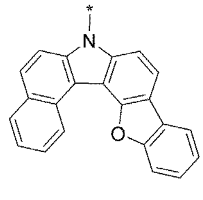

D198

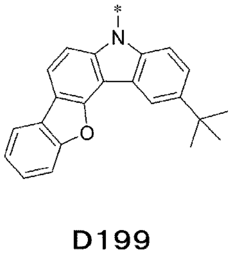

D199

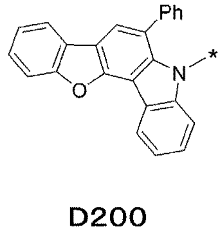

D200

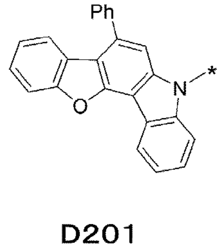

D201

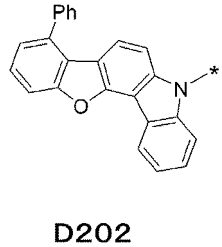

D202

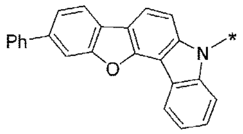
D203

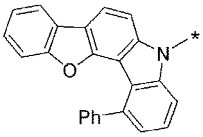
D204

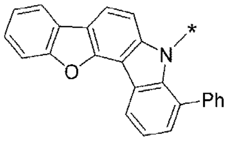
D205

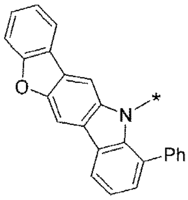
D206

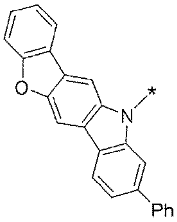
D207

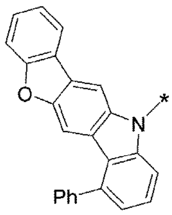
D208

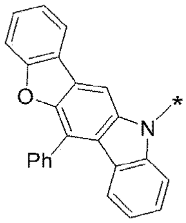
D209

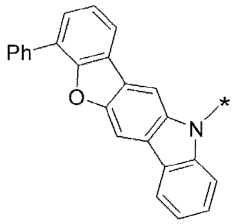
D210

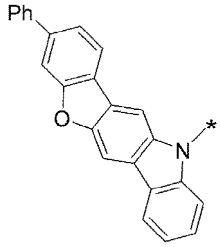
D211

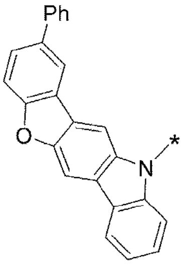
D212

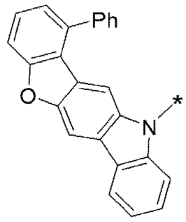
D213

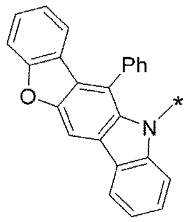
D214

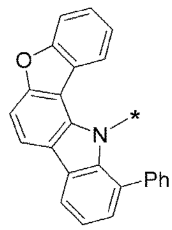
D215

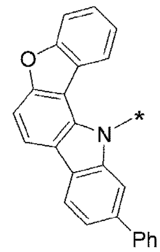
D216

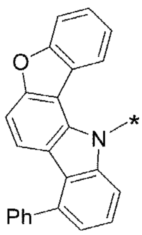
D217

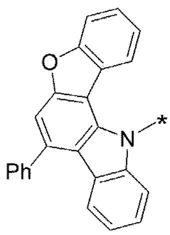
D218

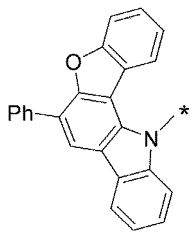
D219

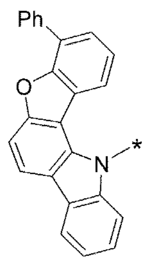
D220

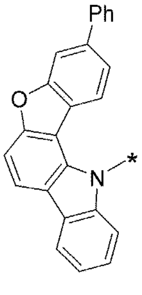
D221

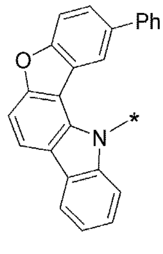
D222

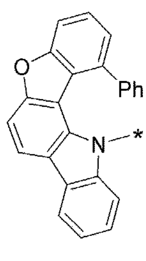
D223

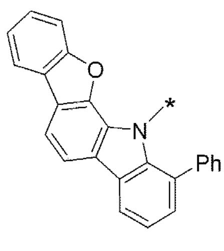
D224

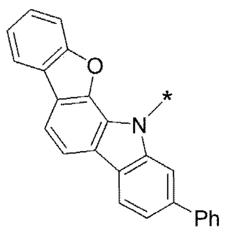
D225

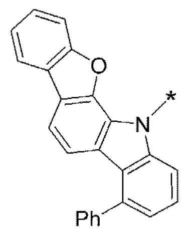
D226

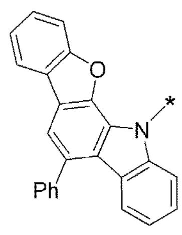
D227

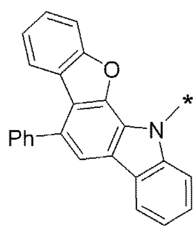
D228

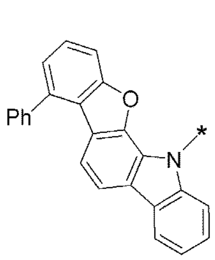
D229

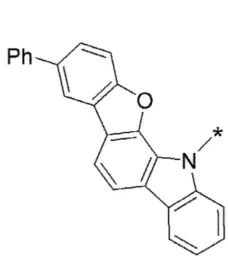
D230

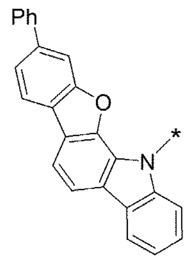
D231

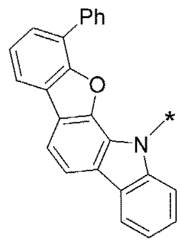
D232

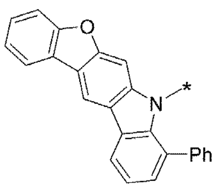
D233

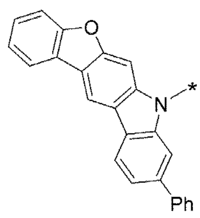
D234

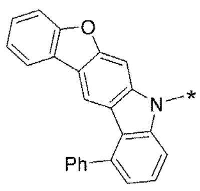
D235

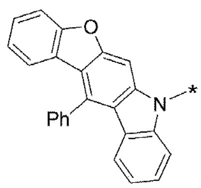
D236

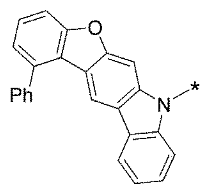
D237

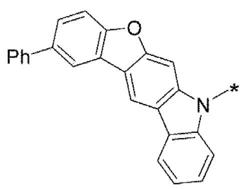
D238

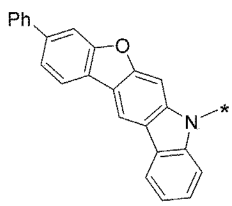
D239

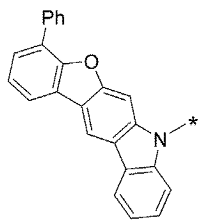
D240

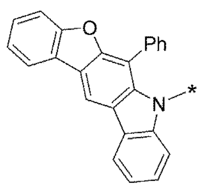
D241

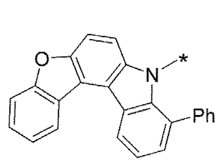
D242

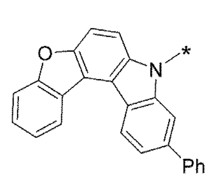
D243

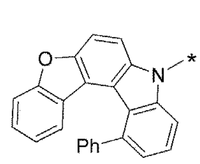
D244

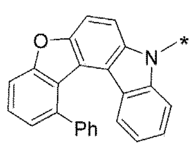
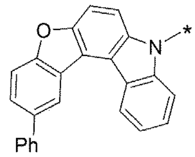
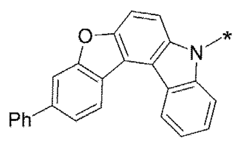
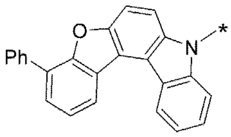

D245 D246 D247 D248

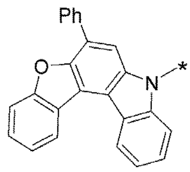
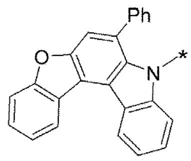
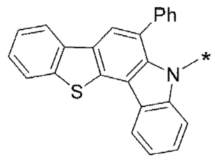
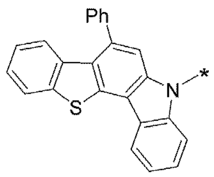

D249 D250 D251 D252

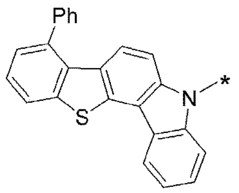
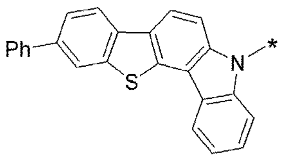
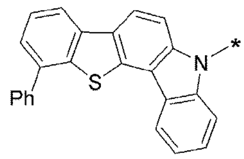
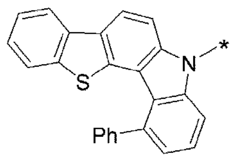

D253 D254 D255 D256

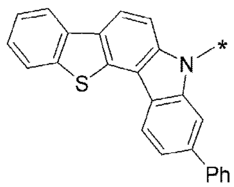
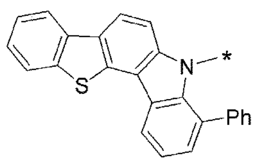
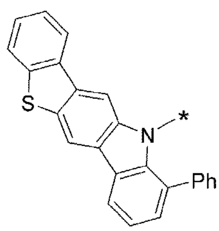
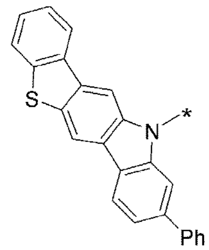

D257 D258 D259 D260

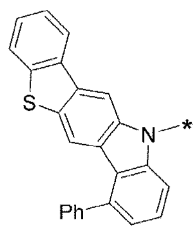
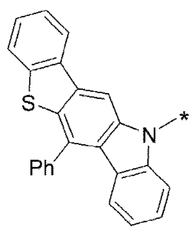
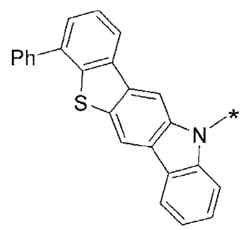
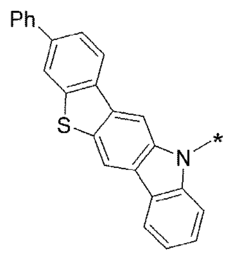

D261 D262 D263 D264

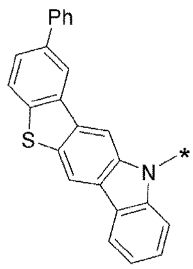
D265

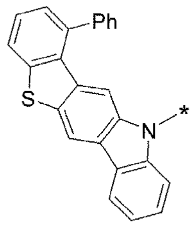
D266

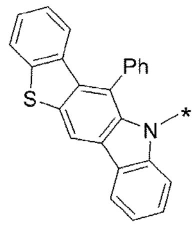
D267

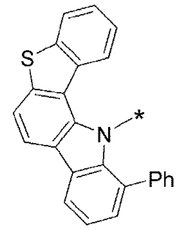
D268

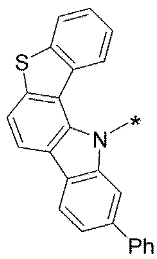
D269

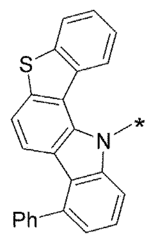
D270

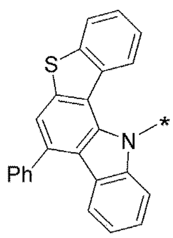
D271

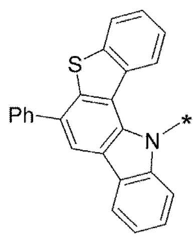
D272

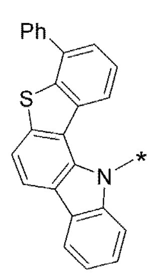
D273

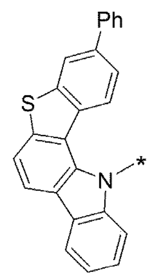
D274

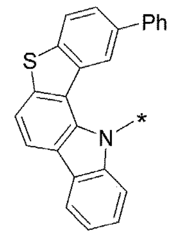
D275

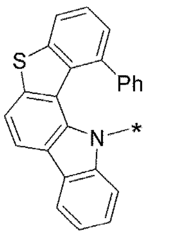
D276

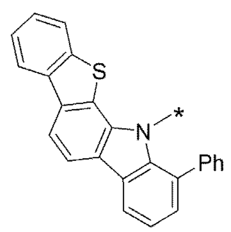
D277

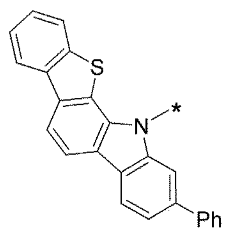
D278

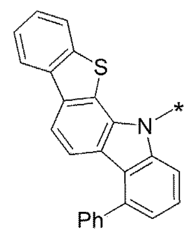
D279

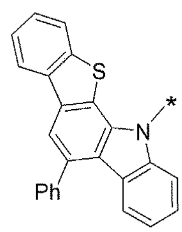
D280

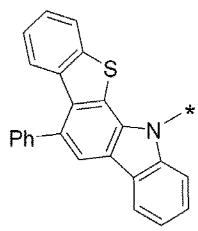
D281

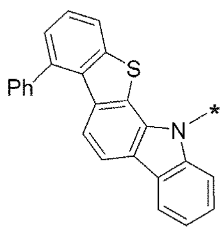
D282

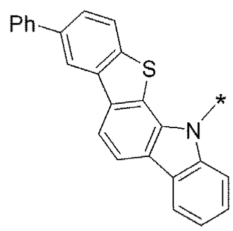
D283

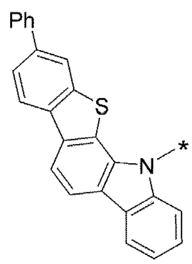
D284

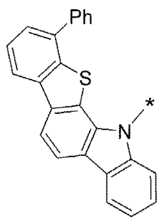
D285

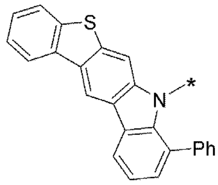
D286

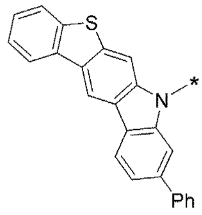
D287

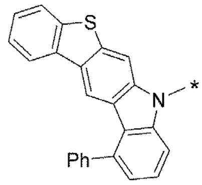
D288

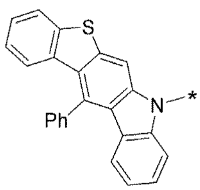
D289

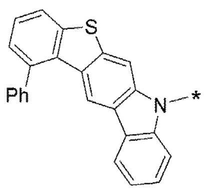
D290

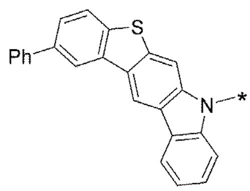
D291

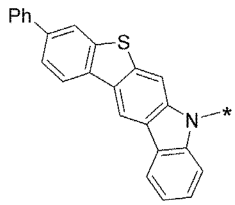
D292

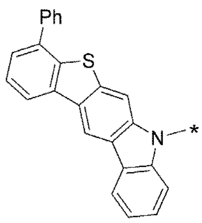
D293

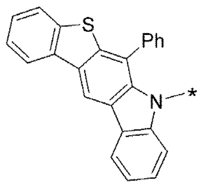
D294

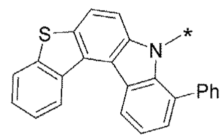
D295

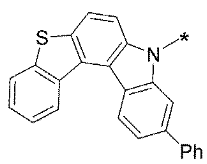
D296

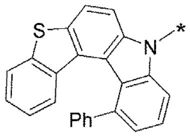
D297

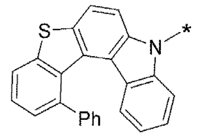
D298

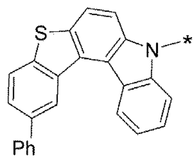
D299

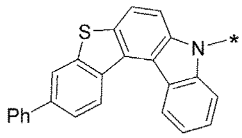
D300

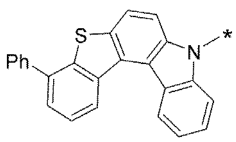
D301

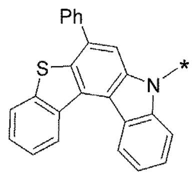
D302

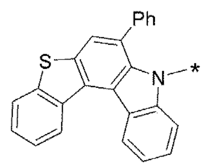
D303

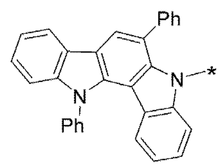
D304

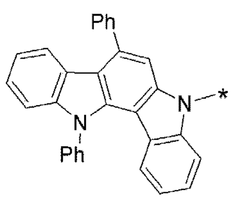
D305

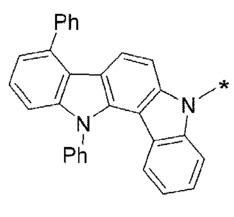
D306

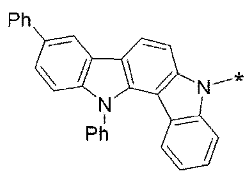
D307

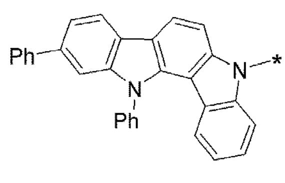
D308

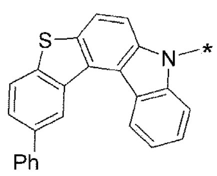 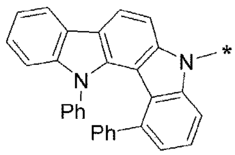 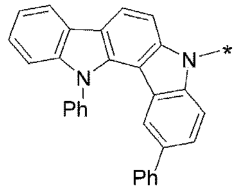 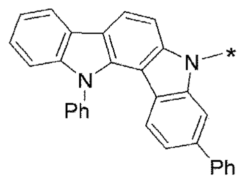

D309 D310 D311 D312

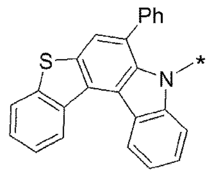 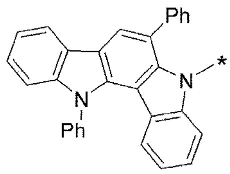 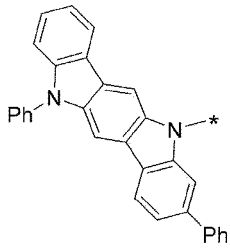 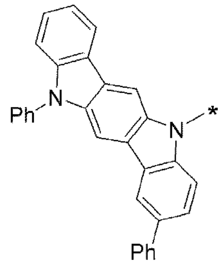

D313 D314 D315 D316

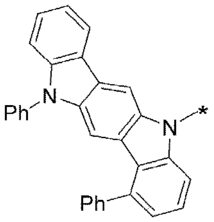 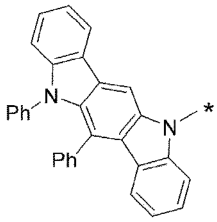 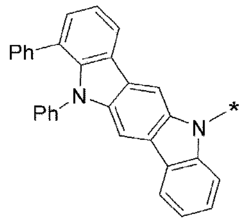 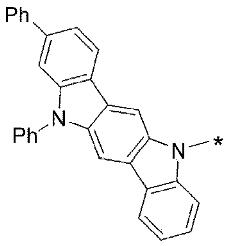

D317 D318 D319 D320

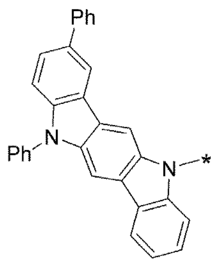 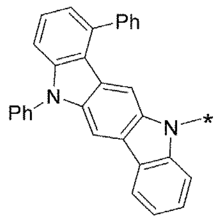 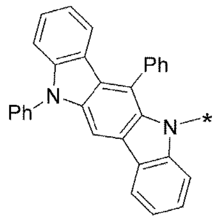 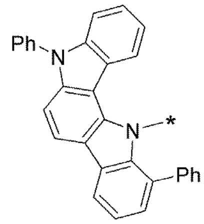

D321 D322 D323 D324

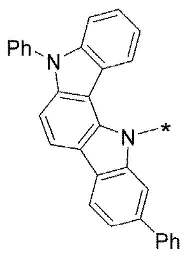 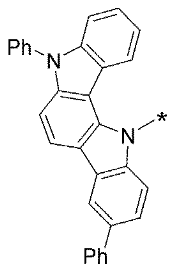 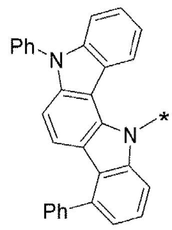 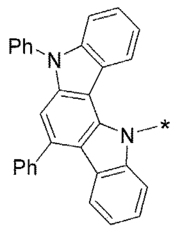

D325 D326 D327 D328

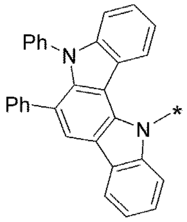
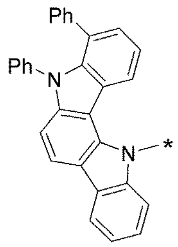
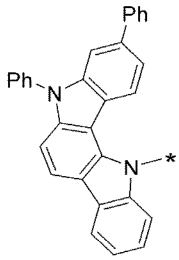
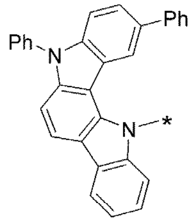

D329 D330 D331 D332

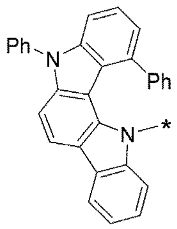
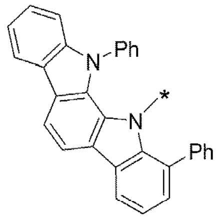
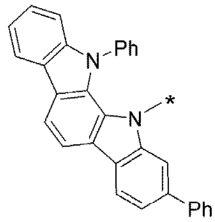
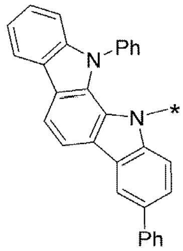

D333 D334 D335 D336

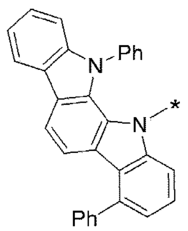
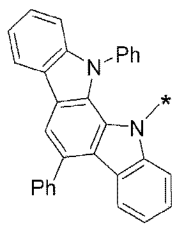
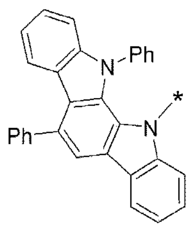
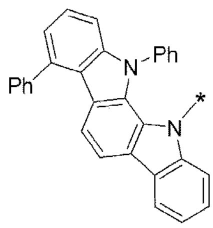

D337 D338 D339 D340

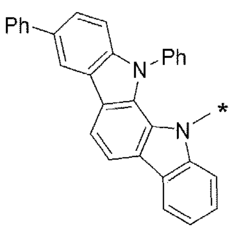
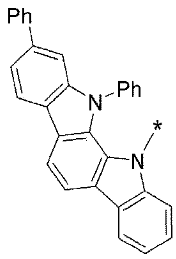
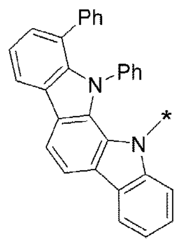
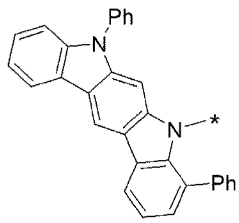

D341 D342 D343 D344

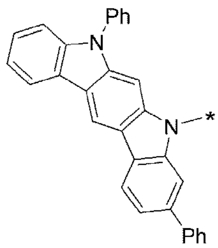
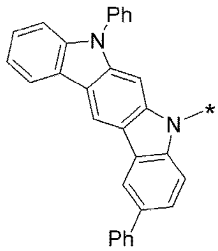
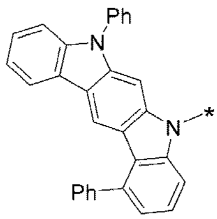
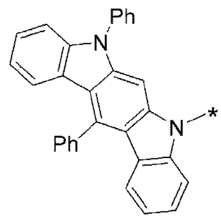

D345 D346 D347 D348

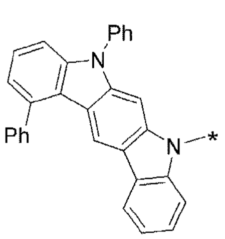
D349

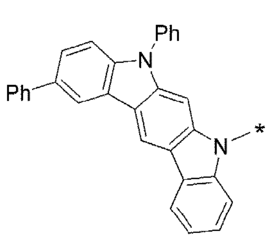
D350

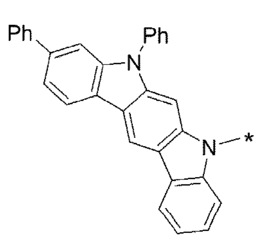
D351

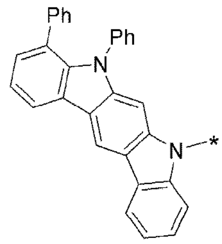
D352

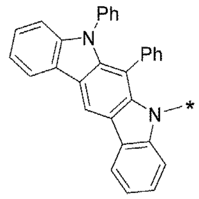
D353

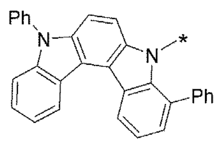
D354

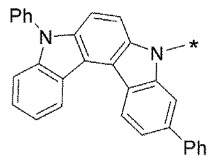
D355

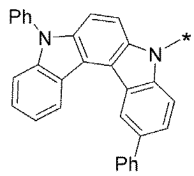
D356

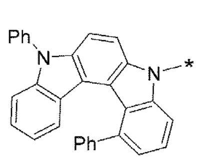
D357

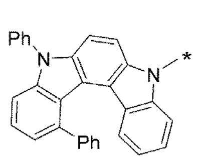
D358

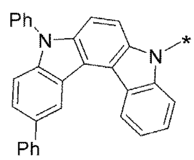
D359

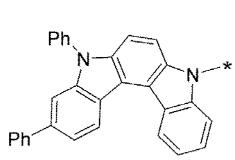
D360

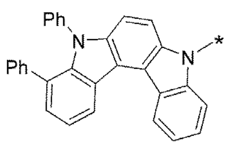
D361

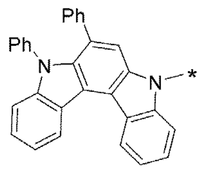
D362

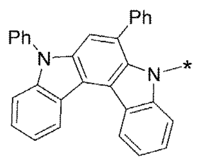
D363

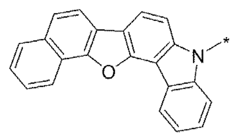
D364

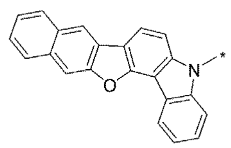
D365

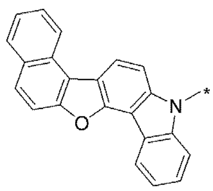
D366

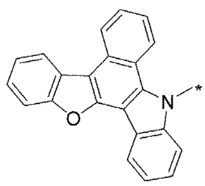
D367

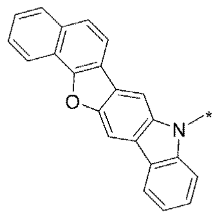
D368

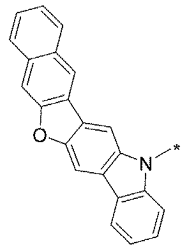
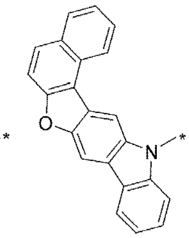
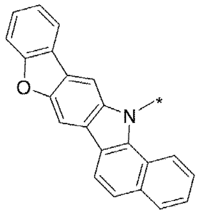
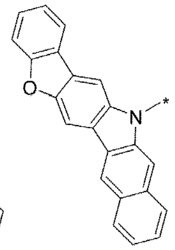
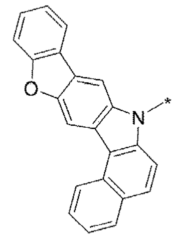

D369 D370 D371 D372 D373

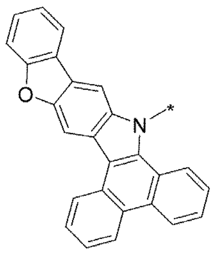
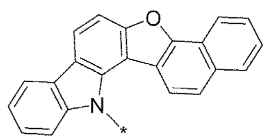
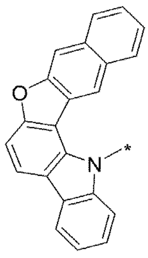
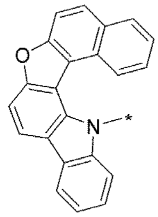

D374 D375 D376 D377

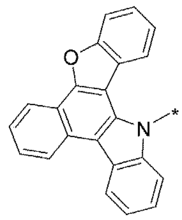
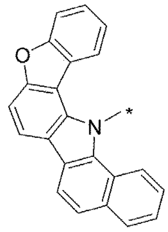
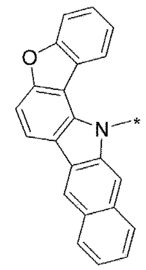
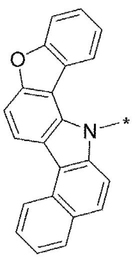

D378 D379 D380 D381

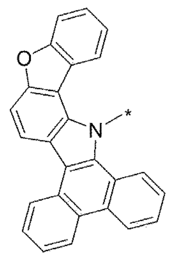
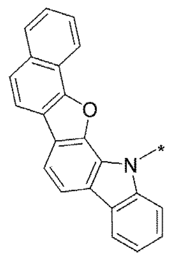
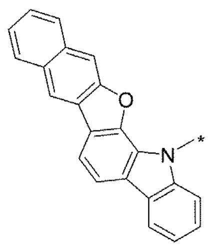
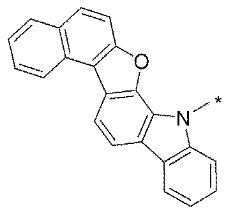

D382 D383 D384 D385

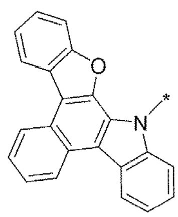
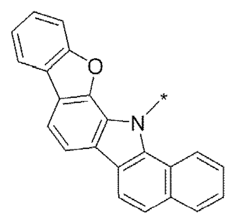
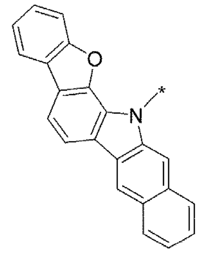
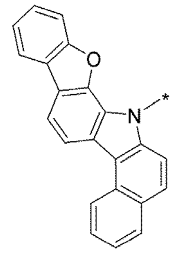

D386 D387 D388 D389

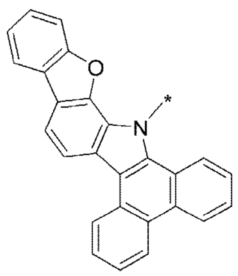
D390

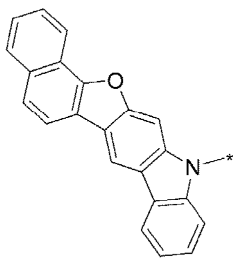
D391

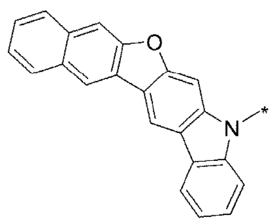
D392

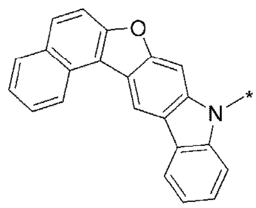
D393

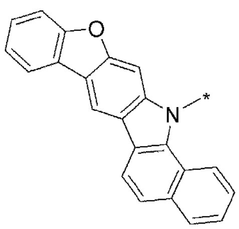
D394

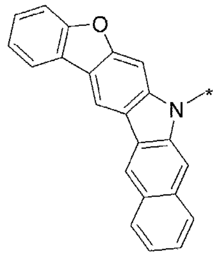
D395

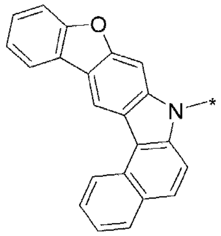
D396

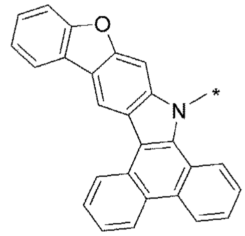
D397

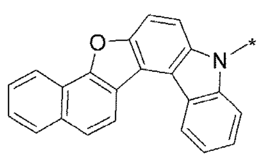
D398

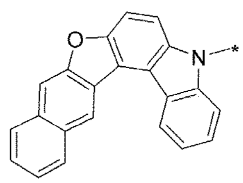
D399

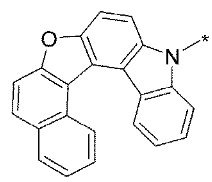
D400

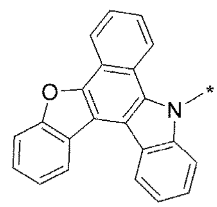
D401

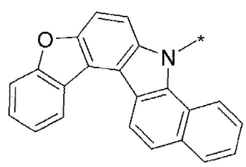
D402

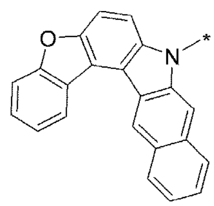
D403

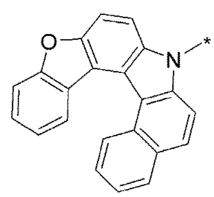
D404

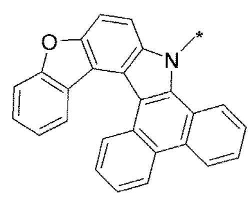
D405

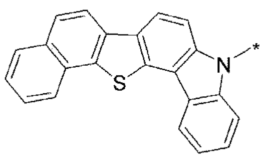
D406

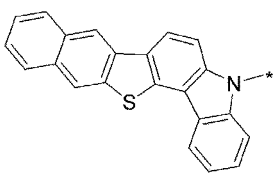
D407

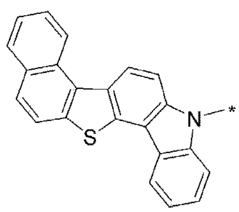
D408

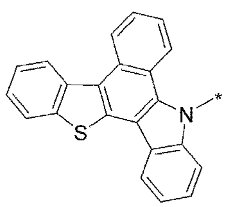
D409

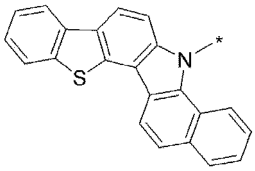 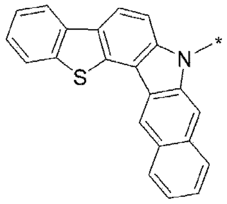 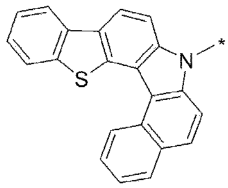 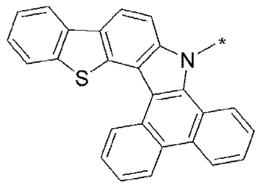

D410 D411 D412 D413

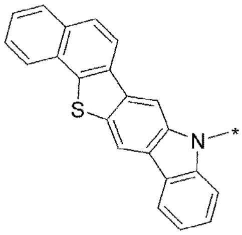 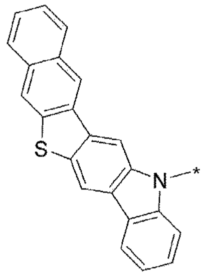 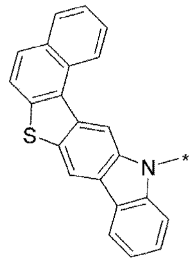 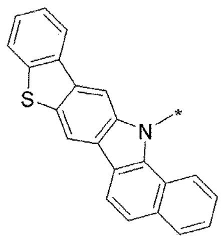

D414 D415 D416 D417

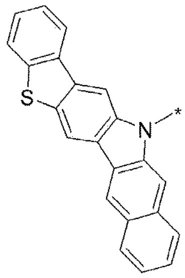 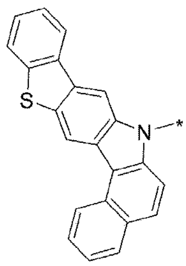 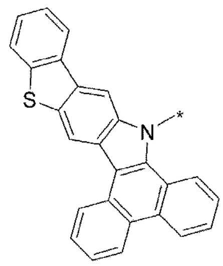 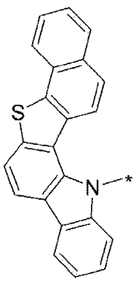

D418 D419 D420 D421

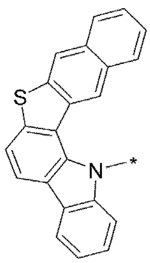 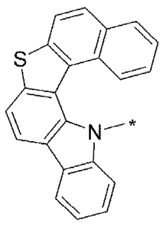 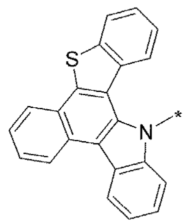 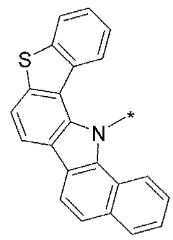

D422 D423 D424 D425

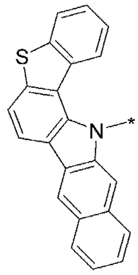 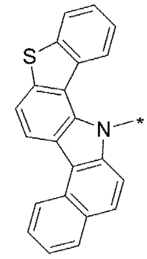 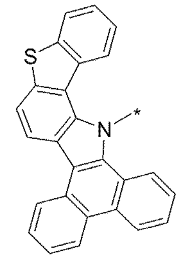 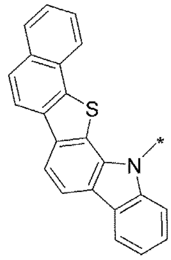

D426 D427 D428 D429

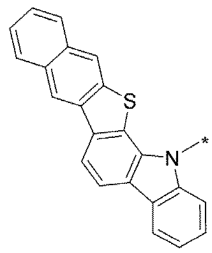 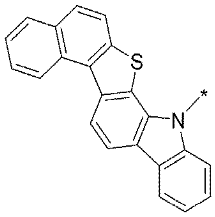 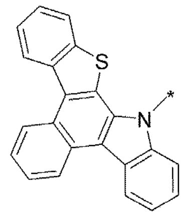 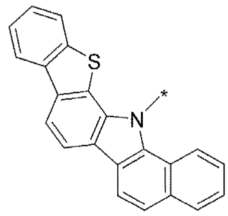

D430 D431 D432 D433

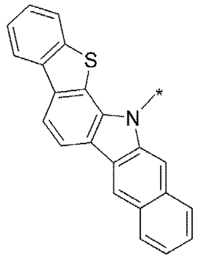 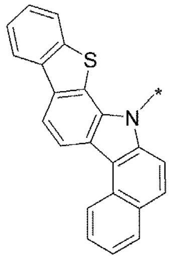 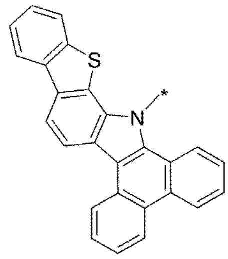 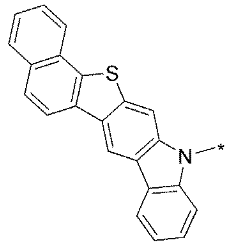

D434 D435 D436 D437

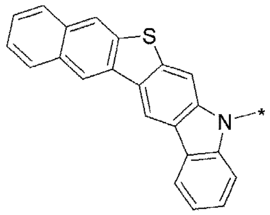 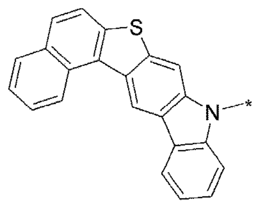 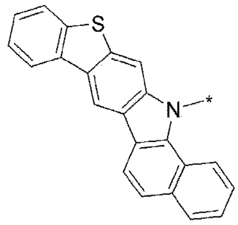 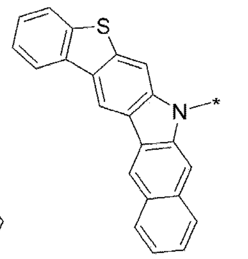

D438 D439 D440 D441

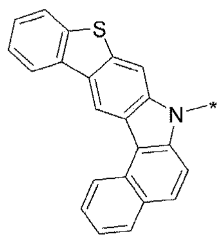 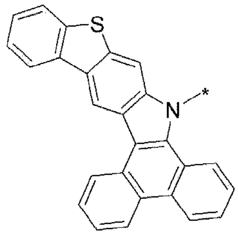 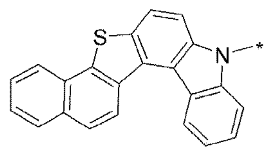 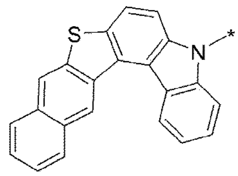

D442 D443 D444 D445

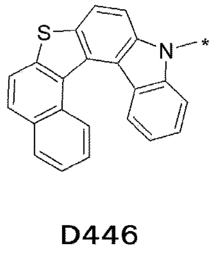 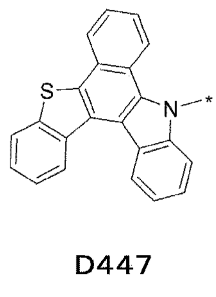 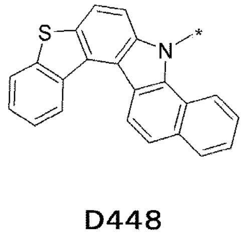 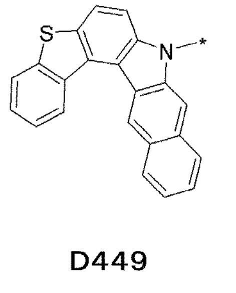

D446 D447 D448 D449

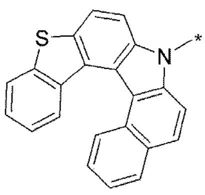
D450

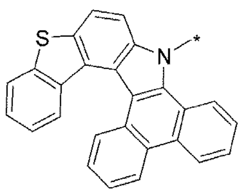
D451

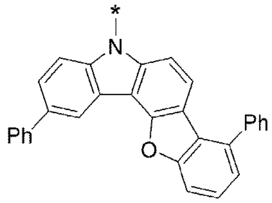
D452

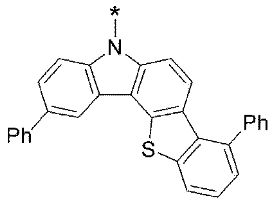
D453

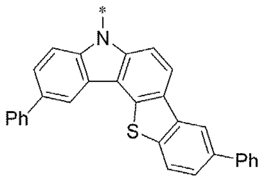
D454

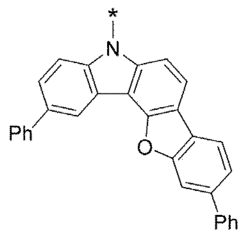
D455

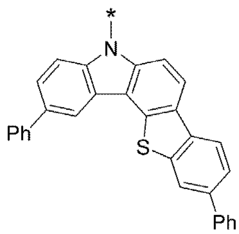
D456

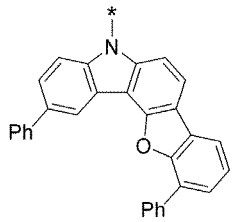
D457

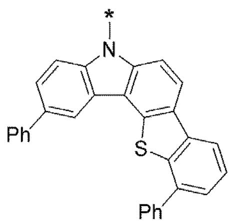
D458

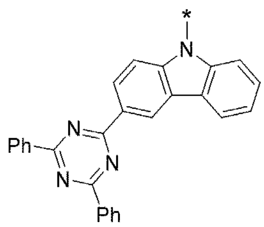
D459

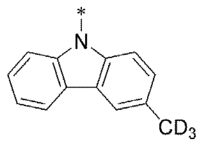
D460

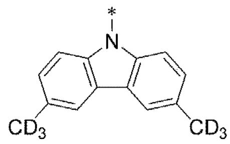
D461

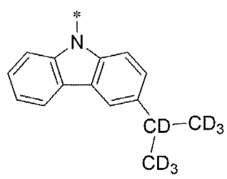
D462

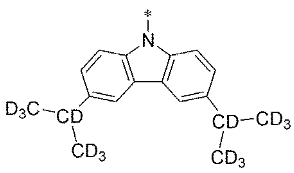
D463

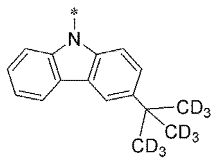
D464

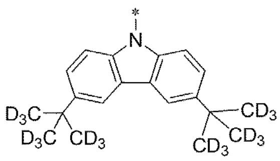
D465

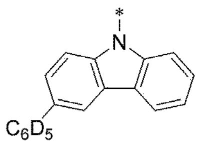
D466

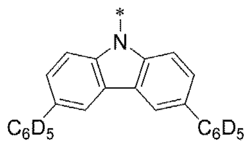
D467

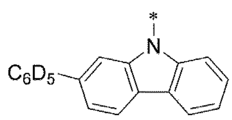
D468

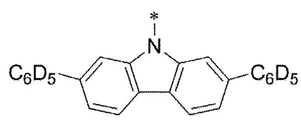
D469

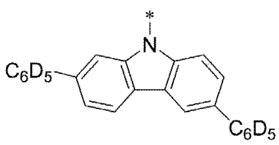
D470

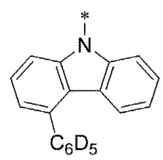
D471

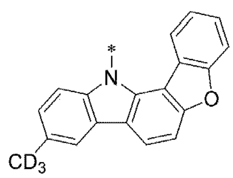
D472

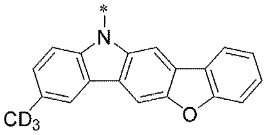
D473

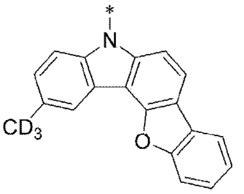 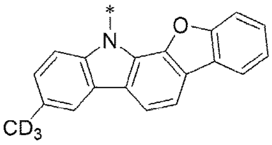 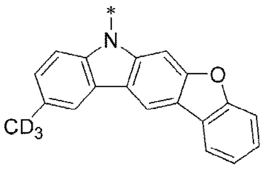 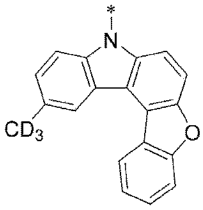

D474 D475 D476 D477

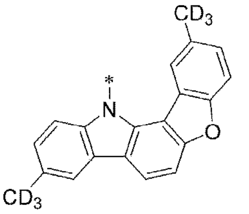 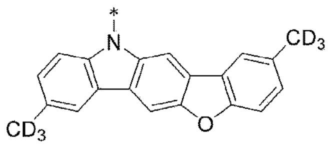 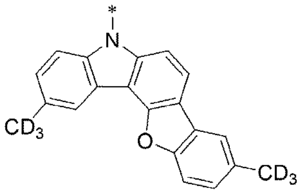

D478 D479 D480

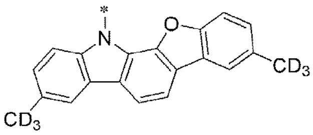 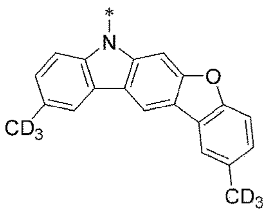 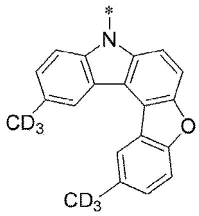 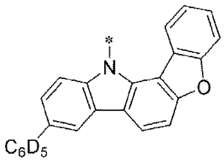

D481 D482 D483 D484

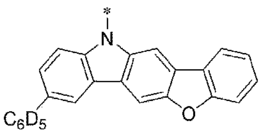 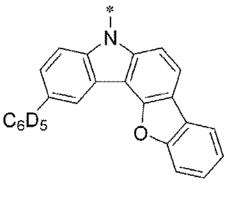 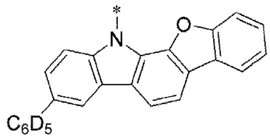 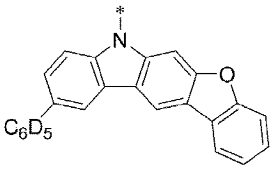

D485 D486 D487 D488

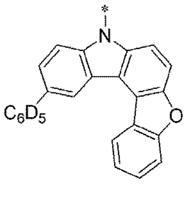 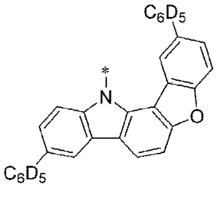 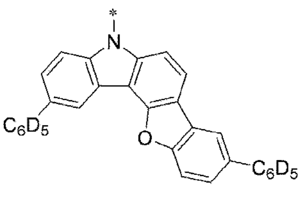 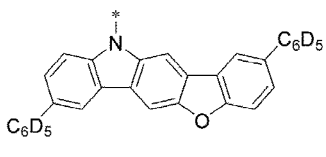

D489 D490 D491 D492

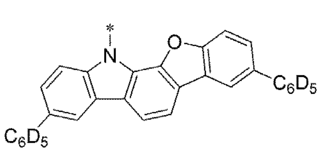 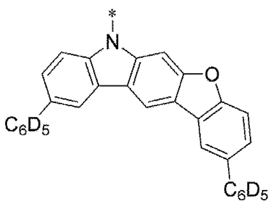 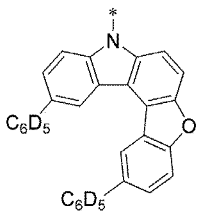 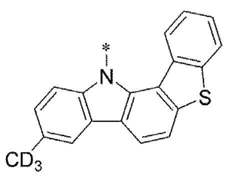

D493 D494 D495 D496

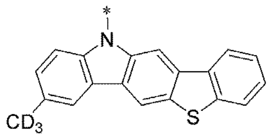 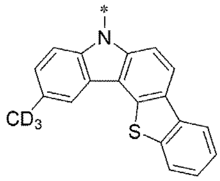 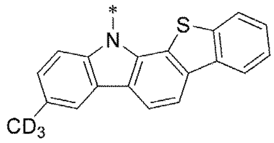 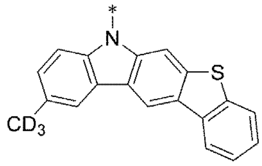

D497 D498 D499 D500

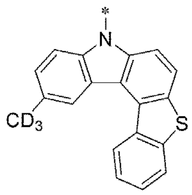 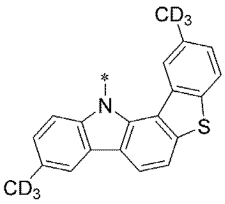 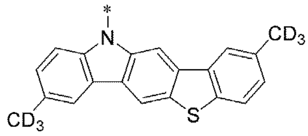 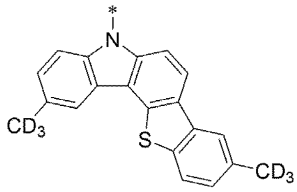

D501 D502 D503 D504

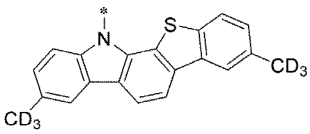 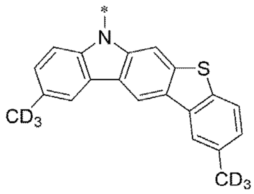 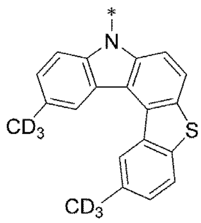 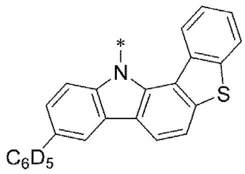

D505 D506 D507 D508

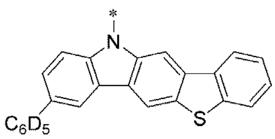 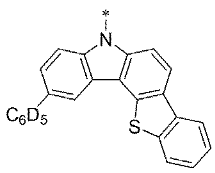 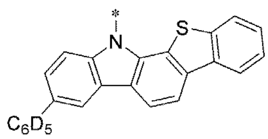 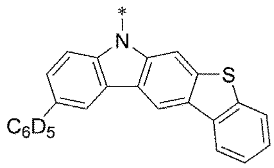

D509 D510 D511 D512

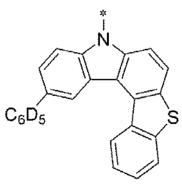 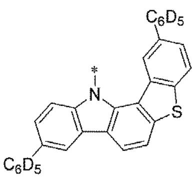 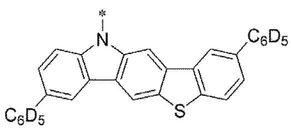 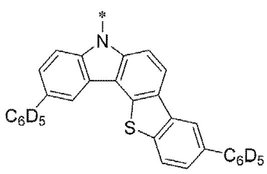

D513 D514 D515 D516

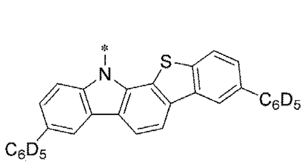 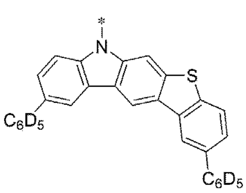 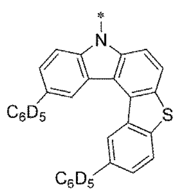 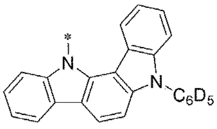

D517 D518 D519 D520

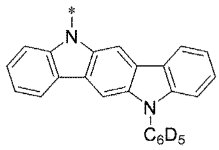
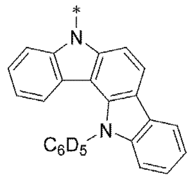
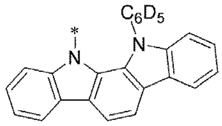
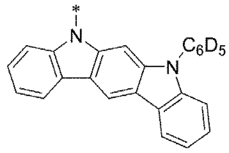

D521 D522 D523 D524

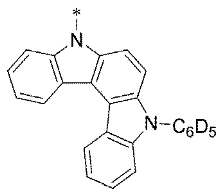
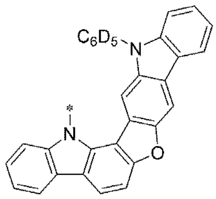
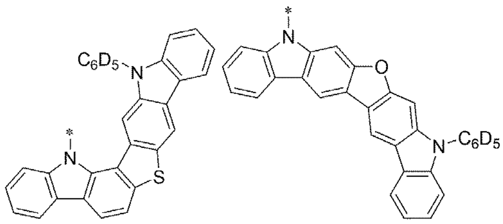

D525 D526 D527 D528

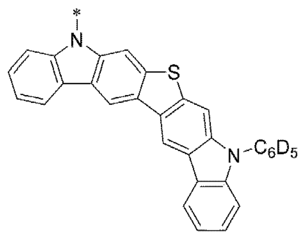
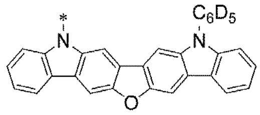
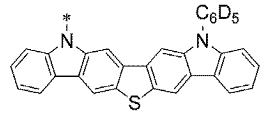

D529 D530 D531

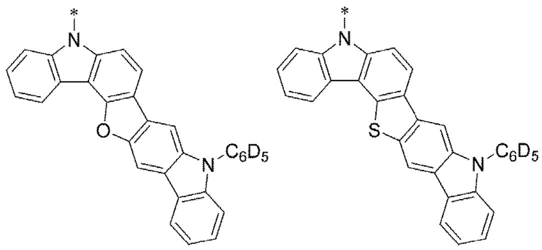
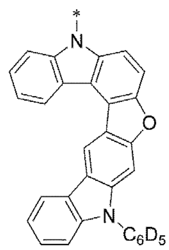
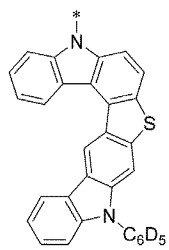

D532 D533 D534 D535

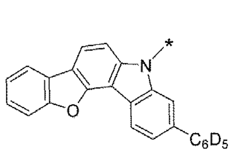
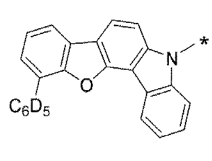
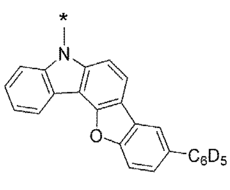
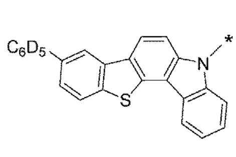

D536 D537 D538 D539

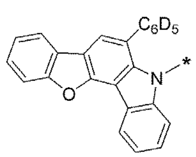
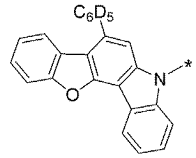
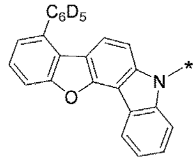
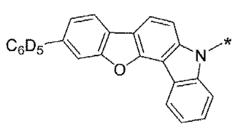

D540 D541 D542 D543

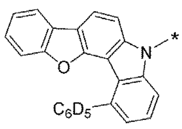
D544

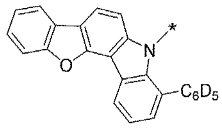
D545

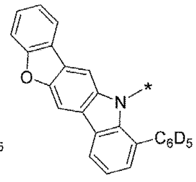
D546

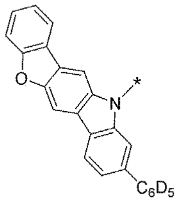
D547

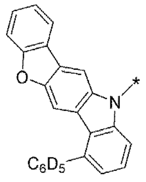
D548

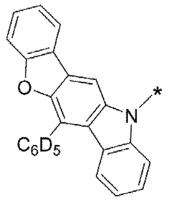
D549

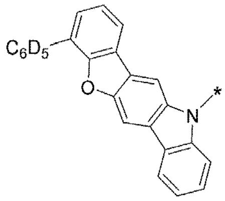
D550

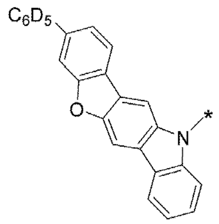
D551

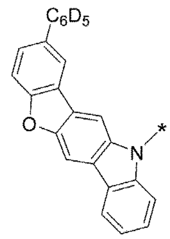
D552

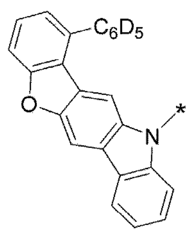
D553

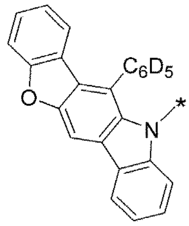
D554

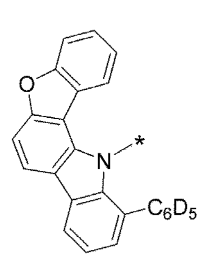
D555

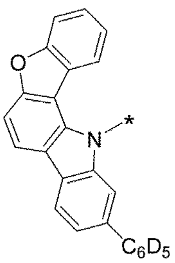
D556

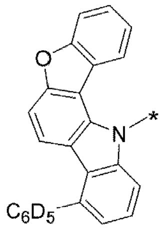
D557

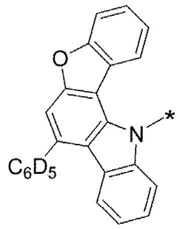
D558

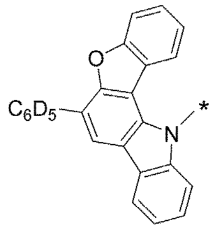
D559

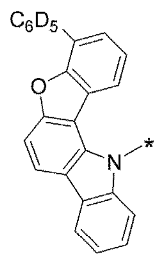
D560

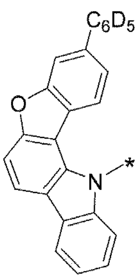
D561

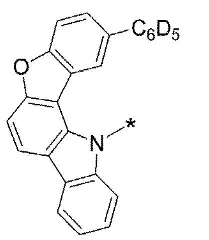
D562

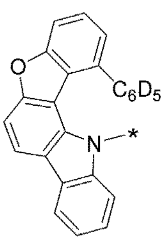
D563

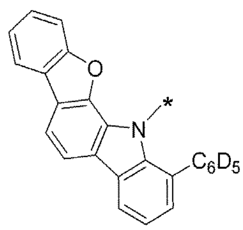
D564

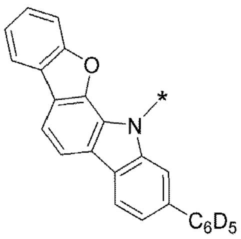 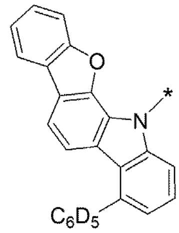 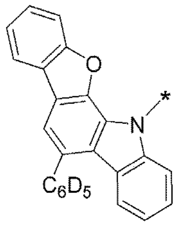 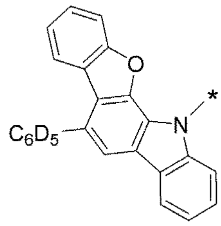

D565 D566 D567 D568

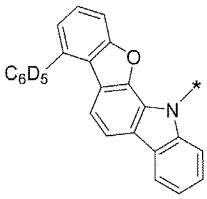 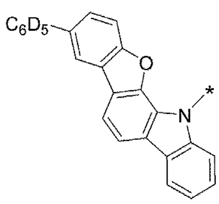 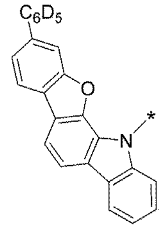 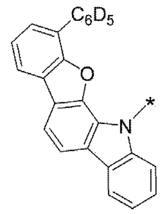

D569 D570 D571 D572

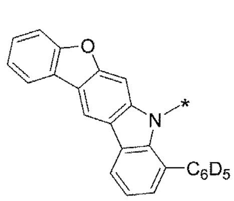 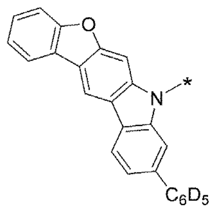 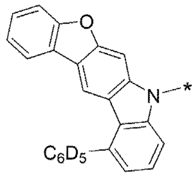 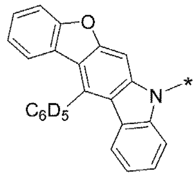

D573 D574 D575 D576

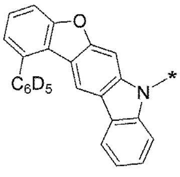 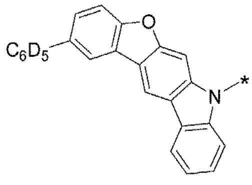 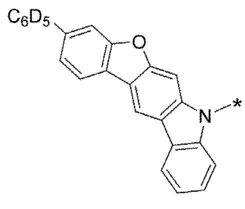 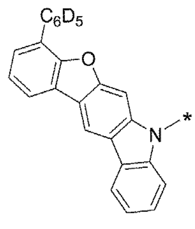

D577 D578 D579 D580

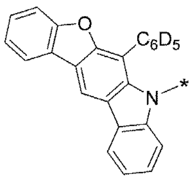 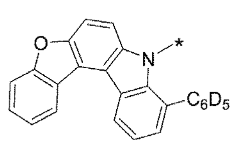 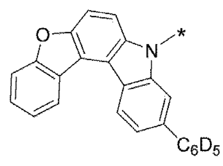 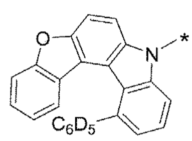

D581 D582 D583 D584

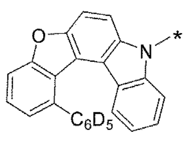 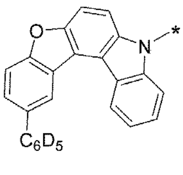 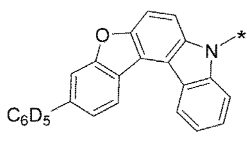 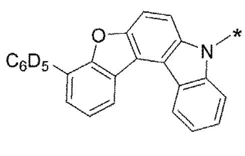

D585 D586 D587 D588

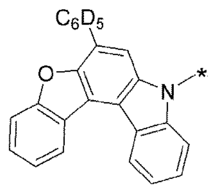 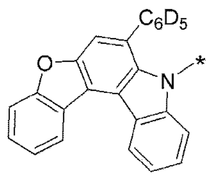 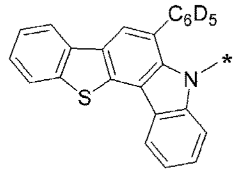 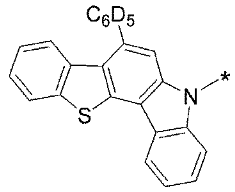

D589 D590 D591 D592

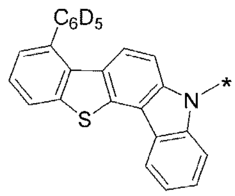 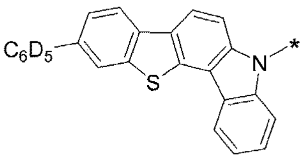 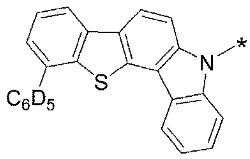 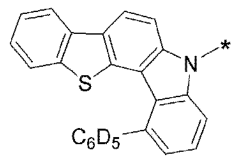

D593 D594 D595 D596

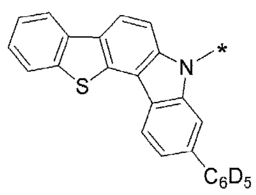 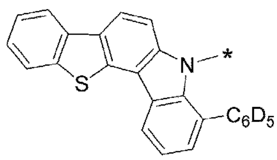 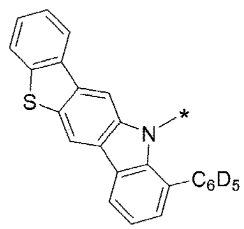 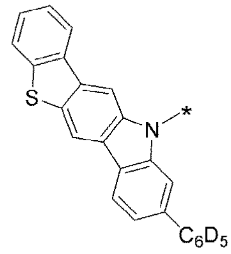

D597 D598 D599 D600

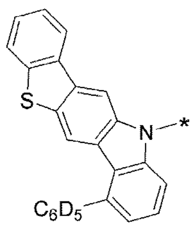 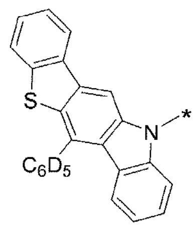 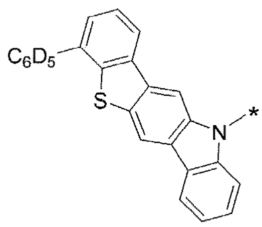 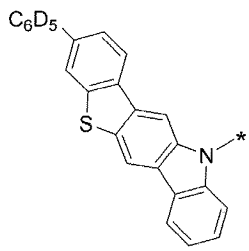

D601 D602 D603 D604

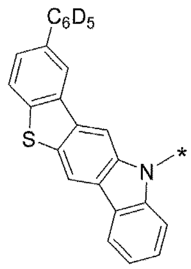 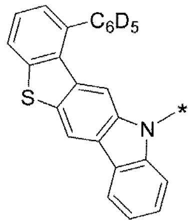 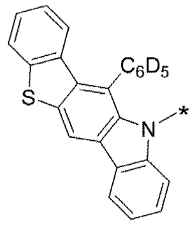 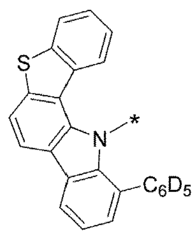

D605 D606 D607 D608

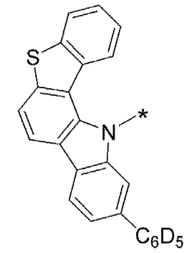 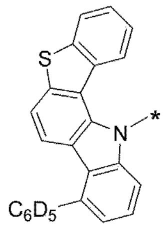 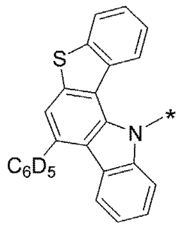 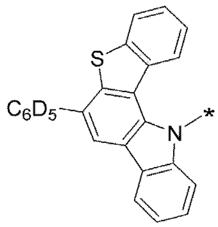

D609 D610 D611 D612

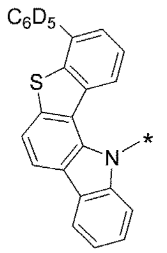 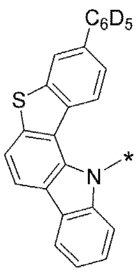 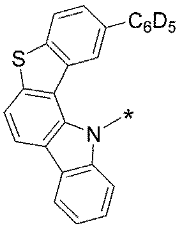 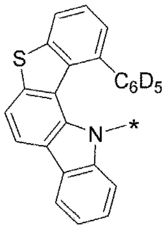

D613 D614 D615 D616

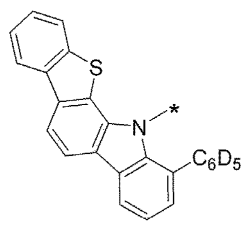 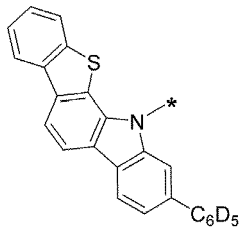 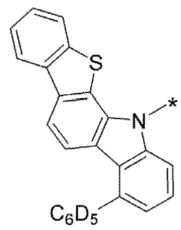 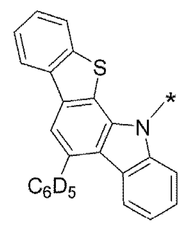

D617 D618 D619 D620

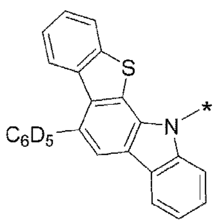 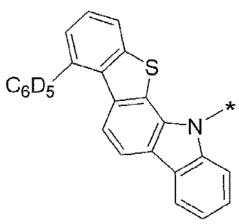 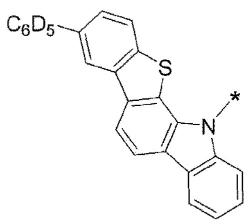 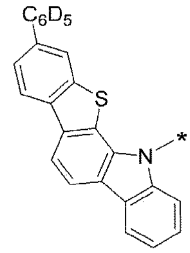

D621 D622 D623 D624

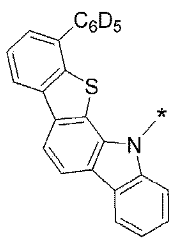 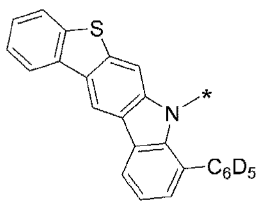 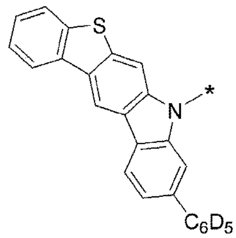 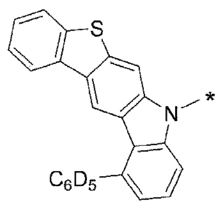

D625 D626 D627 D628

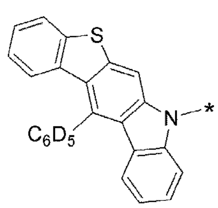
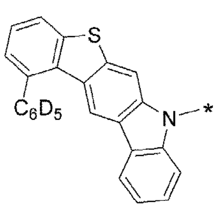
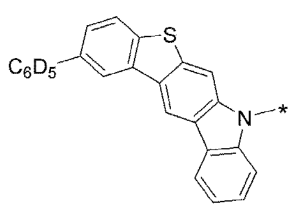
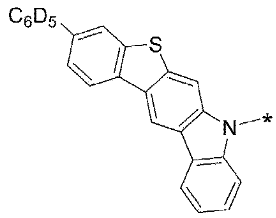

D629 D630 D631 D632

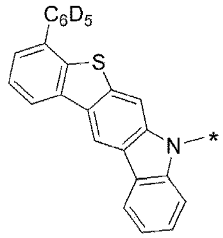
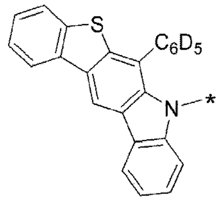
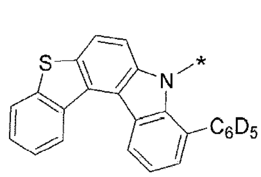
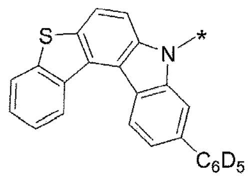

D633 D634 D635 D636

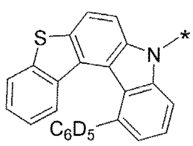
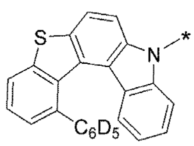
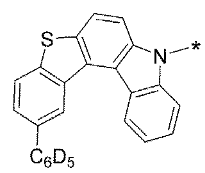
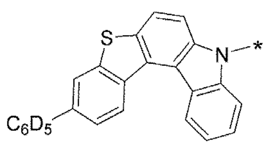

D637 D638 D639 D640

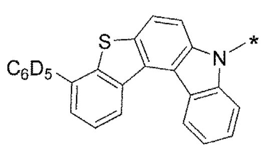
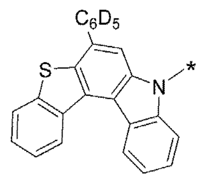
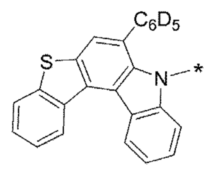
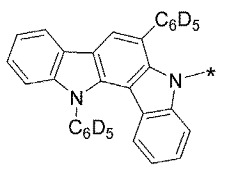

D641 D642 D643 D644

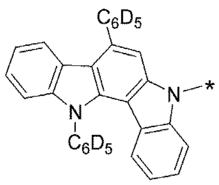
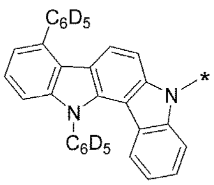
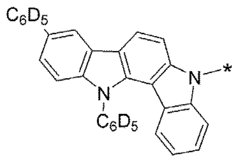
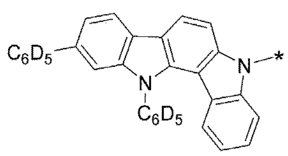

D645 D646 D647 D648

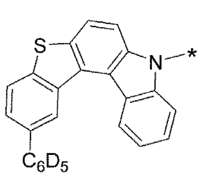
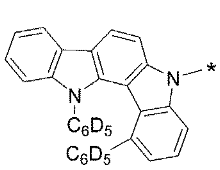
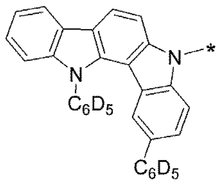
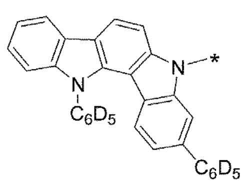

D649 D650 D651 D652

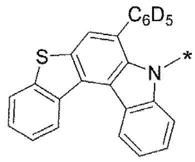

D653

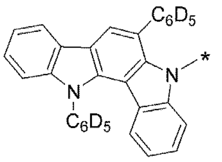

D654

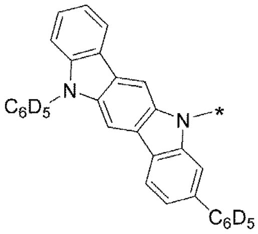

D655

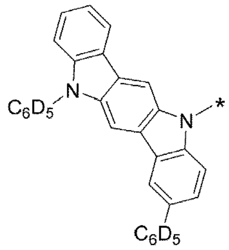

D656

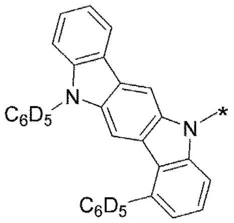

D657

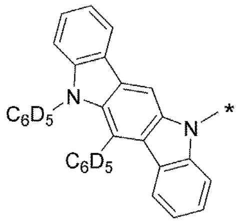

D658

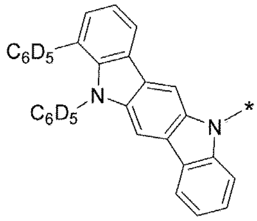

D659

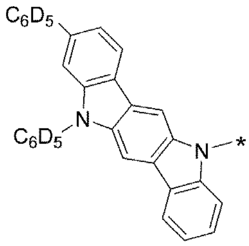

D660

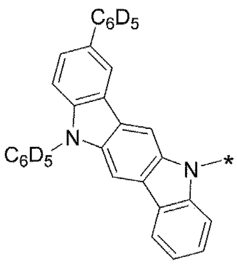

D661

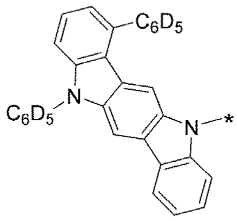

D662

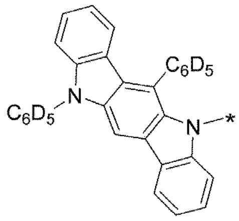

D663

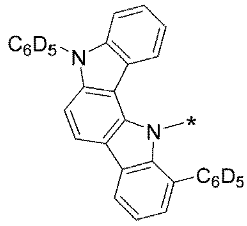

D664

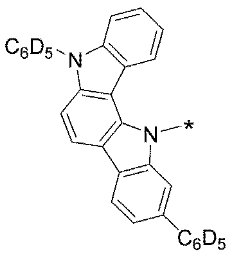

D665

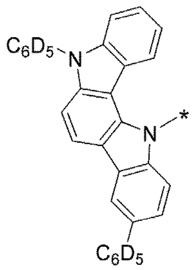

D666

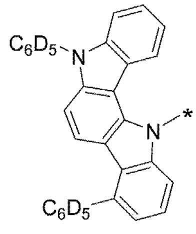

D667

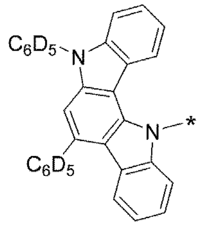

D668

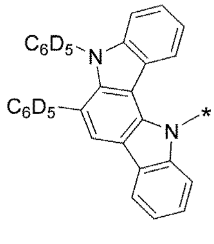

D669

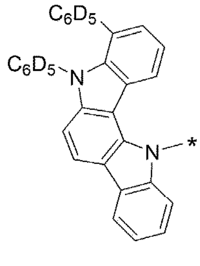

D670

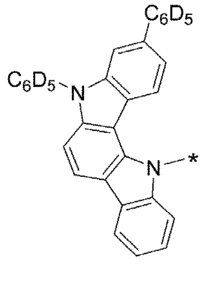

D671

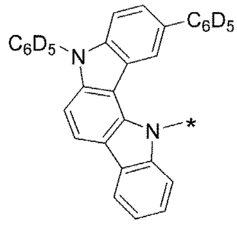

D672

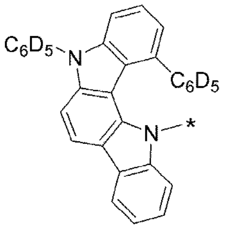

D673

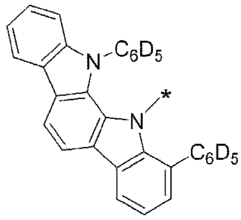

D674

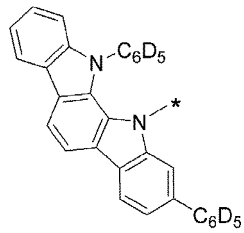

D675

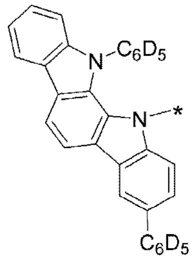

D676

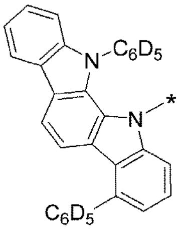

D677

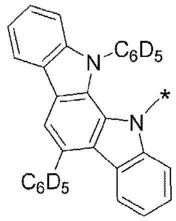

D678

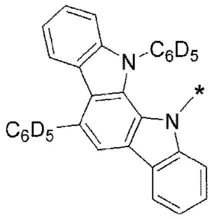

D679

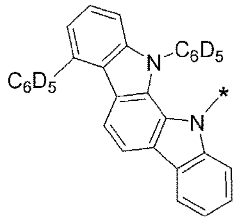

D680

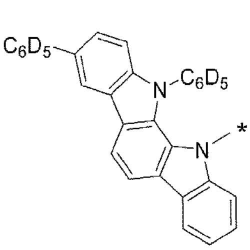

D681

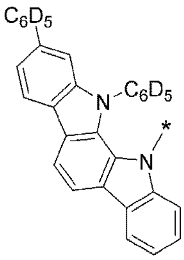

D682

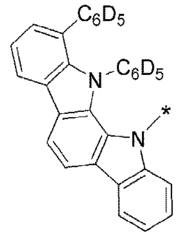

D683

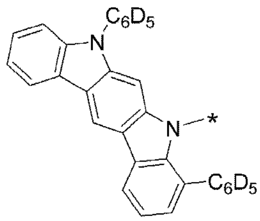

D684

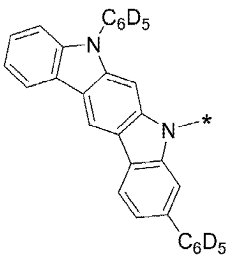

D685

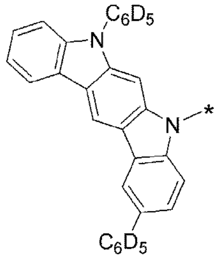

D686

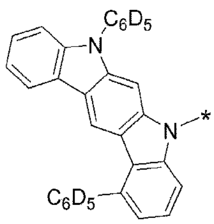

D687

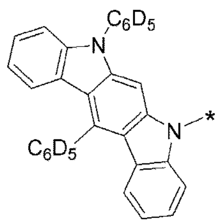

D688

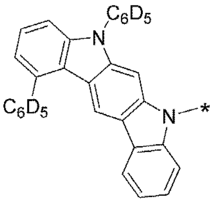

D689

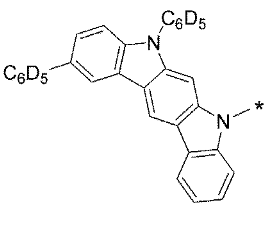

D690

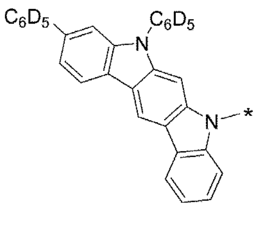

D691

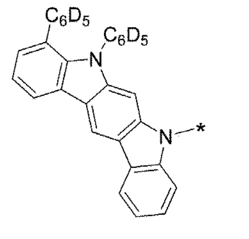

D692

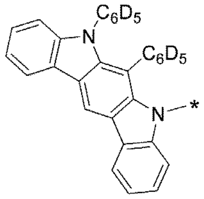 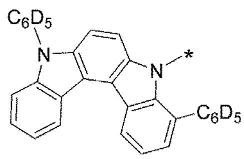 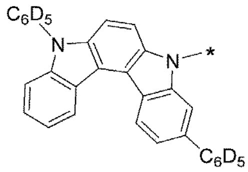 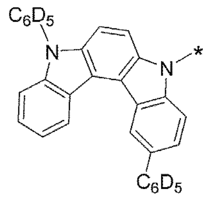

D693 D694 D695 D696

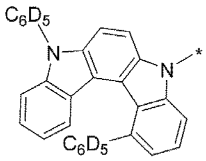 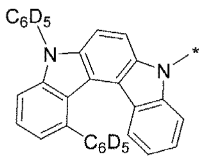 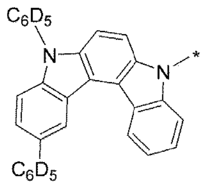 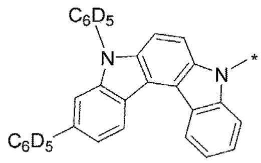

D697 D698 D699 D700

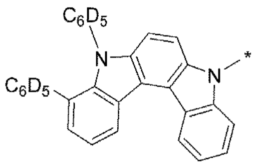 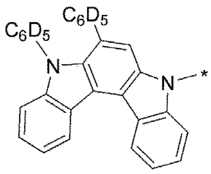 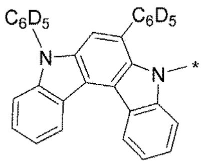 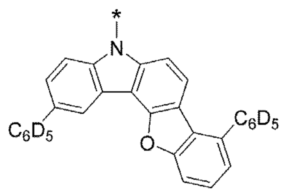

D701 D702 D703 D704

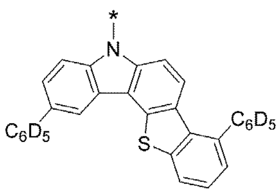 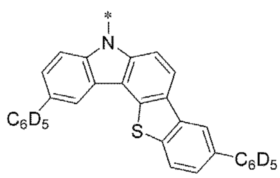 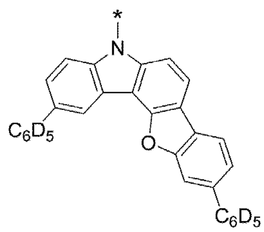

D705 D706 D707

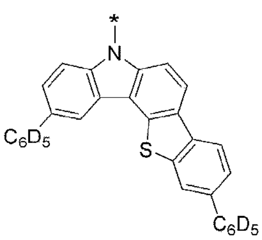 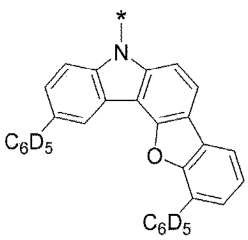 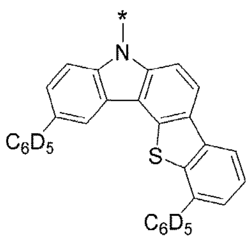 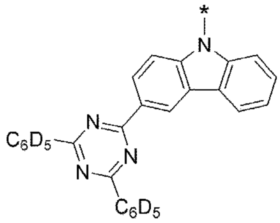

D708 D709 D710 D711

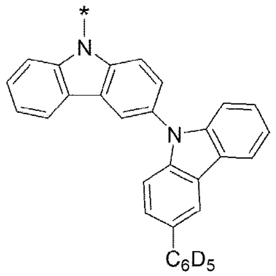

D712

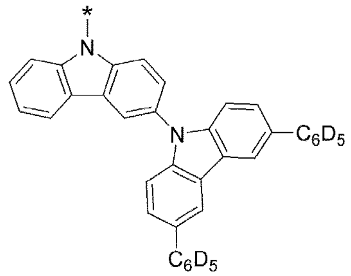

D713

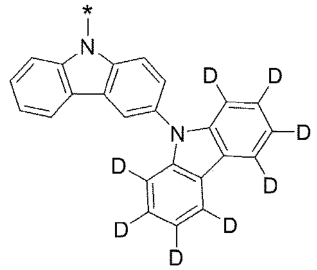

D714

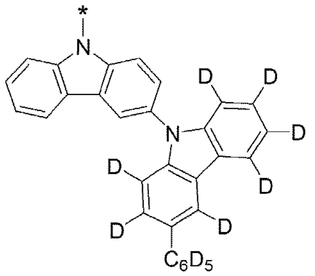

D715

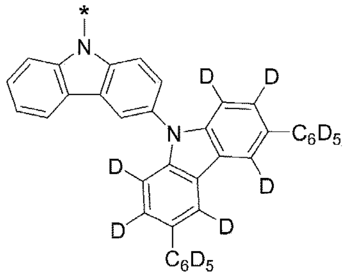

D716

Groups obtained by substituting all hydrogen atoms present in the above D1 to D459 with deuterium atoms are disclosed as D717 to D1175. Phenyl groups with any of the above D1 to D1175 bonding to the 3-position (that is, groups with a metaphenylene group further bonding to * of D1 to D1175) are disclosed as D1(m) to D1175(m). Phenyl groups with any of the above D1 to D1175 bonding to the 4-position (that is, groups with a paraphenylene group further bonding to * of D1 to D1175) are disclosed as D1(p) to D1175(p).

In one preferred aspect of the present invention, the donor group that $R^1$ to $R^5$ in the general formula (1) can take is selected from the group consisting of D1 to D1175. In one aspect of the present invention, the donor group that $R^1$ to $R^5$ can take is selected from the group consisting of D460 to D1175. In one aspect of the present invention, the donor group that $R^1$ to $R^5$ can take is selected from the group consisting of D1(m) to D1175(m). In one aspect of the present invention, the donor group that $R^1$ to $R^5$ can take is selected from the group consisting of D1(p) to D1175(p).

In one aspect of the present invention, the donor group that $R^1$ to $R^5$ can take is selected from the group consisting of D1 to D13, and D717 to D729. In one aspect of the present invention, the donor group that $R^1$ to $R^5$ can take is selected from the group consisting of D14 to D16, and D730 to D732. In one aspect of the present invention, the donor group that $R^1$ to $R^5$ can take is selected from the group consisting of D17 to D87, and D733 to D803. In one aspect of the present invention, the donor group that $R^1$ to $R^5$ can take is selected from the group consisting of D88 to D123, and D804 to D839. In one aspect of the present invention, the donor group that $R^1$ to $R^5$ can take is selected from the group consisting of D124 to D189, and D840 to D905. In one aspect of the present invention, the donor group that $R^1$ to $R^5$ can take is selected from the group consisting of D190 to D363, D452 to D459, D906 to D1079, and D1168 to D1175. In one aspect of the present invention, the donor group that $R^1$ to $R^5$ can take is selected from the group consisting of D364 to D451, and D1080 to D1167. In one aspect of the present invention, the donor group that $R^1$ to $R^5$ can take is selected from the group consisting of D460 to D716.

One or more of $R^1$ to $R^5$ in the general formula (1) is a cyano group. In one aspect of the present invention, one or two of $R^1$ to $R^5$ is a cyano group. In one preferred aspect of the present invention, at least $R^2$ is a cyano group. In one aspect of the present invention, at least $R^1$ is a cyano group. In one aspect of the present invention, at least $R^3$ is a cyano group. In one preferred aspect of the present invention, only $R^2$ is a cyano group. In one aspect of the present invention, only $R^1$ is a cyano group. In one aspect of the present invention, only $R^3$ is a cyano group. In one aspect of the present invention, only $R^2$ and $R^4$ are cyano groups. In one aspect of the present invention, only $R^2$ and $R^5$ are cyano groups. In one aspect of the present invention, only $R^1$ and $R^3$ are cyano groups.

One or more of $R^1$ to $R^5$ in the general formula (1) is a donor group. In one aspect of the present invention, one to three of $R^1$ to $R^5$ is a donor group. In one aspect of the present invention, two or three of $R^1$ to $R^5$ are donor groups. In one preferred aspect of the present invention, two of $R^1$ to $R^5$ are donor groups. In one preferred aspect of the present invention, three of $R^1$ to $R^5$ are donor groups. In one aspect of the present invention, at least $R^3$ is a donor group. In one aspect of the present invention, at least $R^4$ is a donor group. In one aspect of the present invention, at least $R^5$ is a donor group. In one aspect of the present invention, only $R^3$ is a donor group. In one aspect of the present invention, only $R^4$ is a donor group. In one aspect of the present invention, only $R^5$ is a donor group. In one aspect of the present invention, only $R^3$ and $R^5$ are donor groups. In one aspect of the present invention, only $R^2$ and $R^5$ are donor groups. In one aspect of the

present invention, only $R^2$ and $R^4$ are donor groups. In one aspect of the present invention, only $R^3$, $R^4$ and $R^5$ are donor groups. In one aspect of the present invention, only $R^2$, $R^4$ and $R^5$ are donor groups. When 2 or more of $R^1$ to $R^5$ are donor groups, these can be the same or different.

The number of $R^1$ to $R^5$ that are hydrogen atoms or deuterium atoms is 0 to 2, preferably 0 or 1, and is, for example, 1, or for example, 0. These show more excellent light emission characteristics than the compounds where the number of $R^1$ to $R^5$ that are hydrogen atoms or deuterium atoms is 3. The number of $R^1$ to $R^5$ that are substituted or unsubstituted aryl groups is 0 or 1, and is preferably 1. The number can be 0. The number of $R^1$ to $R^5$ that are substituted or unsubstituted alkyl groups is 0 to 3, preferably 0 to 2, and can be 1 or can be 0.

In one preferred aspect of the present invention, one of $R^1$ to $R^5$ is a cyano group, two are donor groups, one is a substituted or unsubstituted aryl group, and one is a hydrogen atom or a deuterium atom. In one aspect of the present invention, two donor groups are the same. In one aspect of the present invention, two donor groups are different from each other. In one aspect of the present invention, $R^1$ is a hydrogen atom or a deuterium atom, and $R^2$ or $R^3$ is a cyano group. In one preferred aspect of the present invention, $R^1$ is a hydrogen atom or a deuterium atom, $R^2$ is a cyano group, $R^3$ and $R^5$ are donor groups, and $R^4$ is a substituted or unsubstituted aryl group. In one aspect of the present invention, $R^1$ is a hydrogen atom or a deuterium atom, $R^2$ is a cyano group, $R^4$ and $R^5$ are donor groups, and $R^3$ is a substituted or unsubstituted aryl group. In one aspect of the present invention, $R^1$ is a hydrogen atom or a deuterium atom, $R^2$ is a cyano group, $R^3$ and $R^4$ are donor groups, and $R^5$ is a substituted or unsubstituted aryl group.

In one preferred aspect of the present invention, one of $R^1$ to $R^5$ is a cyano group, three are donor groups, and one is a hydrogen atom or a deuterium atom. In one aspect of the present invention, three donor groups are the same. In one aspect of the present invention, two of three donor groups are the same, and one differs. In one aspect of the present invention, $R^1$ is a hydrogen atom or a deuterium atom, $R^2$ is a cyano group, and $R^3$ to $R^5$ are donor groups. In one aspect of the present invention, $R^1$ is a hydrogen atom or a deuterium atom, $R^3$ is a cyano group, $R^2$, $R^4$ and $R^5$ are donor groups.

In one aspect of the present invention, one of $R^1$ to $R^5$ is a cyano group, one is a donor group, one is a substituted or unsubstituted aryl group, and two are hydrogen atoms or deuterium atoms. In one aspect of the present invention, $R^1$ and $R^3$ are hydrogen atoms or deuterium atoms, $R^2$ is a cyano group, $R^4$ is a substituted or unsubstituted aryl group, and $R^5$ is a donor group. In one aspect of the present invention, $R^1$ and $R^3$ are hydrogen atoms or deuterium atoms, $R^2$ is a cyano group, $R^4$ is a donor group, and $R^5$ is a substituted or unsubstituted aryl group. In one aspect of the present invention, $R^1$ and $R^5$ are hydrogen atoms or deuterium atoms, $R^2$ is a cyano group, $R^3$ is a donor group, and $R^4$ is a substituted or unsubstituted aryl group. In one aspect of the present invention, $R^1$ and $R^5$ are hydrogen atoms or deuterium atoms, $R^2$ is a cyano group, $R^3$ is a donor group, and $R^4$ is a substituted or unsubstituted aryl group. In one aspect of the present invention, $R^1$ and $R^4$ are hydrogen atoms or deuterium atoms, $R^2$ is a cyano group, $R^3$ is a donor group, and $R^5$ is a substituted or unsubstituted aryl group. In one aspect of the present invention, $R^1$ and $R^4$ are hydrogen atoms or deuterium atoms, $R^2$ is a cyano group, $R^3$ is a donor group, and $R^5$ is a substituted or unsubstituted aryl group.

In one aspect of the present invention, one of $R^1$ to $R^5$ is a cyano group, two are donor groups, and two are hydrogen atoms or deuterium atoms. In one aspect of the present invention, $R^1$ and $R^4$ are hydrogen atoms or deuterium atoms, $R^2$ is a cyano group, and $R^3$ and $R^5$ are donor groups. In one aspect of the present invention, $R^1$ and $R^5$ are hydrogen atoms or deuterium atoms, $R^2$ is a cyano group, and $R^3$ and $R^4$ are donor groups. In one aspect of the present invention, $R^1$ and $R^3$ are hydrogen atoms or deuterium atoms, $R^2$ is a cyano group, and $R^4$ and $R^5$ are donor groups. In one aspect of the present invention, $R^1$ and $R^4$ are hydrogen atoms or deuterium atoms, $R^3$ is a cyano group, and $R^2$ and $R^5$ are donor groups.

The heteroaryl group that $Ar^1$ and $Ar^2$ in the general formula (1) can take each can be a monocyclic ring or can be a fused ring of two or more kinds of rings. In the case of a fused ring, the number of fused rings is preferably 2 to 6, and can be selected from, for example, 2 to 4. Specific examples of the ring include a pyridine ring, a pyrimidine ring and a pyrrole ring, and these rings can be fused with any other ring. Specific examples of the heteroaryl group include a 2-pyridyl group, a 3-pyridyl group, a 4-pyridyl group, a carbazol-9-yl group, a carbazol-1-yl group, a carbazol-2-yl group, a carbazol-3-yl group, and a carbazol-4-yl group. The number of the ring skeleton-constituting atoms of the heteroaryl group is preferably 4 to 40, more preferably 5 to 20, and can be selected from a range of 5 to 16, or can be selected from a range of 5 to 12.

At least one of $Ar^1$ an $Ar^2$ in the general formula (1) is a substituted or unsubstituted heteroaryl group bonding via a nitrogen atom. Specifically, the group is a substituted or unsubstituted heteroaryl group containing a nitrogen atom as a ring skeleton-constituting atom, which bonds via the nitrogen atom which is one of the ring skeleton-constituting atoms. A typical example of the group is a substituted or unsubstituted pyrrol-1-yl group, preferably a substituted or unsubstituted ring-fused pyrrol-1-yl group, more preferably a substituted or unsubstituted carbazol-9-yl group, of which the carbazole skeleton can be fused with any other ring. Specific examples of the substituted or unsubstituted heteroaryl group bonding via a nitrogen atom, which at least one of $Ar^1$ and $Ar^2$ can take, include the above-mentioned D1 to D1176.

In one aspect of the present invention, the group that at least one of $Ar^1$ and $Ar^2$ can take is selected from the group consisting of D1 to D13, and D717 to D729. In one aspect of the present invention, the group that at least one of $Ar^1$ and $Ar^2$ can take is selected from the group consisting of D14 to D16, and D730 to D732. In one aspect of the present invention, the group that at least one of $Ar^1$ and $Ar^2$ can take is selected from the group consisting of D17 to D87, and D733

to D803. In one aspect of the present invention, the group that at least one of $Ar^1$ and $Ar^2$ can take is selected from the group consisting of D88 to D123, and D804 to D839. In one aspect of the present invention, the group that at least one of $Ar^1$ and $Ar^2$ can take is selected from the group consisting of D124 to D189, and D840 to D905. In one aspect of the present invention, the group that at least one of $Ar^1$ and $Ar^2$ can take is selected from the group consisting of D190 to D363, D452 to D459, D906 to D1079, and D1168 to D1175. In one aspect of the present invention, the group that at least one of $Ar^1$ and $Ar^2$ can take is selected from the group consisting of D364 to D451, and D1080 to D1167. In one aspect of the present invention, the group that at least one of $Ar^1$ and $Ar^2$ can take is selected from the group consisting of D460 to D716.

In one aspect of the present invention, $Ar^1$ and $Ar^2$ each are independently a substituted or unsubstituted heteroaryl group that bonds via a nitrogen atom. In one aspect of the present invention, $Ar^1$ and $Ar^2$ are substituted or unsubstituted heteroaryl groups bonding via a nitrogen atom, which have the same structure. In one aspect of the present invention, only $Ar^1$ is a substituted or unsubstituted heteroaryl group bonding via a nitrogen atom, and $Ar^2$ is a substituted or unsubstituted aryl group.

In the general formula (1), $L^1$ represents a single bond or a divalent linking group. The divalent linking group includes a substituted or unsubstituted arylene group, and a substituted or unsubstituted heteroarylene group. In one preferred aspect of the present invention, $L^1$ is a single bond. In one aspect of the present invention, $L^1$ is a substituted or unsubstituted arylene group. In one aspect of the present invention, $L^1$ is a substituted or unsubstituted heteroarylene group. Regarding the aryl moiety constituting the arylene group, reference can be made to the description and the preferred range of the aryl group in the description section of $R^1$ to $R^5$ mentioned above. The heteroarylene group includes a linking group formed by substituting at least one ring skeleton carbon atom constituting the arylene group with a nitrogen atom.

Specific examples of $L^1$ are shown below. However, $L^1$ which can be employed in the present invention shall not be construed as being limited by the following specific examples. In the following specific examples, expression of a methyl group is omitted. Consequently, for example, L3 to L5 are substituted with a methyl group. * indicates a bonding site. L1 is a single bond.

none (direct bond)

L1

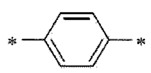

L2

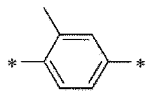

L3

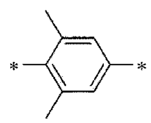

L4

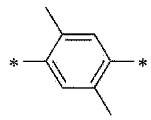

L5

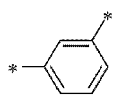

L6

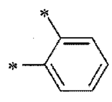

L7

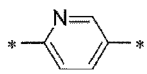

L8

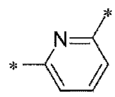

L9

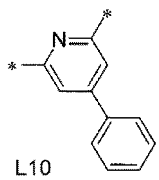

L10

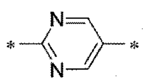

L11

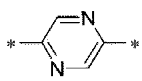

L12

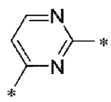

L13

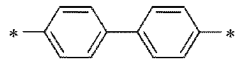

L14

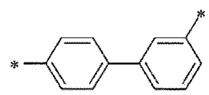

L15

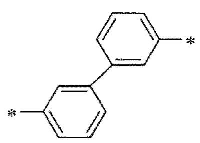

L16

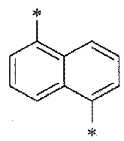

L17

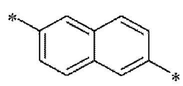

L18

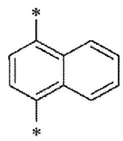

L19

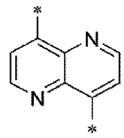

L20

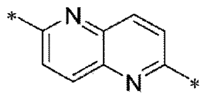

L21

Groups obtained by substituting all hydrogen atoms present in the above L2 to L21 with deuterium atoms are disclosed as L22 to L41. In one aspect of the present invention, $L^1$ is selected from the group consisting of L1 to L7, and L22 to L27. In one aspect of the present invention, $L^1$ is selected from the group consisting of L2 to L7, and L22 to L27. In one aspect of the present invention, $L^1$ is selected from the group consisting of L1, L8 to L13, L20, L21, L28 to L33, L40 and L41. In one aspect of the present invention, $L^1$ is selected from the group consisting of L8 to L13, L20, L21, L28 to L33, L40 and L41. In one aspect of the present invention, $L^1$ is selected from the group consisting of L1, L14 to L19, and L34 to L39. In one aspect of the present invention, $L^1$ is selected from the group consisting of L14 to L19, and L34 to L39.

In the general formula (1), $X^1$ to $X^3$ each independently represent N or C(R). However, at least one of $X^1$ to $X^3$ is N. R represents a hydrogen atom, a deuterium atom or a substituent. As referred to herein, the substituent can be selected from Substituent Group A, can be selected from Substituent Group B, can be selected from Substituent Group C, can be selected from Substituent Group D, or can be selected from Substituent Group E. In one preferred aspect of the present invention, $X^1$ to $X^3$ are N. In one aspect of the present invention, $X^1$ and $X^3$ are N, and $X^2$ is C(R). In one aspect of the present invention, $X^1$ and $X^2$ are N, and $X^3$ is C(R). In one aspect of the present invention, $X^1$ is N, and $X^2$ and $X^3$ are C(R). In one aspect of the present invention, $X^2$ is N, and $X^1$ and $X^3$ are C(R). In one aspect of the present invention, R is a hydrogen atom or a deuterium atom. In one aspect of the present invention, R is an alkyl group optionally substituted with a deuterium atom. In one aspect of the present invention, R is an aryl group optionally substituted with a deuterium atom, an alkyl group or an aryl group.

In one preferred aspect of the present invention, $X^1$ to $X^3$ are N, $L^1$ is a single bond, $Ar^1$ and $Ar^2$ each are independently a substituted or unsubstituted heteroaryl group bonding via a nitrogen atom (preferably, a substituted or unsubstituted carbazol-9-yl group), $R^2$ is a cyano group, two of $R^1$ and $R^3$ to $R^5$ are donor groups (preferably, substituted or unsubstituted carbazol-9-yl groups), one is a substituted or unsubstituted aryl group, and one (preferably $R^1$) is a hydrogen atom or a deuterium atom.

In one preferred aspect of the present invention, $X^1$ to $X^3$ are N, $L^1$ is a single bond, $Ar^1$ is a substituted or unsubstituted heteroaryl group bonding via a nitrogen atom (preferably, a substituted or unsubstituted carbazol-9-yl group), $Ar^2$ is a substituted or unsubstituted aryl group, $R^2$ is a cyano group, two of $R^1$ and $R^3$ to $R^5$ are donor groups (preferably, substituted or unsubstituted carbazol-9-yl groups), one is a substituted or unsubstituted aryl group, and one (preferably $R^1$) is a hydrogen atom or a deuterium atom.

In one preferred aspect of the present invention, $X^1$ to $X^3$ are N, $L^1$ is a single bond, $Ar^1$ and $Ar^2$ each are independently a substituted or unsubstituted heteroaryl group bonding via a nitrogen atom (preferably, a substituted or unsubstituted carbazol-9-yl group), $R^2$ is a cyano group, three of $R^1$ and $R^3$ to $R^5$ are donor groups (preferably, substituted or unsubstituted carbazol-9-yl groups), and one (preferably $R^1$) is a hydrogen atom or a deuterium atom.

In one preferred aspect of the present invention, $X^1$ to $X^3$ are N, $L^1$ is a single bond, $Ar^1$ is a substituted or unsubstituted heteroaryl group bonding via a nitrogen atom (preferably, a substituted or unsubstituted carbazol-9-yl group), $Ar^2$ is a substituted or unsubstituted aryl group, $R^2$ is a cyano group, three of $R^1$ and $R^3$ to $R^5$ are donor groups (preferably, substituted or unsubstituted carbazol-9-yl groups), and one (preferably $R^1$) is a hydrogen atom or a deuterium atom.

In one preferred aspect of the present invention, $X^1$ to $X^3$ are N, $L^1$ is a single bond, $Ar^1$ and $Ar^2$ each are independently a substituted or unsubstituted heteroaryl group bonding via a nitrogen atom (preferably, a substituted or unsubstituted carbazol-9-yl group), $R^3$ is a cyano group, two to three of $R^1$, $R^2$, $R^4$ and $R^5$ are donor groups (preferably, substituted or unsubstituted carbazol-9-yl groups), one or two (preferably at least $R^1$) is a hydrogen atom or a deuterium atom, and zero or one is a substituted or unsubstituted aryl group.

In one aspect of the present invention, $X^1$ to $X^3$ are N, $L^1$ is a single bond, $Ar^1$ is a substituted or unsubstituted heteroaryl group bonding via a nitrogen atom (preferably, a substituted or unsubstituted carbazol-9-yl group), $Ar^2$ is a substituted or unsubstituted aryl group, $R^3$ is a cyano group, two to three of $R^1$, $R^2$, $R^4$ and $R^5$ are donor groups (preferably, substituted or unsubstituted carbazol-9-yl groups), one or two (preferably at least $R^1$) is a hydrogen atom or a deuterium atom, and zero or one is a substituted or unsubstituted aryl group.

The compound represented by the general formula (1) preferably does not contain a metal atom, and can be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the

general formula (1) can be a compound composed only of atoms selected from the group consisting of a carbon atom, a hydrogen atom, a deuterium atom, a nitrogen atom, and a sulfur atom. The compound represented by the general formula (1) can be a compound composed only of atoms selected from the group consisting of a carbon atom, a hydrogen atom, a deuterium atom, and a nitrogen atom. The compound represented by the general formula (1) can be a compound composed only of atoms selected from the group consisting of a carbon atom, a hydrogen atom, and a nitrogen atom. Further, the compound represented by the general formula (1) can be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), and a diarylaminoamino group (for example, having 0 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" can be selected, for example, from Substituent Group A, can be selected from Substituent Group B, can be selected from Substituent Group C, can be selected from Substituent Group D, or can be selected from Substituent Group E.

Specific examples of the compound represented by the general formula (1) are shown in the following Tables 1 to 4. However, the compound represented by the general formula (1) that can be used in the present invention should not be construed as being limited by these specific examples.

In Table 1, structures of the compounds are individually shown by specifying $R^3$ to $R^5$ of the following general formula (1a) for each compound. Specifically, the structures where $Ar^1$ and $Ar^2$ are deuterated carbazolyl groups (D717), $X^1$ to $X^3$ are nitrogen atoms (N), $L^1$ is a single bond, $R^1$ is a hydrogen atom, $R^2$ is a cyano group, and $R^3$ to $R^5$ are the groups specified in Table 1 are individually shown as structures of Compounds 1 to 1175.

General Formula (1a)

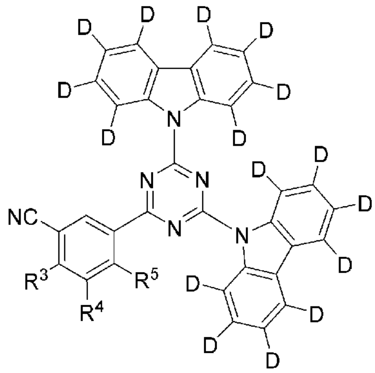

In Table 2, structures of Compounds 1 to 1015655 are shown by collectively displaying $R^3$ to $R^5$ of a plurality of compounds in each row. For example, in the row of Compounds 1 to 1175 in Table 2, compounds in which $R^4$ is fixed to Ar1, and $R^3$ and $R^5$ are both D1 to D1175 are referred to as Compounds 1 to 1175 in that order. $R^3$ and $R^5$ are the same. That is, the row of Compounds 1 to 1175 in Table 2 collectively represents Compounds 1 to 1175 specified in Table 1. Similarly, in the row of Compounds 1176 to 2350 in Table 2, those in which $R^4$ is fixed to Ar2, and $R^3$ and $R^5$ are both D1 to D1175 are referred to as Compounds 1176 to 2350 in that order. In the same manner, Compounds 2351 to 1015655 in Table 2 are also specified.

Table 1

| No. | $R^3$ | $R^4$ | $R^5$ |
|---|---|---|---|
| 1 | D1 | Ar1 | D1 |
| 2 | D2 | Ar1 | D2 |
| 3 | D3 | Ar1 | D3 |
| 4 | D4 | Ar1 | D4 |
| 5 | D5 | Ar1 | D5 |
| 6 | D6 | Ar1 | D6 |
| 7 | D7 | Ar1 | D7 |
| 8 | D8 | Ar1 | D8 |
| 9 | D9 | Ar1 | D9 |
| 10 | D10 | Ar1 | D10 |
| 11 | D11 | Ar1 | D11 |
| 12 | D12 | Ar1 | D12 |
| 13 | D13 | Ar1 | D13 |
| 14 | D14 | Ar1 | D14 |
| 15 | D15 | Ar1 | D15 |
| 16 | D16 | Ar1 | D16 |
| 17 | D17 | Ar1 | D17 |
| 18 | D18 | Ar1 | D18 |
| 19 | D19 | Ar1 | D19 |
| 20 | D20 | Ar1 | D20 |
| 21 | D21 | Ar1 | D21 |
| 22 | D22 | Ar1 | D22 |
| 23 | D23 | Ar1 | D23 |
| 24 | D24 | Ar1 | D24 |

(continued)

| No. | $R^3$ | $R^4$ | $R^5$ |
|---|---|---|---|
| 25 | D25 | Ar1 | D25 |
| 26 | D26 | Ar1 | D26 |
| 27 | D27 | Ar1 | D27 |
| 28 | D28 | Ar1 | D28 |
| 29 | D29 | Ar1 | D29 |
| 30 | D30 | Ar1 | D30 |
| 31 | D31 | Ar1 | D31 |
| 32 | D32 | Ar1 | D32 |
| 33 | D33 | Ar1 | D33 |
| 34 | D34 | Ar1 | D34 |
| 35 | D35 | Ar1 | D35 |
| 36 | D36 | Ar1 | D36 |
| 37 | D37 | Ar1 | D37 |
| 38 | D38 | Ar1 | D38 |
| 39 | D39 | Ar1 | D39 |
| 40 | D40 | Ar1 | D40 |
| 41 | D41 | Ar1 | D41 |
| 42 | D42 | Ar1 | D42 |
| 43 | D43 | Ar1 | D43 |
| 44 | D44 | Ar1 | D44 |
| 45 | D45 | Ar1 | D45 |
| 46 | D46 | Ar1 | D46 |
| 47 | D47 | Ar1 | D47 |
| 48 | D48 | Ar1 | D48 |
| 49 | D49 | Ar1 | D49 |
| 50 | D50 | Ar1 | D50 |
| 51 | D51 | Ar1 | D51 |
| 52 | D52 | Ar1 | D52 |
| 53 | D53 | Ar1 | D53 |
| 54 | D54 | Ar1 | D54 |
| 55 | D55 | Ar1 | D55 |
| 56 | D56 | Ar1 | D56 |
| 57 | D57 | Ar1 | D57 |
| 58 | D58 | Ar1 | D58 |
| 59 | D59 | Ar1 | D59 |
| 60 | D60 | Ar1 | D60 |
| 61 | D61 | Ar1 | D61 |
| 62 | D62 | Ar1 | D62 |
| 63 | D63 | Ar1 | D63 |

(continued)

| No. | $R^3$ | $R^4$ | $R^5$ |
|---|---|---|---|
| 64 | D64 | Ar1 | D64 |
| 65 | D65 | Ar1 | D65 |
| 66 | D66 | Ar1 | D66 |
| 67 | D67 | Ar1 | D67 |
| 68 | D68 | Ar1 | D68 |
| 69 | D69 | Ar1 | D69 |
| 70 | D70 | Ar1 | D70 |
| 71 | D71 | Ar1 | D71 |
| 72 | D72 | Ar1 | D72 |
| 73 | D73 | Ar1 | D73 |
| 74 | D74 | Ar1 | D74 |
| 75 | D75 | Ar1 | D75 |
| 76 | D76 | Ar1 | D76 |
| 77 | D77 | Ar1 | D77 |
| 78 | D78 | Ar1 | D78 |
| 79 | D79 | Ar1 | D79 |
| 80 | D80 | Ar1 | D80 |
| 81 | D81 | Ar1 | D81 |
| 82 | D82 | Ar1 | D82 |
| 83 | D83 | Ar1 | D83 |
| 84 | D84 | Ar1 | D84 |
| 85 | D85 | Ar1 | D85 |
| 86 | D86 | Ar1 | D86 |
| 87 | D87 | Ar1 | D87 |
| 88 | D88 | Ar1 | D88 |
| 89 | D89 | Ar1 | D89 |
| 90 | D90 | Ar1 | D90 |
| 91 | D91 | Ar1 | D91 |
| 92 | D92 | Ar1 | D92 |
| 93 | D93 | Ar1 | D93 |
| 94 | D94 | Ar1 | D94 |
| 95 | D95 | Ar1 | D95 |
| 96 | D96 | Ar1 | D96 |
| 97 | D97 | Ar1 | D97 |
| 98 | D98 | Ar1 | D98 |
| 99 | D99 | Ar1 | D99 |
| 100 | D100 | Ar1 | D100 |
| 101 | D101 | Ar1 | D101 |
| 102 | D102 | Ar1 | D102 |

(continued)

| No. | $R^3$ | $R^4$ | $R^5$ |
|---|---|---|---|
| 103 | D103 | Ar1 | D103 |
| 104 | D104 | Ar1 | D104 |
| 105 | D105 | Ar1 | D105 |
| 106 | D106 | Ar1 | D106 |
| 107 | D107 | Ar1 | D107 |
| 108 | D108 | Ar1 | D108 |
| 109 | D109 | Ar1 | D109 |
| 110 | D110 | Ar1 | D110 |
| 111 | D111 | Ar1 | D111 |
| 112 | D112 | Ar1 | D112 |
| 113 | D113 | Ar1 | D113 |
| 114 | D114 | Ar1 | D114 |
| 115 | D115 | Ar1 | D115 |
| 116 | D116 | Ar1 | D116 |
| 117 | D117 | Ar1 | D117 |
| 118 | D118 | Ar1 | D118 |
| 119 | D119 | Ar1 | D119 |
| 120 | D120 | Ar1 | D120 |
| 121 | D121 | Ar1 | D121 |
| 122 | D122 | Ar1 | D122 |
| 123 | D123 | Ar1 | D123 |
| 124 | D124 | Ar1 | D124 |
| 125 | D125 | Ar1 | D125 |
| 126 | D126 | Ar1 | D126 |
| 127 | D127 | Ar1 | D127 |
| 128 | D128 | Ar1 | D128 |
| 129 | D129 | Ar1 | D129 |
| 130 | D130 | Ar1 | D130 |
| 131 | D131 | Ar1 | D131 |
| 132 | D132 | Ar1 | D132 |
| 133 | D133 | Ar1 | D133 |
| 134 | D134 | Ar1 | D134 |
| 135 | D135 | Ar1 | D135 |
| 136 | D136 | Ar1 | D136 |
| 137 | D137 | Ar1 | D137 |
| 138 | D138 | Ar1 | D138 |
| 139 | D139 | Ar1 | D139 |
| 140 | D140 | Ar1 | D140 |
| 141 | D141 | Ar1 | D141 |

(continued)

| No. | $R^3$ | $R^4$ | $R^5$ |
|---|---|---|---|
| 142 | D142 | Ar1 | D142 |
| 143 | D143 | Ar1 | D143 |
| 144 | D144 | Ar1 | D144 |
| 145 | D145 | Ar1 | D145 |
| 146 | D146 | Ar1 | D146 |
| 147 | D147 | Ar1 | D147 |
| 148 | D148 | Ar1 | D148 |
| 149 | D149 | Ar1 | D149 |
| 150 | D150 | Ar1 | D150 |
| 151 | D151 | Ar1 | D151 |
| 152 | D152 | Ar1 | D152 |
| 153 | D153 | Ar1 | D153 |
| 154 | D154 | Ar1 | D154 |
| 155 | D155 | Ar1 | D155 |
| 156 | D156 | Ar1 | D156 |
| 157 | D157 | Ar1 | D157 |
| 158 | D158 | Ar1 | D158 |
| 159 | D159 | Ar1 | D159 |
| 160 | D160 | Ar1 | D160 |
| 161 | D161 | Ar1 | D161 |
| 162 | D162 | Ar1 | D162 |
| 163 | D163 | Ar1 | D163 |
| 164 | D164 | Ar1 | D164 |
| 165 | D165 | Ar1 | D165 |
| 166 | D166 | Ar1 | D166 |
| 167 | D167 | Ar1 | D167 |
| 168 | D168 | Ar1 | D168 |
| 169 | D169 | Ar1 | D169 |
| 170 | D170 | Ar1 | D170 |
| 171 | D171 | Ar1 | D171 |
| 172 | D172 | Ar1 | D172 |
| 173 | D173 | Ar1 | D173 |
| 174 | D174 | Ar1 | D174 |
| 175 | D175 | Ar1 | D175 |
| 176 | D176 | Ar1 | D176 |
| 177 | D177 | Ar1 | D177 |
| 178 | D178 | Ar1 | D178 |
| 179 | D179 | Ar1 | D179 |
| 180 | D180 | Ar1 | D180 |

(continued)

| No. | $R^3$ | $R^4$ | $R^5$ |
|---|---|---|---|
| 181 | D181 | Ar1 | D181 |
| 182 | D182 | Ar1 | D182 |
| 183 | D183 | Ar1 | D183 |
| 184 | D184 | Ar1 | D184 |
| 185 | D185 | Ar1 | D185 |
| 186 | D186 | Ar1 | D186 |
| 187 | D187 | Ar1 | D187 |
| 188 | D188 | Ar1 | D188 |
| 189 | D189 | Ar1 | D189 |
| 190 | D190 | Ar1 | D190 |
| 191 | D191 | Ar1 | D191 |
| 192 | D192 | Ar1 | D192 |
| 193 | D193 | Ar1 | D193 |
| 194 | D194 | Ar1 | D194 |
| 195 | D195 | Ar1 | D195 |
| 196 | D196 | Ar1 | D196 |
| 197 | D197 | Ar1 | D197 |
| 198 | D198 | Ar1 | D198 |
| 199 | D199 | Ar1 | D199 |
| 200 | D200 | Ar1 | D200 |
| 201 | D201 | Ar1 | D201 |
| 202 | D202 | Ar1 | D202 |
| 203 | D203 | Ar1 | D203 |
| 204 | D204 | Ar1 | D204 |
| 205 | D205 | Ar1 | D205 |
| 206 | D206 | Ar1 | D206 |
| 207 | D207 | Ar1 | D207 |
| 208 | D208 | Ar1 | D208 |
| 209 | D209 | Ar1 | D209 |
| 210 | D210 | Ar1 | D210 |
| 211 | D211 | Ar1 | D211 |
| 212 | D212 | Ar1 | D212 |
| 213 | D213 | Ar1 | D213 |
| 214 | D214 | Ar1 | D214 |
| 215 | D215 | Ar1 | D215 |
| 216 | D216 | Ar1 | D216 |
| 217 | D217 | Ar1 | D217 |
| 218 | D218 | Ar1 | D218 |
| 219 | D219 | Ar1 | D219 |

(continued)

| No. | $R^3$ | $R^4$ | $R^5$ |
|---|---|---|---|
| 220 | D220 | Ar1 | D220 |
| 221 | D221 | Ar1 | D221 |
| 222 | D222 | Ar1 | D222 |
| 223 | D223 | Ar1 | D223 |
| 224 | D224 | Ar1 | D224 |
| 225 | D225 | Ar1 | D225 |
| 226 | D226 | Ar1 | D226 |
| 227 | D227 | Ar1 | D227 |
| 228 | D228 | Ar1 | D228 |
| 229 | D229 | Ar1 | D229 |
| 230 | D230 | Ar1 | D230 |
| 231 | D231 | Ar1 | D231 |
| 232 | D232 | Ar1 | D232 |
| 233 | D233 | Ar1 | D233 |
| 234 | D234 | Ar1 | D234 |
| 235 | D235 | Ar1 | D235 |
| 236 | D236 | Ar1 | D236 |
| 237 | D237 | Ar1 | D237 |
| 238 | D238 | Ar1 | D238 |
| 239 | D239 | Ar1 | D239 |
| 240 | D240 | Ar1 | D240 |
| 241 | D241 | Ar1 | D241 |
| 242 | D242 | Ar1 | D242 |
| 243 | D243 | Ar1 | D243 |
| 244 | D244 | Ar1 | D244 |
| 245 | D245 | Ar1 | D245 |
| 246 | D246 | Ar1 | D246 |
| 247 | D247 | Ar1 | D247 |
| 248 | D248 | Ar1 | D248 |
| 249 | D249 | Ar1 | D249 |
| 250 | D250 | Ar1 | D250 |
| 251 | D251 | Ar1 | D251 |
| 252 | D252 | Ar1 | D252 |
| 253 | D253 | Ar1 | D253 |
| 254 | D254 | Ar1 | D254 |
| 255 | D255 | Ar1 | D255 |
| 256 | D256 | Ar1 | D256 |
| 257 | D257 | Ar1 | D257 |
| 258 | D258 | Ar1 | D258 |

(continued)

| No. | $R^3$ | $R^4$ | $R^5$ |
|---|---|---|---|
| 259 | D259 | Ar1 | D259 |
| 260 | D260 | Ar1 | D260 |
| 261 | D261 | Ar1 | D261 |
| 262 | D262 | Ar1 | D262 |
| 263 | D263 | Ar1 | D263 |
| 264 | D264 | Ar1 | D264 |
| 265 | D265 | Ar1 | D265 |
| 266 | D266 | Ar1 | D266 |
| 267 | D267 | Ar1 | D267 |
| 268 | D268 | Ar1 | D268 |
| 269 | D269 | Ar1 | D269 |
| 270 | D270 | Ar1 | D270 |
| 271 | D271 | Ar1 | D271 |
| 272 | D272 | Ar1 | D272 |
| 273 | D273 | Ar1 | D273 |
| 274 | D274 | Ar1 | D274 |
| 275 | D275 | Ar1 | D275 |
| 276 | D276 | Ar1 | D276 |
| 277 | D277 | Ar1 | D277 |
| 278 | D278 | Ar1 | D278 |
| 279 | D279 | Ar1 | D279 |
| 280 | D280 | Ar1 | D280 |
| 281 | D281 | Ar1 | D281 |
| 282 | D282 | Ar1 | D282 |
| 283 | D283 | Ar1 | D283 |
| 284 | D284 | Ar1 | D284 |
| 285 | D285 | Ar1 | D285 |
| 286 | D286 | Ar1 | D286 |
| 287 | D287 | Ar1 | D287 |
| 288 | D288 | Ar1 | D288 |
| 289 | D289 | Ar1 | D289 |
| 290 | D290 | Ar1 | D290 |
| 291 | D291 | Ar1 | D291 |
| 292 | D292 | Ar1 | D292 |
| 293 | D293 | Ar1 | D293 |
| 294 | D294 | Ar1 | D294 |
| 295 | D295 | Ar1 | D295 |
| 296 | D296 | Ar1 | D296 |
| 297 | D297 | Ar1 | D297 |

(continued)

| No. | $R^3$ | $R^4$ | $R^5$ |
|---|---|---|---|
| 298 | D298 | Ar1 | D298 |
| 299 | D299 | Ar1 | D299 |
| 300 | D300 | Ar1 | D300 |
| 301 | D301 | Ar1 | D301 |
| 302 | D302 | Ar1 | D302 |
| 303 | D303 | Ar1 | D303 |
| 304 | D304 | Ar1 | D304 |
| 305 | D305 | Ar1 | D305 |
| 306 | D306 | Ar1 | D306 |
| 307 | D307 | Ar1 | D307 |
| 308 | D308 | Ar1 | D308 |
| 309 | D309 | Ar1 | D309 |
| 310 | D310 | Ar1 | D310 |
| 311 | D311 | Ar1 | D311 |
| 312 | D312 | Ar1 | D312 |
| 313 | D313 | Ar1 | D313 |
| 314 | D314 | Ar1 | D314 |
| 315 | D315 | Ar1 | D315 |
| 316 | D316 | Ar1 | D316 |
| 317 | D317 | Ar1 | D317 |
| 318 | D318 | Ar1 | D318 |
| 319 | D319 | Ar1 | D319 |
| 320 | D320 | Ar1 | D320 |
| 321 | D321 | Ar1 | D321 |
| 322 | D322 | Ar1 | D322 |
| 323 | D323 | Ar1 | D323 |
| 324 | D324 | Ar1 | D324 |
| 325 | D325 | Ar1 | D325 |
| 326 | D326 | Ar1 | D326 |
| 327 | D327 | Ar1 | D327 |
| 328 | D328 | Ar1 | D328 |
| 329 | D329 | Ar1 | D329 |
| 330 | D330 | Ar1 | D330 |
| 331 | D331 | Ar1 | D331 |
| 332 | D332 | Ar1 | D332 |
| 333 | D333 | Ar1 | D333 |
| 334 | D334 | Ar1 | D334 |
| 335 | D335 | Ar1 | D335 |
| 336 | D336 | Ar1 | D336 |

(continued)

| No. | $R^3$ | $R^4$ | $R^5$ |
|---|---|---|---|
| 337 | D337 | Ar1 | D337 |
| 338 | D338 | Ar1 | D338 |
| 339 | D339 | Ar1 | D339 |
| 340 | D340 | Ar1 | D340 |
| 341 | D341 | Ar1 | D341 |
| 342 | D342 | Ar1 | D342 |
| 343 | D343 | Ar1 | D343 |
| 344 | D344 | Ar1 | D344 |
| 345 | D345 | Ar1 | D345 |
| 346 | D346 | Ar1 | D346 |
| 347 | D347 | Ar1 | D347 |
| 348 | D348 | Ar1 | D348 |
| 349 | D349 | Ar1 | D349 |
| 350 | D350 | Ar1 | D350 |
| 351 | D351 | Ar1 | D351 |
| 352 | D352 | Ar1 | D352 |
| 353 | D353 | Ar1 | D353 |
| 354 | D354 | Ar1 | D354 |
| 355 | D355 | Ar1 | D355 |
| 356 | D356 | Ar1 | D356 |
| 357 | D357 | Ar1 | D357 |
| 358 | D358 | Ar1 | D358 |
| 359 | D359 | Ar1 | D359 |
| 360 | D360 | Ar1 | D360 |
| 361 | D361 | Ar1 | D361 |
| 362 | D362 | Ar1 | D362 |
| 363 | D363 | Ar1 | D363 |
| 364 | D364 | Ar1 | D364 |
| 365 | D365 | Ar1 | D365 |
| 366 | D366 | Ar1 | D366 |
| 367 | D367 | Ar1 | D367 |
| 368 | D368 | Ar1 | D368 |
| 369 | D369 | Ar1 | D369 |
| 370 | D370 | Ar1 | D370 |
| 371 | D371 | Ar1 | D371 |
| 372 | D372 | Ar1 | D372 |
| 373 | D373 | Ar1 | D373 |
| 374 | D374 | Ar1 | D374 |
| 375 | D375 | Ar1 | D375 |

(continued)

| No. | $R^3$ | $R^4$ | $R^5$ |
|---|---|---|---|
| 376 | D376 | Ar1 | D376 |
| 377 | D377 | Ar1 | D377 |
| 378 | D378 | Ar1 | D378 |
| 379 | D379 | Ar1 | D379 |
| 380 | D380 | Ar1 | D380 |
| 381 | D381 | Ar1 | D381 |
| 382 | D382 | Ar1 | D382 |
| 383 | D383 | Ar1 | D383 |
| 384 | D384 | Ar1 | D384 |
| 385 | D385 | Ar1 | D385 |
| 386 | D386 | Ar1 | D386 |
| 387 | D387 | Ar1 | D387 |
| 388 | D388 | Ar1 | D388 |
| 389 | D389 | Ar1 | D389 |
| 390 | D390 | Ar1 | D390 |
| 391 | D391 | Ar1 | D391 |
| 392 | D392 | Ar1 | D392 |
| 393 | D393 | Ar1 | D393 |
| 394 | D394 | Ar1 | D394 |
| 395 | D395 | Ar1 | D395 |
| 396 | D396 | Ar1 | D396 |
| 397 | D397 | Ar1 | D397 |
| 398 | D398 | Ar1 | D398 |
| 399 | D399 | Ar1 | D399 |
| 400 | D400 | Ar1 | D400 |
| 401 | D401 | Ar1 | D401 |
| 402 | D402 | Ar1 | D402 |
| 403 | D403 | Ar1 | D403 |
| 404 | D404 | Ar1 | D404 |
| 405 | D405 | Ar1 | D405 |
| 406 | D406 | Ar1 | D406 |
| 407 | D407 | Ar1 | D407 |
| 408 | D408 | Ar1 | D408 |
| 409 | D409 | Ar1 | D409 |
| 410 | D410 | Ar1 | D410 |
| 411 | D411 | Ar1 | D411 |
| 412 | D412 | Ar1 | D412 |
| 413 | D413 | Ar1 | D413 |
| 414 | D414 | Ar1 | D414 |

(continued)

| No. | $R^3$ | $R^4$ | $R^5$ |
|---|---|---|---|
| 415 | D415 | Ar1 | D415 |
| 416 | D416 | Ar1 | D416 |
| 417 | D417 | Ar1 | D417 |
| 418 | D418 | Ar1 | D418 |
| 419 | D419 | Ar1 | D419 |
| 420 | D420 | Ar1 | D420 |
| 421 | D421 | Ar1 | D421 |
| 422 | D422 | Ar1 | D422 |
| 423 | D423 | Ar1 | D423 |
| 424 | D424 | Ar1 | D424 |
| 425 | D425 | Ar1 | D425 |
| 426 | D426 | Ar1 | D426 |
| 427 | D427 | Ar1 | D427 |
| 428 | D428 | Ar1 | D428 |
| 429 | D429 | Ar1 | D429 |
| 430 | D430 | Ar1 | D430 |
| 431 | D431 | Ar1 | D431 |
| 432 | D432 | Ar1 | D432 |
| 433 | D433 | Ar1 | D433 |
| 434 | D434 | Ar1 | D434 |
| 435 | D435 | Ar1 | D435 |
| 436 | D436 | Ar1 | D436 |
| 437 | D437 | Ar1 | D437 |
| 438 | D438 | Ar1 | D438 |
| 439 | D439 | Ar1 | D439 |
| 440 | D440 | Ar1 | D440 |
| 441 | D441 | Ar1 | D441 |
| 442 | D442 | Ar1 | D442 |
| 443 | D443 | Ar1 | D443 |
| 444 | D444 | Ar1 | D444 |
| 445 | D445 | Ar1 | D445 |
| 446 | D446 | Ar1 | D446 |
| 447 | D447 | Ar1 | D447 |
| 448 | D448 | Ar1 | D448 |
| 449 | D449 | Ar1 | D449 |
| 450 | D450 | Ar1 | D450 |
| 451 | D451 | Ar1 | D451 |
| 452 | D452 | Ar1 | D452 |
| 453 | D453 | Ar1 | D453 |

(continued)

| No. | $R^3$ | $R^4$ | $R^5$ |
|---|---|---|---|
| 454 | D454 | Ar1 | D454 |
| 455 | D455 | Ar1 | D455 |
| 456 | D456 | Ar1 | D456 |
| 457 | D457 | Ar1 | D457 |
| 458 | D458 | Ar1 | D458 |
| 459 | D459 | Ar1 | D459 |
| 460 | D460 | Ar1 | D460 |
| 461 | D461 | Ar1 | D461 |
| 462 | D462 | Ar1 | D462 |
| 463 | D463 | Ar1 | D463 |
| 464 | D464 | Ar1 | D464 |
| 465 | D465 | Ar1 | D465 |
| 466 | D466 | Ar1 | D466 |
| 467 | D467 | Ar1 | D467 |
| 468 | D468 | Ar1 | D468 |
| 469 | D469 | Ar1 | D469 |
| 470 | D470 | Ar1 | D470 |
| 471 | D471 | Ar1 | D471 |
| 472 | D472 | Ar1 | D472 |
| 473 | D473 | Ar1 | D473 |
| 474 | D474 | Ar1 | D474 |
| 475 | D475 | Ar1 | D475 |
| 476 | D476 | Ar1 | D476 |
| 477 | D477 | Ar1 | D477 |
| 478 | D478 | Ar1 | D478 |
| 479 | D479 | Ar1 | D479 |
| 480 | D480 | Ar1 | D480 |
| 481 | D481 | Ar1 | D481 |
| 482 | D482 | Ar1 | D482 |
| 483 | D483 | Ar1 | D483 |
| 484 | D484 | Ar1 | D484 |
| 485 | D485 | Ar1 | D485 |
| 486 | D486 | Ar1 | D486 |
| 487 | D487 | Ar1 | D487 |
| 488 | D488 | Ar1 | D488 |
| 489 | D489 | Ar1 | D489 |
| 490 | D490 | Ar1 | D490 |
| 491 | D491 | Ar1 | D491 |
| 492 | D492 | Ar1 | D492 |

(continued)

| No. | $R^3$ | $R^4$ | $R^5$ |
|---|---|---|---|
| 493 | D493 | Ar1 | D493 |
| 494 | D494 | Ar1 | D494 |
| 495 | D495 | Ar1 | D495 |
| 496 | D496 | Ar1 | D496 |
| 497 | D497 | Ar1 | D497 |
| 498 | D498 | Ar1 | D498 |
| 499 | D499 | Ar1 | D499 |
| 500 | D500 | Ar1 | D500 |
| 501 | D501 | Ar1 | D501 |
| 502 | D502 | Ar1 | D502 |
| 503 | D503 | Ar1 | D503 |
| 504 | D504 | Ar1 | D504 |
| 505 | D505 | Ar1 | D505 |
| 506 | D506 | Ar1 | D506 |
| 507 | D507 | Ar1 | D507 |
| 508 | D508 | Ar1 | D508 |
| 509 | D509 | Ar1 | D509 |
| 510 | D510 | Ar1 | D510 |
| 511 | D511 | Ar1 | D511 |
| 512 | D512 | Ar1 | D512 |
| 513 | D513 | Ar1 | D513 |
| 514 | D514 | Ar1 | D514 |
| 515 | D515 | Ar1 | D515 |
| 516 | D516 | Ar1 | D516 |
| 517 | D517 | Ar1 | D517 |
| 518 | D518 | Ar1 | D518 |
| 519 | D519 | Ar1 | D519 |
| 520 | D520 | Ar1 | D520 |
| 521 | D521 | Ar1 | D521 |
| 522 | D522 | Ar1 | D522 |
| 523 | D523 | Ar1 | D523 |
| 524 | D524 | Ar1 | D524 |
| 525 | D525 | Ar1 | D525 |
| 526 | D526 | Ar1 | D526 |
| 527 | D527 | Ar1 | D527 |
| 528 | D528 | Ar1 | D528 |
| 529 | D529 | Ar1 | D529 |
| 530 | D530 | Ar1 | D530 |
| 531 | D531 | Ar1 | D531 |

(continued)

| No. | $R^3$ | $R^4$ | $R^5$ |
|---|---|---|---|
| 532 | D532 | Ar1 | D532 |
| 533 | D533 | Ar1 | D533 |
| 534 | D534 | Ar1 | D534 |
| 535 | D535 | Ar1 | D535 |
| 536 | D536 | Ar1 | D536 |
| 537 | D537 | Ar1 | D537 |
| 538 | D538 | Ar1 | D538 |
| 539 | D539 | Ar1 | D539 |
| 540 | D540 | Ar1 | D540 |
| 541 | D541 | Ar1 | D541 |
| 542 | D542 | Ar1 | D542 |
| 543 | D543 | Ar1 | D543 |
| 544 | D544 | Ar1 | D544 |
| 545 | D545 | Ar1 | D545 |
| 546 | D546 | Ar1 | D546 |
| 547 | D547 | Ar1 | D547 |
| 548 | D548 | Ar1 | D548 |
| 549 | D549 | Ar1 | D549 |
| 550 | D550 | Ar1 | D550 |
| 551 | D551 | Ar1 | D551 |
| 552 | D552 | Ar1 | D552 |
| 553 | D553 | Ar1 | D553 |
| 554 | D554 | Ar1 | D554 |
| 555 | D555 | Ar1 | D555 |
| 556 | D556 | Ar1 | D556 |
| 557 | D557 | Ar1 | D557 |
| 558 | D558 | Ar1 | D558 |
| 559 | D559 | Ar1 | D559 |
| 560 | D560 | Ar1 | D560 |
| 561 | D561 | Ar1 | D561 |
| 562 | D562 | Ar1 | D562 |
| 563 | D563 | Ar1 | D563 |
| 564 | D564 | Ar1 | D564 |
| 565 | D565 | Ar1 | D565 |
| 566 | D566 | Ar1 | D566 |
| 567 | D567 | Ar1 | D567 |
| 568 | D568 | Ar1 | D568 |
| 569 | D569 | Ar1 | D569 |
| 570 | D570 | Ar1 | D570 |

(continued)

| No. | $R^3$ | $R^4$ | $R^5$ |
|---|---|---|---|
| 571 | D571 | Ar1 | D571 |
| 572 | D572 | Ar1 | D572 |
| 573 | D573 | Ar1 | D573 |
| 574 | D574 | Ar1 | D574 |
| 575 | D575 | Ar1 | D575 |
| 576 | D576 | Ar1 | D576 |
| 577 | D577 | Ar1 | D577 |
| 578 | D578 | Ar1 | D578 |
| 579 | D579 | Ar1 | D579 |
| 580 | D580 | Ar1 | D580 |
| 581 | D581 | Ar1 | D581 |
| 582 | D582 | Ar1 | D582 |
| 583 | D583 | Ar1 | D583 |
| 584 | D584 | Ar1 | D584 |
| 585 | D585 | Ar1 | D585 |
| 586 | D586 | Ar1 | D586 |
| 587 | D587 | Ar1 | D587 |
| 588 | D588 | Ar1 | D588 |
| 589 | D589 | Ar1 | D589 |
| 590 | D590 | Ar1 | D590 |
| 591 | D591 | Ar1 | D591 |
| 592 | D592 | Ar1 | D592 |
| 593 | D593 | Ar1 | D593 |
| 594 | D594 | Ar1 | D594 |
| 595 | D595 | Ar1 | D595 |
| 596 | D596 | Ar1 | D596 |
| 597 | D597 | Ar1 | D597 |
| 598 | D598 | Ar1 | D598 |
| 599 | D599 | Ar1 | D599 |
| 600 | D600 | Ar1 | D600 |
| 601 | D601 | Ar1 | D601 |
| 602 | D602 | Ar1 | D602 |
| 603 | D603 | Ar1 | D603 |
| 604 | D604 | Ar1 | D604 |
| 605 | D605 | Ar1 | D605 |
| 606 | D606 | Ar1 | D606 |
| 607 | D607 | Ar1 | D607 |
| 608 | D608 | Ar1 | D608 |
| 609 | D609 | Ar1 | D609 |

(continued)

| No. | $R^3$ | $R^4$ | $R^5$ |
|---|---|---|---|
| 610 | D610 | Ar1 | D610 |
| 611 | D611 | Ar1 | D611 |
| 612 | D612 | Ar1 | D612 |
| 613 | D613 | Ar1 | D613 |
| 614 | D614 | Ar1 | D614 |
| 615 | D615 | Ar1 | D615 |
| 616 | D616 | Ar1 | D616 |
| 617 | D617 | Ar1 | D617 |
| 618 | D618 | Ar1 | D618 |
| 619 | D619 | Ar1 | D619 |
| 620 | D620 | Ar1 | D620 |
| 621 | D621 | Ar1 | D621 |
| 622 | D622 | Ar1 | D622 |
| 623 | D623 | Ar1 | D623 |
| 624 | D624 | Ar1 | D624 |
| 625 | D625 | Ar1 | D625 |
| 626 | D626 | Ar1 | D626 |
| 627 | D627 | Ar1 | D627 |
| 628 | D628 | Ar1 | D628 |
| 629 | D629 | Ar1 | D629 |
| 630 | D630 | Ar1 | D630 |
| 631 | D631 | Ar1 | D631 |
| 632 | D632 | Ar1 | D632 |
| 633 | D633 | Ar1 | D633 |
| 634 | D634 | Ar1 | D634 |
| 635 | D635 | Ar1 | D635 |
| 636 | D636 | Ar1 | D636 |
| 637 | D637 | Ar1 | D637 |
| 638 | D638 | Ar1 | D638 |
| 639 | D639 | Ar1 | D639 |
| 640 | D640 | Ar1 | D640 |
| 641 | D641 | Ar1 | D641 |
| 642 | D642 | Ar1 | D642 |
| 643 | D643 | Ar1 | D643 |
| 644 | D644 | Ar1 | D644 |
| 645 | D645 | Ar1 | D645 |
| 646 | D646 | Ar1 | D646 |
| 647 | D647 | Ar1 | D647 |
| 648 | D648 | Ar1 | D648 |

(continued)

| No. | $R^3$ | $R^4$ | $R^5$ |
|---|---|---|---|
| 649 | D649 | Ar1 | D649 |
| 650 | D650 | Ar1 | D650 |
| 651 | D651 | Ar1 | D651 |
| 652 | D652 | Ar1 | D652 |
| 653 | D653 | Ar1 | D653 |
| 654 | D654 | Ar1 | D654 |
| 655 | D655 | Ar1 | D655 |
| 656 | D656 | Ar1 | D656 |
| 657 | D657 | Ar1 | D657 |
| 658 | D658 | Ar1 | D658 |
| 659 | D659 | Ar1 | D659 |
| 660 | D660 | Ar1 | D660 |
| 661 | D661 | Ar1 | D661 |
| 662 | D662 | Ar1 | D662 |
| 663 | D663 | Ar1 | D663 |
| 664 | D664 | Ar1 | D664 |
| 665 | D665 | Ar1 | D665 |
| 666 | D666 | Ar1 | D666 |
| 667 | D667 | Ar1 | D667 |
| 668 | D668 | Ar1 | D668 |
| 669 | D669 | Ar1 | D669 |
| 670 | D670 | Ar1 | D670 |
| 671 | D671 | Ar1 | D671 |
| 672 | D672 | Ar1 | D672 |
| 673 | D673 | Ar1 | D673 |
| 674 | D674 | Ar1 | D674 |
| 675 | D675 | Ar1 | D675 |
| 676 | D676 | Ar1 | D676 |
| 677 | D677 | Ar1 | D677 |
| 678 | D678 | Ar1 | D678 |
| 679 | D679 | Ar1 | D679 |
| 680 | D680 | Ar1 | D680 |
| 681 | D681 | Ar1 | D681 |
| 682 | D682 | Ar1 | D682 |
| 683 | D683 | Ar1 | D683 |
| 684 | D684 | Ar1 | D684 |
| 685 | D685 | Ar1 | D685 |
| 686 | D686 | Ar1 | D686 |
| 687 | D687 | Ar1 | D687 |

(continued)

| No. | $R^3$ | $R^4$ | $R^5$ |
|---|---|---|---|
| 688 | D688 | Ar1 | D688 |
| 689 | D689 | Ar1 | D689 |
| 690 | D690 | Ar1 | D690 |
| 691 | D691 | Ar1 | D691 |
| 692 | D692 | Ar1 | D692 |
| 693 | D693 | Ar1 | D693 |
| 694 | D694 | Ar1 | D694 |
| 695 | D695 | Ar1 | D695 |
| 696 | D696 | Ar1 | D696 |
| 697 | D697 | Ar1 | D697 |
| 698 | D698 | Ar1 | D698 |
| 699 | D699 | Ar1 | D699 |
| 700 | D700 | Ar1 | D700 |
| 701 | D701 | Ar1 | D701 |
| 702 | D702 | Ar1 | D702 |
| 703 | D703 | Ar1 | D703 |
| 704 | D704 | Ar1 | D704 |
| 705 | D705 | Ar1 | D705 |
| 706 | D706 | Ar1 | D706 |
| 707 | D707 | Ar1 | D707 |
| 708 | D708 | Ar1 | D708 |
| 709 | D709 | Ar1 | D709 |
| 710 | D710 | Ar1 | D710 |
| 711 | D711 | Ar1 | D711 |
| 712 | D712 | Ar1 | D712 |
| 713 | D713 | Ar1 | D713 |
| 714 | D714 | Ar1 | D714 |
| 715 | D715 | Ar1 | D715 |
| 716 | D716 | Ar1 | D716 |
| 717 | D717 | Ar1 | D717 |
| 718 | D718 | Ar1 | D718 |
| 719 | D719 | Ar1 | D719 |
| 720 | D720 | Ar1 | D720 |
| 721 | D721 | Ar1 | D721 |
| 722 | D722 | Ar1 | D722 |
| 723 | D723 | Ar1 | D723 |
| 724 | D724 | Ar1 | D724 |
| 725 | D725 | Ar1 | D725 |
| 726 | D726 | Ar1 | D726 |

(continued)

| No. | $R^3$ | $R^4$ | $R^5$ |
|---|---|---|---|
| 727 | D727 | Ar1 | D727 |
| 728 | D728 | Ar1 | D728 |
| 729 | D729 | Ar1 | D729 |
| 730 | D730 | Ar1 | D730 |
| 731 | D731 | Ar1 | D731 |
| 732 | D732 | Ar1 | D732 |
| 733 | D733 | Ar1 | D733 |
| 734 | D734 | Ar1 | D734 |
| 735 | D735 | Ar1 | D735 |
| 736 | D736 | Ar1 | D736 |
| 737 | D737 | Ar1 | D737 |
| 738 | D738 | Ar1 | D738 |
| 739 | D739 | Ar1 | D739 |
| 740 | D740 | Ar1 | D740 |
| 741 | D741 | Ar1 | D741 |
| 742 | D742 | Ar1 | D742 |
| 743 | D743 | Ar1 | D743 |
| 744 | D744 | Ar1 | D744 |
| 745 | D745 | Ar1 | D745 |
| 746 | D746 | Ar1 | D746 |
| 747 | D747 | Ar1 | D747 |
| 748 | D748 | Ar1 | D748 |
| 749 | D749 | Ar1 | D749 |
| 750 | D750 | Ar1 | D750 |
| 751 | D751 | Ar1 | D751 |
| 752 | D752 | Ar1 | D752 |
| 753 | D753 | Ar1 | D753 |
| 754 | D754 | Ar1 | D754 |
| 755 | D755 | Ar1 | D755 |
| 756 | D756 | Ar1 | D756 |
| 757 | D757 | Ar1 | D757 |
| 758 | D758 | Ar1 | D758 |
| 759 | D759 | Ar1 | D759 |
| 760 | D760 | Ar1 | D760 |
| 761 | D761 | Ar1 | D761 |
| 762 | D762 | Ar1 | D762 |
| 763 | D763 | Ar1 | D763 |
| 764 | D764 | Ar1 | D764 |
| 765 | D765 | Ar1 | D765 |

(continued)

| No. | $R^3$ | $R^4$ | $R^5$ |
|---|---|---|---|
| 766 | D766 | Ar1 | D766 |
| 767 | D767 | Ar1 | D767 |
| 768 | D768 | Ar1 | D768 |
| 769 | D769 | Ar1 | D769 |
| 770 | D770 | Ar1 | D770 |
| 771 | D771 | Ar1 | D771 |
| 772 | D772 | Ar1 | D772 |
| 773 | D773 | Ar1 | D773 |
| 774 | D774 | Ar1 | D774 |
| 775 | D775 | Ar1 | D775 |
| 776 | D776 | Ar1 | D776 |
| 777 | D777 | Ar1 | D777 |
| 778 | D778 | Ar1 | D778 |
| 779 | D779 | Ar1 | D779 |
| 780 | D780 | Ar1 | D780 |
| 781 | D781 | Ar1 | D781 |
| 782 | D782 | Ar1 | D782 |
| 783 | D783 | Ar1 | D783 |
| 784 | D784 | Ar1 | D784 |
| 785 | D785 | Ar1 | D785 |
| 786 | D786 | Ar1 | D786 |
| 787 | D787 | Ar1 | D787 |
| 788 | D788 | Ar1 | D788 |
| 789 | D789 | Ar1 | D789 |
| 790 | D790 | Ar1 | D790 |
| 791 | D791 | Ar1 | D791 |
| 792 | D792 | Ar1 | D792 |
| 793 | D793 | Ar1 | D793 |
| 794 | D794 | Ar1 | D794 |
| 795 | D795 | Ar1 | D795 |
| 796 | D796 | Ar1 | D796 |
| 797 | D797 | Ar1 | D797 |
| 798 | D798 | Ar1 | D798 |
| 799 | D799 | Ar1 | D799 |
| 800 | D800 | Ar1 | D800 |
| 801 | D801 | Ar1 | D801 |
| 802 | D802 | Ar1 | D802 |
| 803 | D803 | Ar1 | D803 |
| 804 | D804 | Ar1 | D804 |

(continued)

| No. | $R^3$ | $R^4$ | $R^5$ |
|---|---|---|---|
| 805 | D805 | Ar1 | D805 |
| 806 | D806 | Ar1 | D806 |
| 807 | D807 | Ar1 | D807 |
| 808 | D808 | Ar1 | D808 |
| 809 | D809 | Ar1 | D809 |
| 810 | D810 | Ar1 | D810 |
| 811 | D811 | Ar1 | D811 |
| 812 | D812 | Ar1 | D812 |
| 813 | D813 | Ar1 | D813 |
| 814 | D814 | Ar1 | D814 |
| 815 | D815 | Ar1 | D815 |
| 816 | D816 | Ar1 | D816 |
| 817 | D817 | Ar1 | D817 |
| 818 | D818 | Ar1 | D818 |
| 819 | D819 | Ar1 | D819 |
| 820 | D820 | Ar1 | D820 |
| 821 | D821 | Ar1 | D821 |
| 822 | D822 | Ar1 | D822 |
| 823 | D823 | Ar1 | D823 |
| 824 | D824 | Ar1 | D824 |
| 825 | D825 | Ar1 | D825 |
| 826 | D826 | Ar1 | D826 |
| 827 | D827 | Ar1 | D827 |
| 828 | D828 | Ar1 | D828 |
| 829 | D829 | Ar1 | D829 |
| 830 | D830 | Ar1 | D830 |
| 831 | D831 | Ar1 | D831 |
| 832 | D832 | Ar1 | D832 |
| 833 | D833 | Ar1 | D833 |
| 834 | D834 | Ar1 | D834 |
| 835 | D835 | Ar1 | D835 |
| 836 | D836 | Ar1 | D836 |
| 837 | D837 | Ar1 | D837 |
| 838 | D838 | Ar1 | D838 |
| 839 | D839 | Ar1 | D839 |
| 840 | D840 | Ar1 | D840 |
| 841 | D841 | Ar1 | D841 |
| 842 | D842 | Ar1 | D842 |
| 843 | D843 | Ar1 | D843 |

(continued)

| No. | $R^3$ | $R^4$ | $R^5$ |
|---|---|---|---|
| 844 | D844 | Ar1 | D844 |
| 845 | D845 | Ar1 | D845 |
| 846 | D846 | Ar1 | D846 |
| 847 | D847 | Ar1 | D847 |
| 848 | D848 | Ar1 | D848 |
| 849 | D849 | Ar1 | D849 |
| 850 | D850 | Ar1 | D850 |
| 851 | D851 | Ar1 | D851 |
| 852 | D852 | Ar1 | D852 |
| 853 | D853 | Ar1 | D853 |
| 854 | D854 | Ar1 | D854 |
| 855 | D855 | Ar1 | D855 |
| 856 | D856 | Ar1 | D856 |
| 857 | D857 | Ar1 | D857 |
| 858 | D858 | Ar1 | D858 |
| 859 | D859 | Ar1 | D859 |
| 860 | D860 | Ar1 | D860 |
| 861 | D861 | Ar1 | D861 |
| 862 | D862 | Ar1 | D862 |
| 863 | D863 | Ar1 | D863 |
| 864 | D864 | Ar1 | D864 |
| 865 | D865 | Ar1 | D865 |
| 866 | D866 | Ar1 | D866 |
| 867 | D867 | Ar1 | D867 |
| 868 | D868 | Ar1 | D868 |
| 869 | D869 | Ar1 | D869 |
| 870 | D870 | Ar1 | D870 |
| 871 | D871 | Ar1 | D871 |
| 872 | D872 | Ar1 | D872 |
| 873 | D873 | Ar1 | D873 |
| 874 | D874 | Ar1 | D874 |
| 875 | D875 | Ar1 | D875 |
| 876 | D876 | Ar1 | D876 |
| 877 | D877 | Ar1 | D877 |
| 878 | D878 | Ar1 | D878 |
| 879 | D879 | Ar1 | D879 |
| 880 | D880 | Ar1 | D880 |
| 881 | D881 | Ar1 | D881 |
| 882 | D882 | Ar1 | D882 |

(continued)

| No. | $R^3$ | $R^4$ | $R^5$ |
|---|---|---|---|
| 883 | D883 | Ar1 | D883 |
| 884 | D884 | Ar1 | D884 |
| 885 | D885 | Ar1 | D885 |
| 886 | D886 | Ar1 | D886 |
| 887 | D887 | Ar1 | D887 |
| 888 | D888 | Ar1 | D888 |
| 889 | D889 | Ar1 | D889 |
| 890 | D890 | Ar1 | D890 |
| 891 | D891 | Ar1 | D891 |
| 892 | D892 | Ar1 | D892 |
| 893 | D893 | Ar1 | D893 |
| 894 | D894 | Ar1 | D894 |
| 895 | D895 | Ar1 | D895 |
| 896 | D896 | Ar1 | D896 |
| 897 | D897 | Ar1 | D897 |
| 898 | D898 | Ar1 | D898 |
| 899 | D899 | Ar1 | D899 |
| 900 | D900 | Ar1 | D900 |
| 901 | D901 | Ar1 | D901 |
| 902 | D902 | Ar1 | D902 |
| 903 | D903 | Ar1 | D903 |
| 904 | D904 | Ar1 | D904 |
| 905 | D905 | Ar1 | D905 |
| 906 | D906 | Ar1 | D906 |
| 907 | D907 | Ar1 | D907 |
| 908 | D908 | Ar1 | D908 |
| 909 | D909 | Ar1 | D909 |
| 910 | D910 | Ar1 | D910 |
| 911 | D911 | Ar1 | D911 |
| 912 | D912 | Ar1 | D912 |
| 913 | D913 | Ar1 | D913 |
| 914 | D914 | Ar1 | D914 |
| 915 | D915 | Ar1 | D915 |
| 916 | D916 | Ar1 | D916 |
| 917 | D917 | Ar1 | D917 |
| 918 | D918 | Ar1 | D918 |
| 919 | D919 | Ar1 | D919 |
| 920 | D920 | Ar1 | D920 |
| 921 | D921 | Ar1 | D921 |

(continued)

| No. | $R^3$ | $R^4$ | $R^5$ |
|---|---|---|---|
| 922 | D922 | Ar1 | D922 |
| 923 | D923 | Ar1 | D923 |
| 924 | D924 | Ar1 | D924 |
| 925 | D925 | Ar1 | D925 |
| 926 | D926 | Ar1 | D926 |
| 927 | D927 | Ar1 | D927 |
| 928 | D928 | Ar1 | D928 |
| 929 | D929 | Ar1 | D929 |
| 930 | D930 | Ar1 | D930 |
| 931 | D931 | Ar1 | D931 |
| 932 | D932 | Ar1 | D932 |
| 933 | D933 | Ar1 | D933 |
| 934 | D934 | Ar1 | D934 |
| 935 | D935 | Ar1 | D935 |
| 936 | D936 | Ar1 | D936 |
| 937 | D937 | Ar1 | D937 |
| 938 | D938 | Ar1 | D938 |
| 939 | D939 | Ar1 | D939 |
| 940 | D940 | Ar1 | D940 |
| 941 | D941 | Ar1 | D941 |
| 942 | D942 | Ar1 | D942 |
| 943 | D943 | Ar1 | D943 |
| 944 | D944 | Ar1 | D944 |
| 945 | D945 | Ar1 | D945 |
| 946 | D946 | Ar1 | D946 |
| 947 | D947 | Ar1 | D947 |
| 948 | D948 | Ar1 | D948 |
| 949 | D949 | Ar1 | D949 |
| 950 | D950 | Ar1 | D950 |
| 951 | D951 | Ar1 | D951 |
| 952 | D952 | Ar1 | D952 |
| 953 | D953 | Ar1 | D953 |
| 954 | D954 | Ar1 | D954 |
| 955 | D955 | Ar1 | D955 |
| 956 | D956 | Ar1 | D956 |
| 957 | D957 | Ar1 | D957 |
| 958 | D958 | Ar1 | D958 |
| 959 | D959 | Ar1 | D959 |
| 960 | D960 | Ar1 | D960 |

(continued)

| No. | $R^3$ | $R^4$ | $R^5$ |
|---|---|---|---|
| 961 | D961 | Ar1 | D961 |
| 962 | D962 | Ar1 | D962 |
| 963 | D963 | Ar1 | D963 |
| 964 | D964 | Ar1 | D964 |
| 965 | D965 | Ar1 | D965 |
| 966 | D966 | Ar1 | D966 |
| 967 | D967 | Ar1 | D967 |
| 968 | D968 | Ar1 | D968 |
| 969 | D969 | Ar1 | D969 |
| 970 | D970 | Ar1 | D970 |
| 971 | D971 | Ar1 | D971 |
| 972 | D972 | Ar1 | D972 |
| 973 | D973 | Ar1 | D973 |
| 974 | D974 | Ar1 | D974 |
| 975 | D975 | Ar1 | D975 |
| 976 | D976 | Ar1 | D976 |
| 977 | D977 | Ar1 | D977 |
| 978 | D978 | Ar1 | D978 |
| 979 | D979 | Ar1 | D979 |
| 980 | D980 | Ar1 | D980 |
| 981 | D981 | Ar1 | D981 |
| 982 | D982 | Ar1 | D982 |
| 983 | D983 | Ar1 | D983 |
| 984 | D984 | Ar1 | D984 |
| 985 | D985 | Ar1 | D985 |
| 986 | D986 | Ar1 | D986 |
| 987 | D987 | Ar1 | D987 |
| 988 | D988 | Ar1 | D988 |
| 989 | D989 | Ar1 | D989 |
| 990 | D990 | Ar1 | D990 |
| 991 | D991 | Ar1 | D991 |
| 992 | D992 | Ar1 | D992 |
| 993 | D993 | Ar1 | D993 |
| 994 | D994 | Ar1 | D994 |
| 995 | D995 | Ar1 | D995 |
| 996 | D996 | Ar1 | D996 |
| 997 | D997 | Ar1 | D997 |
| 998 | D998 | Ar1 | D998 |
| 999 | D999 | Ar1 | D999 |

(continued)

| No. | $R^3$ | $R^4$ | $R^5$ |
|---|---|---|---|
| 1000 | D1000 | Ar1 | D1000 |
| 1001 | D1001 | Ar1 | D1001 |
| 1002 | D1002 | Ar1 | D1002 |
| 1003 | D1003 | Ar1 | D1003 |
| 1004 | D1004 | Ar1 | D1004 |
| 1005 | D1005 | Ar1 | D1005 |
| 1006 | D1006 | Ar1 | D1006 |
| 1007 | D1007 | Ar1 | D1007 |
| 1008 | D1008 | Ar1 | D1008 |
| 1009 | D1009 | Ar1 | D1009 |
| 1010 | D1010 | Ar1 | D1010 |
| 1011 | D1011 | Ar1 | D1011 |
| 1012 | D1012 | Ar1 | D1012 |
| 1013 | D1013 | Ar1 | D1013 |
| 1014 | D1014 | Ar1 | D1014 |
| 1015 | D1015 | Ar1 | D1015 |
| 1016 | D1016 | Ar1 | D1016 |
| 1017 | D1017 | Ar1 | D1017 |
| 1018 | D1018 | Ar1 | D1018 |
| 1019 | D1019 | Ar1 | D1019 |
| 1020 | D1020 | Ar1 | D1020 |
| 1021 | D1021 | Ar1 | D1021 |
| 1022 | D1022 | Ar1 | D1022 |
| 1023 | D1023 | Ar1 | D1023 |
| 1024 | D1024 | Ar1 | D1024 |
| 1025 | D1025 | Ar1 | D1025 |
| 1026 | D1026 | Ar1 | D1026 |
| 1027 | D1027 | Ar1 | D1027 |
| 1028 | D1028 | Ar1 | D1028 |
| 1029 | D1029 | Ar1 | D1029 |
| 1030 | D1030 | Ar1 | D1030 |
| 1031 | D1031 | Ar1 | D1031 |
| 1032 | D1032 | Ar1 | D1032 |
| 1033 | D1033 | Ar1 | D1033 |
| 1034 | D1034 | Ar1 | D1034 |
| 1035 | D1035 | Ar1 | D1035 |
| 1036 | D1036 | Ar1 | D1036 |
| 1037 | D1037 | Ar1 | D1037 |
| 1038 | D1038 | Ar1 | D1038 |

(continued)

| No. | $R^3$ | $R^4$ | $R^5$ |
|---|---|---|---|
| 1039 | D1039 | Ar1 | D1039 |
| 1040 | D1040 | Ar1 | D1040 |
| 1041 | D1041 | Ar1 | D1041 |
| 1042 | D1042 | Ar1 | D1042 |
| 1043 | D1043 | Ar1 | D1043 |
| 1044 | D1044 | Ar1 | D1044 |
| 1045 | D1045 | Ar1 | D1045 |
| 1046 | D1046 | Ar1 | D1046 |
| 1047 | D1047 | Ar1 | D1047 |
| 1048 | D1048 | Ar1 | D1048 |
| 1049 | D1049 | Ar1 | D1049 |
| 1050 | D1050 | Ar1 | D1050 |
| 1051 | D1051 | Ar1 | D1051 |
| 1052 | D1052 | Ar1 | D1052 |
| 1053 | D1053 | Ar1 | D1053 |
| 1054 | D1054 | Ar1 | D1054 |
| 1055 | D1055 | Ar1 | D1055 |
| 1056 | D1056 | Ar1 | D1056 |
| 1057 | D1057 | Ar1 | D1057 |
| 1058 | D1058 | Ar1 | D1058 |
| 1059 | D1059 | Ar1 | D1059 |
| 1060 | D1060 | Ar1 | D1060 |
| 1061 | D1061 | Ar1 | D1061 |
| 1062 | D1062 | Ar1 | D1062 |
| 1063 | D1063 | Ar1 | D1063 |
| 1064 | D1064 | Ar1 | D1064 |
| 1065 | D1065 | Ar1 | D1065 |
| 1066 | D1066 | Ar1 | D1066 |
| 1067 | D1067 | Ar1 | D1067 |
| 1068 | D1068 | Ar1 | D1068 |
| 1069 | D1069 | Ar1 | D1069 |
| 1070 | D1070 | Ar1 | D1070 |
| 1071 | D1071 | Ar1 | D1071 |
| 1072 | D1072 | Ar1 | D1072 |
| 1073 | D1073 | Ar1 | D1073 |
| 1074 | D1074 | Ar1 | D1074 |
| 1075 | D1075 | Ar1 | D1075 |
| 1076 | D1076 | Ar1 | D1076 |
| 1077 | D1077 | Ar1 | D1077 |

(continued)

| No. | $R^3$ | $R^4$ | $R^5$ |
|---|---|---|---|
| 1078 | D1078 | Ar1 | D1078 |
| 1079 | D1079 | Ar1 | D1079 |
| 1080 | D1080 | Ar1 | D1080 |
| 1081 | D1081 | Ar1 | D1081 |
| 1082 | D1082 | Ar1 | D1082 |
| 1083 | D1083 | Ar1 | D1083 |
| 1084 | D1084 | Ar1 | D1084 |
| 1085 | D1085 | Ar1 | D1085 |
| 1086 | D1086 | Ar1 | D1086 |
| 1087 | D1087 | Ar1 | D1087 |
| 1088 | D1088 | Ar1 | D1088 |
| 1089 | D1089 | Ar1 | D1089 |
| 1090 | D1090 | Ar1 | D1090 |
| 1091 | D1091 | Ar1 | D1091 |
| 1092 | D1092 | Ar1 | D1092 |
| 1093 | D1093 | Ar1 | D1093 |
| 1094 | D1094 | Ar1 | D1094 |
| 1095 | D1095 | Ar1 | D1095 |
| 1096 | D1096 | Ar1 | D1096 |
| 1097 | D1097 | Ar1 | D1097 |
| 1098 | D1098 | Ar1 | D1098 |
| 1099 | D1099 | Ar1 | D1099 |
| 1100 | D1100 | Ar1 | D1100 |
| 1101 | D1101 | Ar1 | D1101 |
| 1102 | D1102 | Ar1 | D1102 |
| 1103 | D1103 | Ar1 | D1103 |
| 1104 | D1104 | Ar1 | D1104 |
| 1105 | D1105 | Ar1 | D1105 |
| 1106 | D1106 | Ar1 | D1106 |
| 1107 | D1107 | Ar1 | D1107 |
| 1108 | D1108 | Ar1 | D1108 |
| 1109 | D1109 | Ar1 | D1109 |
| 1110 | D1110 | Ar1 | D1110 |
| 1111 | D1111 | Ar1 | D1111 |
| 1112 | D1112 | Ar1 | D1112 |
| 1113 | D1113 | Ar1 | D1113 |
| 1114 | D1114 | Ar1 | D1114 |
| 1115 | D1115 | Ar1 | D1115 |
| 1116 | D1116 | Ar1 | D1116 |

(continued)

| No. | $R^3$ | $R^4$ | $R^5$ |
|---|---|---|---|
| 1117 | D1117 | Ar1 | D1117 |
| 1118 | D1118 | Ar1 | D1118 |
| 1119 | D1119 | Ar1 | D1119 |
| 1120 | D1120 | Ar1 | D1120 |
| 1121 | D1121 | Ar1 | D1121 |
| 1122 | D1122 | Ar1 | D1122 |
| 1123 | D1123 | Ar1 | D1123 |
| 1124 | D1124 | Ar1 | D1124 |
| 1125 | D1125 | Ar1 | D1125 |
| 1126 | D1126 | Ar1 | D1126 |
| 1127 | D1127 | Ar1 | D1127 |
| 1128 | D1128 | Ar1 | D1128 |
| 1129 | D1129 | Ar1 | D1129 |
| 1130 | D1130 | Ar1 | D1130 |
| 1131 | D1131 | Ar1 | D1131 |
| 1132 | D1132 | Ar1 | D1132 |
| 1133 | D1133 | Ar1 | D1133 |
| 1134 | D1134 | Ar1 | D1134 |
| 1135 | D1135 | Ar1 | D1135 |
| 1136 | D1136 | Ar1 | D1136 |
| 1137 | D1137 | Ar1 | D1137 |
| 1138 | D1138 | Ar1 | D1138 |
| 1139 | D1139 | Ar1 | D1139 |
| 1140 | D1140 | Ar1 | D1140 |
| 1141 | D1141 | Ar1 | D1141 |
| 1142 | D1142 | Ar1 | D1142 |
| 1143 | D1143 | Ar1 | D1143 |
| 1144 | D1144 | Ar1 | D1144 |
| 1145 | D1145 | Ar1 | D1145 |
| 1146 | D1146 | Ar1 | D1146 |
| 1147 | D1147 | Ar1 | D1147 |
| 1148 | D1148 | Ar1 | D1148 |
| 1149 | D1149 | Ar1 | D1149 |
| 1150 | D1150 | Ar1 | D1150 |
| 1151 | D1151 | Ar1 | D1151 |
| 1152 | D1152 | Ar1 | D1152 |
| 1153 | D1153 | Ar1 | D1153 |
| 1154 | D1154 | Ar1 | D1154 |
| 1155 | D1155 | Ar1 | D1155 |

(continued)

| No. | $R^3$ | $R^4$ | $R^5$ |
|---|---|---|---|
| 1156 | D1156 | Ar1 | D1156 |
| 1157 | D1157 | Ar1 | D1157 |
| 1158 | D1158 | Ar1 | D1158 |
| 1159 | D1159 | Ar1 | D1159 |
| 1160 | D1160 | Ar1 | D1160 |
| 1161 | D1161 | Ar1 | D1161 |
| 1162 | D1162 | Ar1 | D1162 |
| 1163 | D1163 | Ar1 | D1163 |
| 1164 | D1164 | Ar1 | D1164 |
| 1165 | D1165 | Ar1 | D1165 |
| 1166 | D1166 | Ar1 | D1166 |
| 1167 | D1167 | Ar1 | D1167 |
| 1168 | D1168 | Ar1 | D1168 |
| 1169 | D1169 | Ar1 | D1169 |
| 1170 | D1170 | Ar1 | D1170 |
| 1171 | D1171 | Ar1 | D1171 |
| 1172 | D1172 | Ar1 | D1172 |
| 1173 | D1173 | Ar1 | D1173 |
| 1174 | D1174 | Ar1 | D1174 |
| 1175 | D1175 | Ar1 | D1175 |

Table 2

| No. | $R^3$ | $R^4$ | $R^5$ | = |
|---|---|---|---|---|
| 1 ~ 1175 | D1~D1175 | Ar1 | D1~D1175 | $R^3 = R^5$ |
| 1176 ~ 2350 | D1~D1175 | Ar2 | D1~D1175 | |
| 2351 ~ 3525 | D1~D1175 | Ar3 | D1~D1175 | |
| 3526 ~ 4700 | D1~D1175 | Ar4 | D1~D1175 | |
| 4701 ~ 5875 | D1~D1175 | Ar5 | D1~D1175 | |
| 5876 ~ 7050 | D1~D1175 | Ar6 | D1~D1175 | |
| 7051 ~ 8225 | D1~D1175 | Ar7 | D1~D1175 | |
| 8226 ~ 9400 | D1~D1175 | Ar8 | D1~D1175 | |
| 9401 ~ 10575 | D1~D1175 | Ar9 | D1~D1175 | |
| 10576 ~ 11750 | D1~D1175 | Ar10 | D1~D1175 | |
| 11751 ~ 12925 | D1~D1175 | Ar11 | D1~D1175 | |
| 12926 ~ 14100 | D1~D1175 | Ar12 | D1~D1175 | |
| 14101 ~ 15275 | D1~D1175 | Ar13 | D1~D1175 | |
| 15276 ~ 16450 | D1~D1175 | Ar14 | D1~D1175 | |
| 16451 ~ 17625 | D1~D1175 | Ar15 | D1~D1175 | |
| 17626 ~ 18800 | D1~D1175 | Ar16 | D1~D1175 | |
| 18801 ~ 19975 | D1~D1175 | Ar17 | D1~D1175 | |
| 19976 ~ 21150 | D1~D1175 | Ar18 | D1~D1175 | |
| 21151 ~ 22325 | D1~D1175 | Ar19 | D1~D1175 | |
| 22326 ~ 23500 | D1~D1175 | Ar20 | D1~D1175 | |
| 23501 ~ 24675 | D1~D1175 | Ar21 | D1~D1175 | |
| 24676 ~ 25850 | D1~D1175 | Ar22 | D1~D1175 | |
| 25851 ~ 27025 | D1~D1175 | Ar23 | D1~D1175 | |
| 27026 ~ 28200 | D1~D1175 | Ar24 | D1~D1175 | |
| 28201 ~ 29375 | D1~D1175 | Ar25 | D1~D1175 | |
| 29376 ~ 30550 | D1~D1175 | Ar26 | D1~D1175 | |
| 30551 ~ 31725 | D1~D1175 | Ar27 | D1~D1175 | |
| 31726 ~ 32900 | D1~D1175 | Ar28 | D1~D1175 | |
| 32901 ~ 34075 | D1~D1175 | Ar29 | D1~D1175 | |
| 34076 ~ 35250 | D1~D1175 | Ar30 | D1~D1175 | |
| 35251 ~ 36425 | D1~D1175 | Ar31 | D1~D1175 | |
| 36426 ~ 37600 | D1~D1175 | Ar32 | D1~D1175 | |
| 37601 ~ 38775 | D1~D1175 | Ar33 | D1~D1175 | |
| 38776 ~ 39950 | D1~D1175 | Ar34 | D1~D1175 | |
| 39951 ~ 41125 | D1~D1175 | Ar35 | D1~D1175 | |
| 41126 ~ 42300 | D1~D1175 | Ar36 | D1~D1175 | |
| 42301 ~ 43475 | D1~D1175 | Ar37 | D1~D1175 | |
| 43476 ~ 44650 | D1~D1175 | Ar38 | D1~D1175 | |
| 44651 ~ 45825 | D1~D1175 | Ar39 | D1~D1175 | |
| 45826 ~ 47000 | D1~D1175 | Ar40 | D1~D1175 | |
| 47001 ~ 48175 | D1~D1175 | Ar41 | D1~D1175 | |
| 48176 ~ 49350 | D1~D1175 | Ar42 | D1~D1175 | |
| 49351 ~ 50525 | D1~D1175 | Ar43 | D1~D1175 | |
| 50526 ~ 51700 | D1~D1175 | Ar44 | D1~D1175 | |
| 51701 ~ 52875 | D1~D1175 | Ar45 | D1~D1175 | |
| 52876 ~ 54050 | D1~D1175 | Ar46 | D1~D1175 | |
| 54051 ~ 55225 | D1~D1175 | Ar47 | D1~D1175 | |
| 55226 ~ 56400 | D1~D1175 | Ar48 | D1~D1175 | |
| 56401 ~ 57575 | D1~D1175 | Ar49 | D1~D1175 | |
| 57576 ~ 58750 | D1~D1175 | Ar50 | D1~D1175 | |
| 58751 ~ 59925 | D1~D1175 | Ar51 | D1~D1175 | |
| 59926 ~ 61100 | D1~D1175 | Ar52 | D1~D1175 | |
| 61101 ~ 62275 | D1~D1175 | Ar53 | D1~D1175 | |
| 62276 ~ 63450 | D1~D1175 | Ar54 | D1~D1175 | |
| 63451 ~ 64625 | D1~D1175 | Ar55 | D1~D1175 | |
| 64626 ~ 65800 | D1~D1175 | Ar56 | D1~D1175 | |
| 65801 ~ 66975 | D1~D1175 | Ar57 | D1~D1175 | |
| 66976 ~ 68150 | D1~D1175 | Ar58 | D1~D1175 | |
| 68151 ~ 69325 | D1~D1175 | Ar59 | D1~D1175 | |
| 69326 ~ 70500 | D1~D1175 | H | D1~D1175 | |

| No. | $R^3$ | $R^4$ | $R^5$ | No. | $R^3$ | $R^4$ | $R^5$ |
|---|---|---|---|---|---|---|---|
| 70501 ～ 71674 | D1 | Ar1 | D2～D1175 | 140941 ～ 142114 | D7 | Ar1 | D1～6,D8～1175 |
| 71675 ～ 72848 | D1 | Ar2 | D2～D1175 | 142115 ～ 143288 | D7 | Ar2 | D1～6,D8～1175 |
| 72849 ～ 74022 | D1 | Ar3 | D2～D1175 | 143289 ～ 144462 | D7 | Ar3 | D1～6,D8～1175 |
| 74023 ～ 75196 | D1 | Ar4 | D2～D1175 | 144463 ～ 145636 | D7 | Ar4 | D1～6,D8～1175 |
| 75197 ～ 76370 | D1 | Ar5 | D2～D1175 | 145637 ～ 146810 | D7 | Ar5 | D1～6,D8～1175 |
| 76371 ～ 77544 | D1 | Ar6 | D2～D1175 | 146811 ～ 147984 | D7 | Ar6 | D1～6,D8～1175 |
| 77545 ～ 78718 | D1 | Ar7 | D2～D1175 | 147985 ～ 149158 | D7 | Ar7 | D1～6,D8～1175 |
| 78719 ～ 79892 | D1 | Ar8 | D2～D1175 | 149159 ～ 150332 | D7 | Ar8 | D1～6,D8～1175 |
| 79893 ～ 81066 | D1 | Ar9 | D2～D1175 | 150333 ～ 151506 | D7 | Ar9 | D1～6,D8～1175 |
| 81067 ～ 82240 | D1 | Ar10 | D2～D1175 | 151507 ～ 152680 | D7 | Ar10 | D1～6,D8～1175 |
| 82241 ～ 83414 | D1 | Ar11 | D2～D1175 | 152681 ～ 153854 | D7 | Ar11 | D1～6,D8～1175 |
| 83415 ～ 84588 | D1 | Ar12 | D2～D1175 | 153855 ～ 155028 | D7 | Ar12 | D1～6,D8～1175 |
| 84589 ～ 85762 | D1 | Ar13 | D2～D1175 | 155029 ～ 156202 | D7 | Ar13 | D1～6,D8～1175 |
| 85763 ～ 86936 | D1 | Ar14 | D2～D1175 | 156203 ～ 157376 | D7 | Ar14 | D1～6,D8～1175 |
| 86937 ～ 88110 | D1 | Ar15 | D2～D1175 | 157377 ～ 158550 | D7 | Ar15 | D1～6,D8～1175 |
| 88111 ～ 89284 | D1 | Ar16 | D2～D1175 | 158551 ～ 159724 | D7 | Ar16 | D1～6,D8～1175 |
| 89285 ～ 90458 | D1 | Ar17 | D2～D1175 | 159725 ～ 160898 | D7 | Ar17 | D1～6,D8～1175 |
| 90459 ～ 91632 | D1 | Ar18 | D2～D1175 | 160899 ～ 162072 | D7 | Ar18 | D1～6,D8～1175 |
| 91633 ～ 92806 | D1 | Ar19 | D2～D1175 | 162073 ～ 163246 | D7 | Ar19 | D1～6,D8～1175 |
| 92807 ～ 93980 | D1 | Ar20 | D2～D1175 | 163247 ～ 164420 | D7 | Ar20 | D1～6,D8～1175 |
| 93981 ～ 95154 | D1 | Ar21 | D2～D1175 | 164421 ～ 165594 | D7 | Ar21 | D1～6,D8～1175 |
| 95155 ～ 96328 | D1 | Ar22 | D2～D1175 | 165595 ～ 166768 | D7 | Ar22 | D1～6,D8～1175 |
| 96329 ～ 97502 | D1 | Ar23 | D2～D1175 | 166769 ～ 167942 | D7 | Ar23 | D1～6,D8～1175 |
| 97503 ～ 98676 | D1 | Ar24 | D2～D1175 | 167943 ～ 169116 | D7 | Ar24 | D1～6,D8～1175 |
| 98677 ～ 99850 | D1 | Ar25 | D2～D1175 | 169117 ～ 170290 | D7 | Ar25 | D1～6,D8～1175 |
| 99851 ～ 101024 | D1 | Ar26 | D2～D1175 | 170291 ～ 171464 | D7 | Ar26 | D1～6,D8～1175 |
| 101025 ～ 102198 | D1 | Ar27 | D2～D1175 | 171465 ～ 172638 | D7 | Ar27 | D1～6,D8～1175 |
| 102199 ～ 103372 | D1 | Ar28 | D2～D1175 | 172639 ～ 173812 | D7 | Ar28 | D1～6,D8～1175 |
| 103373 ～ 104546 | D1 | Ar29 | D2～D1175 | 173813 ～ 174986 | D7 | Ar29 | D1～6,D8～1175 |
| 104547 ～ 105720 | D1 | Ar30 | D2～D1175 | 174987 ～ 176160 | D7 | Ar30 | D1～6,D8～1175 |
| 105721 ～ 106894 | D1 | Ar31 | D2～D1175 | 176161 ～ 177334 | D7 | Ar31 | D1～6,D8～1175 |
| 106895 ～ 108068 | D1 | Ar32 | D2～D1175 | 177335 ～ 178508 | D7 | Ar32 | D1～6,D8～1175 |
| 108069 ～ 109242 | D1 | Ar33 | D2～D1175 | 178509 ～ 179682 | D7 | Ar33 | D1～6,D8～1175 |
| 109243 ～ 110416 | D1 | Ar34 | D2～D1175 | 179683 ～ 180856 | D7 | Ar34 | D1～6,D8～1175 |
| 110417 ～ 111590 | D1 | Ar35 | D2～D1175 | 180857 ～ 182030 | D7 | Ar35 | D1～6,D8～1175 |
| 111591 ～ 112764 | D1 | Ar36 | D2～D1175 | 182031 ～ 183204 | D7 | Ar36 | D1～6,D8～1175 |
| 112765 ～ 113938 | D1 | Ar37 | D2～D1175 | 183205 ～ 184378 | D7 | Ar37 | D1～6,D8～1175 |
| 113939 ～ 115112 | D1 | Ar38 | D2～D1175 | 184379 ～ 185552 | D7 | Ar38 | D1～6,D8～1175 |
| 115113 ～ 116286 | D1 | Ar39 | D2～D1175 | 185553 ～ 186726 | D7 | Ar39 | D1～6,D8～1175 |
| 116287 ～ 117460 | D1 | Ar40 | D2～D1175 | 186727 ～ 187900 | D7 | Ar40 | D1～6,D8～1175 |
| 117461 ～ 118634 | D1 | Ar41 | D2～D1175 | 187901 ～ 189074 | D7 | Ar41 | D1～6,D8～1175 |
| 118635 ～ 119808 | D1 | Ar42 | D2～D1175 | 189075 ～ 190248 | D7 | Ar42 | D1～6,D8～1175 |
| 119809 ～ 120982 | D1 | Ar43 | D2～D1175 | 190249 ～ 191422 | D7 | Ar43 | D1～6,D8～1175 |
| 120983 ～ 122156 | D1 | Ar44 | D2～D1175 | 191423 ～ 192596 | D7 | Ar44 | D1～6,D8～1175 |
| 122157 ～ 123330 | D1 | Ar45 | D2～D1175 | 192597 ～ 193770 | D7 | Ar45 | D1～6,D8～1175 |
| 123331 ～ 124504 | D1 | Ar46 | D2～D1175 | 193771 ～ 194944 | D7 | Ar46 | D1～6,D8～1175 |
| 124505 ～ 125678 | D1 | Ar47 | D2～D1175 | 194945 ～ 196118 | D7 | Ar47 | D1～6,D8～1175 |
| 125679 ～ 126852 | D1 | Ar48 | D2～D1175 | 196119 ～ 197292 | D7 | Ar48 | D1～6,D8～1175 |
| 126853 ～ 128026 | D1 | Ar49 | D2～D1175 | 197293 ～ 198466 | D7 | Ar49 | D1～6,D8～1175 |
| 128027 ～ 129200 | D1 | Ar50 | D2～D1175 | 198467 ～ 199640 | D7 | Ar50 | D1～6,D8～1175 |
| 129201 ～ 130374 | D1 | Ar51 | D2～D1175 | 199641 ～ 200814 | D7 | Ar51 | D1～6,D8～1175 |
| 130375 ～ 131548 | D1 | Ar52 | D2～D1175 | 200815 ～ 201988 | D7 | Ar52 | D1～6,D8～1175 |
| 131549 ～ 132722 | D1 | Ar53 | D2～D1175 | 201989 ～ 203162 | D7 | Ar53 | D1～6,D8～1175 |
| 132723 ～ 133896 | D1 | Ar54 | D2～D1175 | 203163 ～ 204336 | D7 | Ar54 | D1～6,D8～1175 |
| 133897 ～ 135070 | D1 | Ar55 | D2～D1175 | 204337 ～ 205510 | D7 | Ar55 | D1～6,D8～1175 |
| 135071 ～ 136244 | D1 | Ar56 | D2～D1175 | 205511 ～ 206684 | D7 | Ar56 | D1～6,D8～1175 |
| 136245 ～ 137418 | D1 | Ar57 | D2～D1175 | 206685 ～ 207858 | D7 | Ar57 | D1～6,D8～1175 |
| 137419 ～ 138592 | D1 | Ar58 | D2～D1175 | 207859 ～ 209032 | D7 | Ar58 | D1～6,D8～1175 |
| 138593 ～ 139766 | D1 | Ar59 | D2～D1175 | 209033 ～ 210206 | D7 | Ar59 | D1～6,D8～1175 |
| 139767 ～ 140940 | D1 | H | D2～D1175 | 210207 ～ 211380 | D7 | H | D1～6,D8～1175 |

| No. | $R^3$ | $R^4$ | $R^5$ | No. | $R^3$ | $R^4$ | $R^5$ |
|---|---|---|---|---|---|---|---|
| 211381 ～212554 | D8 | Ar1 | D1～7,D9～1175 | 281821 ～282994 | D9 | Ar1 | D1～8,D10～1175 |
| 212555 ～213728 | D8 | Ar2 | D1～7,D9～1175 | 282995 ～284168 | D9 | Ar2 | D1～8,D10～1175 |
| 213729 ～214902 | D8 | Ar3 | D1～7,D9～1175 | 284169 ～285342 | D9 | Ar3 | D1～8,D10～1175 |
| 214903 ～216076 | D8 | Ar4 | D1～7,D9～1175 | 285343 ～286516 | D9 | Ar4 | D1～8,D10～1175 |
| 216077 ～217250 | D8 | Ar5 | D1～7,D9～1175 | 286517 ～287690 | D9 | Ar5 | D1～8,D10～1175 |
| 217251 ～218424 | D8 | Ar6 | D1～7,D9～1175 | 287691 ～288864 | D9 | Ar6 | D1～8,D10～1175 |
| 218425 ～219598 | D8 | Ar7 | D1～7,D9～1175 | 288865 ～290038 | D9 | Ar7 | D1～8,D10～1175 |
| 219599 ～220772 | D8 | Ar8 | D1～7,D9～1175 | 290039 ～291212 | D9 | Ar8 | D1～8,D10～1175 |
| 220773 ～221946 | D8 | Ar9 | D1～7,D9～1175 | 291213 ～292386 | D9 | Ar9 | D1～8,D10～1175 |
| 221947 ～223120 | D8 | Ar10 | D1～7,D9～1175 | 292387 ～293560 | D9 | Ar10 | D1～8,D10～1175 |
| 223121 ～224294 | D8 | Ar11 | D1～7,D9～1175 | 293561 ～294734 | D9 | Ar11 | D1～8,D10～1175 |
| 224295 ～225468 | D8 | Ar12 | D1～7,D9～1175 | 294735 ～295908 | D9 | Ar12 | D1～8,D10～1175 |
| 225469 ～226642 | D8 | Ar13 | D1～7,D9～1175 | 295909 ～297082 | D9 | Ar13 | D1～8,D10～1175 |
| 226643 ～227816 | D8 | Ar14 | D1～7,D9～1175 | 297083 ～298256 | D9 | Ar14 | D1～8,D10～1175 |
| 227817 ～228990 | D8 | Ar15 | D1～7,D9～1175 | 298257 ～299430 | D9 | Ar15 | D1～8,D10～1175 |
| 228991 ～230164 | D8 | Ar16 | D1～7,D9～1175 | 299431 ～300604 | D9 | Ar16 | D1～8,D10～1175 |
| 230165 ～231338 | D8 | Ar17 | D1～7,D9～1175 | 300605 ～301778 | D9 | Ar17 | D1～8,D10～1175 |
| 231339 ～232512 | D8 | Ar18 | D1～7,D9～1175 | 301779 ～302952 | D9 | Ar18 | D1～8,D10～1175 |
| 232513 ～233686 | D8 | Ar19 | D1～7,D9～1175 | 302953 ～304126 | D9 | Ar19 | D1～8,D10～1175 |
| 233687 ～234860 | D8 | Ar20 | D1～7,D9～1175 | 304127 ～305300 | D9 | Ar20 | D1～8,D10～1175 |
| 234861 ～236034 | D8 | Ar21 | D1～7,D9～1175 | 305301 ～306474 | D9 | Ar21 | D1～8,D10～1175 |
| 236035 ～237208 | D8 | Ar22 | D1～7,D9～1175 | 306475 ～307648 | D9 | Ar22 | D1～8,D10～1175 |
| 237209 ～238382 | D8 | Ar23 | D1～7,D9～1175 | 307649 ～308822 | D9 | Ar23 | D1～8,D10～1175 |
| 238383 ～239556 | D8 | Ar24 | D1～7,D9～1175 | 308823 ～309996 | D9 | Ar24 | D1～8,D10～1175 |
| 239557 ～240730 | D8 | Ar25 | D1～7,D9～1175 | 309997 ～311170 | D9 | Ar25 | D1～8,D10～1175 |
| 240731 ～241904 | D8 | Ar26 | D1～7,D9～1175 | 311171 ～312344 | D9 | Ar26 | D1～8,D10～1175 |
| 241905 ～243078 | D8 | Ar27 | D1～7,D9～1175 | 312345 ～313518 | D9 | Ar27 | D1～8,D10～1175 |
| 243079 ～244252 | D8 | Ar28 | D1～7,D9～1175 | 313519 ～314692 | D9 | Ar28 | D1～8,D10～1175 |
| 244253 ～245426 | D8 | Ar29 | D1～7,D9～1175 | 314693 ～315866 | D9 | Ar29 | D1～8,D10～1175 |
| 245427 ～246600 | D8 | Ar30 | D1～7,D9～1175 | 315867 ～317040 | D9 | Ar30 | D1～8,D10～1175 |
| 246601 ～247774 | D8 | Ar31 | D1～7,D9～1175 | 317041 ～318214 | D9 | Ar31 | D1～8,D10～1175 |
| 247775 ～248948 | D8 | Ar32 | D1～7,D9～1175 | 318215 ～319388 | D9 | Ar32 | D1～8,D10～1175 |
| 248949 ～250122 | D8 | Ar33 | D1～7,D9～1175 | 319389 ～320562 | D9 | Ar33 | D1～8,D10～1175 |
| 250123 ～251296 | D8 | Ar34 | D1～7,D9～1175 | 320563 ～321736 | D9 | Ar34 | D1～8,D10～1175 |
| 251297 ～252470 | D8 | Ar35 | D1～7,D9～1175 | 321737 ～322910 | D9 | Ar35 | D1～8,D10～1175 |
| 252471 ～253644 | D8 | Ar36 | D1～7,D9～1175 | 322911 ～324084 | D9 | Ar36 | D1～8,D10～1175 |
| 253645 ～254818 | D8 | Ar37 | D1～7,D9～1175 | 324085 ～325258 | D9 | Ar37 | D1～8,D10～1175 |
| 254819 ～255992 | D8 | Ar38 | D1～7,D9～1175 | 325259 ～326432 | D9 | Ar38 | D1～8,D10～1175 |
| 255993 ～257166 | D8 | Ar39 | D1～7,D9～1175 | 326433 ～327606 | D9 | Ar39 | D1～8,D10～1175 |
| 257167 ～258340 | D8 | Ar40 | D1～7,D9～1175 | 327607 ～328780 | D9 | Ar40 | D1～8,D10～1175 |
| 258341 ～259514 | D8 | Ar41 | D1～7,D9～1175 | 328781 ～329954 | D9 | Ar41 | D1～8,D10～1175 |
| 259515 ～260688 | D8 | Ar42 | D1～7,D9～1175 | 329955 ～331128 | D9 | Ar42 | D1～8,D10～1175 |
| 260689 ～261862 | D8 | Ar43 | D1～7,D9～1175 | 331129 ～332302 | D9 | Ar43 | D1～8,D10～1175 |
| 261863 ～263036 | D8 | Ar44 | D1～7,D9～1175 | 332303 ～333476 | D9 | Ar44 | D1～8,D10～1175 |
| 263037 ～264210 | D8 | Ar45 | D1～7,D9～1175 | 333477 ～334650 | D9 | Ar45 | D1～8,D10～1175 |
| 264211 ～265384 | D8 | Ar46 | D1～7,D9～1175 | 334651 ～335824 | D9 | Ar46 | D1～8,D10～1175 |
| 265385 ～266558 | D8 | Ar47 | D1～7,D9～1175 | 335825 ～336998 | D9 | Ar47 | D1～8,D10～1175 |
| 266559 ～267732 | D8 | Ar48 | D1～7,D9～1175 | 336999 ～338172 | D9 | Ar48 | D1～8,D10～1175 |
| 267733 ～268906 | D8 | Ar49 | D1～7,D9～1175 | 338173 ～339346 | D9 | Ar49 | D1～8,D10～1175 |
| 268907 ～270080 | D8 | Ar50 | D1～7,D9～1175 | 339347 ～340520 | D9 | Ar50 | D1～8,D10～1175 |
| 270081 ～271254 | D8 | Ar51 | D1～7,D9～1175 | 340521 ～341694 | D9 | Ar51 | D1～8,D10～1175 |
| 271255 ～272428 | D8 | Ar52 | D1～7,D9～1175 | 341695 ～342868 | D9 | Ar52 | D1～8,D10～1175 |
| 272429 ～273602 | D8 | Ar53 | D1～7,D9～1175 | 342869 ～344042 | D9 | Ar53 | D1～8,D10～1175 |
| 273603 ～274776 | D8 | Ar54 | D1～7,D9～1175 | 344043 ～345216 | D9 | Ar54 | D1～8,D10～1175 |
| 274777 ～275950 | D8 | Ar55 | D1～7,D9～1175 | 345217 ～346390 | D9 | Ar55 | D1～8,D10～1175 |
| 275951 ～277124 | D8 | Ar56 | D1～7,D9～1175 | 346391 ～347564 | D9 | Ar56 | D1～8,D10～1175 |
| 277125 ～278298 | D8 | Ar57 | D1～7,D9～1175 | 347565 ～348738 | D9 | Ar57 | D1～8,D10～1175 |
| 278299 ～279472 | D8 | Ar58 | D1～7,D9～1175 | 348739 ～349912 | D9 | Ar58 | D1～8,D10～1175 |
| 279473 ～280646 | D8 | Ar59 | D1～7,D9～1175 | 349913 ～351086 | D9 | Ar59 | D1～8,D10～1175 |
| 280647 ～281820 | D8 | H | D1～7,D9～1175 | 351087 ～352260 | D9 | H | D1～8,D10～1175 |

| No. | $R^3$ | $R^4$ | $R^5$ |
|---|---|---|---|
| 352261 ～353434 | D19 | Ar1 | D1～18,D20～1175 |
| 353435 ～354608 | D19 | Ar2 | D1～18,D20～1175 |
| 354609 ～355782 | D19 | Ar3 | D1～18,D20～1175 |
| 355783 ～356956 | D19 | Ar4 | D1～18,D20～1175 |
| 356957 ～358130 | D19 | Ar5 | D1～18,D20～1175 |
| 358131 ～359304 | D19 | Ar6 | D1～18,D20～1175 |
| 359305 ～360478 | D19 | Ar7 | D1～18,D20～1175 |
| 360479 ～361652 | D19 | Ar8 | D1～18,D20～1175 |
| 361653 ～362826 | D19 | Ar9 | D1～18,D20～1175 |
| 362827 ～364000 | D19 | Ar10 | D1～18,D20～1175 |
| 364001 ～365174 | D19 | Ar11 | D1～18,D20～1175 |
| 365175 ～366348 | D19 | Ar12 | D1～18,D20～1175 |
| 366349 ～367522 | D19 | Ar13 | D1～18,D20～1175 |
| 367523 ～368696 | D19 | Ar14 | D1～18,D20～1175 |
| 368697 ～369870 | D19 | Ar15 | D1～18,D20～1175 |
| 369871 ～371044 | D19 | Ar16 | D1～18,D20～1175 |
| 371045 ～372218 | D19 | Ar17 | D1～18,D20～1175 |
| 372219 ～373392 | D19 | Ar18 | D1～18,D20～1175 |
| 373393 ～374566 | D19 | Ar19 | D1～18,D20～1175 |
| 374567 ～375740 | D19 | Ar20 | D1～18,D20～1175 |
| 375741 ～376914 | D19 | Ar21 | D1～18,D20～1175 |
| 376915 ～378088 | D19 | Ar22 | D1～18,D20～1175 |
| 378089 ～379262 | D19 | Ar23 | D1～18,D20～1175 |
| 379263 ～380436 | D19 | Ar24 | D1～18,D20～1175 |
| 380437 ～381610 | D19 | Ar25 | D1～18,D20～1175 |
| 381611 ～382784 | D19 | Ar26 | D1～18,D20～1175 |
| 382785 ～383958 | D19 | Ar27 | D1～18,D20～1175 |
| 383959 ～385132 | D19 | Ar28 | D1～18,D20～1175 |
| 385133 ～386306 | D19 | Ar29 | D1～18,D20～1175 |
| 386307 ～387480 | D19 | Ar30 | D1～18,D20～1175 |
| 387481 ～388654 | D19 | Ar31 | D1～18,D20～1175 |
| 388655 ～389828 | D19 | Ar32 | D1～18,D20～1175 |
| 389829 ～391002 | D19 | Ar33 | D1～18,D20～1175 |
| 391003 ～392176 | D19 | Ar34 | D1～18,D20～1175 |
| 392177 ～393350 | D19 | Ar35 | D1～18,D20～1175 |
| 393351 ～394524 | D19 | Ar36 | D1～18,D20～1175 |
| 394525 ～395698 | D19 | Ar37 | D1～18,D20～1175 |
| 395699 ～396872 | D19 | Ar38 | D1～18,D20～1175 |
| 396873 ～398046 | D19 | Ar39 | D1～18,D20～1175 |
| 398047 ～399220 | D19 | Ar40 | D1～18,D20～1175 |
| 399221 ～400394 | D19 | Ar41 | D1～18,D20～1175 |
| 400395 ～401568 | D19 | Ar42 | D1～18,D20～1175 |
| 401569 ～402742 | D19 | Ar43 | D1～18,D20～1175 |
| 402743 ～403916 | D19 | Ar44 | D1～18,D20～1175 |
| 403917 ～405090 | D19 | Ar45 | D1～18,D20～1175 |
| 405091 ～406264 | D19 | Ar46 | D1～18,D20～1175 |
| 406265 ～407438 | D19 | Ar47 | D1～18,D20～1175 |
| 407439 ～408612 | D19 | Ar48 | D1～18,D20～1175 |
| 408613 ～409786 | D19 | Ar49 | D1～18,D20～1175 |
| 409787 ～410960 | D19 | Ar50 | D1～18,D20～1175 |
| 410961 ～412134 | D19 | Ar51 | D1～18,D20～1175 |
| 412135 ～413308 | D19 | Ar52 | D1～18,D20～1175 |
| 413309 ～414482 | D19 | Ar53 | D1～18,D20～1175 |
| 414483 ～415656 | D19 | Ar54 | D1～18,D20～1175 |
| 415657 ～416830 | D19 | Ar55 | D1～18,D20～1175 |
| 416831 ～418004 | D19 | Ar56 | D1～18,D20～1175 |
| 418005 ～419178 | D19 | Ar57 | D1～18,D20～1175 |
| 419179 ～420352 | D19 | Ar58 | D1～18,D20～1175 |
| 420353 ～421526 | D19 | Ar59 | D1～18,D20～1175 |
| 421527 ～422700 | D19 | H | D1～18,D20～1175 |

| No. | $R^3$ | $R^4$ | $R^5$ |
|---|---|---|---|
| 422701 ～423874 | D37 | Ar1 | D1～36,D38～1175 |
| 423875 ～425048 | D37 | Ar2 | D1～36,D38～1175 |
| 425049 ～426222 | D37 | Ar3 | D1～36,D38～1175 |
| 426223 ～427396 | D37 | Ar4 | D1～36,D38～1175 |
| 427397 ～428570 | D37 | Ar5 | D1～36,D38～1175 |
| 428571 ～429744 | D37 | Ar6 | D1～36,D38～1175 |
| 429745 ～430918 | D37 | Ar7 | D1～36,D38～1175 |
| 430919 ～432092 | D37 | Ar8 | D1～36,D38～1175 |
| 432093 ～433266 | D37 | Ar9 | D1～36,D38～1175 |
| 433267 ～434440 | D37 | Ar10 | D1～36,D38～1175 |
| 434441 ～435614 | D37 | Ar11 | D1～36,D38～1175 |
| 435615 ～436788 | D37 | Ar12 | D1～36,D38～1175 |
| 436789 ～437962 | D37 | Ar13 | D1～36,D38～1175 |
| 437963 ～439136 | D37 | Ar14 | D1～36,D38～1175 |
| 439137 ～440310 | D37 | Ar15 | D1～36,D38～1175 |
| 440311 ～441484 | D37 | Ar16 | D1～36,D38～1175 |
| 441485 ～442658 | D37 | Ar17 | D1～36,D38～1175 |
| 442659 ～443832 | D37 | Ar18 | D1～36,D38～1175 |
| 443833 ～445006 | D37 | Ar19 | D1～36,D38～1175 |
| 445007 ～446180 | D37 | Ar20 | D1～36,D38～1175 |
| 446181 ～447354 | D37 | Ar21 | D1～36,D38～1175 |
| 447355 ～448528 | D37 | Ar22 | D1～36,D38～1175 |
| 448529 ～449702 | D37 | Ar23 | D1～36,D38～1175 |
| 449703 ～450876 | D37 | Ar24 | D1～36,D38～1175 |
| 450877 ～452050 | D37 | Ar25 | D1～36,D38～1175 |
| 452051 ～453224 | D37 | Ar26 | D1～36,D38～1175 |
| 453225 ～454398 | D37 | Ar27 | D1～36,D38～1175 |
| 454399 ～455572 | D37 | Ar28 | D1～36,D38～1175 |
| 455573 ～456746 | D37 | Ar29 | D1～36,D38～1175 |
| 456747 ～457920 | D37 | Ar30 | D1～36,D38～1175 |
| 457921 ～459094 | D37 | Ar31 | D1～36,D38～1175 |
| 459095 ～460268 | D37 | Ar32 | D1～36,D38～1175 |
| 460269 ～461442 | D37 | Ar33 | D1～36,D38～1175 |
| 461443 ～462616 | D37 | Ar34 | D1～36,D38～1175 |
| 462617 ～463790 | D37 | Ar35 | D1～36,D38～1175 |
| 463791 ～464964 | D37 | Ar36 | D1～36,D38～1175 |
| 464965 ～466138 | D37 | Ar37 | D1～36,D38～1175 |
| 466139 ～467312 | D37 | Ar38 | D1～36,D38～1175 |
| 467313 ～468486 | D37 | Ar39 | D1～36,D38～1175 |
| 468487 ～469660 | D37 | Ar40 | D1～36,D38～1175 |
| 469661 ～470834 | D37 | Ar41 | D1～36,D38～1175 |
| 470835 ～472008 | D37 | Ar42 | D1～36,D38～1175 |
| 472009 ～473182 | D37 | Ar43 | D1～36,D38～1175 |
| 473183 ～474356 | D37 | Ar44 | D1～36,D38～1175 |
| 474357 ～475530 | D37 | Ar45 | D1～36,D38～1175 |
| 475531 ～476704 | D37 | Ar46 | D1～36,D38～1175 |
| 476705 ～477878 | D37 | Ar47 | D1～36,D38～1175 |
| 477879 ～479052 | D37 | Ar48 | D1～36,D38～1175 |
| 479053 ～480226 | D37 | Ar49 | D1～36,D38～1175 |
| 480227 ～481400 | D37 | Ar50 | D1～36,D38～1175 |
| 481401 ～482574 | D37 | Ar51 | D1～36,D38～1175 |
| 482575 ～483748 | D37 | Ar52 | D1～36,D38～1175 |
| 483749 ～484922 | D37 | Ar53 | D1～36,D38～1175 |
| 484923 ～486096 | D37 | Ar54 | D1～36,D38～1175 |
| 486097 ～487270 | D37 | Ar55 | D1～36,D38～1175 |
| 487271 ～488444 | D37 | Ar56 | D1～36,D38～1175 |
| 488445 ～489618 | D37 | Ar57 | D1～36,D38～1175 |
| 489619 ～490792 | D37 | Ar58 | D1～36,D38～1175 |
| 490793 ～491966 | D37 | Ar59 | D1～36,D38～1175 |
| 491967 ～493140 | D37 | H | D1～36,D38～1175 |

| No. | $R^3$ | $R^4$ | $R^5$ |
|---|---|---|---|
| 493141 ～494314 | D50 | Ar1 | D1～49,D51～1175 |
| 494315 ～495488 | D50 | Ar2 | D1～49,D51～1175 |
| 495489 ～496662 | D50 | Ar3 | D1～49,D51～1175 |
| 496663 ～497836 | D50 | Ar4 | D1～49,D51～1175 |
| 497837 ～499010 | D50 | Ar5 | D1～49,D51～1175 |
| 499011 ～500184 | D50 | Ar6 | D1～49,D51～1175 |
| 500185 ～501358 | D50 | Ar7 | D1～49,D51～1175 |
| 501359 ～502532 | D50 | Ar8 | D1～49,D51～1175 |
| 502533 ～503706 | D50 | Ar9 | D1～49,D51～1175 |
| 503707 ～504880 | D50 | Ar10 | D1～49,D51～1175 |
| 504881 ～506054 | D50 | Ar11 | D1～49,D51～1175 |
| 506055 ～507228 | D50 | Ar12 | D1～49,D51～1175 |
| 507229 ～508402 | D50 | Ar13 | D1～49,D51～1175 |
| 508403 ～509576 | D50 | Ar14 | D1～49,D51～1175 |
| 509577 ～510750 | D50 | Ar15 | D1～49,D51～1175 |
| 510751 ～511924 | D50 | Ar16 | D1～49,D51～1175 |
| 511925 ～513098 | D50 | Ar17 | D1～49,D51～1175 |
| 513099 ～514272 | D50 | Ar18 | D1～49,D51～1175 |
| 514273 ～515446 | D50 | Ar19 | D1～49,D51～1175 |
| 515447 ～516620 | D50 | Ar20 | D1～49,D51～1175 |
| 516621 ～517794 | D50 | Ar21 | D1～49,D51～1175 |
| 517795 ～518968 | D50 | Ar22 | D1～49,D51～1175 |
| 518969 ～520142 | D50 | Ar23 | D1～49,D51～1175 |
| 520143 ～521316 | D50 | Ar24 | D1～49,D51～1175 |
| 521317 ～522490 | D50 | Ar25 | D1～49,D51～1175 |
| 522491 ～523664 | D50 | Ar26 | D1～49,D51～1175 |
| 523665 ～524838 | D50 | Ar27 | D1～49,D51～1175 |
| 524839 ～526012 | D50 | Ar28 | D1～49,D51～1175 |
| 526013 ～527186 | D50 | Ar29 | D1～49,D51～1175 |
| 527187 ～528360 | D50 | Ar30 | D1～49,D51～1175 |
| 528361 ～529534 | D50 | Ar31 | D1～49,D51～1175 |
| 529535 ～530708 | D50 | Ar32 | D1～49,D51～1175 |
| 530709 ～531882 | D50 | Ar33 | D1～49,D51～1175 |
| 531883 ～533056 | D50 | Ar34 | D1～49,D51～1175 |
| 533057 ～534230 | D50 | Ar35 | D1～49,D51～1175 |
| 534231 ～535404 | D50 | Ar36 | D1～49,D51～1175 |
| 535405 ～536578 | D50 | Ar37 | D1～49,D51～1175 |
| 536579 ～537752 | D50 | Ar38 | D1～49,D51～1175 |
| 537753 ～538926 | D50 | Ar39 | D1～49,D51～1175 |
| 538927 ～540100 | D50 | Ar40 | D1～49,D51～1175 |
| 540101 ～541274 | D50 | Ar41 | D1～49,D51～1175 |
| 541275 ～542448 | D50 | Ar42 | D1～49,D51～1175 |
| 542449 ～543622 | D50 | Ar43 | D1～49,D51～1175 |
| 543623 ～544796 | D50 | Ar44 | D1～49,D51～1175 |
| 544797 ～545970 | D50 | Ar45 | D1～49,D51～1175 |
| 545971 ～547144 | D50 | Ar46 | D1～49,D51～1175 |
| 547145 ～548318 | D50 | Ar47 | D1～49,D51～1175 |
| 548319 ～549492 | D50 | Ar48 | D1～49,D51～1175 |
| 549493 ～550666 | D50 | Ar49 | D1～49,D51～1175 |
| 550667 ～551840 | D50 | Ar50 | D1～49,D51～1175 |
| 551841 ～553014 | D50 | Ar51 | D1～49,D51～1175 |
| 553015 ～554188 | D50 | Ar52 | D1～49,D51～1175 |
| 554189 ～555362 | D50 | Ar53 | D1～49,D51～1175 |
| 555363 ～556536 | D50 | Ar54 | D1～49,D51～1175 |
| 556537 ～557710 | D50 | Ar55 | D1～49,D51～1175 |
| 557711 ～558884 | D50 | Ar56 | D1～49,D51～1175 |
| 558885 ～560058 | D50 | Ar57 | D1～49,D51～1175 |
| 560059 ～561232 | D50 | Ar58 | D1～49,D51～1175 |
| 561233 ～562406 | D50 | Ar59 | D1～49,D51～1175 |
| 562407 ～563580 | D50 | H | D1～49,D51～1175 |
| 563581 ～564754 | D465 | Ar1 | D1～464,D466～1175 |
| 564755 ～565928 | D465 | Ar2 | D1～464,D466～1175 |
| 565929 ～567102 | D465 | Ar3 | D1～464,D466～1175 |
| 567103 ～568276 | D465 | Ar4 | D1～464,D466～1175 |
| 568277 ～569450 | D465 | Ar5 | D1～464,D466～1175 |
| 569451 ～570624 | D465 | Ar6 | D1～464,D466～1175 |
| 570625 ～571798 | D465 | Ar7 | D1～464,D466～1175 |
| 571799 ～572972 | D465 | Ar8 | D1～464,D466～1175 |
| 572973 ～574146 | D465 | Ar9 | D1～464,D466～1175 |
| 574147 ～575320 | D465 | Ar10 | D1～464,D466～1175 |
| 575321 ～576494 | D465 | Ar11 | D1～464,D466～1175 |
| 576495 ～577668 | D465 | Ar12 | D1～464,D466～1175 |
| 577669 ～578842 | D465 | Ar13 | D1～464,D466～1175 |
| 578843 ～580016 | D465 | Ar14 | D1～464,D466～1175 |
| 580017 ～581190 | D465 | Ar15 | D1～464,D466～1175 |
| 581191 ～582364 | D465 | Ar16 | D1～464,D466～1175 |
| 582365 ～583538 | D465 | Ar17 | D1～464,D466～1175 |
| 583539 ～584712 | D465 | Ar18 | D1～464,D466～1175 |
| 584713 ～585886 | D465 | Ar19 | D1～464,D466～1175 |
| 585887 ～587060 | D465 | Ar20 | D1～464,D466～1175 |
| 587061 ～588234 | D465 | Ar21 | D1～464,D466～1175 |
| 588235 ～589408 | D465 | Ar22 | D1～464,D466～1175 |
| 589409 ～590582 | D465 | Ar23 | D1～464,D466～1175 |
| 590583 ～591756 | D465 | Ar24 | D1～464,D466～1175 |
| 591757 ～592930 | D465 | Ar25 | D1～464,D466～1175 |
| 592931 ～594104 | D465 | Ar26 | D1～464,D466～1175 |
| 594105 ～595278 | D465 | Ar27 | D1～464,D466～1175 |
| 595279 ～596452 | D465 | Ar28 | D1～464,D466～1175 |
| 596453 ～597626 | D465 | Ar29 | D1～464,D466～1175 |
| 597627 ～598800 | D465 | Ar30 | D1～464,D466～1175 |
| 598801 ～599974 | D465 | Ar31 | D1～464,D466～1175 |
| 599975 ～601148 | D465 | Ar32 | D1～464,D466～1175 |
| 601149 ～602322 | D465 | Ar33 | D1～464,D466～1175 |
| 602323 ～603496 | D465 | Ar34 | D1～464,D466～1175 |
| 603497 ～604670 | D465 | Ar35 | D1～464,D466～1175 |
| 604671 ～605844 | D465 | Ar36 | D1～464,D466～1175 |
| 605845 ～607018 | D465 | Ar37 | D1～464,D466～1175 |
| 607019 ～608192 | D465 | Ar38 | D1～464,D466～1175 |
| 608193 ～609366 | D465 | Ar39 | D1～464,D466～1175 |
| 609367 ～610540 | D465 | Ar40 | D1～464,D466～1175 |
| 610541 ～611714 | D465 | Ar41 | D1～464,D466～1175 |
| 611715 ～612888 | D465 | Ar42 | D1～464,D466～1175 |
| 612889 ～614062 | D465 | Ar43 | D1～464,D466～1175 |
| 614063 ～615236 | D465 | Ar44 | D1～464,D466～1175 |
| 615237 ～616410 | D465 | Ar45 | D1～464,D466～1175 |
| 616411 ～617584 | D465 | Ar46 | D1～464,D466～1175 |
| 617585 ～618758 | D465 | Ar47 | D1～464,D466～1175 |
| 618759 ～619932 | D465 | Ar48 | D1～464,D466～1175 |
| 619933 ～621106 | D465 | Ar49 | D1～464,D466～1175 |
| 621107 ～622280 | D465 | Ar50 | D1～464,D466～1175 |
| 622281 ～623454 | D465 | Ar51 | D1～464,D466～1175 |
| 623455 ～624628 | D465 | Ar52 | D1～464,D466～1175 |
| 624629 ～625802 | D465 | Ar53 | D1～464,D466～1175 |
| 625803 ～626976 | D465 | Ar54 | D1～464,D466～1175 |
| 626977 ～628150 | D465 | Ar55 | D1～464,D466～1175 |
| 628151 ～629324 | D465 | Ar56 | D1～464,D466～1175 |
| 629325 ～630498 | D465 | Ar57 | D1～464,D466～1175 |
| 630499 ～631672 | D465 | Ar58 | D1～464,D466～1175 |
| 631673 ～632846 | D465 | Ar59 | D1～464,D466～1175 |
| 632847 ～634020 | D465 | H | D1～464,D466～1175 |

| No. | $R^3$ | $R^4$ | $R^5$ |
|---|---|---|---|
| 634021 ～635194 | D466 | Ar1 | D1～465,D467～1175 |
| 635195 ～636368 | D466 | Ar2 | D1～465,D467～1175 |
| 636369 ～637542 | D466 | Ar3 | D1～465,D467～1175 |
| 637543 ～638716 | D466 | Ar4 | D1～465,D467～1175 |
| 638717 ～639890 | D466 | Ar5 | D1～465,D467～1175 |
| 639891 ～641064 | D466 | Ar6 | D1～465,D467～1175 |
| 641065 ～642238 | D466 | Ar7 | D1～465,D467～1175 |
| 642239 ～643412 | D466 | Ar8 | D1～465,D467～1175 |
| 643413 ～644586 | D466 | Ar9 | D1～465,D467～1175 |
| 644587 ～645760 | D466 | Ar10 | D1～465,D467～1175 |
| 645761 ～646934 | D466 | Ar11 | D1～465,D467～1175 |
| 646935 ～648108 | D466 | Ar12 | D1～465,D467～1175 |
| 648109 ～649282 | D466 | Ar13 | D1～465,D467～1175 |
| 649283 ～650456 | D466 | Ar14 | D1～465,D467～1175 |
| 650457 ～651630 | D466 | Ar15 | D1～465,D467～1175 |
| 651631 ～652804 | D466 | Ar16 | D1～465,D467～1175 |
| 652805 ～653978 | D466 | Ar17 | D1～465,D467～1175 |
| 653979 ～655152 | D466 | Ar18 | D1～465,D467～1175 |
| 655153 ～656326 | D466 | Ar19 | D1～465,D467～1175 |
| 656327 ～657500 | D466 | Ar20 | D1～465,D467～1175 |
| 657501 ～658674 | D466 | Ar21 | D1～465,D467～1175 |
| 658675 ～659848 | D466 | Ar22 | D1～465,D467～1175 |
| 659849 ～661022 | D466 | Ar23 | D1～465,D467～1175 |
| 661023 ～662196 | D466 | Ar24 | D1～465,D467～1175 |
| 662197 ～663370 | D466 | Ar25 | D1～465,D467～1175 |
| 663371 ～664544 | D466 | Ar26 | D1～465,D467～1175 |
| 664545 ～665718 | D466 | Ar27 | D1～465,D467～1175 |
| 665719 ～666892 | D466 | Ar28 | D1～465,D467～1175 |
| 666893 ～668066 | D466 | Ar29 | D1～465,D467～1175 |
| 668067 ～669240 | D466 | Ar30 | D1～465,D467～1175 |
| 669241 ～670414 | D466 | Ar31 | D1～465,D467～1175 |
| 670415 ～671588 | D466 | Ar32 | D1～465,D467～1175 |
| 671589 ～672762 | D466 | Ar33 | D1～465,D467～1175 |
| 672763 ～673936 | D466 | Ar34 | D1～465,D467～1175 |
| 673937 ～675110 | D466 | Ar35 | D1～465,D467～1175 |
| 675111 ～676284 | D466 | Ar36 | D1～465,D467～1175 |
| 676285 ～677458 | D466 | Ar37 | D1～465,D467～1175 |
| 677459 ～678632 | D466 | Ar38 | D1～465,D467～1175 |
| 678633 ～679806 | D466 | Ar39 | D1～465,D467～1175 |
| 679807 ～680980 | D466 | Ar40 | D1～465,D467～1175 |
| 680981 ～682154 | D466 | Ar41 | D1～465,D467～1175 |
| 682155 ～683328 | D466 | Ar42 | D1～465,D467～1175 |
| 683329 ～684502 | D466 | Ar43 | D1～465,D467～1175 |
| 684503 ～685676 | D466 | Ar44 | D1～465,D467～1175 |
| 685677 ～686850 | D466 | Ar45 | D1～465,D467～1175 |
| 686851 ～688024 | D466 | Ar46 | D1～465,D467～1175 |
| 688025 ～689198 | D466 | Ar47 | D1～465,D467～1175 |
| 689199 ～690372 | D466 | Ar48 | D1～465,D467～1175 |
| 690373 ～691546 | D466 | Ar49 | D1～465,D467～1175 |
| 691547 ～692720 | D466 | Ar50 | D1～465,D467～1175 |
| 692721 ～693894 | D466 | Ar51 | D1～465,D467～1175 |
| 693895 ～695068 | D466 | Ar52 | D1～465,D467～1175 |
| 695069 ～696242 | D466 | Ar53 | D1～465,D467～1175 |
| 696243 ～697416 | D466 | Ar54 | D1～465,D467～1175 |
| 697417 ～698590 | D466 | Ar55 | D1～465,D467～1175 |
| 698591 ～699764 | D466 | Ar56 | D1～465,D467～1175 |
| 699765 ～700938 | D466 | Ar57 | D1～465,D467～1175 |
| 700939 ～702112 | D466 | Ar58 | D1～465,D467～1175 |
| 702113 ～703286 | D466 | Ar59 | D1～465,D467～1175 |
| 703287 ～704460 | D466 | H | D1～465,D467～1175 |
| 704461 ～705634 | D467 | Ar1 | D1～466,D468～1175 |
| 705635 ～706808 | D467 | Ar2 | D1～466,D468～1175 |
| 706809 ～707982 | D467 | Ar3 | D1～466,D468～1175 |
| 707983 ～709156 | D467 | Ar4 | D1～466,D468～1175 |
| 709157 ～710330 | D467 | Ar5 | D1～466,D468～1175 |
| 710331 ～711504 | D467 | Ar6 | D1～466,D468～1175 |
| 711505 ～712678 | D467 | Ar7 | D1～466,D468～1175 |
| 712679 ～713852 | D467 | Ar8 | D1～466,D468～1175 |
| 713853 ～715026 | D467 | Ar9 | D1～466,D468～1175 |
| 715027 ～716200 | D467 | Ar10 | D1～466,D468～1175 |
| 716201 ～717374 | D467 | Ar11 | D1～466,D468～1175 |
| 717375 ～718548 | D467 | Ar12 | D1～466,D468～1175 |
| 718549 ～719722 | D467 | Ar13 | D1～466,D468～1175 |
| 719723 ～720896 | D467 | Ar14 | D1～466,D468～1175 |
| 720897 ～722070 | D467 | Ar15 | D1～466,D468～1175 |
| 722071 ～723244 | D467 | Ar16 | D1～466,D468～1175 |
| 723245 ～724418 | D467 | Ar17 | D1～466,D468～1175 |
| 724419 ～725592 | D467 | Ar18 | D1～466,D468～1175 |
| 725593 ～726766 | D467 | Ar19 | D1～466,D468～1175 |
| 726767 ～727940 | D467 | Ar20 | D1～466,D468～1175 |
| 727941 ～729114 | D467 | Ar21 | D1～466,D468～1175 |
| 729115 ～730288 | D467 | Ar22 | D1～466,D468～1175 |
| 730289 ～731462 | D467 | Ar23 | D1～466,D468～1175 |
| 731463 ～732636 | D467 | Ar24 | D1～466,D468～1175 |
| 732637 ～733810 | D467 | Ar25 | D1～466,D468～1175 |
| 733811 ～734984 | D467 | Ar26 | D1～466,D468～1175 |
| 734985 ～736158 | D467 | Ar27 | D1～466,D468～1175 |
| 736159 ～737332 | D467 | Ar28 | D1～466,D468～1175 |
| 737333 ～738506 | D467 | Ar29 | D1～466,D468～1175 |
| 738507 ～739680 | D467 | Ar30 | D1～466,D468～1175 |
| 739681 ～740854 | D467 | Ar31 | D1～466,D468～1175 |
| 740855 ～742028 | D467 | Ar32 | D1～466,D468～1175 |
| 742029 ～743202 | D467 | Ar33 | D1～466,D468～1175 |
| 743203 ～744376 | D467 | Ar34 | D1～466,D468～1175 |
| 744377 ～745550 | D467 | Ar35 | D1～466,D468～1175 |
| 745551 ～746724 | D467 | Ar36 | D1～466,D468～1175 |
| 746725 ～747898 | D467 | Ar37 | D1～466,D468～1175 |
| 747899 ～749072 | D467 | Ar38 | D1～466,D468～1175 |
| 749073 ～750246 | D467 | Ar39 | D1～466,D468～1175 |
| 750247 ～751420 | D467 | Ar40 | D1～466,D468～1175 |
| 751421 ～752594 | D467 | Ar41 | D1～466,D468～1175 |
| 752595 ～753768 | D467 | Ar42 | D1～466,D468～1175 |
| 753769 ～754942 | D467 | Ar43 | D1～466,D468～1175 |
| 754943 ～756116 | D467 | Ar44 | D1～466,D468～1175 |
| 756117 ～757290 | D467 | Ar45 | D1～466,D468～1175 |
| 757291 ～758464 | D467 | Ar46 | D1～466,D468～1175 |
| 758465 ～759638 | D467 | Ar47 | D1～466,D468～1175 |
| 759639 ～760812 | D467 | Ar48 | D1～466,D468～1175 |
| 760813 ～761986 | D467 | Ar49 | D1～466,D468～1175 |
| 761987 ～763160 | D467 | Ar50 | D1～466,D468～1175 |
| 763161 ～764334 | D467 | Ar51 | D1～466,D468～1175 |
| 764335 ～765508 | D467 | Ar52 | D1～466,D468～1175 |
| 765509 ～766682 | D467 | Ar53 | D1～466,D468～1175 |
| 766683 ～767856 | D467 | Ar54 | D1～466,D468～1175 |
| 767857 ～769030 | D467 | Ar55 | D1～466,D468～1175 |
| 769031 ～770204 | D467 | Ar56 | D1～466,D468～1175 |
| 770205 ～771378 | D467 | Ar57 | D1～466,D468～1175 |
| 771379 ～772552 | D467 | Ar58 | D1～466,D468～1175 |
| 772553 ～773726 | D467 | Ar59 | D1～466,D468～1175 |
| 773727 ～774900 | D467 | H | D1～466,D468～1175 |

| No. | $R^3$ | $R^4$ | $R^5$ |
|---|---|---|---|
| 774901 ~ 776074 | D486 | Ar1 | D1~485,D487~1175 |
| 776075 ~ 777248 | D486 | Ar2 | D1~485,D487~1175 |
| 777249 ~ 778422 | D486 | Ar3 | D1~485,D487~1175 |
| 778423 ~ 779596 | D486 | Ar4 | D1~485,D487~1175 |
| 779597 ~ 780770 | D486 | Ar5 | D1~485,D487~1175 |
| 780771 ~ 781944 | D486 | Ar6 | D1~485,D487~1175 |
| 781945 ~ 783118 | D486 | Ar7 | D1~485,D487~1175 |
| 783119 ~ 784292 | D486 | Ar8 | D1~485,D487~1175 |
| 784293 ~ 785466 | D486 | Ar9 | D1~485,D487~1175 |
| 785467 ~ 786640 | D486 | Ar10 | D1~485,D487~1175 |
| 786641 ~ 787814 | D486 | Ar11 | D1~485,D487~1175 |
| 787815 ~ 788988 | D486 | Ar12 | D1~485,D487~1175 |
| 788989 ~ 790162 | D486 | Ar13 | D1~485,D487~1175 |
| 790163 ~ 791336 | D486 | Ar14 | D1~485,D487~1175 |
| 791337 ~ 792510 | D486 | Ar15 | D1~485,D487~1175 |
| 792511 ~ 793684 | D486 | Ar16 | D1~485,D487~1175 |
| 793685 ~ 794858 | D486 | Ar17 | D1~485,D487~1175 |
| 794859 ~ 796032 | D486 | Ar18 | D1~485,D487~1175 |
| 796033 ~ 797206 | D486 | Ar19 | D1~485,D487~1175 |
| 797207 ~ 798380 | D486 | Ar20 | D1~485,D487~1175 |
| 798381 ~ 799554 | D486 | Ar21 | D1~485,D487~1175 |
| 799555 ~ 800728 | D486 | Ar22 | D1~485,D487~1175 |
| 800729 ~ 801902 | D486 | Ar23 | D1~485,D487~1175 |
| 801903 ~ 803076 | D486 | Ar24 | D1~485,D487~1175 |
| 803077 ~ 804250 | D486 | Ar25 | D1~485,D487~1175 |
| 804251 ~ 805424 | D486 | Ar26 | D1~485,D487~1175 |
| 805425 ~ 806598 | D486 | Ar27 | D1~485,D487~1175 |
| 806599 ~ 807772 | D486 | Ar28 | D1~485,D487~1175 |
| 807773 ~ 808946 | D486 | Ar29 | D1~485,D487~1175 |
| 808947 ~ 810120 | D486 | Ar30 | D1~485,D487~1175 |
| 810121 ~ 811294 | D486 | Ar31 | D1~485,D487~1175 |
| 811295 ~ 812468 | D486 | Ar32 | D1~485,D487~1175 |
| 812469 ~ 813642 | D486 | Ar33 | D1~485,D487~1175 |
| 813643 ~ 814816 | D486 | Ar34 | D1~485,D487~1175 |
| 814817 ~ 815990 | D486 | Ar35 | D1~485,D487~1175 |
| 815991 ~ 817164 | D486 | Ar36 | D1~485,D487~1175 |
| 817165 ~ 818338 | D486 | Ar37 | D1~485,D487~1175 |
| 818339 ~ 819512 | D486 | Ar38 | D1~485,D487~1175 |
| 819513 ~ 820686 | D486 | Ar39 | D1~485,D487~1175 |
| 820687 ~ 821860 | D486 | Ar40 | D1~485,D487~1175 |
| 821861 ~ 823034 | D486 | Ar41 | D1~485,D487~1175 |
| 823035 ~ 824208 | D486 | Ar42 | D1~485,D487~1175 |
| 824209 ~ 825382 | D486 | Ar43 | D1~485,D487~1175 |
| 825383 ~ 826556 | D486 | Ar44 | D1~485,D487~1175 |
| 826557 ~ 827730 | D486 | Ar45 | D1~485,D487~1175 |
| 827731 ~ 828904 | D486 | Ar46 | D1~485,D487~1175 |
| 828905 ~ 830078 | D486 | Ar47 | D1~485,D487~1175 |
| 830079 ~ 831252 | D486 | Ar48 | D1~485,D487~1175 |
| 831253 ~ 832426 | D486 | Ar49 | D1~485,D487~1175 |
| 832427 ~ 833600 | D486 | Ar50 | D1~485,D487~1175 |
| 833601 ~ 834774 | D486 | Ar51 | D1~485,D487~1175 |
| 834775 ~ 835948 | D486 | Ar52 | D1~485,D487~1175 |
| 835949 ~ 837122 | D486 | Ar53 | D1~485,D487~1175 |
| 837123 ~ 838296 | D486 | Ar54 | D1~485,D487~1175 |
| 838297 ~ 839470 | D486 | Ar55 | D1~485,D487~1175 |
| 839471 ~ 840644 | D486 | Ar56 | D1~485,D487~1175 |
| 840645 ~ 841818 | D486 | Ar57 | D1~485,D487~1175 |
| 841819 ~ 842992 | D486 | Ar58 | D1~485,D487~1175 |
| 842993 ~ 844166 | D486 | Ar59 | D1~485,D487~1175 |
| 844167 ~ 845340 | D486 | H | D1~485,D487~1175 |

| No. | $R^3$ | $R^4$ | $R^5$ |
|---|---|---|---|
| 845341 ~ 846514 | D717 | Ar1 | D1~716,D718~1175 |
| 846515 ~ 847688 | D717 | Ar2 | D1~716,D718~1175 |
| 847689 ~ 848862 | D717 | Ar3 | D1~716,D718~1175 |
| 848863 ~ 850036 | D717 | Ar4 | D1~716,D718~1175 |
| 850037 ~ 851210 | D717 | Ar5 | D1~716,D718~1175 |
| 851211 ~ 852384 | D717 | Ar6 | D1~716,D718~1175 |
| 852385 ~ 853558 | D717 | Ar7 | D1~716,D718~1175 |
| 853559 ~ 854732 | D717 | Ar8 | D1~716,D718~1175 |
| 854733 ~ 855906 | D717 | Ar9 | D1~716,D718~1175 |
| 855907 ~ 857080 | D717 | Ar10 | D1~716,D718~1175 |
| 857081 ~ 858254 | D717 | Ar11 | D1~716,D718~1175 |
| 858255 ~ 859428 | D717 | Ar12 | D1~716,D718~1175 |
| 859429 ~ 860602 | D717 | Ar13 | D1~716,D718~1175 |
| 860603 ~ 861776 | D717 | Ar14 | D1~716,D718~1175 |
| 861777 ~ 862950 | D717 | Ar15 | D1~716,D718~1175 |
| 862951 ~ 864124 | D717 | Ar16 | D1~716,D718~1175 |
| 864125 ~ 865298 | D717 | Ar17 | D1~716,D718~1175 |
| 865299 ~ 866472 | D717 | Ar18 | D1~716,D718~1175 |
| 866473 ~ 867646 | D717 | Ar19 | D1~716,D718~1175 |
| 867647 ~ 868820 | D717 | Ar20 | D1~716,D718~1175 |
| 868821 ~ 869994 | D717 | Ar21 | D1~716,D718~1175 |
| 869995 ~ 871168 | D717 | Ar22 | D1~716,D718~1175 |
| 871169 ~ 872342 | D717 | Ar23 | D1~716,D718~1175 |
| 872343 ~ 873516 | D717 | Ar24 | D1~716,D718~1175 |
| 873517 ~ 874690 | D717 | Ar25 | D1~716,D718~1175 |
| 874691 ~ 875864 | D717 | Ar26 | D1~716,D718~1175 |
| 875865 ~ 877038 | D717 | Ar27 | D1~716,D718~1175 |
| 877039 ~ 878212 | D717 | Ar28 | D1~716,D718~1175 |
| 878213 ~ 879386 | D717 | Ar29 | D1~716,D718~1175 |
| 879387 ~ 880560 | D717 | Ar30 | D1~716,D718~1175 |
| 880561 ~ 881734 | D717 | Ar31 | D1~716,D718~1175 |
| 881735 ~ 882908 | D717 | Ar32 | D1~716,D718~1175 |
| 882909 ~ 884082 | D717 | Ar33 | D1~716,D718~1175 |
| 884083 ~ 885256 | D717 | Ar34 | D1~716,D718~1175 |
| 885257 ~ 886430 | D717 | Ar35 | D1~716,D718~1175 |
| 886431 ~ 887604 | D717 | Ar36 | D1~716,D718~1175 |
| 887605 ~ 888778 | D717 | Ar37 | D1~716,D718~1175 |
| 888779 ~ 889952 | D717 | Ar38 | D1~716,D718~1175 |
| 889953 ~ 891126 | D717 | Ar39 | D1~716,D718~1175 |
| 891127 ~ 892300 | D717 | Ar40 | D1~716,D718~1175 |
| 892301 ~ 893474 | D717 | Ar41 | D1~716,D718~1175 |
| 893475 ~ 894648 | D717 | Ar42 | D1~716,D718~1175 |
| 894649 ~ 895822 | D717 | Ar43 | D1~716,D718~1175 |
| 895823 ~ 896996 | D717 | Ar44 | D1~716,D718~1175 |
| 896997 ~ 898170 | D717 | Ar45 | D1~716,D718~1175 |
| 898171 ~ 899344 | D717 | Ar46 | D1~716,D718~1175 |
| 899345 ~ 900518 | D717 | Ar47 | D1~716,D718~1175 |
| 900519 ~ 901692 | D717 | Ar48 | D1~716,D718~1175 |
| 901693 ~ 902866 | D717 | Ar49 | D1~716,D718~1175 |
| 902867 ~ 904040 | D717 | Ar50 | D1~716,D718~1175 |
| 904041 ~ 905214 | D717 | Ar51 | D1~716,D718~1175 |
| 905215 ~ 906388 | D717 | Ar52 | D1~716,D718~1175 |
| 906389 ~ 907562 | D717 | Ar53 | D1~716,D718~1175 |
| 907563 ~ 908736 | D717 | Ar54 | D1~716,D718~1175 |
| 908737 ~ 909910 | D717 | Ar55 | D1~716,D718~1175 |
| 909911 ~ 911084 | D717 | Ar56 | D1~716,D718~1175 |
| 911085 ~ 912258 | D717 | Ar57 | D1~716,D718~1175 |
| 912259 ~ 913432 | D717 | Ar58 | D1~716,D718~1175 |
| 913433 ~ 914606 | D717 | Ar59 | D1~716,D718~1175 |
| 914607 ~ 915780 | D717 | H | D1~716,D718~1175 |

| No. | $R^3$ | $R^4$ | $R^5$ | = |
|---|---|---|---|---|
| 915781 ~ 916955 | D1~D1175 | D1 | D1~D1175 | $R^3 = R^5$ |
| 916956 ~ 918130 | D1~D1175 | D7 | D1~D1175 | |
| 918131 ~ 919305 | D1~D1175 | D8 | D1~D1175 | |
| 919306 ~ 920480 | D1~D1175 | D9 | D1~D1175 | |
| 920481 ~ 921655 | D1~D1175 | D19 | D1~D1175 | |
| 921656 ~ 922830 | D1~D1175 | D37 | D1~D1175 | |
| 922831 ~ 924005 | D1~D1175 | D50 | D1~D1175 | |
| 924006 ~ 925180 | D1~D1175 | D67 | D1~D1175 | |
| 925181 ~ 926355 | D1~D1175 | D77 | D1~D1175 | |
| 926356 ~ 927530 | D1~D1175 | D78 | D1~D1175 | |
| 927531 ~ 928705 | D1~D1175 | D79 | D1~D1175 | |
| 928706 ~ 929880 | D1~D1175 | D80 | D1~D1175 | |
| 929881 ~ 931055 | D1~D1175 | D81 | D1~D1175 | |
| 931056 ~ 932230 | D1~D1175 | D465 | D1~D1175 | |
| 932231 ~ 933405 | D1~D1175 | D466 | D1~D1175 | |
| 933406 ~ 934580 | D1~D1175 | D467 | D1~D1175 | |
| 934581 ~ 935755 | D1~D1175 | D471 | D1~D1175 | |
| 935756 ~ 936930 | D1~D1175 | D486 | D1~D1175 | |
| 936931 ~ 938105 | D1~D1175 | D717 | D1~D1175 | |
| 938106 ~ 939280 | D1~D1175 | D735 | D1~D1175 | |
| 939281 ~ 940455 | D1~D1175 | D783 | D1~D1175 | |
| 940456 ~ 941630 | D1~D1175 | D793 | D1~D1175 | |
| 941631 ~ 942805 | D1~D1175 | D794 | D1~D1175 | |
| 942806 ~ 943980 | D1~D1175 | D795 | D1~D1175 | |
| 943981 ~ 945155 | D1~D1175 | D796 | D1~D1175 | |
| 945156 ~ 946330 | D1~D1175 | D797 | D1~D1175 | |
| 946331 ~ 947505 | H | Ar1 | D1~D1175 | |
| 947506 ~ 948680 | H | Ar2 | D1~D1175 | |
| 948681 ~ 949855 | H | Ar3 | D1~D1175 | |
| 949856 ~ 951030 | H | Ar4 | D1~D1175 | |
| 951031 ~ 952205 | H | Ar5 | D1~D1175 | |
| 952206 ~ 953380 | H | Ar6 | D1~D1175 | |
| 953381 ~ 954555 | H | Ar7 | D1~D1175 | |
| 954556 ~ 955730 | H | Ar8 | D1~D1175 | |
| 955731 ~ 956905 | H | Ar9 | D1~D1175 | |
| 956906 ~ 958080 | H | Ar10 | D1~D1175 | |
| 958081 ~ 959255 | H | Ar11 | D1~D1175 | |
| 959256 ~ 960430 | H | Ar12 | D1~D1175 | |
| 960431 ~ 961605 | H | Ar13 | D1~D1175 | |
| 961606 ~ 962780 | H | Ar14 | D1~D1175 | |
| 962781 ~ 963955 | H | Ar15 | D1~D1175 | |
| 963956 ~ 965130 | H | Ar16 | D1~D1175 | |
| 965131 ~ 966305 | H | Ar17 | D1~D1175 | |
| 966306 ~ 967480 | H | Ar18 | D1~D1175 | |
| 967481 ~ 968655 | H | Ar19 | D1~D1175 | |
| 968656 ~ 969830 | H | Ar20 | D1~D1175 | |
| 969831 ~ 971005 | H | Ar21 | D1~D1175 | |
| 971006 ~ 972180 | H | Ar22 | D1~D1175 | |
| 972181 ~ 973355 | H | Ar23 | D1~D1175 | |
| 973356 ~ 974530 | H | Ar24 | D1~D1175 | |
| 974531 ~ 975705 | H | Ar25 | D1~D1175 | |
| 975706 ~ 976880 | H | Ar26 | D1~D1175 | |
| 976881 ~ 978055 | H | Ar27 | D1~D1175 | |
| 978056 ~ 979230 | H | Ar28 | D1~D1175 | |
| 979231 ~ 980405 | H | Ar29 | D1~D1175 | |
| 980406 ~ 981580 | H | Ar30 | D1~D1175 | |
| 981581 ~ 982755 | H | Ar31 | D1~D1175 | |
| 982756 ~ 983930 | H | Ar32 | D1~D1175 | |
| 983931 ~ 985105 | H | Ar33 | D1~D1175 | |
| 985106 ~ 986280 | H | Ar34 | D1~D1175 | |

| No. | $R^3$ | $R^4$ | $R^5$ |
|---|---|---|---|
| 986281 ~ 987455 | H | Ar35 | D1~D1175 |
| 987456 ~ 988630 | H | Ar36 | D1~D1175 |
| 988631 ~ 989805 | H | Ar37 | D1~D1175 |
| 989806 ~ 990980 | H | Ar38 | D1~D1175 |
| 990981 ~ 992155 | H | Ar39 | D1~D1175 |
| 992156 ~ 993330 | H | Ar40 | D1~D1175 |
| 993331 ~ 994505 | H | Ar41 | D1~D1175 |
| 994506 ~ 995680 | H | Ar42 | D1~D1175 |
| 995681 ~ 996855 | H | Ar43 | D1~D1175 |
| 996856 ~ 998030 | H | Ar44 | D1~D1175 |
| 998031 ~ 999205 | H | Ar45 | D1~D1175 |
| 999206 ~ 1000380 | H | Ar46 | D1~D1175 |
| 1000381 ~ 1001555 | H | Ar47 | D1~D1175 |
| 1001556 ~ 1002730 | H | Ar48 | D1~D1175 |
| 1002731 ~ 1003905 | H | Ar49 | D1~D1175 |
| 1003906 ~ 1005080 | H | Ar50 | D1~D1175 |
| 1005081 ~ 1006255 | H | Ar51 | D1~D1175 |
| 1006256 ~ 1007430 | H | Ar52 | D1~D1175 |
| 1007431 ~ 1008605 | H | Ar53 | D1~D1175 |
| 1008606 ~ 1009780 | H | Ar54 | D1~D1175 |
| 1009781 ~ 1010955 | H | Ar55 | D1~D1175 |
| 1010956 ~ 1012130 | H | Ar56 | D1~D1175 |
| 1012131 ~ 1013305 | H | Ar57 | D1~D1175 |
| 1013306 ~ 1014480 | H | Ar58 | D1~D1175 |
| 1014481 ~ 1015655 | H | Ar59 | D1~D1175 |

Next, specific examples of the compound having a structure represented by the following general formula (1b) are shown in Table 3. In Table 3, structures of the compounds are shown in the same manner as in Table 2.

General Formula (1b)

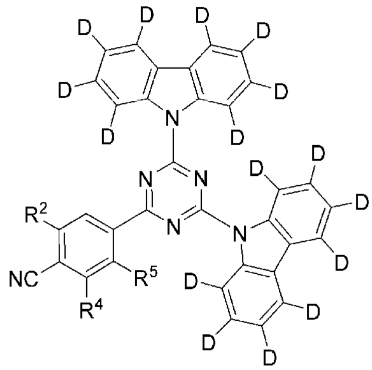

Table 3

| No. | R^2 | R^4 | R^5 | = |
|---|---|---|---|---|
| 1015656 ~ 1016830 | D1~D1175 | Ar1 | D1~D1175 | $R^2 = R^5$ |
| 1016831 ~ 1018005 | D1~D1175 | Ar2 | D1~D1175 | |
| 1018006 ~ 1019180 | D1~D1175 | Ar3 | D1~D1175 | |
| 1019181 ~ 1020355 | D1~D1175 | Ar4 | D1~D1175 | |
| 1020356 ~ 1021530 | D1~D1175 | Ar5 | D1~D1175 | |
| 1021531 ~ 1022705 | D1~D1175 | Ar6 | D1~D1175 | |
| 1022706 ~ 1023880 | D1~D1175 | Ar7 | D1~D1175 | |
| 1023881 ~ 1025055 | D1~D1175 | Ar8 | D1~D1175 | |
| 1025056 ~ 1026230 | D1~D1175 | Ar9 | D1~D1175 | |
| 1026231 ~ 1027405 | D1~D1175 | Ar10 | D1~D1175 | |
| 1027406 ~ 1028580 | D1~D1175 | Ar11 | D1~D1175 | |
| 1028581 ~ 1029755 | D1~D1175 | Ar12 | D1~D1175 | |
| 1029756 ~ 1030930 | D1~D1175 | Ar13 | D1~D1175 | |
| 1030931 ~ 1032105 | D1~D1175 | Ar14 | D1~D1175 | |
| 1032106 ~ 1033280 | D1~D1175 | Ar15 | D1~D1175 | |
| 1033281 ~ 1034455 | D1~D1175 | Ar16 | D1~D1175 | |
| 1034456 ~ 1035630 | D1~D1175 | Ar17 | D1~D1175 | |
| 1035631 ~ 1036805 | D1~D1175 | Ar18 | D1~D1175 | |
| 1036806 ~ 1037980 | D1~D1175 | Ar19 | D1~D1175 | |
| 1037981 ~ 1039155 | D1~D1175 | Ar20 | D1~D1175 | |
| 1039156 ~ 1040330 | D1~D1175 | Ar21 | D1~D1175 | |
| 1040331 ~ 1041505 | D1~D1175 | Ar22 | D1~D1175 | |
| 1041506 ~ 1042680 | D1~D1175 | Ar23 | D1~D1175 | |
| 1042681 ~ 1043855 | D1~D1175 | Ar24 | D1~D1175 | |
| 1043856 ~ 1045030 | D1~D1175 | Ar25 | D1~D1175 | |
| 1045031 ~ 1046205 | D1~D1175 | Ar26 | D1~D1175 | |
| 1046206 ~ 1047380 | D1~D1175 | Ar27 | D1~D1175 | |
| 1047381 ~ 1048555 | D1~D1175 | Ar28 | D1~D1175 | |
| 1048556 ~ 1049730 | D1~D1175 | Ar29 | D1~D1175 | |
| 1049731 ~ 1050905 | D1~D1175 | Ar30 | D1~D1175 | |
| 1050906 ~ 1052080 | D1~D1175 | Ar31 | D1~D1175 | |
| 1052081 ~ 1053255 | D1~D1175 | Ar32 | D1~D1175 | |
| 1053256 ~ 1054430 | D1~D1175 | Ar33 | D1~D1175 | |
| 1054431 ~ 1055605 | D1~D1175 | Ar34 | D1~D1175 | |
| 1055606 ~ 1056780 | D1~D1175 | Ar35 | D1~D1175 | |
| 1056781 ~ 1057955 | D1~D1175 | Ar36 | D1~D1175 | |
| 1057956 ~ 1059130 | D1~D1175 | Ar37 | D1~D1175 | |
| 1059131 ~ 1060305 | D1~D1175 | Ar38 | D1~D1175 | |
| 1060306 ~ 1061480 | D1~D1175 | Ar39 | D1~D1175 | |
| 1061481 ~ 1062655 | D1~D1175 | Ar40 | D1~D1175 | |
| 1062656 ~ 1063830 | D1~D1175 | Ar41 | D1~D1175 | |
| 1063831 ~ 1065005 | D1~D1175 | Ar42 | D1~D1175 | |
| 1065006 ~ 1066180 | D1~D1175 | Ar43 | D1~D1175 | |
| 1066181 ~ 1067355 | D1~D1175 | Ar44 | D1~D1175 | |
| 1067356 ~ 1068530 | D1~D1175 | Ar45 | D1~D1175 | |
| 1068531 ~ 1069705 | D1~D1175 | Ar46 | D1~D1175 | |
| 1069706 ~ 1070880 | D1~D1175 | Ar47 | D1~D1175 | |
| 1070881 ~ 1072055 | D1~D1175 | Ar48 | D1~D1175 | |
| 1072056 ~ 1073230 | D1~D1175 | Ar49 | D1~D1175 | |
| 1073231 ~ 1074405 | D1~D1175 | Ar50 | D1~D1175 | |
| 1074406 ~ 1075580 | D1~D1175 | Ar51 | D1~D1175 | |
| 1075581 ~ 1076755 | D1~D1175 | Ar52 | D1~D1175 | |
| 1076756 ~ 1077930 | D1~D1175 | Ar53 | D1~D1175 | |
| 1077931 ~ 1079105 | D1~D1175 | Ar54 | D1~D1175 | |
| 1079106 ~ 1080280 | D1~D1175 | Ar55 | D1~D1175 | |
| 1080281 ~ 1081455 | D1~D1175 | Ar56 | D1~D1175 | |
| 1081456 ~ 1082630 | D1~D1175 | Ar57 | D1~D1175 | |
| 1082631 ~ 1083805 | D1~D1175 | Ar58 | D1~D1175 | |
| 1083806 ~ 1084980 | D1~D1175 | Ar59 | D1~D1175 | |
| 1084981 ~ 1086155 | D1~D1175 | H | D1~D1175 | |

| No. | $R^2$ | $R^4$ | $R^5$ |
|---|---|---|---|
| 1086156 ～ 1087329 | D1 | Ar1 | D2～D1175 |
| 1087330 ～ 1088503 | D1 | Ar2 | D2～D1175 |
| 1088504 ～ 1089677 | D1 | Ar3 | D2～D1175 |
| 1089678 ～ 1090851 | D1 | Ar4 | D2～D1175 |
| 1090852 ～ 1092025 | D1 | Ar5 | D2～D1175 |
| 1092026 ～ 1093199 | D1 | Ar6 | D2～D1175 |
| 1093200 ～ 1094373 | D1 | Ar7 | D2～D1175 |
| 1094374 ～ 1095547 | D1 | Ar8 | D2～D1175 |
| 1095548 ～ 1096721 | D1 | Ar9 | D2～D1175 |
| 1096722 ～ 1097895 | D1 | Ar10 | D2～D1175 |
| 1097896 ～ 1099069 | D1 | Ar11 | D2～D1175 |
| 1099070 ～ 1100243 | D1 | Ar12 | D2～D1175 |
| 1100244 ～ 1101417 | D1 | Ar13 | D2～D1175 |
| 1101418 ～ 1102591 | D1 | Ar14 | D2～D1175 |
| 1102592 ～ 1103765 | D1 | Ar15 | D2～D1175 |
| 1103766 ～ 1104939 | D1 | Ar16 | D2～D1175 |
| 1104940 ～ 1106113 | D1 | Ar17 | D2～D1175 |
| 1106114 ～ 1107287 | D1 | Ar18 | D2～D1175 |
| 1107288 ～ 1108461 | D1 | Ar19 | D2～D1175 |
| 1108462 ～ 1109635 | D1 | Ar20 | D2～D1175 |
| 1109636 ～ 1110809 | D1 | Ar21 | D2～D1175 |
| 1110810 ～ 1111983 | D1 | Ar22 | D2～D1175 |
| 1111984 ～ 1113157 | D1 | Ar23 | D2～D1175 |
| 1113158 ～ 1114331 | D1 | Ar24 | D2～D1175 |
| 1114332 ～ 1115505 | D1 | Ar25 | D2～D1175 |
| 1115506 ～ 1116679 | D1 | Ar26 | D2～D1175 |
| 1116680 ～ 1117853 | D1 | Ar27 | D2～D1175 |
| 1117854 ～ 1119027 | D1 | Ar28 | D2～D1175 |
| 1119028 ～ 1120201 | D1 | Ar29 | D2～D1175 |
| 1120202 ～ 1121375 | D1 | Ar30 | D2～D1175 |
| 1121376 ～ 1122549 | D1 | Ar31 | D2～D1175 |
| 1122550 ～ 1123723 | D1 | Ar32 | D2～D1175 |
| 1123724 ～ 1124897 | D1 | Ar33 | D2～D1175 |
| 1124898 ～ 1126071 | D1 | Ar34 | D2～D1175 |
| 1126072 ～ 1127245 | D1 | Ar35 | D2～D1175 |
| 1127246 ～ 1128419 | D1 | Ar36 | D2～D1175 |
| 1128420 ～ 1129593 | D1 | Ar37 | D2～D1175 |
| 1129594 ～ 1130767 | D1 | Ar38 | D2～D1175 |
| 1130768 ～ 1131941 | D1 | Ar39 | D2～D1175 |
| 1131942 ～ 1133115 | D1 | Ar40 | D2～D1175 |
| 1133116 ～ 1134289 | D1 | Ar41 | D2～D1175 |
| 1134290 ～ 1135463 | D1 | Ar42 | D2～D1175 |
| 1135464 ～ 1136637 | D1 | Ar43 | D2～D1175 |
| 1136638 ～ 1137811 | D1 | Ar44 | D2～D1175 |
| 1137812 ～ 1138985 | D1 | Ar45 | D2～D1175 |
| 1138986 ～ 1140159 | D1 | Ar46 | D2～D1175 |
| 1140160 ～ 1141333 | D1 | Ar47 | D2～D1175 |
| 1141334 ～ 1142507 | D1 | Ar48 | D2～D1175 |
| 1142508 ～ 1143681 | D1 | Ar49 | D2～D1175 |
| 1143682 ～ 1144855 | D1 | Ar50 | D2～D1175 |
| 1144856 ～ 1146029 | D1 | Ar51 | D2～D1175 |
| 1146030 ～ 1147203 | D1 | Ar52 | D2～D1175 |
| 1147204 ～ 1148377 | D1 | Ar53 | D2～D1175 |
| 1148378 ～ 1149551 | D1 | Ar54 | D2～D1175 |
| 1149552 ～ 1150725 | D1 | Ar55 | D2～D1175 |
| 1150726 ～ 1151899 | D1 | Ar56 | D2～D1175 |
| 1151900 ～ 1153073 | D1 | Ar57 | D2～D1175 |
| 1153074 ～ 1154247 | D1 | Ar58 | D2～D1175 |
| 1154248 ～ 1155421 | D1 | Ar59 | D2～D1175 |
| 1155422 ～ 1156595 | D1 | H | D2～D1175 |

| No. | $R^2$ | $R^4$ | $R^5$ |
|---|---|---|---|
| 1156596 ～ 1157769 | D7 | Ar1 | D1～6,D8～1175 |
| 1157770 ～ 1158943 | D7 | Ar2 | D1～6,D8～1175 |
| 1158944 ～ 1160117 | D7 | Ar3 | D1～6,D8～1175 |
| 1160118 ～ 1161291 | D7 | Ar4 | D1～6,D8～1175 |
| 1161292 ～ 1162465 | D7 | Ar5 | D1～6,D8～1175 |
| 1162466 ～ 1163639 | D7 | Ar6 | D1～6,D8～1175 |
| 1163640 ～ 1164813 | D7 | Ar7 | D1～6,D8～1175 |
| 1164814 ～ 1165987 | D7 | Ar8 | D1～6,D8～1175 |
| 1165988 ～ 1167161 | D7 | Ar9 | D1～6,D8～1175 |
| 1167162 ～ 1168335 | D7 | Ar10 | D1～6,D8～1175 |
| 1168336 ～ 1169509 | D7 | Ar11 | D1～6,D8～1175 |
| 1169510 ～ 1170683 | D7 | Ar12 | D1～6,D8～1175 |
| 1170684 ～ 1171857 | D7 | Ar13 | D1～6,D8～1175 |
| 1171858 ～ 1173031 | D7 | Ar14 | D1～6,D8～1175 |
| 1173032 ～ 1174205 | D7 | Ar15 | D1～6,D8～1175 |
| 1174206 ～ 1175379 | D7 | Ar16 | D1～6,D8～1175 |
| 1175380 ～ 1176553 | D7 | Ar17 | D1～6,D8～1175 |
| 1176554 ～ 1177727 | D7 | Ar18 | D1～6,D8～1175 |
| 1177728 ～ 1178901 | D7 | Ar19 | D1～6,D8～1175 |
| 1178902 ～ 1180075 | D7 | Ar20 | D1～6,D8～1175 |
| 1180076 ～ 1181249 | D7 | Ar21 | D1～6,D8～1175 |
| 1181250 ～ 1182423 | D7 | Ar22 | D1～6,D8～1175 |
| 1182424 ～ 1183597 | D7 | Ar23 | D1～6,D8～1175 |
| 1183598 ～ 1184771 | D7 | Ar24 | D1～6,D8～1175 |
| 1184772 ～ 1185945 | D7 | Ar25 | D1～6,D8～1175 |
| 1185946 ～ 1187119 | D7 | Ar26 | D1～6,D8～1175 |
| 1187120 ～ 1188293 | D7 | Ar27 | D1～6,D8～1175 |
| 1188294 ～ 1189467 | D7 | Ar28 | D1～6,D8～1175 |
| 1189468 ～ 1190641 | D7 | Ar29 | D1～6,D8～1175 |
| 1190642 ～ 1191815 | D7 | Ar30 | D1～6,D8～1175 |
| 1191816 ～ 1192989 | D7 | Ar31 | D1～6,D8～1175 |
| 1192990 ～ 1194163 | D7 | Ar32 | D1～6,D8～1175 |
| 1194164 ～ 1195337 | D7 | Ar33 | D1～6,D8～1175 |
| 1195338 ～ 1196511 | D7 | Ar34 | D1～6,D8～1175 |
| 1196512 ～ 1197685 | D7 | Ar35 | D1～6,D8～1175 |
| 1197686 ～ 1198859 | D7 | Ar36 | D1～6,D8～1175 |
| 1198860 ～ 1200033 | D7 | Ar37 | D1～6,D8～1175 |
| 1200034 ～ 1201207 | D7 | Ar38 | D1～6,D8～1175 |
| 1201208 ～ 1202381 | D7 | Ar39 | D1～6,D8～1175 |
| 1202382 ～ 1203555 | D7 | Ar40 | D1～6,D8～1175 |
| 1203556 ～ 1204729 | D7 | Ar41 | D1～6,D8～1175 |
| 1204730 ～ 1205903 | D7 | Ar42 | D1～6,D8～1175 |
| 1205904 ～ 1207077 | D7 | Ar43 | D1～6,D8～1175 |
| 1207078 ～ 1208251 | D7 | Ar44 | D1～6,D8～1175 |
| 1208252 ～ 1209425 | D7 | Ar45 | D1～6,D8～1175 |
| 1209426 ～ 1210599 | D7 | Ar46 | D1～6,D8～1175 |
| 1210600 ～ 1211773 | D7 | Ar47 | D1～6,D8～1175 |
| 1211774 ～ 1212947 | D7 | Ar48 | D1～6,D8～1175 |
| 1212948 ～ 1214121 | D7 | Ar49 | D1～6,D8～1175 |
| 1214122 ～ 1215295 | D7 | Ar50 | D1～6,D8～1175 |
| 1215296 ～ 1216469 | D7 | Ar51 | D1～6,D8～1175 |
| 1216470 ～ 1217643 | D7 | Ar52 | D1～6,D8～1175 |
| 1217644 ～ 1218817 | D7 | Ar53 | D1～6,D8～1175 |
| 1218818 ～ 1219991 | D7 | Ar54 | D1～6,D8～1175 |
| 1219992 ～ 1221165 | D7 | Ar55 | D1～6,D8～1175 |
| 1221166 ～ 1222339 | D7 | Ar56 | D1～6,D8～1175 |
| 1222340 ～ 1223513 | D7 | Ar57 | D1～6,D8～1175 |
| 1223514 ～ 1224687 | D7 | Ar58 | D1～6,D8～1175 |
| 1224688 ～ 1225861 | D7 | Ar59 | D1～6,D8～1175 |
| 1225862 ～ 1227035 | D7 | H | D1～6,D8～1175 |

| No. | $R^2$ | $R^4$ | $R^5$ |
|---|---|---|---|
| 1227036 ~ 1228209 | D8 | Ar1 | D1~7,D9~1175 |
| 1228210 ~ 1229383 | D8 | Ar2 | D1~7,D9~1175 |
| 1229384 ~ 1230557 | D8 | Ar3 | D1~7,D9~1175 |
| 1230558 ~ 1231731 | D8 | Ar4 | D1~7,D9~1175 |
| 1231732 ~ 1232905 | D8 | Ar5 | D1~7,D9~1175 |
| 1232906 ~ 1234079 | D8 | Ar6 | D1~7,D9~1175 |
| 1234080 ~ 1235253 | D8 | Ar7 | D1~7,D9~1175 |
| 1235254 ~ 1236427 | D8 | Ar8 | D1~7,D9~1175 |
| 1236428 ~ 1237601 | D8 | Ar9 | D1~7,D9~1175 |
| 1237602 ~ 1238775 | D8 | Ar10 | D1~7,D9~1175 |
| 1238776 ~ 1239949 | D8 | Ar11 | D1~7,D9~1175 |
| 1239950 ~ 1241123 | D8 | Ar12 | D1~7,D9~1175 |
| 1241124 ~ 1242297 | D8 | Ar13 | D1~7,D9~1175 |
| 1242298 ~ 1243471 | D8 | Ar14 | D1~7,D9~1175 |
| 1243472 ~ 1244645 | D8 | Ar15 | D1~7,D9~1175 |
| 1244646 ~ 1245819 | D8 | Ar16 | D1~7,D9~1175 |
| 1245820 ~ 1246993 | D8 | Ar17 | D1~7,D9~1175 |
| 1246994 ~ 1248167 | D8 | Ar18 | D1~7,D9~1175 |
| 1248168 ~ 1249341 | D8 | Ar19 | D1~7,D9~1175 |
| 1249342 ~ 1250515 | D8 | Ar20 | D1~7,D9~1175 |
| 1250516 ~ 1251689 | D8 | Ar21 | D1~7,D9~1175 |
| 1251690 ~ 1252863 | D8 | Ar22 | D1~7,D9~1175 |
| 1252864 ~ 1254037 | D8 | Ar23 | D1~7,D9~1175 |
| 1254038 ~ 1255211 | D8 | Ar24 | D1~7,D9~1175 |
| 1255212 ~ 1256385 | D8 | Ar25 | D1~7,D9~1175 |
| 1256386 ~ 1257559 | D8 | Ar26 | D1~7,D9~1175 |
| 1257560 ~ 1258733 | D8 | Ar27 | D1~7,D9~1175 |
| 1258734 ~ 1259907 | D8 | Ar28 | D1~7,D9~1175 |
| 1259908 ~ 1261081 | D8 | Ar29 | D1~7,D9~1175 |
| 1261082 ~ 1262255 | D8 | Ar30 | D1~7,D9~1175 |
| 1262256 ~ 1263429 | D8 | Ar31 | D1~7,D9~1175 |
| 1263430 ~ 1264603 | D8 | Ar32 | D1~7,D9~1175 |
| 1264604 ~ 1265777 | D8 | Ar33 | D1~7,D9~1175 |
| 1265778 ~ 1266951 | D8 | Ar34 | D1~7,D9~1175 |
| 1266952 ~ 1268125 | D8 | Ar35 | D1~7,D9~1175 |
| 1268126 ~ 1269299 | D8 | Ar36 | D1~7,D9~1175 |
| 1269300 ~ 1270473 | D8 | Ar37 | D1~7,D9~1175 |
| 1270474 ~ 1271647 | D8 | Ar38 | D1~7,D9~1175 |
| 1271648 ~ 1272821 | D8 | Ar39 | D1~7,D9~1175 |
| 1272822 ~ 1273995 | D8 | Ar40 | D1~7,D9~1175 |
| 1273996 ~ 1275169 | D8 | Ar41 | D1~7,D9~1175 |
| 1275170 ~ 1276343 | D8 | Ar42 | D1~7,D9~1175 |
| 1276344 ~ 1277517 | D8 | Ar43 | D1~7,D9~1175 |
| 1277518 ~ 1278691 | D8 | Ar44 | D1~7,D9~1175 |
| 1278692 ~ 1279865 | D8 | Ar45 | D1~7,D9~1175 |
| 1279866 ~ 1281039 | D8 | Ar46 | D1~7,D9~1175 |
| 1281040 ~ 1282213 | D8 | Ar47 | D1~7,D9~1175 |
| 1282214 ~ 1283387 | D8 | Ar48 | D1~7,D9~1175 |
| 1283388 ~ 1284561 | D8 | Ar49 | D1~7,D9~1175 |
| 1284562 ~ 1285735 | D8 | Ar50 | D1~7,D9~1175 |
| 1285736 ~ 1286909 | D8 | Ar51 | D1~7,D9~1175 |
| 1286910 ~ 1288083 | D8 | Ar52 | D1~7,D9~1175 |
| 1288084 ~ 1289257 | D8 | Ar53 | D1~7,D9~1175 |
| 1289258 ~ 1290431 | D8 | Ar54 | D1~7,D9~1175 |
| 1290432 ~ 1291605 | D8 | Ar55 | D1~7,D9~1175 |
| 1291606 ~ 1292779 | D8 | Ar56 | D1~7,D9~1175 |
| 1292780 ~ 1293953 | D8 | Ar57 | D1~7,D9~1175 |
| 1293954 ~ 1295127 | D8 | Ar58 | D1~7,D9~1175 |
| 1295128 ~ 1296301 | D8 | Ar59 | D1~7,D9~1175 |
| 1296302 ~ 1297475 | D8 | H | D1~7,D9~1175 |

| No. | $R^2$ | $R^4$ | $R^5$ |
|---|---|---|---|
| 1297476 ~ 1298649 | D9 | Ar1 | D1~8,D10~1175 |
| 1298650 ~ 1299823 | D9 | Ar2 | D1~8,D10~1175 |
| 1299824 ~ 1300997 | D9 | Ar3 | D1~8,D10~1175 |
| 1300998 ~ 1302171 | D9 | Ar4 | D1~8,D10~1175 |
| 1302172 ~ 1303345 | D9 | Ar5 | D1~8,D10~1175 |
| 1303346 ~ 1304519 | D9 | Ar6 | D1~8,D10~1175 |
| 1304520 ~ 1305693 | D9 | Ar7 | D1~8,D10~1175 |
| 1305694 ~ 1306867 | D9 | Ar8 | D1~8,D10~1175 |
| 1306868 ~ 1308041 | D9 | Ar9 | D1~8,D10~1175 |
| 1308042 ~ 1309215 | D9 | Ar10 | D1~8,D10~1175 |
| 1309216 ~ 1310389 | D9 | Ar11 | D1~8,D10~1175 |
| 1310390 ~ 1311563 | D9 | Ar12 | D1~8,D10~1175 |
| 1311564 ~ 1312737 | D9 | Ar13 | D1~8,D10~1175 |
| 1312738 ~ 1313911 | D9 | Ar14 | D1~8,D10~1175 |
| 1313912 ~ 1315085 | D9 | Ar15 | D1~8,D10~1175 |
| 1315086 ~ 1316259 | D9 | Ar16 | D1~8,D10~1175 |
| 1316260 ~ 1317433 | D9 | Ar17 | D1~8,D10~1175 |
| 1317434 ~ 1318607 | D9 | Ar18 | D1~8,D10~1175 |
| 1318608 ~ 1319781 | D9 | Ar19 | D1~8,D10~1175 |
| 1319782 ~ 1320955 | D9 | Ar20 | D1~8,D10~1175 |
| 1320956 ~ 1322129 | D9 | Ar21 | D1~8,D10~1175 |
| 1322130 ~ 1323303 | D9 | Ar22 | D1~8,D10~1175 |
| 1323304 ~ 1324477 | D9 | Ar23 | D1~8,D10~1175 |
| 1324478 ~ 1325651 | D9 | Ar24 | D1~8,D10~1175 |
| 1325652 ~ 1326825 | D9 | Ar25 | D1~8,D10~1175 |
| 1326826 ~ 1327999 | D9 | Ar26 | D1~8,D10~1175 |
| 1328000 ~ 1329173 | D9 | Ar27 | D1~8,D10~1175 |
| 1329174 ~ 1330347 | D9 | Ar28 | D1~8,D10~1175 |
| 1330348 ~ 1331521 | D9 | Ar29 | D1~8,D10~1175 |
| 1331522 ~ 1332695 | D9 | Ar30 | D1~8,D10~1175 |
| 1332696 ~ 1333869 | D9 | Ar31 | D1~8,D10~1175 |
| 1333870 ~ 1335043 | D9 | Ar32 | D1~8,D10~1175 |
| 1335044 ~ 1336217 | D9 | Ar33 | D1~8,D10~1175 |
| 1336218 ~ 1337391 | D9 | Ar34 | D1~8,D10~1175 |
| 1337392 ~ 1338565 | D9 | Ar35 | D1~8,D10~1175 |
| 1338566 ~ 1339739 | D9 | Ar36 | D1~8,D10~1175 |
| 1339740 ~ 1340913 | D9 | Ar37 | D1~8,D10~1175 |
| 1340914 ~ 1342087 | D9 | Ar38 | D1~8,D10~1175 |
| 1342088 ~ 1343261 | D9 | Ar39 | D1~8,D10~1175 |
| 1343262 ~ 1344435 | D9 | Ar40 | D1~8,D10~1175 |
| 1344436 ~ 1345609 | D9 | Ar41 | D1~8,D10~1175 |
| 1345610 ~ 1346783 | D9 | Ar42 | D1~8,D10~1175 |
| 1346784 ~ 1347957 | D9 | Ar43 | D1~8,D10~1175 |
| 1347958 ~ 1349131 | D9 | Ar44 | D1~8,D10~1175 |
| 1349132 ~ 1350305 | D9 | Ar45 | D1~8,D10~1175 |
| 1350306 ~ 1351479 | D9 | Ar46 | D1~8,D10~1175 |
| 1351480 ~ 1352653 | D9 | Ar47 | D1~8,D10~1175 |
| 1352654 ~ 1353827 | D9 | Ar48 | D1~8,D10~1175 |
| 1353828 ~ 1355001 | D9 | Ar49 | D1~8,D10~1175 |
| 1355002 ~ 1356175 | D9 | Ar50 | D1~8,D10~1175 |
| 1356176 ~ 1357349 | D9 | Ar51 | D1~8,D10~1175 |
| 1357350 ~ 1358523 | D9 | Ar52 | D1~8,D10~1175 |
| 1358524 ~ 1359697 | D9 | Ar53 | D1~8,D10~1175 |
| 1359698 ~ 1360871 | D9 | Ar54 | D1~8,D10~1175 |
| 1360872 ~ 1362045 | D9 | Ar55 | D1~8,D10~1175 |
| 1362046 ~ 1363219 | D9 | Ar56 | D1~8,D10~1175 |
| 1363220 ~ 1364393 | D9 | Ar57 | D1~8,D10~1175 |
| 1364394 ~ 1365567 | D9 | Ar58 | D1~8,D10~1175 |
| 1365568 ~ 1366741 | D9 | Ar59 | D1~8,D10~1175 |
| 1366742 ~ 1367915 | D9 | H | D1~8,D10~1175 |

| No. | $R^2$ | $R^4$ | $R^5$ |
|---|---|---|---|
| 1367916 ～ 1369089 | D19 | Ar1 | D1～18,D20～1175 |
| 1369090 ～ 1370263 | D19 | Ar2 | D1～18,D20～1175 |
| 1370264 ～ 1371437 | D19 | Ar3 | D1～18,D20～1175 |
| 1371438 ～ 1372611 | D19 | Ar4 | D1～18,D20～1175 |
| 1372612 ～ 1373785 | D19 | Ar5 | D1～18,D20～1175 |
| 1373786 ～ 1374959 | D19 | Ar6 | D1～18,D20～1175 |
| 1374960 ～ 1376133 | D19 | Ar7 | D1～18,D20～1175 |
| 1376134 ～ 1377307 | D19 | Ar8 | D1～18,D20～1175 |
| 1377308 ～ 1378481 | D19 | Ar9 | D1～18,D20～1175 |
| 1378482 ～ 1379655 | D19 | Ar10 | D1～18,D20～1175 |
| 1379656 ～ 1380829 | D19 | Ar11 | D1～18,D20～1175 |
| 1380830 ～ 1382003 | D19 | Ar12 | D1～18,D20～1175 |
| 1382004 ～ 1383177 | D19 | Ar13 | D1～18,D20～1175 |
| 1383178 ～ 1384351 | D19 | Ar14 | D1～18,D20～1175 |
| 1384352 ～ 1385525 | D19 | Ar15 | D1～18,D20～1175 |
| 1385526 ～ 1386699 | D19 | Ar16 | D1～18,D20～1175 |
| 1386700 ～ 1387873 | D19 | Ar17 | D1～18,D20～1175 |
| 1387874 ～ 1389047 | D19 | Ar18 | D1～18,D20～1175 |
| 1389048 ～ 1390221 | D19 | Ar19 | D1～18,D20～1175 |
| 1390222 ～ 1391395 | D19 | Ar20 | D1～18,D20～1175 |
| 1391396 ～ 1392569 | D19 | Ar21 | D1～18,D20～1175 |
| 1392570 ～ 1393743 | D19 | Ar22 | D1～18,D20～1175 |
| 1393744 ～ 1394917 | D19 | Ar23 | D1～18,D20～1175 |
| 1394918 ～ 1396091 | D19 | Ar24 | D1～18,D20～1175 |
| 1396092 ～ 1397265 | D19 | Ar25 | D1～18,D20～1175 |
| 1397266 ～ 1398439 | D19 | Ar26 | D1～18,D20～1175 |
| 1398440 ～ 1399613 | D19 | Ar27 | D1～18,D20～1175 |
| 1399614 ～ 1400787 | D19 | Ar28 | D1～18,D20～1175 |
| 1400788 ～ 1401961 | D19 | Ar29 | D1～18,D20～1175 |
| 1401962 ～ 1403135 | D19 | Ar30 | D1～18,D20～1175 |
| 1403136 ～ 1404309 | D19 | Ar31 | D1～18,D20～1175 |
| 1404310 ～ 1405483 | D19 | Ar32 | D1～18,D20～1175 |
| 1405484 ～ 1406657 | D19 | Ar33 | D1～18,D20～1175 |
| 1406658 ～ 1407831 | D19 | Ar34 | D1～18,D20～1175 |
| 1407832 ～ 1409005 | D19 | Ar35 | D1～18,D20～1175 |
| 1409006 ～ 1410179 | D19 | Ar36 | D1～18,D20～1175 |
| 1410180 ～ 1411353 | D19 | Ar37 | D1～18,D20～1175 |
| 1411354 ～ 1412527 | D19 | Ar38 | D1～18,D20～1175 |
| 1412528 ～ 1413701 | D19 | Ar39 | D1～18,D20～1175 |
| 1413702 ～ 1414875 | D19 | Ar40 | D1～18,D20～1175 |
| 1414876 ～ 1416049 | D19 | Ar41 | D1～18,D20～1175 |
| 1416050 ～ 1417223 | D19 | Ar42 | D1～18,D20～1175 |
| 1417224 ～ 1418397 | D19 | Ar43 | D1～18,D20～1175 |
| 1418398 ～ 1419571 | D19 | Ar44 | D1～18,D20～1175 |
| 1419572 ～ 1420745 | D19 | Ar45 | D1～18,D20～1175 |
| 1420746 ～ 1421919 | D19 | Ar46 | D1～18,D20～1175 |
| 1421920 ～ 1423093 | D19 | Ar47 | D1～18,D20～1175 |
| 1423094 ～ 1424267 | D19 | Ar48 | D1～18,D20～1175 |
| 1424268 ～ 1425441 | D19 | Ar49 | D1～18,D20～1175 |
| 1425442 ～ 1426615 | D19 | Ar50 | D1～18,D20～1175 |
| 1426616 ～ 1427789 | D19 | Ar51 | D1～18,D20～1175 |
| 1427790 ～ 1428963 | D19 | Ar52 | D1～18,D20～1175 |
| 1428964 ～ 1430137 | D19 | Ar53 | D1～18,D20～1175 |
| 1430138 ～ 1431311 | D19 | Ar54 | D1～18,D20～1175 |
| 1431312 ～ 1432485 | D19 | Ar55 | D1～18,D20～1175 |
| 1432486 ～ 1433659 | D19 | Ar56 | D1～18,D20～1175 |
| 1433660 ～ 1434833 | D19 | Ar57 | D1～18,D20～1175 |
| 1434834 ～ 1436007 | D19 | Ar58 | D1～18,D20～1175 |
| 1436008 ～ 1437181 | D19 | Ar59 | D1～18,D20～1175 |
| 1437182 ～ 1438355 | D19 | H | D1～18,D20～1175 |

| No. | $R^2$ | $R^4$ | $R^5$ |
|---|---|---|---|
| 1438356 ～ 1439529 | D37 | Ar1 | D1～36,D38～1175 |
| 1439530 ～ 1440703 | D37 | Ar2 | D1～36,D38～1175 |
| 1440704 ～ 1441877 | D37 | Ar3 | D1～36,D38～1175 |
| 1441878 ～ 1443051 | D37 | Ar4 | D1～36,D38～1175 |
| 1443052 ～ 1444225 | D37 | Ar5 | D1～36,D38～1175 |
| 1444226 ～ 1445399 | D37 | Ar6 | D1～36,D38～1175 |
| 1445400 ～ 1446573 | D37 | Ar7 | D1～36,D38～1175 |
| 1446574 ～ 1447747 | D37 | Ar8 | D1～36,D38～1175 |
| 1447748 ～ 1448921 | D37 | Ar9 | D1～36,D38～1175 |
| 1448922 ～ 1450095 | D37 | Ar10 | D1～36,D38～1175 |
| 1450096 ～ 1451269 | D37 | Ar11 | D1～36,D38～1175 |
| 1451270 ～ 1452443 | D37 | Ar12 | D1～36,D38～1175 |
| 1452444 ～ 1453617 | D37 | Ar13 | D1～36,D38～1175 |
| 1453618 ～ 1454791 | D37 | Ar14 | D1～36,D38～1175 |
| 1454792 ～ 1455965 | D37 | Ar15 | D1～36,D38～1175 |
| 1455966 ～ 1457139 | D37 | Ar16 | D1～36,D38～1175 |
| 1457140 ～ 1458313 | D37 | Ar17 | D1～36,D38～1175 |
| 1458314 ～ 1459487 | D37 | Ar18 | D1～36,D38～1175 |
| 1459488 ～ 1460661 | D37 | Ar19 | D1～36,D38～1175 |
| 1460662 ～ 1461835 | D37 | Ar20 | D1～36,D38～1175 |
| 1461836 ～ 1463009 | D37 | Ar21 | D1～36,D38～1175 |
| 1463010 ～ 1464183 | D37 | Ar22 | D1～36,D38～1175 |
| 1464184 ～ 1465357 | D37 | Ar23 | D1～36,D38～1175 |
| 1465358 ～ 1466531 | D37 | Ar24 | D1～36,D38～1175 |
| 1466532 ～ 1467705 | D37 | Ar25 | D1～36,D38～1175 |
| 1467706 ～ 1468879 | D37 | Ar26 | D1～36,D38～1175 |
| 1468880 ～ 1470053 | D37 | Ar27 | D1～36,D38～1175 |
| 1470054 ～ 1471227 | D37 | Ar28 | D1～36,D38～1175 |
| 1471228 ～ 1472401 | D37 | Ar29 | D1～36,D38～1175 |
| 1472402 ～ 1473575 | D37 | Ar30 | D1～36,D38～1175 |
| 1473576 ～ 1474749 | D37 | Ar31 | D1～36,D38～1175 |
| 1474750 ～ 1475923 | D37 | Ar32 | D1～36,D38～1175 |
| 1475924 ～ 1477097 | D37 | Ar33 | D1～36,D38～1175 |
| 1477098 ～ 1478271 | D37 | Ar34 | D1～36,D38～1175 |
| 1478272 ～ 1479445 | D37 | Ar35 | D1～36,D38～1175 |
| 1479446 ～ 1480619 | D37 | Ar36 | D1～36,D38～1175 |
| 1480620 ～ 1481793 | D37 | Ar37 | D1～36,D38～1175 |
| 1481794 ～ 1482967 | D37 | Ar38 | D1～36,D38～1175 |
| 1482968 ～ 1484141 | D37 | Ar39 | D1～36,D38～1175 |
| 1484142 ～ 1485315 | D37 | Ar40 | D1～36,D38～1175 |
| 1485316 ～ 1486489 | D37 | Ar41 | D1～36,D38～1175 |
| 1486490 ～ 1487663 | D37 | Ar42 | D1～36,D38～1175 |
| 1487664 ～ 1488837 | D37 | Ar43 | D1～36,D38～1175 |
| 1488838 ～ 1490011 | D37 | Ar44 | D1～36,D38～1175 |
| 1490012 ～ 1491185 | D37 | Ar45 | D1～36,D38～1175 |
| 1491186 ～ 1492359 | D37 | Ar46 | D1～36,D38～1175 |
| 1492360 ～ 1493533 | D37 | Ar47 | D1～36,D38～1175 |
| 1493534 ～ 1494707 | D37 | Ar48 | D1～36,D38～1175 |
| 1494708 ～ 1495881 | D37 | Ar49 | D1～36,D38～1175 |
| 1495882 ～ 1497055 | D37 | Ar50 | D1～36,D38～1175 |
| 1497056 ～ 1498229 | D37 | Ar51 | D1～36,D38～1175 |
| 1498230 ～ 1499403 | D37 | Ar52 | D1～36,D38～1175 |
| 1499404 ～ 1500577 | D37 | Ar53 | D1～36,D38～1175 |
| 1500578 ～ 1501751 | D37 | Ar54 | D1～36,D38～1175 |
| 1501752 ～ 1502925 | D37 | Ar55 | D1～36,D38～1175 |
| 1502926 ～ 1504099 | D37 | Ar56 | D1～36,D38～1175 |
| 1504100 ～ 1505273 | D37 | Ar57 | D1～36,D38～1175 |
| 1505274 ～ 1506447 | D37 | Ar58 | D1～36,D38～1175 |
| 1506448 ～ 1507621 | D37 | Ar59 | D1～36,D38～1175 |
| 1507622 ～ 1508795 | D37 | H | D1～36,D38～1175 |

| No. | $R^2$ | $R^4$ | $R^5$ |
|---|---|---|---|
| 1508796 ～ 1509969 | D50 | Ar1 | D1～49,D51～1175 |
| 1509970 ～ 1511143 | D50 | Ar2 | D1～49,D51～1175 |
| 1511144 ～ 1512317 | D50 | Ar3 | D1～49,D51～1175 |
| 1512318 ～ 1513491 | D50 | Ar4 | D1～49,D51～1175 |
| 1513492 ～ 1514665 | D50 | Ar5 | D1～49,D51～1175 |
| 1514666 ～ 1515839 | D50 | Ar6 | D1～49,D51～1175 |
| 1515840 ～ 1517013 | D50 | Ar7 | D1～49,D51～1175 |
| 1517014 ～ 1518187 | D50 | Ar8 | D1～49,D51～1175 |
| 1518188 ～ 1519361 | D50 | Ar9 | D1～49,D51～1175 |
| 1519362 ～ 1520535 | D50 | Ar10 | D1～49,D51～1175 |
| 1520536 ～ 1521709 | D50 | Ar11 | D1～49,D51～1175 |
| 1521710 ～ 1522883 | D50 | Ar12 | D1～49,D51～1175 |
| 1522884 ～ 1524057 | D50 | Ar13 | D1～49,D51～1175 |
| 1524058 ～ 1525231 | D50 | Ar14 | D1～49,D51～1175 |
| 1525232 ～ 1526405 | D50 | Ar15 | D1～49,D51～1175 |
| 1526406 ～ 1527579 | D50 | Ar16 | D1～49,D51～1175 |
| 1527580 ～ 1528753 | D50 | Ar17 | D1～49,D51～1175 |
| 1528754 ～ 1529927 | D50 | Ar18 | D1～49,D51～1175 |
| 1529928 ～ 1531101 | D50 | Ar19 | D1～49,D51～1175 |
| 1531102 ～ 1532275 | D50 | Ar20 | D1～49,D51～1175 |
| 1532276 ～ 1533449 | D50 | Ar21 | D1～49,D51～1175 |
| 1533450 ～ 1534623 | D50 | Ar22 | D1～49,D51～1175 |
| 1534624 ～ 1535797 | D50 | Ar23 | D1～49,D51～1175 |
| 1535798 ～ 1536971 | D50 | Ar24 | D1～49,D51～1175 |
| 1536972 ～ 1538145 | D50 | Ar25 | D1～49,D51～1175 |
| 1538146 ～ 1539319 | D50 | Ar26 | D1～49,D51～1175 |
| 1539320 ～ 1540493 | D50 | Ar27 | D1～49,D51～1175 |
| 1540494 ～ 1541667 | D50 | Ar28 | D1～49,D51～1175 |
| 1541668 ～ 1542841 | D50 | Ar29 | D1～49,D51～1175 |
| 1542842 ～ 1544015 | D50 | Ar30 | D1～49,D51～1175 |
| 1544016 ～ 1545189 | D50 | Ar31 | D1～49,D51～1175 |
| 1545190 ～ 1546363 | D50 | Ar32 | D1～49,D51～1175 |
| 1546364 ～ 1547537 | D50 | Ar33 | D1～49,D51～1175 |
| 1547538 ～ 1548711 | D50 | Ar34 | D1～49,D51～1175 |
| 1548712 ～ 1549885 | D50 | Ar35 | D1～49,D51～1175 |
| 1549886 ～ 1551059 | D50 | Ar36 | D1～49,D51～1175 |
| 1551060 ～ 1552233 | D50 | Ar37 | D1～49,D51～1175 |
| 1552234 ～ 1553407 | D50 | Ar38 | D1～49,D51～1175 |
| 1553408 ～ 1554581 | D50 | Ar39 | D1～49,D51～1175 |
| 1554582 ～ 1555755 | D50 | Ar40 | D1～49,D51～1175 |
| 1555756 ～ 1556929 | D50 | Ar41 | D1～49,D51～1175 |
| 1556930 ～ 1558103 | D50 | Ar42 | D1～49,D51～1175 |
| 1558104 ～ 1559277 | D50 | Ar43 | D1～49,D51～1175 |
| 1559278 ～ 1560451 | D50 | Ar44 | D1～49,D51～1175 |
| 1560452 ～ 1561625 | D50 | Ar45 | D1～49,D51～1175 |
| 1561626 ～ 1562799 | D50 | Ar46 | D1～49,D51～1175 |
| 1562800 ～ 1563973 | D50 | Ar47 | D1～49,D51～1175 |
| 1563974 ～ 1565147 | D50 | Ar48 | D1～49,D51～1175 |
| 1565148 ～ 1566321 | D50 | Ar49 | D1～49,D51～1175 |
| 1566322 ～ 1567495 | D50 | Ar50 | D1～49,D51～1175 |
| 1567496 ～ 1568669 | D50 | Ar51 | D1～49,D51～1175 |
| 1568670 ～ 1569843 | D50 | Ar52 | D1～49,D51～1175 |
| 1569844 ～ 1571017 | D50 | Ar53 | D1～49,D51～1175 |
| 1571018 ～ 1572191 | D50 | Ar54 | D1～49,D51～1175 |
| 1572192 ～ 1573365 | D50 | Ar55 | D1～49,D51～1175 |
| 1573366 ～ 1574539 | D50 | Ar56 | D1～49,D51～1175 |
| 1574540 ～ 1575713 | D50 | Ar57 | D1～49,D51～1175 |
| 1575714 ～ 1576887 | D50 | Ar58 | D1～49,D51～1175 |
| 1576888 ～ 1578061 | D50 | Ar59 | D1～49,D51～1175 |
| 1578062 ～ 1579235 | D50 | H | D1～49,D51～1175 |

| No. | $R^2$ | $R^4$ | $R^5$ |
|---|---|---|---|
| 1579236 ～ 1580409 | D465 | Ar1 | D1～464,D466～1175 |
| 1580410 ～ 1581583 | D465 | Ar2 | D1～464,D466～1175 |
| 1581584 ～ 1582757 | D465 | Ar3 | D1～464,D466～1175 |
| 1582758 ～ 1583931 | D465 | Ar4 | D1～464,D466～1175 |
| 1583932 ～ 1585105 | D465 | Ar5 | D1～464,D466～1175 |
| 1585106 ～ 1586279 | D465 | Ar6 | D1～464,D466～1175 |
| 1586280 ～ 1587453 | D465 | Ar7 | D1～464,D466～1175 |
| 1587454 ～ 1588627 | D465 | Ar8 | D1～464,D466～1175 |
| 1588628 ～ 1589801 | D465 | Ar9 | D1～464,D466～1175 |
| 1589802 ～ 1590975 | D465 | Ar10 | D1～464,D466～1175 |
| 1590976 ～ 1592149 | D465 | Ar11 | D1～464,D466～1175 |
| 1592150 ～ 1593323 | D465 | Ar12 | D1～464,D466～1175 |
| 1593324 ～ 1594497 | D465 | Ar13 | D1～464,D466～1175 |
| 1594498 ～ 1595671 | D465 | Ar14 | D1～464,D466～1175 |
| 1595672 ～ 1596845 | D465 | Ar15 | D1～464,D466～1175 |
| 1596846 ～ 1598019 | D465 | Ar16 | D1～464,D466～1175 |
| 1598020 ～ 1599193 | D465 | Ar17 | D1～464,D466～1175 |
| 1599194 ～ 1600367 | D465 | Ar18 | D1～464,D466～1175 |
| 1600368 ～ 1601541 | D465 | Ar19 | D1～464,D466～1175 |
| 1601542 ～ 1602715 | D465 | Ar20 | D1～464,D466～1175 |
| 1602716 ～ 1603889 | D465 | Ar21 | D1～464,D466～1175 |
| 1603890 ～ 1605063 | D465 | Ar22 | D1～464,D466～1175 |
| 1605064 ～ 1606237 | D465 | Ar23 | D1～464,D466～1175 |
| 1606238 ～ 1607411 | D465 | Ar24 | D1～464,D466～1175 |
| 1607412 ～ 1608585 | D465 | Ar25 | D1～464,D466～1175 |
| 1608586 ～ 1609759 | D465 | Ar26 | D1～464,D466～1175 |
| 1609760 ～ 1610933 | D465 | Ar27 | D1～464,D466～1175 |
| 1610934 ～ 1612107 | D465 | Ar28 | D1～464,D466～1175 |
| 1612108 ～ 1613281 | D465 | Ar29 | D1～464,D466～1175 |
| 1613282 ～ 1614455 | D465 | Ar30 | D1～464,D466～1175 |
| 1614456 ～ 1615629 | D465 | Ar31 | D1～464,D466～1175 |
| 1615630 ～ 1616803 | D465 | Ar32 | D1～464,D466～1175 |
| 1616804 ～ 1617977 | D465 | Ar33 | D1～464,D466～1175 |
| 1617978 ～ 1619151 | D465 | Ar34 | D1～464,D466～1175 |
| 1619152 ～ 1620325 | D465 | Ar35 | D1～464,D466～1175 |
| 1620326 ～ 1621499 | D465 | Ar36 | D1～464,D466～1175 |
| 1621500 ～ 1622673 | D465 | Ar37 | D1～464,D466～1175 |
| 1622674 ～ 1623847 | D465 | Ar38 | D1～464,D466～1175 |
| 1623848 ～ 1625021 | D465 | Ar39 | D1～464,D466～1175 |
| 1625022 ～ 1626195 | D465 | Ar40 | D1～464,D466～1175 |
| 1626196 ～ 1627369 | D465 | Ar41 | D1～464,D466～1175 |
| 1627370 ～ 1628543 | D465 | Ar42 | D1～464,D466～1175 |
| 1628544 ～ 1629717 | D465 | Ar43 | D1～464,D466～1175 |
| 1629718 ～ 1630891 | D465 | Ar44 | D1～464,D466～1175 |
| 1630892 ～ 1632065 | D465 | Ar45 | D1～464,D466～1175 |
| 1632066 ～ 1633239 | D465 | Ar46 | D1～464,D466～1175 |
| 1633240 ～ 1634413 | D465 | Ar47 | D1～464,D466～1175 |
| 1634414 ～ 1635587 | D465 | Ar48 | D1～464,D466～1175 |
| 1635588 ～ 1636761 | D465 | Ar49 | D1～464,D466～1175 |
| 1636762 ～ 1637935 | D465 | Ar50 | D1～464,D466～1175 |
| 1637936 ～ 1639109 | D465 | Ar51 | D1～464,D466～1175 |
| 1639110 ～ 1640283 | D465 | Ar52 | D1～464,D466～1175 |
| 1640284 ～ 1641457 | D465 | Ar53 | D1～464,D466～1175 |
| 1641458 ～ 1642631 | D465 | Ar54 | D1～464,D466～1175 |
| 1642632 ～ 1643805 | D465 | Ar55 | D1～464,D466～1175 |
| 1643806 ～ 1644979 | D465 | Ar56 | D1～464,D466～1175 |
| 1644980 ～ 1646153 | D465 | Ar57 | D1～464,D466～1175 |
| 1646154 ～ 1647327 | D465 | Ar58 | D1～464,D466～1175 |
| 1647328 ～ 1648501 | D465 | Ar59 | D1～464,D466～1175 |
| 1648502 ～ 1649675 | D465 | H | D1～464,D466～1175 |

| No. | $R^2$ | $R^4$ | $R^5$ |
|---|---|---|---|
| 1649676 ~ 1650849 | D466 | Ar1 | D1~465,D467~1175 |
| 1650850 ~ 1652023 | D466 | Ar2 | D1~465,D467~1175 |
| 1652024 ~ 1653197 | D466 | Ar3 | D1~465,D467~1175 |
| 1653198 ~ 1654371 | D466 | Ar4 | D1~465,D467~1175 |
| 1654372 ~ 1655545 | D466 | Ar5 | D1~465,D467~1175 |
| 1655546 ~ 1656719 | D466 | Ar6 | D1~465,D467~1175 |
| 1656720 ~ 1657893 | D466 | Ar7 | D1~465,D467~1175 |
| 1657894 ~ 1659067 | D466 | Ar8 | D1~465,D467~1175 |
| 1659068 ~ 1660241 | D466 | Ar9 | D1~465,D467~1175 |
| 1660242 ~ 1661415 | D466 | Ar10 | D1~465,D467~1175 |
| 1661416 ~ 1662589 | D466 | Ar11 | D1~465,D467~1175 |
| 1662590 ~ 1663763 | D466 | Ar12 | D1~465,D467~1175 |
| 1663764 ~ 1664937 | D466 | Ar13 | D1~465,D467~1175 |
| 1664938 ~ 1666111 | D466 | Ar14 | D1~465,D467~1175 |
| 1666112 ~ 1667285 | D466 | Ar15 | D1~465,D467~1175 |
| 1667286 ~ 1668459 | D466 | Ar16 | D1~465,D467~1175 |
| 1668460 ~ 1669633 | D466 | Ar17 | D1~465,D467~1175 |
| 1669634 ~ 1670807 | D466 | Ar18 | D1~465,D467~1175 |
| 1670808 ~ 1671981 | D466 | Ar19 | D1~465,D467~1175 |
| 1671982 ~ 1673155 | D466 | Ar20 | D1~465,D467~1175 |
| 1673156 ~ 1674329 | D466 | Ar21 | D1~465,D467~1175 |
| 1674330 ~ 1675503 | D466 | Ar22 | D1~465,D467~1175 |
| 1675504 ~ 1676677 | D466 | Ar23 | D1~465,D467~1175 |
| 1676678 ~ 1677851 | D466 | Ar24 | D1~465,D467~1175 |
| 1677852 ~ 1679025 | D466 | Ar25 | D1~465,D467~1175 |
| 1679026 ~ 1680199 | D466 | Ar26 | D1~465,D467~1175 |
| 1680200 ~ 1681373 | D466 | Ar27 | D1~465,D467~1175 |
| 1681374 ~ 1682547 | D466 | Ar28 | D1~465,D467~1175 |
| 1682548 ~ 1683721 | D466 | Ar29 | D1~465,D467~1175 |
| 1683722 ~ 1684895 | D466 | Ar30 | D1~465,D467~1175 |
| 1684896 ~ 1686069 | D466 | Ar31 | D1~465,D467~1175 |
| 1686070 ~ 1687243 | D466 | Ar32 | D1~465,D467~1175 |
| 1687244 ~ 1688417 | D466 | Ar33 | D1~465,D467~1175 |
| 1688418 ~ 1689591 | D466 | Ar34 | D1~465,D467~1175 |
| 1689592 ~ 1690765 | D466 | Ar35 | D1~465,D467~1175 |
| 1690766 ~ 1691939 | D466 | Ar36 | D1~465,D467~1175 |
| 1691940 ~ 1693113 | D466 | Ar37 | D1~465,D467~1175 |
| 1693114 ~ 1694287 | D466 | Ar38 | D1~465,D467~1175 |
| 1694288 ~ 1695461 | D466 | Ar39 | D1~465,D467~1175 |
| 1695462 ~ 1696635 | D466 | Ar40 | D1~465,D467~1175 |
| 1696636 ~ 1697809 | D466 | Ar41 | D1~465,D467~1175 |
| 1697810 ~ 1698983 | D466 | Ar42 | D1~465,D467~1175 |
| 1698984 ~ 1700157 | D466 | Ar43 | D1~465,D467~1175 |
| 1700158 ~ 1701331 | D466 | Ar44 | D1~465,D467~1175 |
| 1701332 ~ 1702505 | D466 | Ar45 | D1~465,D467~1175 |
| 1702506 ~ 1703679 | D466 | Ar46 | D1~465,D467~1175 |
| 1703680 ~ 1704853 | D466 | Ar47 | D1~465,D467~1175 |
| 1704854 ~ 1706027 | D466 | Ar48 | D1~465,D467~1175 |
| 1706028 ~ 1707201 | D466 | Ar49 | D1~465,D467~1175 |
| 1707202 ~ 1708375 | D466 | Ar50 | D1~465,D467~1175 |
| 1708376 ~ 1709549 | D466 | Ar51 | D1~465,D467~1175 |
| 1709550 ~ 1710723 | D466 | Ar52 | D1~465,D467~1175 |
| 1710724 ~ 1711897 | D466 | Ar53 | D1~465,D467~1175 |
| 1711898 ~ 1713071 | D466 | Ar54 | D1~465,D467~1175 |
| 1713072 ~ 1714245 | D466 | Ar55 | D1~465,D467~1175 |
| 1714246 ~ 1715419 | D466 | Ar56 | D1~465,D467~1175 |
| 1715420 ~ 1716593 | D466 | Ar57 | D1~465,D467~1175 |
| 1716594 ~ 1717767 | D466 | Ar58 | D1~465,D467~1175 |
| 1717768 ~ 1718941 | D466 | Ar59 | D1~465,D467~1175 |
| 1718942 ~ 1720115 | D466 | H | D1~465,D467~1175 |

| No. | $R^2$ | $R^4$ | $R^5$ |
|---|---|---|---|
| 1720116 ~ 1721289 | D467 | Ar1 | D1~466,D468~1175 |
| 1721290 ~ 1722463 | D467 | Ar2 | D1~466,D468~1175 |
| 1722464 ~ 1723637 | D467 | Ar3 | D1~466,D468~1175 |
| 1723638 ~ 1724811 | D467 | Ar4 | D1~466,D468~1175 |
| 1724812 ~ 1725985 | D467 | Ar5 | D1~466,D468~1175 |
| 1725986 ~ 1727159 | D467 | Ar6 | D1~466,D468~1175 |
| 1727160 ~ 1728333 | D467 | Ar7 | D1~466,D468~1175 |
| 1728334 ~ 1729507 | D467 | Ar8 | D1~466,D468~1175 |
| 1729508 ~ 1730681 | D467 | Ar9 | D1~466,D468~1175 |
| 1730682 ~ 1731855 | D467 | Ar10 | D1~466,D468~1175 |
| 1731856 ~ 1733029 | D467 | Ar11 | D1~466,D468~1175 |
| 1733030 ~ 1734203 | D467 | Ar12 | D1~466,D468~1175 |
| 1734204 ~ 1735377 | D467 | Ar13 | D1~466,D468~1175 |
| 1735378 ~ 1736551 | D467 | Ar14 | D1~466,D468~1175 |
| 1736552 ~ 1737725 | D467 | Ar15 | D1~466,D468~1175 |
| 1737726 ~ 1738899 | D467 | Ar16 | D1~466,D468~1175 |
| 1738900 ~ 1740073 | D467 | Ar17 | D1~466,D468~1175 |
| 1740074 ~ 1741247 | D467 | Ar18 | D1~466,D468~1175 |
| 1741248 ~ 1742421 | D467 | Ar19 | D1~466,D468~1175 |
| 1742422 ~ 1743595 | D467 | Ar20 | D1~466,D468~1175 |
| 1743596 ~ 1744769 | D467 | Ar21 | D1~466,D468~1175 |
| 1744770 ~ 1745943 | D467 | Ar22 | D1~466,D468~1175 |
| 1745944 ~ 1747117 | D467 | Ar23 | D1~466,D468~1175 |
| 1747118 ~ 1748291 | D467 | Ar24 | D1~466,D468~1175 |
| 1748292 ~ 1749465 | D467 | Ar25 | D1~466,D468~1175 |
| 1749466 ~ 1750639 | D467 | Ar26 | D1~466,D468~1175 |
| 1750640 ~ 1751813 | D467 | Ar27 | D1~466,D468~1175 |
| 1751814 ~ 1752987 | D467 | Ar28 | D1~466,D468~1175 |
| 1752988 ~ 1754161 | D467 | Ar29 | D1~466,D468~1175 |
| 1754162 ~ 1755335 | D467 | Ar30 | D1~466,D468~1175 |
| 1755336 ~ 1756509 | D467 | Ar31 | D1~466,D468~1175 |
| 1756510 ~ 1757683 | D467 | Ar32 | D1~466,D468~1175 |
| 1757684 ~ 1758857 | D467 | Ar33 | D1~466,D468~1175 |
| 1758858 ~ 1760031 | D467 | Ar34 | D1~466,D468~1175 |
| 1760032 ~ 1761205 | D467 | Ar35 | D1~466,D468~1175 |
| 1761206 ~ 1762379 | D467 | Ar36 | D1~466,D468~1175 |
| 1762380 ~ 1763553 | D467 | Ar37 | D1~466,D468~1175 |
| 1763554 ~ 1764727 | D467 | Ar38 | D1~466,D468~1175 |
| 1764728 ~ 1765901 | D467 | Ar39 | D1~466,D468~1175 |
| 1765902 ~ 1767075 | D467 | Ar40 | D1~466,D468~1175 |
| 1767076 ~ 1768249 | D467 | Ar41 | D1~466,D468~1175 |
| 1768250 ~ 1769423 | D467 | Ar42 | D1~466,D468~1175 |
| 1769424 ~ 1770597 | D467 | Ar43 | D1~466,D468~1175 |
| 1770598 ~ 1771771 | D467 | Ar44 | D1~466,D468~1175 |
| 1771772 ~ 1772945 | D467 | Ar45 | D1~466,D468~1175 |
| 1772946 ~ 1774119 | D467 | Ar46 | D1~466,D468~1175 |
| 1774120 ~ 1775293 | D467 | Ar47 | D1~466,D468~1175 |
| 1775294 ~ 1776467 | D467 | Ar48 | D1~466,D468~1175 |
| 1776468 ~ 1777641 | D467 | Ar49 | D1~466,D468~1175 |
| 1777642 ~ 1778815 | D467 | Ar50 | D1~466,D468~1175 |
| 1778816 ~ 1779989 | D467 | Ar51 | D1~466,D468~1175 |
| 1779990 ~ 1781163 | D467 | Ar52 | D1~466,D468~1175 |
| 1781164 ~ 1782337 | D467 | Ar53 | D1~466,D468~1175 |
| 1782338 ~ 1783511 | D467 | Ar54 | D1~466,D468~1175 |
| 1783512 ~ 1784685 | D467 | Ar55 | D1~466,D468~1175 |
| 1784686 ~ 1785859 | D467 | Ar56 | D1~466,D468~1175 |
| 1785860 ~ 1787033 | D467 | Ar57 | D1~466,D468~1175 |
| 1787034 ~ 1788207 | D467 | Ar58 | D1~466,D468~1175 |
| 1788208 ~ 1789381 | D467 | Ar59 | D1~466,D468~1175 |
| 1789382 ~ 1790555 | D467 | H | D1~466,D468~1175 |

| No. | $R^2$ | $R^4$ | $R^5$ |
|---|---|---|---|
| 1790556 ～ 1791729 | D486 | Ar1 | D1～485,D487～1175 |
| 1791730 ～ 1792903 | D486 | Ar2 | D1～485,D487～1175 |
| 1792904 ～ 1794077 | D486 | Ar3 | D1～485,D487～1175 |
| 1794078 ～ 1795251 | D486 | Ar4 | D1～485,D487～1175 |
| 1795252 ～ 1796425 | D486 | Ar5 | D1～485,D487～1175 |
| 1796426 ～ 1797599 | D486 | Ar6 | D1～485,D487～1175 |
| 1797600 ～ 1798773 | D486 | Ar7 | D1～485,D487～1175 |
| 1798774 ～ 1799947 | D486 | Ar8 | D1～485,D487～1175 |
| 1799948 ～ 1801121 | D486 | Ar9 | D1～485,D487～1175 |
| 1801122 ～ 1802295 | D486 | Ar10 | D1～485,D487～1175 |
| 1802296 ～ 1803469 | D486 | Ar11 | D1～485,D487～1175 |
| 1803470 ～ 1804643 | D486 | Ar12 | D1～485,D487～1175 |
| 1804644 ～ 1805817 | D486 | Ar13 | D1～485,D487～1175 |
| 1805818 ～ 1806991 | D486 | Ar14 | D1～485,D487～1175 |
| 1806992 ～ 1808165 | D486 | Ar15 | D1～485,D487～1175 |
| 1808166 ～ 1809339 | D486 | Ar16 | D1～485,D487～1175 |
| 1809340 ～ 1810513 | D486 | Ar17 | D1～485,D487～1175 |
| 1810514 ～ 1811687 | D486 | Ar18 | D1～485,D487～1175 |
| 1811688 ～ 1812861 | D486 | Ar19 | D1～485,D487～1175 |
| 1812862 ～ 1814035 | D486 | Ar20 | D1～485,D487～1175 |
| 1814036 ～ 1815209 | D486 | Ar21 | D1～485,D487～1175 |
| 1815210 ～ 1816383 | D486 | Ar22 | D1～485,D487～1175 |
| 1816384 ～ 1817557 | D486 | Ar23 | D1～485,D487～1175 |
| 1817558 ～ 1818731 | D486 | Ar24 | D1～485,D487～1175 |
| 1818732 ～ 1819905 | D486 | Ar25 | D1～485,D487～1175 |
| 1819906 ～ 1821079 | D486 | Ar26 | D1～485,D487～1175 |
| 1821080 ～ 1822253 | D486 | Ar27 | D1～485,D487～1175 |
| 1822254 ～ 1823427 | D486 | Ar28 | D1～485,D487～1175 |
| 1823428 ～ 1824601 | D486 | Ar29 | D1～485,D487～1175 |
| 1824602 ～ 1825775 | D486 | Ar30 | D1～485,D487～1175 |
| 1825776 ～ 1826949 | D486 | Ar31 | D1～485,D487～1175 |
| 1826950 ～ 1828123 | D486 | Ar32 | D1～485,D487～1175 |
| 1828124 ～ 1829297 | D486 | Ar33 | D1～485,D487～1175 |
| 1829298 ～ 1830471 | D486 | Ar34 | D1～485,D487～1175 |
| 1830472 ～ 1831645 | D486 | Ar35 | D1～485,D487～1175 |
| 1831646 ～ 1832819 | D486 | Ar36 | D1～485,D487～1175 |
| 1832820 ～ 1833993 | D486 | Ar37 | D1～485,D487～1175 |
| 1833994 ～ 1835167 | D486 | Ar38 | D1～485,D487～1175 |
| 1835168 ～ 1836341 | D486 | Ar39 | D1～485,D487～1175 |
| 1836342 ～ 1837515 | D486 | Ar40 | D1～485,D487～1175 |
| 1837516 ～ 1838689 | D486 | Ar41 | D1～485,D487～1175 |
| 1838690 ～ 1839863 | D486 | Ar42 | D1～485,D487～1175 |
| 1839864 ～ 1841037 | D486 | Ar43 | D1～485,D487～1175 |
| 1841038 ～ 1842211 | D486 | Ar44 | D1～485,D487～1175 |
| 1842212 ～ 1843385 | D486 | Ar45 | D1～485,D487～1175 |
| 1843386 ～ 1844559 | D486 | Ar46 | D1～485,D487～1175 |
| 1844560 ～ 1845733 | D486 | Ar47 | D1～485,D487～1175 |
| 1845734 ～ 1846907 | D486 | Ar48 | D1～485,D487～1175 |
| 1846908 ～ 1848081 | D486 | Ar49 | D1～485,D487～1175 |
| 1848082 ～ 1849255 | D486 | Ar50 | D1～485,D487～1175 |
| 1849256 ～ 1850429 | D486 | Ar51 | D1～485,D487～1175 |
| 1850430 ～ 1851603 | D486 | Ar52 | D1～485,D487～1175 |
| 1851604 ～ 1852777 | D486 | Ar53 | D1～485,D487～1175 |
| 1852778 ～ 1853951 | D486 | Ar54 | D1～485,D487～1175 |
| 1853952 ～ 1855125 | D486 | Ar55 | D1～485,D487～1175 |
| 1855126 ～ 1856299 | D486 | Ar56 | D1～485,D487～1175 |
| 1856300 ～ 1857473 | D486 | Ar57 | D1～485,D487～1175 |
| 1857474 ～ 1858647 | D486 | Ar58 | D1～485,D487～1175 |
| 1858648 ～ 1859821 | D486 | Ar59 | D1～485,D487～1175 |
| 1859822 ～ 1860995 | D486 | H | D1～485,D487～1175 |

| No. | $R^2$ | $R^4$ | $R^5$ |
|---|---|---|---|
| 1860996 ～ 1862169 | D717 | Ar1 | D1～716,D718～1175 |
| 1862170 ～ 1863343 | D717 | Ar2 | D1～716,D718～1175 |
| 1863344 ～ 1864517 | D717 | Ar3 | D1～716,D718～1175 |
| 1864518 ～ 1865691 | D717 | Ar4 | D1～716,D718～1175 |
| 1865692 ～ 1866865 | D717 | Ar5 | D1～716,D718～1175 |
| 1866866 ～ 1868039 | D717 | Ar6 | D1～716,D718～1175 |
| 1868040 ～ 1869213 | D717 | Ar7 | D1～716,D718～1175 |
| 1869214 ～ 1870387 | D717 | Ar8 | D1～716,D718～1175 |
| 1870388 ～ 1871561 | D717 | Ar9 | D1～716,D718～1175 |
| 1871562 ～ 1872735 | D717 | Ar10 | D1～716,D718～1175 |
| 1872736 ～ 1873909 | D717 | Ar11 | D1～716,D718～1175 |
| 1873910 ～ 1875083 | D717 | Ar12 | D1～716,D718～1175 |
| 1875084 ～ 1876257 | D717 | Ar13 | D1～716,D718～1175 |
| 1876258 ～ 1877431 | D717 | Ar14 | D1～716,D718～1175 |
| 1877432 ～ 1878605 | D717 | Ar15 | D1～716,D718～1175 |
| 1878606 ～ 1879779 | D717 | Ar16 | D1～716,D718～1175 |
| 1879780 ～ 1880953 | D717 | Ar17 | D1～716,D718～1175 |
| 1880954 ～ 1882127 | D717 | Ar18 | D1～716,D718～1175 |
| 1882128 ～ 1883301 | D717 | Ar19 | D1～716,D718～1175 |
| 1883302 ～ 1884475 | D717 | Ar20 | D1～716,D718～1175 |
| 1884476 ～ 1885649 | D717 | Ar21 | D1～716,D718～1175 |
| 1885650 ～ 1886823 | D717 | Ar22 | D1～716,D718～1175 |
| 1886824 ～ 1887997 | D717 | Ar23 | D1～716,D718～1175 |
| 1887998 ～ 1889171 | D717 | Ar24 | D1～716,D718～1175 |
| 1889172 ～ 1890345 | D717 | Ar25 | D1～716,D718～1175 |
| 1890346 ～ 1891519 | D717 | Ar26 | D1～716,D718～1175 |
| 1891520 ～ 1892693 | D717 | Ar27 | D1～716,D718～1175 |
| 1892694 ～ 1893867 | D717 | Ar28 | D1～716,D718～1175 |
| 1893868 ～ 1895041 | D717 | Ar29 | D1～716,D718～1175 |
| 1895042 ～ 1896215 | D717 | Ar30 | D1～716,D718～1175 |
| 1896216 ～ 1897389 | D717 | Ar31 | D1～716,D718～1175 |
| 1897390 ～ 1898563 | D717 | Ar32 | D1～716,D718～1175 |
| 1898564 ～ 1899737 | D717 | Ar33 | D1～716,D718～1175 |
| 1899738 ～ 1900911 | D717 | Ar34 | D1～716,D718～1175 |
| 1900912 ～ 1902085 | D717 | Ar35 | D1～716,D718～1175 |
| 1902086 ～ 1903259 | D717 | Ar36 | D1～716,D718～1175 |
| 1903260 ～ 1904433 | D717 | Ar37 | D1～716,D718～1175 |
| 1904434 ～ 1905607 | D717 | Ar38 | D1～716,D718～1175 |
| 1905608 ～ 1906781 | D717 | Ar39 | D1～716,D718～1175 |
| 1906782 ～ 1907955 | D717 | Ar40 | D1～716,D718～1175 |
| 1907956 ～ 1909129 | D717 | Ar41 | D1～716,D718～1175 |
| 1909130 ～ 1910303 | D717 | Ar42 | D1～716,D718～1175 |
| 1910304 ～ 1911477 | D717 | Ar43 | D1～716,D718～1175 |
| 1911478 ～ 1912651 | D717 | Ar44 | D1～716,D718～1175 |
| 1912652 ～ 1913825 | D717 | Ar45 | D1～716,D718～1175 |
| 1913826 ～ 1914999 | D717 | Ar46 | D1～716,D718～1175 |
| 1915000 ～ 1916173 | D717 | Ar47 | D1～716,D718～1175 |
| 1916174 ～ 1917347 | D717 | Ar48 | D1～716,D718～1175 |
| 1917348 ～ 1918521 | D717 | Ar49 | D1～716,D718～1175 |
| 1918522 ～ 1919695 | D717 | Ar50 | D1～716,D718～1175 |
| 1919696 ～ 1920869 | D717 | Ar51 | D1～716,D718～1175 |
| 1920870 ～ 1922043 | D717 | Ar52 | D1～716,D718～1175 |
| 1922044 ～ 1923217 | D717 | Ar53 | D1～716,D718～1175 |
| 1923218 ～ 1924391 | D717 | Ar54 | D1～716,D718～1175 |
| 1924392 ～ 1925565 | D717 | Ar55 | D1～716,D718～1175 |
| 1925566 ～ 1926739 | D717 | Ar56 | D1～716,D718～1175 |
| 1926740 ～ 1927913 | D717 | Ar57 | D1～716,D718～1175 |
| 1927914 ～ 1929087 | D717 | Ar58 | D1～716,D718～1175 |
| 1929088 ～ 1930261 | D717 | Ar59 | D1～716,D718～1175 |
| 1930262 ～ 1931435 | D717 | H | D1～716,D718～1175 |

| No. | $R^2$ | $R^4$ | $R^5$ | = |
|---|---|---|---|---|
| 1931436 ~ 1932610 | D1~D1175 | D1 | D1~D1175 | $R^2 = R^5$ |
| 1932611 ~ 1933785 | D1~D1175 | D7 | D1~D1175 | |
| 1933786 ~ 1934960 | D1~D1175 | D8 | D1~D1175 | |
| 1934961 ~ 1936135 | D1~D1175 | D9 | D1~D1175 | |
| 1936136 ~ 1937310 | D1~D1175 | D19 | D1~D1175 | |
| 1937311 ~ 1938485 | D1~D1175 | D37 | D1~D1175 | |
| 1938486 ~ 1939660 | D1~D1175 | D50 | D1~D1175 | |
| 1939661 ~ 1940835 | D1~D1175 | D67 | D1~D1175 | |
| 1940836 ~ 1942010 | D1~D1175 | D77 | D1~D1175 | |
| 1942011 ~ 1943185 | D1~D1175 | D78 | D1~D1175 | |
| 1943186 ~ 1944360 | D1~D1175 | D79 | D1~D1175 | |
| 1944361 ~ 1945535 | D1~D1175 | D80 | D1~D1175 | |
| 1945536 ~ 1946710 | D1~D1175 | D81 | D1~D1175 | |
| 1946711 ~ 1947885 | D1~D1175 | D465 | D1~D1175 | |
| 1947886 ~ 1949060 | D1~D1175 | D466 | D1~D1175 | |
| 1949061 ~ 1950235 | D1~D1175 | D467 | D1~D1175 | |
| 1950236 ~ 1951410 | D1~D1175 | D471 | D1~D1175 | |
| 1951411 ~ 1952585 | D1~D1175 | D486 | D1~D1175 | |
| 1952586 ~ 1953760 | D1~D1175 | D717 | D1~D1175 | |
| 1953761 ~ 1954935 | D1~D1175 | D735 | D1~D1175 | |
| 1954936 ~ 1956110 | D1~D1175 | D783 | D1~D1175 | |
| 1956111 ~ 1957285 | D1~D1175 | D793 | D1~D1175 | |
| 1957286 ~ 1958460 | D1~D1175 | D794 | D1~D1175 | |
| 1958461 ~ 1959635 | D1~D1175 | D795 | D1~D1175 | |
| 1959636 ~ 1960810 | D1~D1175 | D796 | D1~D1175 | |
| 1960811 ~ 1961985 | D1~D1175 | D797 | D1~D1175 | |
| 1961986 ~ 1963160 | H | Ar1 | D1~D1175 | |
| 1963161 ~ 1964335 | H | Ar2 | D1~D1175 | |
| 1964336 ~ 1965510 | H | Ar3 | D1~D1175 | |
| 1965511 ~ 1966685 | H | Ar4 | D1~D1175 | |
| 1966686 ~ 1967860 | H | Ar5 | D1~D1175 | |
| 1967861 ~ 1969035 | H | Ar6 | D1~D1175 | |
| 1969036 ~ 1970210 | H | Ar7 | D1~D1175 | |
| 1970211 ~ 1971385 | H | Ar8 | D1~D1175 | |
| 1971386 ~ 1972560 | H | Ar9 | D1~D1175 | |
| 1972561 ~ 1973735 | H | Ar10 | D1~D1175 | |
| 1973736 ~ 1974910 | H | Ar11 | D1~D1175 | |
| 1974911 ~ 1976085 | H | Ar12 | D1~D1175 | |
| 1976086 ~ 1977260 | H | Ar13 | D1~D1175 | |
| 1977261 ~ 1978435 | H | Ar14 | D1~D1175 | |
| 1978436 ~ 1979610 | H | Ar15 | D1~D1175 | |
| 1979611 ~ 1980785 | H | Ar16 | D1~D1175 | |
| 1980786 ~ 1981960 | H | Ar17 | D1~D1175 | |
| 1981961 ~ 1983135 | H | Ar18 | D1~D1175 | |
| 1983136 ~ 1984310 | H | Ar19 | D1~D1175 | |
| 1984311 ~ 1985485 | H | Ar20 | D1~D1175 | |
| 1985486 ~ 1986660 | H | Ar21 | D1~D1175 | |
| 1986661 ~ 1987835 | H | Ar22 | D1~D1175 | |
| 1987836 ~ 1989010 | H | Ar23 | D1~D1175 | |
| 1989011 ~ 1990185 | H | Ar24 | D1~D1175 | |
| 1990186 ~ 1991360 | H | Ar25 | D1~D1175 | |
| 1991361 ~ 1992535 | H | Ar26 | D1~D1175 | |
| 1992536 ~ 1993710 | H | Ar27 | D1~D1175 | |
| 1993711 ~ 1994885 | H | Ar28 | D1~D1175 | |
| 1994886 ~ 1996060 | H | Ar29 | D1~D1175 | |
| 1996061 ~ 1997235 | H | Ar30 | D1~D1175 | |
| 1997236 ~ 1998410 | H | Ar31 | D1~D1175 | |
| 1998411 ~ 1999585 | H | Ar32 | D1~D1175 | |
| 1999586 ~ 2000760 | H | Ar33 | D1~D1175 | |
| 2000761 ~ 2001935 | H | Ar34 | D1~D1175 | |

| No. | $R^2$ | $R^4$ | $R^5$ |
|---|---|---|---|
| 2001936 ~ 2003110 | H | Ar35 | D1~D1175 |
| 2003111 ~ 2004285 | H | Ar36 | D1~D1175 |
| 2004286 ~ 2005460 | H | Ar37 | D1~D1175 |
| 2005461 ~ 2006635 | H | Ar38 | D1~D1175 |
| 2006636 ~ 2007810 | H | Ar39 | D1~D1175 |
| 2007811 ~ 2008985 | H | Ar40 | D1~D1175 |
| 2008986 ~ 2010160 | H | Ar41 | D1~D1175 |
| 2010161 ~ 2011335 | H | Ar42 | D1~D1175 |
| 2011336 ~ 2012510 | H | Ar43 | D1~D1175 |
| 2012511 ~ 2013685 | H | Ar44 | D1~D1175 |
| 2013686 ~ 2014860 | H | Ar45 | D1~D1175 |
| 2014861 ~ 2016035 | H | Ar46 | D1~D1175 |
| 2016036 ~ 2017210 | H | Ar47 | D1~D1175 |
| 2017211 ~ 2018385 | H | Ar48 | D1~D1175 |
| 2018386 ~ 2019560 | H | Ar49 | D1~D1175 |
| 2019561 ~ 2020735 | H | Ar50 | D1~D1175 |
| 2020736 ~ 2021910 | H | Ar51 | D1~D1175 |
| 2021911 ~ 2023085 | H | Ar52 | D1~D1175 |
| 2023086 ~ 2024260 | H | Ar53 | D1~D1175 |
| 2024261 ~ 2025435 | H | Ar54 | D1~D1175 |
| 2025436 ~ 2026610 | H | Ar55 | D1~D1175 |
| 2026611 ~ 2027785 | H | Ar56 | D1~D1175 |
| 2027786 ~ 2028960 | H | Ar57 | D1~D1175 |
| 2028961 ~ 2030135 | H | Ar58 | D1~D1175 |
| 2030136 ~ 2031310 | H | Ar59 | D1~D1175 |

In Tables 1 to 3, the structures where $Ar^1$ and $Ar^2$ in the general formula (1) are a deuterated carbazolyl group (D717) are specified as the structures of Compounds 1 to 2031310. In Table 4, $Ar^1$ and $Ar^2$ in Compounds 1 to 2031310 were changed as in Table 4, and the resultant compounds were sequentially displayed as in the table format. In Table 4, Compounds 1 to 2031310 are also shown in the first row for clarifying the correspondence relationship. In the second row in Table 4, for example, Compound 1(1) shows a compound having a structure formed by replacing $Ar^2$ in Compound 1 to Ar1. Compound 2(1) indicates a compound having a structure in which $Ar^2$ of Compound 2 is changed to Ar1. Compound 2031310(1) indicates a compound having a structure in which $Ar^2$ of Compound 2031310 is changed to Ar1. Compounds 1(2) to 2031310(2) in the third row in Table 4 and the subsequent compounds are also specified in the same manner. $X^1$ to

$X^3$ of the compounds specified in Table 4 are all nitrogen atoms (N), $L^1$ is a single bond, and $R^1$ is a hydrogen atom.

Table 4

| No. | $Ar^1$ | $Ar^2$ |
|---|---|---|
| 1 ~ 2031310 | D717 | D717 |
| 1(1) ~ 2031310(1) | D717 | Ar1 |
| 1(2) ~ 2031310(2)<br>1(3) ~ 2031310(3) | D717<br>D717 | Ar2<br>Ar3 |
| 1(4) ~ 2031310(4) | D717 | Ar4 |
| 1(5) ~ 2031310(5)<br>1(6) ~ 2031310(6) | D717<br>D717 | Ar5<br>Ar6 |
| 1(7) ~ 2031310(7) | D717 | Ar7 |
| 1(8) ~ 2031310(8)<br>1(9) ~ 2031310(9) | D717<br>D717 | Ar8<br>Ar9 |
| 1(10) ~ 2031310(10) | D717 | Ar10 |
| 1(11) ~ 2031310(11) | D717 | Ar11 |
| 1(12) ~ 2031310(12) | D717 | Ar12 |
| 1(13) ~ 2031310(13) | D717 | Ar13 |
| 1(14) ~ 2031310(14) | D717 | Ar14 |
| 1(15) ~ 2031310(15) | D717 | Ar15 |
| 1(16) ~ 2031310(16) | D717 | Ar16 |
| 1(17) ~ 2031310(17) | D717 | Ar17 |
| 1(18) ~ 2031310(18) | D717 | Ar18 |
| 1(19) ~ 2031310(19) | D717 | Ar19 |
| 1(20) ~ 2031310(20) | D717 | Ar20 |
| 1(21) ~ 2031310(21) | D717 | Ar21 |
| 1(22) ~ 2031310(22) | D717 | Ar22 |
| 1(23) ~ 2031310(23) | D717 | Ar23 |
| 1(24) ~ 2031310(24) | D717 | Ar24 |
| 1(25) ~ 2031310(25) | D717 | Ar25 |
| 1(26) ~ 2031310(26) | D717 | Ar26 |
| 1(27) ~ 2031310(27) | D717 | Ar27 |
| 1(28) ~ 2031310(28) | D717 | Ar28 |
| 1(29) ~ 2031310(29) | D717 | Ar29 |
| 1(30) ~ 2031310(30) | D717 | Ar30 |
| 1(31) ~ 2031310(31) | D717 | Ar31 |
| 1(32) ~ 2031310(32) | D717 | Ar32 |
| 1(33) ~ 2031310(33) | D717 | Ar33 |
| 1(34) ~ 2031310(34) | D717 | Ar34 |
| 1(35) ~ 2031310(35) | D717 | Ar35 |
| 1(36) ~ 2031310(36) | D717 | Ar36 |
| 1(37) ~ 2031310(37) | D717 | Ar37 |
| 1(38) ~ 2031310(38) | D717 | Ar38 |

(continued)

| No. | Ar1 | Ar2 |
|---|---|---|
| 1(39) ~ 2031310(39) | D717 | Ar39 |
| 1(40) ~ 2031310(40) | D717 | Ar40 |
| 1(41) ~ 2031310(41) | D717 | Ar41 |
| 1(42) ~ 2031310(42) | D717 | Ar42 |
| 1(43) ~ 2031310(43) | D717 | Ar43 |
| 1(44) ~ 2031310(44) | D717 | Ar44 |
| 1(45) ~ 2031310(45) | D717 | Ar45 |
| 1(46) ~ 2031310(46) | D717 | Ar46 |
| 1(47) ~ 2031310(47) | D717 | Ar47 |
| 1(48) ~ 2031310(48) | D717 | Ar48 |
| 1(49) ~ 2031310(49) | D717 | Ar49 |
| 1(50) ~ 2031310(50) | D717 | Ar50 |
| 1(51) ~ 2031310(51) | D717 | Ar51 |
| 1(52) ~ 2031310(52) | D717 | Ar52 |
| 1(53) ~ 2031310(53) | D717 | Ar53 |
| 1(54) ~ 2031310(54) | D717 | Ar54 |
| 1(55) ~ 2031310(55) | D717 | Ar55 |
| 1(56) ~ 2031310(56) | D717 | Ar56 |
| 1(57) ~ 2031310(57) | D717 | Ar57 |
| 1(58) ~ 2031310(58) | D717 | Ar58 |
| 1(59) ~ 2031310(59) | D717 | Ar59 |
| 1(60) ~ 2031310(60) | D1 | Ar1 |
| 1(61) ~ 2031310(61) | D1 | Ar2 |
| 1(62) ~ 2031310(62) | D1 | Ar3 |
| 1(63) ~ 2031310(63) | D1 | Ar4 |
| 1(64) ~ 2031310(64) | D1 | Ar5 |
| 1(65) ~ 2031310(65) | D1 | Ar6 |
| 1(66) ~ 2031310(66) | D1 | Ar7 |
| 1(67) ~ 2031310(67) | D1 | Ar8 |
| 1(68) ~ 2031310(68) | D1 | Ar9 |
| 1(69) ~ 2031310(69) | D1 | Ar10 |
| 1(70) ~ 2031310(70) | D1 | Ar11 |
| 1(71) ~ 2031310(71) | D1 | Ar12 |
| 1(72) ~ 2031310(72) | D1 | Ar13 |
| 1(73) ~ 2031310(73) | D1 | Ar14 |
| 1(74) ~ 2031310(74) | D1 | Ar15 |
| 1(75) ~ 2031310(75) | D1 | Ar16 |
| 1(76) ~ 2031310(76) | D1 | Ar17 |
| 1(77) ~ 2031310(77) | D1 | Ar18 |
| 1(78) ~ 2031310(78) | D1 | Ar19 |
| 1(79) ~ 2031310(79) | D1 | Ar20 |

(continued)

| No. | Ar1 | Ar2 |
|---|---|---|
| 1(80) ~ 2031310(80) | D1 | Ar21 |
| 1(81) ~ 2031310(81) | D1 | Ar22 |
| 1(82) ~ 2031310(82) | D1 | Ar23 |
| 1(83) ~ 2031310(83) | D1 | Ar24 |
| 1(84) ~ 2031310(84) | D1 | Ar25 |
| 1(85) ~ 2031310(85) | D1 | Ar26 |
| 1(86) ~ 2031310(86) | D1 | Ar27 |
| 1(87) ~ 2031310(87) | D1 | Ar28 |
| 1(88) ~ 2031310(88) | D1 | Ar29 |
| 1(89) ~ 2031310(89) | D1 | Ar30 |
| 1(90) ~ 2031310(90) | D1 | Ar31 |
| 1(91) ~ 2031310(91) | D1 | Ar32 |
| 1(92) ~ 2031310(92) | D1 | Ar33 |
| 1(93) ~ 2031310(93) | D1 | Ar34 |
| 1(94) ~ 2031310(94) | D1 | Ar35 |
| 1(95) ~ 2031310(95) | D1 | Ar36 |
| 1(96) ~ 2031310(96) | D1 | Ar37 |
| 1(97) ~ 2031310(97) | D1 | Ar38 |
| 1(98) ~ 2031310(98) | D1 | Ar39 |
| 1(99) ~ 2031310(99) | D1 | Ar40 |
| 1(100) ~ 2031310(100) | D1 | Ar41 |
| 1(101) ~ 2031310(101) | D1 | Ar42 |
| 1(102) ~ 2031310(102) | D1 | Ar43 |
| 1(103) ~ 2031310(103) | D1 | Ar44 |
| 1(104) ~ 2031310(104) | D1 | Ar45 |
| 1(105) ~ 2031310(105) | D1 | Ar46 |
| 1(106) ~ 2031310(106) | D1 | Ar47 |
| 1(107) ~ 2031310(107) | D1 | Ar48 |
| 1(108) ~ 2031310(108) | D1 | Ar49 |
| 1(109) ~ 2031310(109) | D1 | Ar50 |
| 1(110) ~ 2031310(110) | D1 | Ar51 |
| 1(111) ~ 2031310(111) | D1 | Ar52 |
| 1(112) ~ 2031310(112) | D1 | Ar53 |
| 1(113) ~ 2031310(113) | D1 | Ar54 |
| 1(114) ~ 2031310(114) | D1 | Ar55 |
| 1(115) ~ 2031310(115) | D1 | Ar56 |
| 1(116) ~ 2031310(116) | D1 | Ar57 |
| 1(117) ~ 2031310(117) | D1 | Ar58 |
| 1(118) ~ 2031310(118) | D1 | Ar59 |
| 1(119) ~ 2031310(119) | D7 | Ar1 |

(continued)

| No. | $Ar^1$ | $Ar^2$ |
|---|---|---|
| 1(120) ~ 2031310(120) | D7 | Ar2 |
| 1(121) ~ 2031310(121)<br>1(122) ~ 2031310(122) | D7<br>D7 | Ar3<br>Ar4 |
| 1(123) ~ 2031310(123) | D7 | Ar5 |
| 1(124) ~ 2031310(124)<br>1(125) ~ 2031310(125) | D7<br>D7 | Ar6<br>Ar7 |
| 1(126) ~ 2031310(126) | D7 | Ar8 |
| 1(127) ~ 2031310(127)<br>1(128) ~ 2031310(128) | D7<br>D7 | Ar9<br>Ar10 |
| 1(129) ~ 2031310(129) | D7 | Ar11 |
| 1(130) ~ 2031310(130) | D7 | Ar12 |
| 1(131) ~ 2031310(131) | D7 | Ar13 |
| 1(132) ~ 2031310(132) | D7 | Ar14 |
| 1(133) ~ 2031310(133) | D7 | Ar15 |
| 1(134) ~ 2031310(134) | D7 | Ar16 |
| 1(135) ~ 2031310(135) | D7 | Ar17 |
| 1(136) ~ 2031310(136) | D7 | Ar18 |
| 1(137) ~ 2031310(137) | D7 | Ar19 |
| 1(138) ~ 2031310(138) | D7 | Ar20 |
| 1(139) ~ 2031310(139) | D7 | Ar21 |
| 1(140) ~ 2031310(140) | D7 | Ar22 |
| 1(141) ~ 2031310(141) | D7 | Ar23 |
| 1(142) ~ 2031310(142) | D7 | Ar24 |
| 1(143) ~ 2031310(143) | D7 | Ar25 |
| 1(144) ~ 2031310(144) | D7 | Ar26 |
| 1(145) ~ 2031310(145) | D7 | Ar27 |
| 1(146) ~ 2031310(146) | D7 | Ar28 |
| 1(147) ~ 2031310(147) | D7 | Ar29 |
| 1(148) ~ 2031310(148) | D7 | Ar30 |
| 1(149) ~ 2031310(149) | D7 | Ar31 |
| 1(150) ~ 2031310(150) | D7 | Ar32 |
| 1(151) ~ 2031310(151) | D7 | Ar33 |
| 1(152) ~ 2031310(152) | D7 | Ar34 |
| 1(153) ~ 2031310(153) | D7 | Ar35 |
| 1(154) ~ 2031310(154) | D7 | Ar36 |
| 1(155) ~ 2031310(155) | D7 | Ar37 |
| 1(156) ~ 2031310(156) | D7 | Ar38 |
| 1(157) ~ 2031310(157) | D7 | Ar39 |
| 1(158) ~ 2031310(158) | D7 | Ar40 |
| 1(159) ~ 2031310(159) | D7 | Ar41 |

(continued)

| No. | $Ar^1$ | $Ar^2$ |
|---|---|---|
| 1(160) ~ 2031310(160) | D7 | Ar42 |
| 1(161) ~ 2031310(161) | D7 | Ar43 |
| 1(162) ~ 2031310(162) | D7 | Ar44 |
| 1(163) ~ 2031310(163) | D7 | Ar45 |
| 1(164) ~ 2031310(164)<br>1(165) ~ 2031310(165)<br>1(166) ~ 2031310(166) | D7<br>D7<br>D7 | Ar46<br>Ar47<br>Ar48 |
| 1(167) - 2031310(167) | D7 | Ar49 |
| 1(168) ~ 2031310(168)<br>1(169) ~ 2031310(169) | D7<br>D7 | Ar50<br>Ar51 |
| 1(170) ~ 2031310(170) | D7 | Ar52 |
| 1(171) ~ 2031310(171) | D7 | Ar53 |
| 1(172) ~ 2031310(172) | D7 | Ar54 |
| 1(173) ~ 2031310(173) | D7 | Ar55 |
| 1(174) ~ 2031310(174) | D7 | Ar56 |
| 1(175) ~ 2031310(175) | D7 | Ar57 |
| 1(176) ~ 2031310(176) | D7 | Ar58 |
| 1(177) ~ 2031310(177) | D7 | Ar59 |
| 1(178) ~ 2031310(178) | D8 | Ar1 |
| 1(179) ~ 2031310(179) | D8 | Ar2 |
| 1(180) ~ 2031310(180) | D8 | Ar3 |
| 1(181) ~ 2031310(181) | D8 | Ar4 |
| 1(182) ~ 2031310(182) | D8 | Ar5 |
| 1(183) ~ 2031310(183) | D8 | Ar6 |
| 1(184) ~ 2031310(184) | D8 | Ar7 |
| 1(185) ~ 2031310(185) | D8 | Ar8 |
| 1(186) ~ 2031310(186) | D8 | Ar9 |
| 1(187) ~ 2031310(187) | D8 | Ar10 |
| 1(188) ~ 2031310(188) | D8 | Ar11 |
| 1(189) ~ 2031310(189) | D8 | Ar12 |
| 1(190) ~ 2031310(190) | D8 | Ar13 |
| 1(191) ~ 2031310(191) | D8 | Ar14 |
| 1(192) ~ 2031310(192) | D8 | Ar15 |
| 1(193) ~ 2031310(193) | D8 | Ar16 |
| 1(194) ~ 2031310(194) | D8 | Ar17 |
| 1(195) ~ 2031310(195) | D8 | Ar18 |
| 1(196) ~ 2031310(196) | D8 | Ar19 |
| 1(197) ~ 2031310(197) | D8 | Ar20 |
| 1(198) ~ 2031310(198) | D8 | Ar21 |
| 1(199) ~ 2031310(199) | D8 | Ar22 |

(continued)

| No. | $Ar^1$ | $Ar^2$ |
|---|---|---|
| 1(200) ~ 2031310(200) | D8 | Ar23 |
| 1(201) ~ 2031310(201) | D8 | Ar24 |
| 1(202) ~ 2031310(202) | D8 | Ar25 |
| 1(203) ~ 2031310(203)<br>1(204) ~ 2031310(204) | D8<br>D8 | Ar26<br>Ar27 |
| 1(205) ~ 2031310(205)<br>1(206) ~ 2031310(206) | D8<br>D8 | Ar28<br>Ar29 |
| 1(207) ~ 2031310(207) | D8 | Ar30 |
| 1(208) ~ 2031310(208) | D8 | Ar31 |
| 1(209) ~ 2031310(209) | D8 | Ar32 |
| 1(210) ~ 2031310(210) | D8 | Ar33 |
| 1(211) ~ 2031310(211) | D8 | Ar34 |
| 1(212) ~ 2031310(212) | D8 | Ar35 |
| 1(213) ~ 2031310(213) | D8 | Ar36 |
| 1(214) ~ 2031310(214) | D8 | Ar37 |
| 1(215) ~ 2031310(215) | D8 | Ar38 |
| 1(216) ~ 2031310(216) | D8 | Ar39 |
| 1(217) ~ 2031310(217) | D8 | Ar40 |
| 1(218) ~ 2031310(218) | D8 | Ar41 |
| 1(219) ~ 2031310(219)<br>1(220) ~ 2031310(220) | D8<br>D8 | Ar42<br>Ar43 |
| 1(221) ~ 2031310(221) | D8 | Ar44 |
| 1(222) ~ 2031310(222) | D8 | Ar45 |
| 1(223) ~ 2031310(223) | D8 | Ar46 |
| 1(224) ~ 2031310(224) | D8 | Ar47 |
| 1(225) ~ 2031310(225) | D8 | Ar48 |
| 1(226) ~ 2031310(226) | D8 | Ar49 |
| 1(227) ~ 2031310(227) | D8 | Ar50 |
| 1(228) ~ 2031310(228) | D8 | Ar51 |
| 1(229) ~ 2031310(229) | D8 | Ar52 |
| 1(230) ~ 2031310(230) | D8 | Ar53 |
| 1(231) ~ 2031310(231) | D8 | Ar54 |
| 1(232) ~ 2031310(232) | D8 | Ar55 |
| 1(233) ~ 2031310(233) | D8 | Ar56 |
| 1(234) ~ 2031310(234) | D8 | Ar57 |
| 1(235) ~ 2031310(235) | D8 | Ar58 |
| 1(236) ~ 2031310(236) | D8 | Ar59 |
| 1(237) ~ 2031310(237) | D9 | Ar1 |
| 1(238) ~ 2031310(238) | D9 | Ar2 |
| 1(239) ~ 2031310(239) | D9 | Ar3 |

(continued)

| No. | $Ar^1$ | $Ar^2$ |
|---|---|---|
| 1(240) ~ 2031310(240) | D9 | Ar4 |
| 1(241) ~ 2031310(241) | D9 | Ar5 |
| 1(242) ~ 2031310(242) | D9 | Ar6 |
| 1(243) ~ 2031310(243) | D9 | Ar7 |
| 1(244) ~ 2031310(244) | D9 | Ar8 |
| 1(245) ~ 2031310(245) | D9 | Ar9 |
| 1(246) ~ 2031310(246) | D9 | Ar10 |
| 1(247) ~ 2031310(247) | D9 | Ar11 |
| 1(248) ~ 2031310(248) | D9 | Ar12 |
| 1(249) ~ 2031310(249) | D9 | Ar13 |
| 1(250) ~ 2031310(250) | D9 | Ar14 |
| 1(251) ~ 2031310(251) | D9 | Ar15 |
| 1(252) ~ 2031310(252) | D9 | Ar16 |
| 1(253) ~ 2031310(253) | D9 | Ar17 |
| 1(254) ~ 2031310(254) | D9 | Ar18 |
| 1(255) ~ 2031310(255) | D9 | Ar19 |
| 1(256) ~ 2031310(256) | D9 | Ar20 |
| 1(257) ~ 2031310(257) | D9 | Ar21 |
| 1(258) ~ 2031310(258) | D9 | Ar22 |
| 1(259) ~ 2031310(259) | D9 | Ar23 |
| 1(260) ~ 2031310(260) | D9 | Ar24 |
| 1(261) ~ 2031310(261) | D9 | Ar25 |
| 1(262) ~ 2031310(262) | D9 | Ar26 |
| 1(263) ~ 2031310(263) | D9 | Ar27 |
| 1(264) ~ 2031310(264) | D9 | Ar28 |
| 1(265) ~ 2031310(265) | D9 | Ar29 |
| 1(266) ~ 2031310(266) | D9 | Ar30 |
| 1(267) ~ 2031310(267) | D9 | Ar31 |
| 1(268) ~ 2031310(268) | D9 | Ar32 |
| 1(269) ~ 2031310(269) | D9 | Ar33 |
| 1(270) ~ 2031310(270) | D9 | Ar34 |
| 1(271) ~ 2031310(271) | D9 | Ar35 |
| 1(272) ~ 2031310(272) | D9 | Ar36 |
| 1(273) ~ 2031310(273) | D9 | Ar37 |
| 1(274) ~ 2031310(274) | D9 | Ar38 |
| 1(275) ~ 2031310(275) | D9 | Ar39 |
| 1(276) ~ 2031310(276) | D9 | Ar40 |
| 1(277) ~ 2031310(277) | D9 | Ar41 |
| 1(278) ~ 2031310(278) | D9 | Ar42 |
| 1(279) ~ 2031310(279) | D9 | Ar43 |

(continued)

| No. | $Ar^1$ | $Ar^2$ |
|---|---|---|
| 1(280) ~ 2031310(280) | D9 | Ar44 |
| 1(281) ~ 2031310(281) | D9 | Ar45 |
| 1(282) ~ 2031310(282) | D9 | Ar46 |
| 1(283) ~ 2031310(283) | D9 | Ar47 |
| 1(284) ~ 2031310(284) | D9 | Ar48 |
| 1(285) ~ 2031310(285) | D9 | Ar49 |
| 1(286) ~ 2031310(286) | D9 | Ar50 |
| 1(287) ~ 2031310(287) | D9 | Ar51 |
| 1(288) ~ 2031310(288) | D9 | Ar52 |
| 1(289) ~ 2031310(289) | D9 | Ar53 |
| 1(290) ~ 2031310(290) | D9 | Ar54 |
| 1(291) ~ 2031310(291) | D9 | Ar55 |
| 1(292) ~ 2031310(292) | D9 | Ar56 |
| 1(293) ~ 2031310(293) | D9 | Ar57 |
| 1(294) ~ 2031310(294) | D9 | Ar58 |
| 1(295) ~ 2031310(295) | D9 | Ar59 |
| 1(296) ~ 2031310(296) | D19 | Ar1 |
| 1(297) ~ 2031310(297) | D19 | Ar2 |
| 1(298) ~ 2031310(298) | D19 | Ar3 |
| 1(299) ~ 2031310(299) | D19 | Ar4 |
| 1(300) ~ 2031310(300) | D19 | Ar5 |
| 1(301) ~ 2031310(301) | D19 | Ar6 |
| 1(302) ~ 2031310(302) | D19 | Ar7 |
| 1(303) ~ 2031310(303) | D19 | Ar8 |
| 1(304) ~ 2031310(304) | D19 | Ar9 |
| 1(305) ~ 2031310(305) | D19 | Ar10 |
| 1(306) ~ 2031310(306) | D19 | Ar11 |
| 1(307) ~ 2031310(307) | D19 | Ar12 |
| 1(308) ~ 2031310(308) | D19 | Ar13 |
| 1(309) ~ 2031310(309) | D19 | Ar14 |
| 1(310) ~ 2031310(310) | D19 | Ar15 |
| 1(311) ~ 2031310(311) | D19 | Ar16 |
| 1(312) ~ 2031310(312) | D19 | Ar17 |
| 1(313) ~ 2031310(313) | D19 | Ar18 |
| 1(314) ~ 2031310(314) | D19 | Ar19 |
| 1(315) ~ 2031310(315) | D19 | Ar20 |
| 1(316) ~ 2031310(316) | D19 | Ar21 |
| 1(317) ~ 2031310(317) | D19 | Ar22 |
| 1(318) ~ 2031310(318) | D19 | Ar23 |

(continued)

| No. | $Ar^1$ | $Ar^2$ |
|---|---|---|
| 1(319) ~ 2031310(319) | D19 | Ar24 |
| 1(320) ~ 2031310(320) | D19 | Ar25 |
| 1(321) ~ 2031310(321) | D19 | Ar26 |
| 1(322) ~ 2031310(322) | D19 | Ar27 |
| 1(323) ~ 2031310(323) | D19 | Ar28 |
| 1(324) ~ 2031310(324) | D19 | Ar29 |
| 1(325) ~ 2031310(325) | D19 | Ar30 |
| 1(326) ~ 2031310(326) | D19 | Ar31 |
| 1(327) ~ 2031310(327) | D19 | Ar32 |
| 1(328) ~ 2031310(328) | D19 | Ar33 |
| 1(329) ~ 2031310(329) | D19 | Ar34 |
| 1(330) ~ 2031310(330) | D19 | Ar35 |
| 1(331) ~ 2031310(331) | D19 | Ar36 |
| 1(332) ~ 2031310(332) | D19 | Ar37 |
| 1(333) ~ 2031310(333) | D19 | Ar38 |
| 1(334) ~ 2031310(334) | D19 | Ar39 |
| 1(335) ~ 2031310(335) | D19 | Ar40 |
| 1(336) ~ 2031310(336) | D19 | Ar41 |
| 1(337) ~ 2031310(337) | D19 | Ar42 |
| 1(338) ~ 2031310(338) | D19 | Ar43 |
| 1(339) ~ 2031310(339) | D19 | Ar44 |
| 1(340) ~ 2031310(340) | D19 | Ar45 |
| 1(341) ~ 2031310(341) | D19 | Ar46 |
| 1(342) ~ 2031310(342) | D19 | Ar47 |
| 1(343) ~ 2031310(343) | D19 | Ar48 |
| 1(344) ~ 2031310(344) | D19 | Ar49 |
| 1(345) ~ 2031310(345) | D19 | Ar50 |
| 1(346) ~ 2031310(346) | D19 | Ar51 |
| 1(347) ~ 2031310(347) | D19 | Ar52 |
| 1(348) ~ 2031310(348) | D19 | Ar53 |
| 1(349) ~ 2031310(349) | D19 | Ar54 |
| 1(350) ~ 2031310(350) | D19 | Ar55 |
| 1(351) ~ 2031310(351) | D19 | Ar56 |
| 1(352) ~ 2031310(352) | D19 | Ar57 |
| 1(353) ~ 2031310(353) | D19 | Ar58 |
| 1(354) ~ 2031310(354) | D19 | Ar59 |
| 1(355) ~ 2031310(355) | D37 | Ar1 |
| 1(356) ~ 2031310(356) | D37 | Ar2 |
| 1(357) ~ 2031310(357) | D37 | Ar3 |
| 1(358) ~ 2031310(358) | D37 | Ar4 |

(continued)

| No. | $Ar^1$ | $Ar^2$ |
|---|---|---|
| 1(359) ~ 2031310(359) | D37 | Ar5 |
| 1(360) ~ 2031310(360) | D37 | Ar6 |
| 1(361) ~ 2031310(361) | D37 | Ar7 |
| 1(362) ~ 2031310(362) | D37 | Ar8 |
| 1(363) ~ 2031310(363) | D37 | Ar9 |
| 1(364) ~ 2031310(364) | D37 | Ar10 |
| 1(365) ~ 2031310(365) | D37 | Ar11 |
| 1(366) ~ 2031310(366) | D37 | Ar12 |
| 1(367) ~ 2031310(367) | D37 | Ar13 |
| 1(368) ~ 2031310(368) | D37 | Ar14 |
| 1(369) ~ 2031310(369) | D37 | Ar15 |
| 1(370) ~ 2031310(370) | D37 | Ar16 |
| 1(371) ~ 2031310(371) | D37 | Ar17 |
| 1(372) ~ 2031310(372) | D37 | Ar18 |
| 1(373) ~ 2031310(373) | D37 | Ar19 |
| 1(374) ~ 2031310(374) | D37 | Ar20 |
| 1(375) ~ 2031310(375) | D37 | Ar21 |
| 1(376) ~ 2031310(376) | D37 | Ar22 |
| 1(377) ~ 2031310(377) | D37 | Ar23 |
| 1(378) ~ 2031310(378) | D37 | Ar24 |
| 1(379) ~ 2031310(379) | D37 | Ar25 |
| 1(380) ~ 2031310(380) | D37 | Ar26 |
| 1(381) ~ 2031310(381) | D37 | Ar27 |
| 1(382) ~ 2031310(382) | D37 | Ar28 |
| 1(383) ~ 2031310(383) | D37 | Ar29 |
| 1(384) ~ 2031310(384) | D37 | Ar30 |
| 1(385) ~ 2031310(385) | D37 | Ar31 |
| 1(386) ~ 2031310(386) | D37 | Ar32 |
| 1(387) ~ 2031310(387) | D37 | Ar33 |
| 1(388) ~ 2031310(388) | D37 | Ar34 |
| 1(389) ~ 2031310(389) | D37 | Ar35 |
| 1(390) ~ 2031310(390) | D37 | Ar36 |
| 1(391) ~ 2031310(391) | D37 | Ar37 |
| 1(392) ~ 2031310(392) | D37 | Ar38 |
| 1(393) ~ 2031310(393) | D37 | Ar39 |
| 1(394) ~ 2031310(394) | D37 | Ar40 |
| 1(395) ~ 2031310(395) | D37 | Ar41 |
| 1(396) ~ 2031310(396) | D37 | Ar42 |
| 1(397) ~ 2031310(397) | D37 | Ar43 |
| 1(398) ~ 2031310(398) | D37 | Ar44 |
| 1(399) ~ 2031310(399) | D37 | Ar45 |

(continued)

| No. | Ar1 | Ar2 |
|---|---|---|
| 1(400) ~ 2031310(400) | D37 | Ar46 |
| 1(401) ~ 2031310(401) | D37 | Ar47 |
| 1(402) ~ 2031310(402) | D37 | Ar48 |
| 1(403) ~ 2031310(403) | D37 | Ar49 |
| 1(404) ~ 2031310(404) | D37 | Ar50 |
| 1(405) ~ 2031310(405) | D37 | Ar51 |
| 1(406) ~ 2031310(406) | D37 | Ar52 |
| 1(407) ~ 2031310(407) | D37 | Ar53 |
| 1(408) ~ 2031310(408) | D37 | Ar54 |
| 1(409) ~ 2031310(409) | D37 | Ar55 |
| 1(410) ~ 2031310(410) | D37 | Ar56 |
| 1(411) ~ 2031310(411) | D37 | Ar57 |
| 1(412) ~ 2031310(412) | D37 | Ar58 |
| 1(413) ~ 2031310(413) | D37 | Ar59 |
| 1(414) ~ 2031310(414) | D50 | Ar1 |
| 1(415) ~ 2031310(415) | D50 | Ar2 |
| 1(416) ~ 2031310(416) | D50 | Ar3 |
| 1(417) ~ 2031310(417) | D50 | Ar4 |
| 1(418) ~ 2031310(418) | D50 | Ar5 |
| 1(419) ~ 2031310(419) | D50 | Ar6 |
| 1(420) ~ 2031310(420) | D50 | Ar7 |
| 1(421) ~ 2031310(421) | D50 | Ar8 |
| 1(422) ~ 2031310(422) | D50 | Ar9 |
| 1(423) ~ 2031310(423) | D50 | Ar10 |
| 1(424) ~ 2031310(424) | D50 | Ar11 |
| 1(425) ~ 2031310(425) | D50 | Ar12 |
| 1(426) ~ 2031310(426) | D50 | Ar13 |
| 1(427) ~ 2031310(427) | D50 | Ar14 |
| 1(428) ~ 2031310(428) | D50 | Ar15 |
| 1(429) ~ 2031310(429) | D50 | Ar16 |
| 1(430) ~ 2031310(430) | D50 | Arl7 |
| 1(431) ~ 2031310(431) | D50 | Ar18 |
| 1(432) ~ 2031310(432) | D50 | Ar19 |
| 1(433) ~ 2031310(433) | D50 | Ar20 |
| 1(434) ~ 2031310(434) | D50 | Ar21 |
| 1(435) ~ 2031310(435) | D50 | Ar22 |
| 1(436) ~ 2031310(436) | D50 | Ar23 |
| 1(437) ~ 2031310(437) | D50 | Ar24 |
| 1(438) ~ 2031310(438) | D50 | Ar25 |
| 1(439) ~ 2031310(439) | D50 | Ar26 |

(continued)

| No. | Ar1 | Ar2 |
|---|---|---|
| 1(440) ~ 2031310(440) | D50 | Ar27 |
| 1(441) ~ 2031310(441) | D50 | Ar28 |
| 1(442) ~ 2031310(442) | D50 | Ar29 |
| 1(443) ~ 2031310(443) | D50 | Ar30 |
| 1(444) ~ 2031310(444) | D50 | Ar31 |
| 1(445) ~ 2031310(445) | D50 | Ar32 |
| 1(446) ~ 2031310(446) | D50 | Ar33 |
| 1(447) ~ 2031310(447) | D50 | Ar34 |
| 1(448) ~ 2031310(448) | D50 | Ar35 |
| 1(449) ~ 2031310(449) | D50 | Ar36 |
| 1(450) ~ 2031310(450) | D50 | Ar37 |
| 1(451) ~ 2031310(451) | D50 | Ar38 |
| 1(452) ~ 2031310(452) | D50 | Ar39 |
| 1(453) ~ 2031310(453) | D50 | Ar40 |
| 1(454) ~ 2031310(454) | D50 | Ar41 |
| 1(455) ~ 2031310(455) | D50 | Ar42 |
| 1(456) ~ 2031310(456) | D50 | Ar43 |
| 1(457) ~ 2031310(457) | D50 | Ar44 |
| 1(458) ~ 2031310(458) | D50 | Ar45 |
| 1(459) ~ 2031310(459) | D50 | Ar46 |
| 1(460) ~ 2031310(460) | D50 | Ar47 |
| 1(461) ~ 2031310(461) | D50 | Ar48 |
| 1(462) ~ 2031310(462) | D50 | Ar49 |
| 1(463) ~ 2031310(463) | D50 | Ar50 |
| 1(464) ~ 2031310(464) | D50 | Ar51 |
| 1(465) ~ 2031310(465) | D50 | Ar52 |
| 1(466) ~ 2031310(466) | D50 | Ar53 |
| 1(467) ~ 2031310(467) | D50 | Ar54 |
| 1(468) ~ 2031310(468) | D50 | Ar55 |
| 1(469) ~ 2031310(469) | D50 | Ar56 |
| 1(470) ~ 2031310(470) | D50 | Ar57 |
| 1(471) ~ 2031310(471) | D50 | Ar58 |
| 1(472) ~ 2031310(472) | D50 | Ar59 |
| 1(473) ~ 2031310(473) | D77 | Ar1 |
| 1(474) ~ 2031310(474) | D77 | Ar2 |
| 1(475) ~ 2031310(475) | D77 | Ar3 |
| 1(476) ~ 2031310(476) | D77 | Ar4 |
| 1(477) ~ 2031310(477) | D77 | Ar5 |
| 1(478) ~ 2031310(478) | D77 | Ar6 |
| 1(479) ~ 2031310(479) | D77 | Ar7 |
| 1(480) ~ 2031310(480) | D77 | Ar8 |

(continued)

| No. | $Ar^1$ | $Ar^2$ |
|---|---|---|
| 1(481) ~ 2031310(481) | D77 | Ar9 |
| 1(482) ~ 2031310(482) | D77 | Ar10 |
| 1(483) ~ 2031310(483) | D77 | Ar11 |
| 1(484) ~ 2031310(484) | D77 | Ar12 |
| 1(485) ~ 2031310(485) | D77 | Ar13 |
| 1(486) ~ 2031310(486) | D77 | Ar14 |
| 1(487) ~ 2031310(487) | D77 | Ar15 |
| 1(488) ~ 2031310(488) | D77 | Ar16 |
| 1(489) ~ 2031310(489) | D77 | Ar17 |
| 1(490) ~ 2031310(490) | D77 | Ar18 |
| 1(491) ~ 2031310(491) | D77 | Ar19 |
| 1(492) ~ 2031310(492) | D77 | Ar20 |
| 1(493) ~ 2031310(493) | D77 | Ar21 |
| 1(494) ~ 2031310(494) | D77 | Ar22 |
| 1(495) ~ 2031310(495) | D77 | Ar23 |
| 1(496) ~ 2031310(496) | D77 | Ar24 |
| 1(497) ~ 2031310(497) | D77 | Ar25 |
| 1(498) ~ 2031310(498) | D77 | Ar26 |
| 1(499) ~ 2031310(499) | D77 | Ar27 |
| 1(500) ~ 2031310(500) | D77 | Ar28 |
| 1(501) ~ 2031310(501) | D77 | Ar29 |
| 1(502) ~ 2031310(502) | D77 | Ar30 |
| 1(503) ~ 2031310(503) | D77 | Ar31 |
| 1(504) ~ 2031310(504) | D77 | Ar32 |
| 1(505) ~ 2031310(505) | D77 | Ar33 |
| 1(506) ~ 2031310(506) | D77 | Ar34 |
| 1(507) ~ 2031310(507) | D77 | Ar35 |
| 1(508) ~ 2031310(508) | D77 | Ar36 |
| 1(509) ~ 2031310(509) | D77 | Ar37 |
| 1(510) ~ 2031310(510) | D77 | Ar38 |
| 1(511) ~ 2031310(511) | D77 | Ar39 |
| 1(512) ~ 2031310(512) | D77 | Ar40 |
| 1(513) ~ 2031310(513) | D77 | Ar41 |
| 1(514) ~ 2031310(514) | D77 | Ar42 |
| 1(515) ~ 2031310(515) | D77 | Ar43 |
| 1(516) ~ 2031310(516) | D77 | Ar44 |
| 1(517) ~ 2031310(517) | D77 | Ar45 |
| 1(518) ~ 2031310(518) | D77 | Ar46 |
| 1(519) ~ 2031310(519) | D77 | Ar47 |
| 1(520) ~ 2031310(520) | D77 | Ar48 |
| 1(521) ~ 2031310(521) | D77 | Ar49 |

(continued)

| No. | $Ar^1$ | $Ar^2$ |
|---|---|---|
| 1(522) ~ 2031310(522) | D77 | Ar50 |
| 1(523) ~ 2031310(523) | D77 | Ar51 |
| 1(524) ~ 2031310(524) | D77 | Ar52 |
| 1(525) ~ 2031310(525) | D77 | Ar53 |
| 1(526) ~ 2031310(526) | D77 | Ar54 |
| 1(527) ~ 2031310(527) | D77 | Ar55 |
| 1(528) ~ 2031310(528) | D77 | Ar56 |
| 1(529) ~ 2031310(529) | D77 | Ar57 |
| 1(530) ~ 2031310(530) | D77 | Ar58 |
| 1(531) ~ 2031310(531) | D77 | Ar59 |
| 1(532) ~ 2031310(532) | D281 | Ar1 |
| 1(533) ~ 2031310(533) | D281 | Ar2 |
| 1(534) ~ 2031310(534) | D281 | Ar3 |
| 1(535) ~ 2031310(535) | D281 | Ar4 |
| 1(536) ~ 2031310(536) | D281 | Ar5 |
| 1(537) ~ 2031310(537) | D281 | Ar6 |
| 1(538) ~ 2031310(538) | D281 | Ar7 |
| 1(539) ~ 2031310(539) | D281 | Ar8 |
| 1(540) ~ 2031310(540) | D281 | Ar9 |
| 1(541) ~ 2031310(541) | D281 | Ar10 |
| 1(542) ~ 2031310(542) | D281 | Ar11 |
| 1(543) ~ 2031310(543) | D281 | Ar12 |
| 1(544) ~ 2031310(544) | D281 | Ar13 |
| 1(545) ~ 2031310(545) | D281 | Ar14 |
| 1(546) ~ 2031310(546) | D281 | Ar15 |
| 1(547) ~ 2031310(547) | D281 | Ar16 |
| 1(548) ~ 2031310(548) | D281 | Ar17 |
| 1(549) ~ 2031310(549) | D281 | Ar18 |
| 1(550) ~ 2031310(550) | D281 | Ar19 |
| 1(551) ~ 2031310(551) | D281 | Ar20 |
| 1(552) ~ 2031310(552) | D281 | Ar21 |
| 1(553) ~ 2031310(553) | D281 | Ar22 |
| 1(554) ~ 2031310(554) | D281 | Ar23 |
| 1(555) ~ 2031310(555) | D281 | Ar24 |
| 1(556) ~ 2031310(556) | D281 | Ar25 |
| 1(557) ~ 2031310(557) | D281 | Ar26 |
| 1(558) ~ 2031310(558) | D281 | Ar27 |
| 1(559) ~ 2031310(559) | D281 | Ar28 |
| 1(560) ~ 2031310(560) | D281 | Ar29 |
| 1(561) ~ 2031310(561) | D281 | Ar30 |

(continued)

| No. | $Ar^1$ | $Ar^2$ |
|---|---|---|
| 1(562) ~ 2031310(562) | D281 | Ar31 |
| 1(563) ~ 2031310(563)<br>1(564) ~ 2031310(564) | D281<br>D281 | Ar32<br>Ar33 |
| 1(565) ~ 2031310(565) | D281 | Ar34 |
| 1(566) ~ 2031310(566)<br>1(567) ~ 2031310(567) | D281<br>D281 | Ar35<br>Ar36 |
| 1(568) ~ 2031310(568) | D281 | Ar37 |
| 1(569) ~ 2031310(569) | D281 | Ar38 |
| 1(570) ~ 2031310(570) | D281 | Ar39 |
| 1(571) ~ 2031310(571) | D281 | Ar40 |
| 1(572) ~ 2031310(572) | D281 | Ar41 |
| 1(573) ~ 2031310(573) | D281 | Ar42 |
| 1(574) ~ 2031310(574) | D281 | Ar43 |
| 1(575) ~ 2031310(575) | D281 | Ar44 |
| 1(576) ~ 2031310(576) | D281 | Ar45 |
| 1(577) ~ 2031310(577)<br>1(578) ~ 2031310(578) | D281<br>D281 | Ar46<br>Ar47 |
| 1(579) ~ 2031310(579) | D281 | Ar48 |
| 1(580) ~ 2031310(580)<br>1(581) ~ 2031310(581) | D281<br>D281 | Ar49<br>Ar50 |
| 1(582) ~ 2031310(582) | D281 | Ar51 |
| 1(583) ~ 2031310(583)<br>1(584) ~ 2031310(584) | D281<br>D281 | Ar52<br>Ar53 |
| 1(585) ~ 2031310(585) | D281 | Ar54 |
| 1(586) ~ 2031310(586) | D281 | Ar55 |
| 1(587) ~ 2031310(587) | D281 | Ar56 |
| 1(588) ~ 2031310(588) | D281 | Ar57 |
| 1(589) ~ 2031310(589) | D281 | Ar58 |
| 1(590) ~ 2031310(590) | D281 | Ar59 |
| 1(591) ~ 2031310(591) | D459 | Ar1 |
| 1(592) ~ 2031310(592) | D459 | Ar2 |
| 1(593) ~ 2031310(593) | D459 | Ar3 |
| 1(594) ~ 2031310(594) | D459 | Ar4 |
| 1(595) ~ 2031310(595) | D459 | Ar5 |
| 1(596) ~ 2031310(596) | D459 | Ar6 |
| 1(597) ~ 2031310(597) | D459 | Ar7 |
| 1(598) ~ 2031310(598) | D459 | Ar8 |
| 1(599) ~ 2031310(599) | D459 | Ar9 |
| 1(600) ~ 2031310(600) | D459 | Ar10 |
| 1(601) ~ 2031310(601) | D459 | Ar11 |

(continued)

| No. | $Ar^1$ | $Ar^2$ |
|---|---|---|
| 1(602) ~ 2031310(602) | D459 | Ar12 |
| 1(603) ~ 2031310(603) | D459 | Ar13 |
| 1(604) ~ 2031310(604) | D459 | Ar14 |
| 1(605) ~ 2031310(605) | D459 | Ar15 |
| 1(606) ~ 2031310(606) | D459 | Ar16 |
| 1(607) ~ 2031310(607) | D459 | Ar17 |
| 1(608) ~ 2031310(608) | D459 | Ar18 |
| 1(609) ~ 2031310(609) | D459 | Ar19 |
| 1(610) ~ 2031310(610) | D459 | Ar20 |
| 1(611) ~ 2031310(611) | D459 | Ar21 |
| 1(612) ~ 2031310(612) | D459 | Ar22 |
| 1(613) ~ 2031310(613) | D459 | Ar23 |
| 1(614) ~ 2031310(614) | D459 | Ar24 |
| 1(615) ~ 2031310(615) | D459 | Ar25 |
| 1(616) ~ 2031310(616) | D459 | Ar26 |
| 1(617) ~ 2031310(617) | D459 | Ar27 |
| 1(618) ~ 2031310(618) | D459 | Ar28 |
| 1(619) ~ 2031310(619) | D459 | Ar29 |
| 1(620) ~ 2031310(620) | D459 | Ar30 |
| 1(621) ~ 2031310(621) | D459 | Ar31 |
| 1(622) ~ 2031310(622) | D459 | Ar32 |
| 1(623) ~ 2031310(623) | D459 | Ar33 |
| 1(624) ~ 2031310(624) | D459 | Ar34 |
| 1(625) ~ 2031310(625) | D459 | Ar35 |
| 1(626) ~ 2031310(626) | D459 | Ar36 |
| 1(627) ~ 2031310(627) | D459 | Ar37 |
| 1(628) ~ 2031310(628) | D459 | Ar38 |
| 1(629) ~ 2031310(629) | D459 | Ar39 |
| 1(630) ~ 2031310(630) | D459 | Ar40 |
| 1(631) ~ 2031310(631) | D459 | Ar41 |
| 1(632) ~ 2031310(632) | D459 | Ar42 |
| 1(633) ~ 2031310(633) | D459 | Ar43 |
| 1(634) ~ 2031310(634) | D459 | Ar44 |
| 1(635) ~ 2031310(635) | D459 | Ar45 |
| 1(636) ~ 2031310(636) | D459 | Ar46 |
| 1(637) ~ 2031310(637) | D459 | Ar47 |
| 1(638) ~ 2031310(638) | D459 | Ar48 |
| 1(639) ~ 2031310(639) | D459 | Ar49 |
| 1(640) ~ 2031310(640) | D459 | Ar50 |
| 1(641) ~ 2031310(641) | D459 | Ar51 |
| 1(642) ~ 2031310(642) | D459 | Ar52 |

(continued)

| No. | $Ar^1$ | $Ar^2$ |
|---|---|---|
| 1(643) ~ 2031310(643) | D459 | Ar53 |
| 1(644) ~ 2031310(644) | D459 | Ar54 |
| 1(645) ~ 2031310(645) | D459 | Ar55 |
| 1(646) ~ 2031310(646) | D459 | Ar56 |
| 1(647) ~ 2031310(647) | D459 | Ar57 |
| 1(648) ~ 2031310(648) | D459 | Ar58 |
| 1(649) ~ 2031310(649) | D459 | Ar59 |
| 1(650) ~ 2031310(650) | D465 | Ar1 |
| 1(651) ~ 2031310(651) | D465 | Ar2 |
| 1(652) ~ 2031310(652) | D465 | Ar3 |
| 1(653) ~ 2031310(653) | D465 | Ar4 |
| 1(654) ~ 2031310(654) | D465 | Ar5 |
| 1(655) ~ 2031310(655) | D465 | Ar6 |
| 1(656) ~ 2031310(656) | D465 | Ar7 |
| 1(657) ~ 2031310(657) | D465 | Ar8 |
| 1(658) ~ 2031310(658) | D465 | Ar9 |
| 1(659) ~ 2031310(659) | D465 | Ar10 |
| 1(660) ~ 2031310(660) | D465 | Ar11 |
| 1(661) ~ 2031310(661) | D465 | Ar12 |
| 1(662) ~ 2031310(662) | D465 | Ar13 |
| 1(663) ~ 2031310(663) | D465 | Ar14 |
| 1(664) ~ 2031310(664) | D465 | Ar15 |
| 1(665) ~ 2031310(665) | D465 | Ar16 |
| 1(666) ~ 2031310(666) | D465 | Ar17 |
| 1(667) ~ 2031310(667) | D465 | Ar18 |
| 1(668) ~ 2031310(668) | D465 | Ar19 |
| 1(669) ~ 2031310(669) | D465 | Ar20 |
| 1(670) ~ 2031310(670) | D465 | Ar21 |
| 1(671) ~ 2031310(671) | D465 | Ar22 |
| 1(672) ~ 2031310(672) | D465 | Ar23 |
| 1(673) ~ 2031310(673) | D465 | Ar24 |
| 1(674) ~ 2031310(674) | D465 | Ar25 |
| 1(675) ~ 2031310(675) | D465 | Ar26 |
| 1(676) ~ 2031310(676) | D465 | Ar27 |
| 1(677) ~ 2031310(677) | D465 | Ar28 |
| 1(678) ~ 2031310(678) | D465 | Ar29 |
| 1(679) ~ 2031310(679) | D465 | Ar30 |
| 1(680) ~ 2031310(680) | D465 | Ar31 |
| 1(681) ~ 2031310(681) | D465 | Ar32 |
| 1(682) ~ 2031310(682) | D465 | Ar33 |

(continued)

| No. | Ar1 | Ar2 |
|---|---|---|
| 1(683) ~ 2031310(683) | D465 | Ar34 |
| 1(684) ~ 2031310(684)<br>1(685) ~ 2031310(685) | D465<br>D465 | Ar35<br>Ar36 |
| 1(686) ~ 2031310(686) | D465 | Ar37 |
| 1(687) ~ 2031310(687) | D465 | Ar38 |
| 1(688) ~ 2031310(688) | D465 | Ar39 |
| 1(689) ~ 2031310(689) | D465 | Ar40 |
| 1(690) ~ 2031310(690) | D465 | Ar41 |
| 1(691) ~ 2031310(691) | D465 | Ar42 |
| 1(692) ~ 2031310(692) | D465 | Ar43 |
| 1(693) ~ 2031310(693) | D465 | Ar44 |
| 1(694) ~ 2031310(694) | D465 | Ar45 |
| 1(695) ~ 2031310(695)<br>1(696) ~ 2031310(696) | D465<br>D465 | Ar46<br>Ar47 |
| 1(697) ~ 2031310(697) | D465 | Ar48 |
| 1(698) ~ 2031310(698)<br>1(699) ~ 2031310(699) | D465<br>D465 | Ar49<br>Ar50 |
| 1(700) ~ 2031310(700) | D465 | Ar51 |
| 1(701) ~ 2031310(701)<br>1(702) ~ 2031310(702) | D465<br>D465 | Ar52<br>Ar53 |
| 1(703) ~ 2031310(703) | D465 | Ar54 |
| 1(704) ~ 2031310(704) | D465 | Ar55 |
| 1(705) ~ 2031310(705) | D465 | Ar56 |
| 1(706) ~ 2031310(706) | D465 | Ar57 |
| 1(707) ~ 2031310(707) | D465 | Ar58 |
| 1(708) ~ 2031310(708) | D465 | Ar59 |
| 1(709) ~ 2031310(709) | D466 | Ar1 |
| 1(710) ~ 2031310(710) | D466 | Ar2 |
| 1(711) ~ 2031310(711) | D466 | Ar3 |
| 1(712) ~ 2031310(712) | D466 | Ar4 |
| 1(713) ~ 2031310(713) | D466 | Ar5 |
| 1(714) ~ 2031310(714) | D466 | Ar6 |
| 1(715) ~ 2031310(715) | D466 | Ar7 |
| 1(716) ~ 2031310(716) | D466 | Ar8 |
| 1(717) ~ 2031310(717) | D466 | Ar9 |
| 1(718) ~ 2031310(718) | D466 | Ar10 |
| 1(719) ~ 2031310(719) | D466 | Ar11 |
| 1(720) ~ 2031310(720)<br>1(721) ~ 2031310(721) | D466<br>D466 | Ar12<br>Ar13 |
| 1(722) ~ 2031310(722) | D466 | Ar14 |

(continued)

| No. | Ar1 | Ar2 |
|---|---|---|
| 1(723) ~ 2031310(723) | D466 | Ar15 |
| 1(724) ~ 2031310(724) | D466 | Ar16 |
| 1(725) ~ 2031310(725) | D466 | Ar17 |
| 1(726) ~ 2031310(726) | D466 | Ar18 |
| 1(727) ~ 2031310(727) | D466 | Ar19 |
| 1(728) ~ 2031310(728) | D466 | Ar20 |
| 1(729) ~ 2031310(729) | D466 | Ar21 |
| 1(730) ~ 2031310(730) | D466 | Ar22 |
| 1(731) ~ 2031310(731) | D466 | Ar23 |
| 1(732) ~ 2031310(732) | D466 | Ar24 |
| 1(733) ~ 2031310(733) | D466 | Ar25 |
| 1(734) ~ 2031310(734) | D466 | Ar26 |
| 1(735) ~ 2031310(735) | D466 | Ar27 |
| 1(736) ~ 2031310(736) | D466 | Ar28 |
| 1(737) ~ 2031310(737) | D466 | Ar29 |
| 1(738) ~ 2031310(738) | D466 | Ar30 |
| 1(739) ~ 2031310(739) | D466 | Ar31 |
| 1(740) ~ 2031310(740) | D466 | Ar32 |
| 1(741) ~ 2031310(741) | D466 | Ar33 |
| 1(742) ~ 2031310(742) | D466 | Ar34 |
| 1(743) ~ 2031310(743) | D466 | Ar35 |
| 1(744) ~ 2031310(744) | D466 | Ar36 |
| 1(745) ~ 2031310(745) | D466 | Ar37 |
| 1(746) ~ 2031310(746) | D466 | Ar38 |
| 1(747) ~ 2031310(747) | D466 | Ar39 |
| 1(748) ~ 2031310(748) | D466 | Ar40 |
| 1(749) ~ 2031310(749) | D466 | Ar41 |
| 1(750) ~ 2031310(750) | D466 | Ar42 |
| 1(751) ~ 2031310(751) | D466 | Ar43 |
| 1(752) ~ 2031310(752) | D466 | Ar44 |
| 1(753) ~ 2031310(753) | D466 | Ar45 |
| 1(754) ~ 2031310(754) | D466 | Ar46 |
| 1(755) ~ 2031310(755) | D466 | Ar47 |
| 1(756) ~ 2031310(756) | D466 | Ar48 |
| 1(757) ~ 2031310(757) | D466 | Ar49 |
| 1(758) ~ 2031310(758) | D466 | Ar50 |
| 1(759) ~ 2031310(759) | D466 | Ar51 |
| 1(760) ~ 2031310(760) | D466 | Ar52 |
| 1(761) ~ 2031310(761) | D466 | Ar53 |
| 1(762) ~ 2031310(762) | D466 | Ar54 |

(continued)

| No. | $Ar^1$ | $Ar^2$ |
|---|---|---|
| 1(763) ~ 2031310(763) | D466 | Ar55 |
| 1(764) ~ 2031310(764) | D466 | Ar56 |
| 1(765) ~ 2031310(765) | D466 | Ar57 |
| 1(766) ~ 2031310(766) | D466 | Ar58 |
| 1(767) ~ 2031310(767) | D466 | Ar59 |
| 1(768) ~ 2031310(768) | D467 | Ar1 |
| 1(769) ~ 2031310(769) | D467 | Ar2 |
| 1(770) ~ 2031310(770) | D467 | Ar3 |
| 1(771) ~ 2031310(771) | D467 | Ar4 |
| 1(772) ~ 2031310(772) | D467 | Ar5 |
| 1(773) ~ 2031310(773) | D467 | Ar6 |
| 1(774) ~ 2031310(774) | D467 | Ar7 |
| 1(775) ~ 2031310(775) | D467 | Ar8 |
| 1(776) ~ 2031310(776) | D467 | Ar9 |
| 1(777) ~ 2031310(777) | D467 | Ar10 |
| 1(778) ~ 2031310(778) | D467 | Ar11 |
| 1(779) ~ 2031310(779) | D467 | Ar12 |
| 1(780) ~ 2031310(780) | D467 | Ar13 |
| 1(781) ~ 2031310(781) | D467 | Ar14 |
| 1(782) ~ 2031310(782) | D467 | Ar15 |
| 1(783) ~ 2031310(783) | D467 | Ar16 |
| 1(784) ~ 2031310(784) | D467 | Ar17 |
| 1(785) ~ 2031310(785) | D467 | Ar18 |
| 1(786) ~ 2031310(786) | D467 | Ar19 |
| 1(787) ~ 2031310(787) | D467 | Ar20 |
| 1(788) ~ 2031310(788) | D467 | Ar21 |
| 1(789) ~ 2031310(789) | D467 | Ar22 |
| 1(790) ~ 2031310(790) | D467 | Ar23 |
| 1(791) ~ 2031310(791) | D467 | Ar24 |
| 1(792) ~ 2031310(792) | D467 | Ar25 |
| 1(793) ~ 2031310(793) | D467 | Ar26 |
| 1(794) ~ 2031310(794) | D467 | Ar27 |
| 1(795) ~ 2031310(795) | D467 | Ar28 |
| 1(796) ~ 2031310(796) | D467 | Ar29 |
| 1(797) ~ 2031310(797) | D467 | Ar30 |
| 1(798) ~ 2031310(798) | D467 | Ar31 |
| 1(799) ~ 2031310(799) | D467 | Ar32 |
| 1(800) ~ 2031310(800) | D467 | Ar33 |
| 1(801) ~ 2031310(801) | D467 | Ar34 |
| 1(802) ~ 2031310(802) | D467 | Ar35 |

(continued)

| No. | Ar1 | Ar2 |
|---|---|---|
| 1(803) ~ 2031310(803) | D467 | Ar36 |
| 1(804) ~ 2031310(804) | D467 | Ar37 |
| 1(805) ~ 2031310(805) | D467 | Ar38 |
| 1(806) ~ 2031310(806) | D467 | Ar39 |
| 1(807) ~ 2031310(807) | D467 | Ar40 |
| 1(808) ~ 2031310(808) | D467 | Ar41 |
| 1(809) ~ 2031310(809) | D467 | Ar42 |
| 1(810) ~ 2031310(810) | D467 | Ar43 |
| 1(811) ~ 2031310(811) | D467 | Ar44 |
| 1(812) ~ 2031310(812) | D467 | Ar45 |
| 1(813) ~ 2031310(813) | D467 | Ar46 |
| 1(814) ~ 2031310(814) | D467 | Ar47 |
| 1(815) ~ 2031310(815) | D467 | Ar48 |
| 1(816) ~ 2031310(816) | D467 | Ar49 |
| 1(817) ~ 2031310(817) | D467 | Ar50 |
| 1(818) ~ 2031310(818) | D467 | Ar51 |
| 1(819) ~ 2031310(819) | D467 | Ar52 |
| 1(820) ~ 2031310(820) | D467 | Ar53 |
| 1(821) ~ 2031310(821) | D467 | Ar54 |
| 1(822) ~ 2031310(822) | D467 | Ar55 |
| 1(823) ~ 2031310(823) | D467 | Ar56 |
| 1(824) ~ 2031310(824) | D467 | Ar57 |
| 1(825) ~ 2031310(825) | D467 | Ar58 |
| 1(826) ~ 2031310(826) | D467 | Ar59 |
| 1(827) ~ 2031310(827) | D471 | Ar1 |
| 1(828) ~ 2031310(828) | D471 | Ar2 |
| 1(829) ~ 2031310(829) | D471 | Ar3 |
| 1(830) ~ 2031310(830) | D471 | Ar4 |
| 1(831) ~ 2031310(831) | D471 | Ar5 |
| 1(832) ~ 2031310(832) | D471 | Ar6 |
| 1(833) ~ 2031310(833) | D471 | Ar7 |
| 1(834) ~ 2031310(834) | D471 | Ar8 |
| 1(835) ~ 2031310(835) | D471 | Ar9 |
| 1(836) ~ 2031310(836) | D471 | Ar10 |
| 1(837) ~ 2031310(837) | D471 | Ar11 |
| 1(838) ~ 2031310(838) | D471 | Ar12 |
| 1(839) ~ 2031310(839) | D471 | Ar13 |
| 1(840) ~ 2031310(840) | D471 | Ar14 |
| 1(841) ~ 2031310(841) | D471 | Ar15 |
| 1(842) ~ 2031310(842) | D471 | Ar16 |

(continued)

| No. | Ar1 | Ar2 |
|---|---|---|
| 1(843) ~ 2031310(843)<br>1(844) ~ 2031310(844) | D471<br>D471 | Ar17<br>Ar18 |
| 1(845) ~ 2031310(845) | D471 | Ar19 |
| 1(846) ~ 2031310(846) | D471 | Ar20 |
| 1(847) ~ 2031310(847) | D471 | Ar21 |
| 1(848) ~ 2031310(848) | D471 | Ar22 |
| 1(849) ~ 2031310(849) | D471 | Ar23 |
| 1(850) ~ 2031310(850) | D471 | Ar24 |
| 1(851) ~ 2031310(851) | D471 | Ar25 |
| 1(852) ~ 2031310(852) | D471 | Ar26 |
| 1(853) ~ 2031310(853) | D471 | Ar27 |
| 1(854) ~ 2031310(854) | D471 | Ar28 |
| 1(855) ~ 2031310(855)<br>1(856) ~ 2031310(856) | D471<br>D471 | Ar29<br>Ar30 |
| 1(857) ~ 2031310(857) | D471 | Ar31 |
| 1(858) ~ 2031310(858) | D471 | Ar32 |
| 1(859) ~ 2031310(859) | D471 | Ar33 |
| 1(860) ~ 2031310(860) | D471 | Ar34 |
| 1(861) ~ 2031310(861) | D471 | Ar35 |
| 1(862) ~ 2031310(862) | D471 | Ar36 |
| 1(863) ~ 2031310(863) | D471 | Ar37 |
| 1(864) ~ 2031310(864) | D471 | Ar38 |
| 1(865) ~ 2031310(865) | D471 | Ar39 |
| 1(866) ~ 2031310(866) | D471 | Ar40 |
| 1(867) ~ 2031310(867) | D471 | Ar41 |
| 1(868) ~ 2031310(868) | D471 | Ar42 |
| 1(869) ~ 2031310(869)<br>1(870) ~ 2031310(870) | D471<br>D471 | Ar43<br>Ar44 |
| 1(871) ~ 2031310(871)<br>1(872) ~ 2031310(872)<br>1(873) ~ 2031310(873) | D471<br>D471<br>D471 | Ar45<br>Ar46<br>Ar47 |
| 1(874) ~ 2031310(874) | D471 | Ar48 |
| 1(875) ~ 2031310(875) | D471 | Ar49 |
| 1(876) ~ 2031310(876) | D471 | Ar50 |
| 1(877) ~ 2031310(877) | D471 | Ar51 |
| 1(878) ~ 2031310(878) | D471 | Ar52 |
| 1(879) ~ 2031310(879) | D471 | Ar53 |
| 1(880) ~ 2031310(880) | D471 | Ar54 |
| 1(881) ~ 2031310(881) | D471 | Ar55 |
| 1(882) ~ 2031310(882) | D471 | Ar56 |

(continued)

| No. | $Ar^1$ | $Ar^2$ |
|---|---|---|
| 1(883) ~ 2031310(883) | D471 | Ar57 |
| 1(884) ~ 2031310(884) | D471 | Ar58 |
| 1(885) ~ 2031310(885) | D471 | Ar59 |
| 1(886) ~ 2031310(886) | D486 | Ar1 |
| 1(887) ~ 2031310(887) | D486 | Ar2 |
| 1(888) ~ 2031310(888) | D486 | Ar3 |
| 1(889) ~ 2031310(889) | D486 | Ar4 |
| 1(890) ~ 2031310(890) | D486 | Ar5 |
| 1(891) ~ 2031310(891) | D486 | Ar6 |
| 1(892) ~ 2031310(892) | D486 | Ar7 |
| 1(893) ~ 2031310(893) | D486 | Ar8 |
| 1(894) ~ 2031310(894) | D486 | Ar9 |
| 1(895) ~ 2031310(895) | D486 | Ar10 |
| 1(896) ~ 2031310(896) | D486 | Ar11 |
| 1(897) ~ 2031310(897) | D486 | Ar12 |
| 1(898) ~ 2031310(898) | D486 | Ar13 |
| 1(899) -- 2031310(899) | D486 | Ar14 |
| 1(900) ~ 2031310(900) | D486 | Ar15 |
| 1(901) ~ 2031310(901) | D486 | Ar16 |
| 1(902) ~ 2031310(902) | D486 | Ar17 |
| 1(903) ~ 2031310(903) | D486 | Ar18 |
| 1(904) ~ 2031310(904) | D486 | Ar19 |
| 1(905) ~ 2031310(905) | D486 | Ar20 |
| 1(906) ~ 2031310(906) | D486 | Ar21 |
| 1(907) ~ 2031310(907) | D486 | Ar22 |
| 1(908) ~ 2031310(908) | D486 | Ar23 |
| 1(909) ~ 2031310(909) | D486 | Ar24 |
| 1(910) ~ 2031310(910) | D486 | Ar25 |
| 1(911) ~ 2031310(911) | D486 | Ar26 |
| 1(912) ~ 2031310(912) | D486 | Ar27 |
| 1(913) ~ 2031310(913) | D486 | Ar28 |
| 1(914) ~ 2031310(914) | D486 | Ar29 |
| 1(915) ~ 2031310(915) | D486 | Ar30 |
| 1(916) ~ 2031310(916) | D486 | Ar31 |
| 1(917) ~ 2031310(917) | D486 | Ar32 |
| 1(918) ~ 2031310(918) | D486 | Ar33 |
| 1(919) ~ 2031310(919) | D486 | Ar34 |
| 1(920) ~ 2031310(920) | D486 | Ar35 |
| 1(921) ~ 2031310(921) | D486 | Ar36 |
| 1(922) ~ 2031310(922) | D486 | Ar37 |

(continued)

| No. | $Ar^1$ | $Ar^2$ |
|---|---|---|
| 1(923) ~ 2031310(923) | D486 | Ar38 |
| 1(924) ~ 2031310(924) | D486 | Ar39 |
| 1(925) ~ 2031310(925) | D486 | Ar40 |
| 1(926) ~ 2031310(926) | D486 | Ar41 |
| 1(927) ~ 2031310(927) | D486 | Ar42 |
| 1(928) ~ 2031310(928) | D486 | Ar43 |
| 1(929) ~ 2031310(929) | D486 | Ar44 |
| 1(930) ~ 2031310(930) | D486 | Ar45 |
| 1(931) ~ 2031310(931) | D486 | Ar46 |
| 1(932) ~ 2031310(932) | D486 | Ar47 |
| 1(933) ~ 2031310(933) | D486 | Ar48 |
| 1(934) ~ 2031310(934) | D486 | Ar49 |
| 1(935) ~ 2031310(935) | D486 | Ar50 |
| 1(936) ~ 2031310(936) | D486 | Ar51 |
| 1(937) ~ 2031310(937) | D486 | Ar52 |
| 1(938) ~ 2031310(938) | D486 | Ar53 |
| 1(939) ~ 2031310(939) | D486 | Ar54 |
| 1(940) ~ 2031310(940) | D486 | Ar55 |
| 1(941) ~ 2031310(941) | D486 | Ar56 |
| 1(942) ~ 2031310(942) | D486 | Ar57 |
| 1(943) ~ 2031310(943) | D486 | Ar58 |
| 1(944) ~ 2031310(944) | D486 | Ar59 |
| 1(945) ~ 2031310(945) | D520 | Ar1 |
| 1(946) ~ 2031310(946) | D520 | Ar2 |
| 1(947) ~ 2031310(947) | D520 | Ar3 |
| 1(948) ~ 2031310(948) | D520 | Ar4 |
| 1(949) ~ 2031310(949) | D520 | Ar5 |
| 1(950) ~ 2031310(950) | D520 | Ar6 |
| 1(951) ~ 2031310(951) | D520 | Ar7 |
| 1(952) ~ 2031310(952) | D520 | Ar8 |
| 1(953) ~ 2031310(953) | D520 | Ar9 |
| 1(954) ~ 2031310(954) | D520 | Ar10 |
| 1(955) ~ 2031310(955) | D520 | Ar11 |
| 1(956) ~ 2031310(956) | D520 | Ar12 |
| 1(957) ~ 2031310(957) | D520 | Ar13 |
| 1(958) ~ 2031310(958) | D520 | Ar14 |
| 1(959) ~ 2031310(959) | D520 | Ar15 |
| 1(960) ~ 2031310(960) | D520 | Ar16 |
| 1(961) ~ 2031310(961) | D520 | Ar17 |
| 1(962) ~ 2031310(962) | D520 | Ar18 |
| 1(963) ~ 2031310(963) | D520 | Ar19 |

(continued)

| No. | $Ar^1$ | $Ar^2$ |
|---|---|---|
| 1(964) ~ 2031310(964) | D520 | Ar20 |
| 1(965) ~ 2031310(965) | D520 | Ar21 |
| 1(966) ~ 2031310(966) | D520 | Ar22 |
| 1(967) ~ 2031310(967) | D520 | Ar23 |
| 1(968) ~ 2031310(968) | D520 | Ar24 |
| 1(969) ~ 2031310(969) | D520 | Ar25 |
| 1(970) ~ 2031310(970) | D520 | Ar26 |
| 1(971) ~ 2031310(971) | D520 | Ar27 |
| 1(972) ~ 2031310(972) | D520 | Ar28 |
| 1(973) ~ 2031310(973) | D520 | Ar29 |
| 1(974) ~ 2031310(974) | D520 | Ar30 |
| 1(975) ~ 2031310(975) | D520 | Ar31 |
| 1(976) ~ 2031310(976) | D520 | Ar32 |
| 1(977) ~ 2031310(977) | D520 | Ar33 |
| 1(978) ~ 2031310(978) | D520 | Ar34 |
| 1(979) ~ 2031310(979) | D520 | Ar35 |
| 1(980) ~ 2031310(980) | D520 | Ar36 |
| 1(981) ~ 2031310(981) | D520 | Ar37 |
| 1(982) ~ 2031310(982) | D520 | Ar38 |
| 1(983) ~ 2031310(983) | D520 | Ar39 |
| 1(984) ~ 2031310(984) | D520 | Ar40 |
| 1(985) ~ 2031310(985) | D520 | Ar41 |
| 1(986) ~ 2031310(986) | D520 | Ar42 |
| 1(987) ~ 2031310(987) | D520 | Ar43 |
| 1(988) ~ 2031310(988) | D520 | Ar44 |
| 1(989) ~ 2031310(989) | D520 | Ar45 |
| 1(990) ~ 2031310(990) | D520 | Ar46 |
| 1(991) ~ 2031310(991) | D520 | Ar47 |
| 1(992) ~ 2031310(992) | D520 | Ar48 |
| 1(993) ~ 2031310(993) | D520 | Ar49 |
| 1(994) ~ 2031310(994) | D520 | Ar50 |
| 1(995) ~ 2031310(995) | D520 | Ar51 |
| 1(996) ~ 2031310(996) | D520 | Ar52 |
| 1(997) ~ 2031310(997) | D520 | Ar53 |
| 1(998) ~ 2031310(998) | D520 | Ar54 |
| 1(999) ~ 2031310(999) | D520 | Ar55 |
| 1(1000) ~ 2031310(1000) | D520 | Ar56 |
| 1(1001) ~ 2031310(1001) | D520 | Ar57 |
| 1(1002) ~ 2031310(1002) | D520 | Ar58 |
| 1(1003) ~ 2031310(1003) | D520 | Ar59 |
| 1(1004) ~ 2031310(1004) | D621 | Ar1 |

(continued)

| No. | Ar1 | Ar2 |
|---|---|---|
| 1(1005) ~ 2031310(1005) | D621 | Ar2 |
| 1(1006) ~ 2031310(1006)<br>1(1007) ~ 2031310(1007) | D621<br>D621 | Ar3<br>Ar4 |
| 1(1008) ~ 2031310(1008) | D621 | Ar5 |
| 1(1009) ~ 2031310(1009) | D621 | Ar6 |
| 1(1010) ~ 2031310(1010) | D621 | Ar7 |
| 1(1011) ~ 2031310(1011) | D621 | Ar8 |
| 1(1012) ~ 2031310(1012)<br>1(1013) ~ 2031310(1013) | D621<br>D621 | Ar9<br>Ar10 |
| 1(1014) ~ 2031310(1014) | D621 | Ar11 |
| 1(1015) ~ 2031310(1015) | D621 | Ar12 |
| 1(1016) ~ 2031310(1016) | D621 | Ar13 |
| 1(1017) ~ 2031310(1017) | D621 | Ar14 |
| 1(1018) ~ 2031310(1018) | D621 | Ar15 |
| 1(1019) ~ 2031310(1019) | D621 | Ar16 |
| 1(1020) ~ 2031310(1020) | D621 | Ar17 |
| 1(1021) ~ 2031310(1021)<br>1(1022) ~ 2031310(1022) | D621<br>D621 | Ar18<br>Ar19 |
| 1(1023) ~ 2031310(1023) | D621 | Ar20 |
| 1(1024) ~ 2031310(1024) | D621 | Ar21 |
| 1(1025) ~ 2031310(1025) | D621 | Ar22 |
| 1(1026) ~ 2031310(1026) | D621 | Ar23 |
| 1(1027) ~ 2031310(1027)<br>1(1028) ~ 2031310(1028) | D621<br>D621 | Ar24<br>Ar25 |
| 1(1029) ~ 2031310(1029) | D621 | Ar26 |
| 1(1030) ~ 2031310(1030) | D621 | Ar27 |
| 1(1031) ~ 2031310(1031) | D621 | Ar28 |
| 1(1032) ~ 2031310(1032) | D621 | Ar29 |
| 1(1033) ~ 2031310(1033) | D621 | Ar30 |
| 1(1034) ~ 2031310(1034) | D621 | Ar31 |
| 1(1035) ~ 2031310(1035) | D621 | Ar32 |
| 1(1036) ~ 2031310(1036) | D621 | Ar33 |
| 1(1037) ~ 2031310(1037) | D621 | Ar34 |
| 1(1038) ~ 2031310(1038) | D621 | Ar35 |
| 1(1039) ~ 2031310(1039) | D621 | Ar36 |
| 1(1040) ~ 2031310(1040)<br>1(1041) ~ 2031310(1041) | D621<br>D621 | Ar37<br>Ar38 |
| 1(1042) ~ 2031310(1042) | D621 | Ar39 |
| 1(1043) ~ 2031310(1043) | D621 | Ar40 |
| 1(1044) ~ 2031310(1044) | D621 | Ar41 |

(continued)

| No. | $Ar^1$ | $Ar^2$ |
|---|---|---|
| 1(1045) ~ 2031310(1045) | D621 | Ar42 |
| 1(1046) ~ 2031310(1046) | D621 | Ar43 |
| 1(1047) ~ 2031310(1047)<br>1(1048) ~ 2031310(1048) | D621<br>D621 | Ar44<br>Ar45 |
| 1(1049) ~ 2031310(1049) | D621 | Ar46 |
| 1(1050) ~ 2031310(1050) | D621 | Ar47 |
| 1(1051) ~ 2031310(1051) | D621 | Ar48 |
| 1(1052) ~ 2031310(1052) | D621 | Ar49 |
| 1(1053) ~ 2031310(1053) | D621 | Ar50 |
| 1(1054) ~ 2031310(1054)<br>1(1055) ~ 2031310(1055)<br>1(1056) ~ 2031310(1056) | D621<br>D621<br>D621 | Ar51<br>Ar52<br>Ar53 |
| 1(1057) ~ 2031310(1057) | D621 | Ar54 |
| 1(1058) ~ 2031310(1058) | D621 | Ar55 |
| 1(1059) ~ 2031310(1059)<br>1(1060) ~ 2031310(1060) | D621<br>D621 | Ar56<br>Ar57 |
| 1(1061) ~ 2031310(1061) | D621 | Ar58 |
| 1(1062) ~ 2031310(1062) | D621 | Ar59 |
| 1(1063) ~ 2031310(1063) | D711 | Ar1 |
| 1(1064) ~ 2031310(1064) | D711 | Ar2 |
| 1(1065) ~ 2031310(1065) | D711 | Ar3 |
| 1(1066) ~ 2031310(1066) | D711 | Ar4 |
| 1(1067) ~ 2031310(1067) | D711 | Ar5 |
| 1(1068) ~ 2031310(1068) | D711 | Ar6 |
| 1(1069) ~ 2031310(1069) | D711 | Ar7 |
| 1(1070) ~ 2031310(1070) | D711 | Ar8 |
| 1(1071) ~ 2031310(1071) | D711 | Ar9 |
| 1(1012) ~ 2031310(1072) | D711 | Ar10 |
| 1(1073) ~ 2031310(1073) | D711 | Ar11 |
| 1(1074) ~ 2031310(1074) | D711 | Ar12 |
| 1(1015) ~ 2031310(1075) | D711 | Ar13 |
| 1(1076) ~ 2031310(1076) | D711 | Ar14 |
| 1(1077) ~ 2031310(1077) | D711 | Ar15 |
| 1(1078) ~ 2031310(1078) | D711 | Ar16 |
| 1(1079) ~ 2031310(1079) | D711 | Ar17 |
| 1(1080) ~ 2031310(1080) | D711 | Ar18 |
| 1(1081) ~ 2031310(1081) | D711 | Ar19 |
| 1(1082) ~ 2031310(1082) | D711 | Ar20 |
| 1(1083) ~ 2031310(1083)<br>1(1084) ~ 2031310(1084)<br>1(1085) ~ 2031310(1085) | D711<br>D711<br>D711 | Ar21<br>Ar22<br>Ar23 |

(continued)

| No. | $Ar^1$ | $Ar^2$ |
|---|---|---|
| 1(1086) ~ 2031310(1086) | D711 | Ar24 |
| 1(1087) ~ 2031310(1087) | D711 | Ar25 |
| 1(1088) ~ 2031310(1088) | D711 | Ar26 |
| 1(1089) ~ 2031310(1089) | D711 | Ar27 |
| 1(1090) ~ 2031310(1090) | D711 | Ar28 |
| 1(1091) ~ 2031310(1091) | D711 | Ar29 |
| 1(1092) ~ 2031310(1092) | D711 | Ar30 |
| 1(1093) ~ 2031310(1093) | D711 | Ar31 |
| 1(1094) ~ 2031310(1094) | D711 | Ar32 |
| 1(1095) ~ 2031310(1095) | D711 | Ar33 |
| 1(1096) ~ 2031310(1096) | D711 | Ar34 |
| 1(1097) ~ 2031310(1097) | D711 | Ar35 |
| 1(1098) ~ 2031310(1098) | D711 | Ar36 |
| 1(1099) ~ 2031310(1099) | D711 | Ar37 |
| 1(1100) ~ 2031310(1100) | D711 | Ar38 |
| 1(1101) ~ 2031310(1101) | D711 | Ar39 |
| 1(1102) ~ 2031310(1102) | D711 | Ar40 |
| 1(1103) ~ 2031310(1103) | D711 | Ar41 |
| 1(1104) ~ 2031310(1104) | D711 | Ar42 |
| 1(1105) ~ 2031310(1105) | D711 | Ar43 |
| 1(1106) ~ 2031310(1106) | D711 | Ar44 |
| 1(1107) ~ 2031310(1107) | D711 | Ar45 |
| 1(1108) ~ 2031310(1108) | D711 | Ar46 |
| 1(1109) ~ 2031310(1109) | D711 | Ar47 |
| 1(1110) ~ 2031310(1110) | D711 | Ar48 |
| 1(1111) ~ 2031310(1111) | D711 | Ar49 |
| 1(1112) ~ 2031310(1112) | D711 | Ar50 |
| 1(1113) ~ 2031310(1113) | D711 | Ar51 |
| 1(1114) ~ 2031310(1114) | D711 | Ar52 |
| 1(1115) ~ 2031310(1115) | D711 | Ar53 |
| 1(1116) ~ 2031310(1116) | D711 | Ar54 |
| 1(1117) ~ 2031310(1117) | D711 | Ar55 |
| 1(1118) ~ 2031310(1118) | D711 | Ar56 |
| 1(1119) ~ 2031310(1119) | D711 | Ar57 |
| 1(1120) ~ 2031310(1120) | D711 | Ar58 |
| 1(1121) ~ 2031310(1121) | D711 | Ar59 |
| 1(1122) ~ 2031310(1122) | D712 | Ar1 |
| 1(1123) ~ 2031310(1123) | D712 | Ar2 |
| 1(1124) ~ 2031310(1124) | D712 | Ar3 |
| 1(1125) ~ 2031310(1125) | D712 | Ar4 |

(continued)

| No. | $Ar^1$ | $Ar^2$ |
|---|---|---|
| 1(1126) ~ 2031310(1126) | D712 | Ar5 |
| 1(1127) ~ 2031310(1127) | D712 | Ar6 |
| 1(1128) ~ 2031310(1128) | D712 | Ar7 |
| 1(1129) ~ 2031310(1129) | D712 | Ar8 |
| 1(1130) ~ 2031310(1130) | D712 | Ar9 |
| 1(1131) ~ 2031310(1131) | D712 | Ar10 |
| 1(1132) ~ 2031310(1132) | D712 | Ar11 |
| 1(1133) ~ 2031310(1133) | D712 | Ar12 |
| 1(1134) ~ 2031310(1134) | D712 | Ar13 |
| 1(1135) ~ 2031310(1135) | D712 | Ar14 |
| 1(1136) ~ 2031310(1136) | D712 | Ar15 |
| 1(1137) ~ 2031310(1137) | D712 | Ar16 |
| 1(1138) ~ 2031310(1138) | D712 | Ar17 |
| 1(1139) ~ 2031310(1139) | D712 | Ar18 |
| 1(1140) ~ 2031310(1140) | D712 | Ar19 |
| 1(1141) ~ 2031310(1141) | D712 | Ar20 |
| 1(1142) ~ 2031310(1142) | D712 | Ar21 |
| 1(1143) ~ 2031310(1143) | D712 | Ar22 |
| 1(1144) ~ 2031310(1144) | D712 | Ar23 |
| 1(1145) ~ 2031310(1145) | D712 | Ar24 |
| 1(1146) ~ 2031310(1146) | D712 | Ar25 |
| 1(1147) ~ 2031310(1147) | D712 | Ar26 |
| 1(1148) ~ 2031310(1148) | D712 | Ar27 |
| 1(1149) ~ 2031310(1149) | D712 | Ar28 |
| 1(1150) ~ 2031310(1150) | D712 | Ar29 |
| 1(1151) ~ 2031310(1151) | D712 | Ar30 |
| 1(1152) ~ 2031310(1152) | D712 | Ar31 |
| 1(1153) ~ 2031310(1153) | D712 | Ar32 |
| 1(1154) ~ 2031310(1154) | D712 | Ar33 |
| 1(1155) ~ 2031310(1155) | D712 | Ar34 |
| 1(1156) ~ 2031310(1156) | D712 | Ar35 |
| 1(1157) ~ 2031310(1157) | D712 | Ar36 |
| 1(1158) ~ 2031310(1158) | D712 | Ar37 |
| 1(1159) ~ 2031310(1159) | D712 | Ar38 |
| 1(1160) ~ 2031310(1160) | D712 | Ar39 |
| 1(1161) ~ 2031310(1161) | D712 | Ar40 |
| 1(1162) ~ 2031310(1162) | D712 | Ar41 |
| 1(1163) ~ 2031310(1163) | D712 | Ar42 |
| 1(1164) ~ 2031310(1164) | D712 | Ar43 |
| 1(1165) ~ 2031310(1165) | D712 | Ar44 |

(continued)

| No. | $Ar^1$ | $Ar^2$ |
|---|---|---|
| 1(1166) ~ 2031310(1166) | D712 | Ar45 |
| 1(1167) ~ 2031310(1167) | D712 | Ar46 |
| 1(1168) ~ 2031310(1168) | D712 | Ar47 |
| 1(1169) ~ 2031310(1169) | D712 | Ar48 |
| 1(1170) ~ 2031310(1170) | D712 | Ar49 |
| 1(1171) ~ 2031310(1171) | D712 | Ar50 |
| 1(1172) ~ 2031310(1172) | D712 | Ar51 |
| 1(1173) ~ 2031310(1173)<br>1(1174) ~ 2031310(1174) | D712<br>D712 | Ar52<br>Ar53 |
| 1(1175) ~ 2031310(1175) | D712 | Ar54 |
| 1(1176) ~ 2031310(1176) | D712 | Ar55 |
| 1(1177) ~ 2031310(1177) | D712 | Ar56 |
| 1(1178) ~ 2031310(1178) | D712 | Ar57 |
| 1(1179) ~ 2031310(1179) | D712 | Ar58 |
| 1(1180) ~ 2031310(1180) | D712 | Ar59 |
| 1(1181) ~ 2031310(1181) | D713 | Ar1 |
| 1(1182) ~ 2031310(1182) | D713 | Ar2 |
| 1(1183) ~ 2031310(1183) | D713 | Ar3 |
| 1(1184) ~ 2031310(1184) | D713 | Ar4 |
| 1(1185) ~ 2031310(1185) | D713 | Ar5 |
| 1(1186) ~ 2031310(1186) | D713 | Ar6 |
| 1(1187) ~ 2031310(1187) | D713 | Ar7 |
| 1(1188) ~ 2031310(1188) | D713 | Ar8 |
| 1(1189) ~ 2031310(1189) | D713 | Ar9 |
| 1(1190) ~ 2031310(1190) | D713 | Ar10 |
| 1(1191) ~ 2031310(1191) | D713 | Ar11 |
| 1(1192) ~ 2031310(1192) | D713 | Ar12 |
| 1(1193) ~ 2031310(1193) | D713 | Ar13 |
| 1(1194) ~ 2031310(1194) | D713 | Ar14 |
| 1(1195) ~ 2031310(1195) | D713 | Ar15 |
| 1(1196) ~ 2031310(1196) | D713 | Ar16 |
| 1(1197) ~ 2031310(1197) | D713 | Ar17 |
| 1(1198) ~ 2031310(1198) | D713 | Ar18 |
| 1(1199) ~ 2031310(1199) | D713 | Ar19 |
| 1(1200) ~ 2031310(1200) | D713 | Ar20 |
| 1(1201) ~ 2031310(1201)<br>1(1202) ~ 2031310(1202)<br>1(1203) ~ 2031310(1203) | D713<br>D713<br>D713 | Ar21<br>Ar22<br>Ar23 |
| 1(1204) ~ 2031310(1204) | D713 | Ar24 |
| 1(1205) ~ 2031310(1205) | D713 | Ar25 |

(continued)

| No. | $Ar^1$ | $Ar^2$ |
|---|---|---|
| 1(1206) ~ 2031310(1206) | D713 | Ar26 |
| 1(1207) ~ 2031310(1207) | D713 | Ar27 |
| 1(1208) ~ 2031310(1208) | D713 | Ar28 |
| 1(1209) ~ 2031310(1209) | D713 | Ar29 |
| 1(1210) ~ 2031310(1210) | D713 | Ar30 |
| 1(1211) ~ 2031310(1211) | D713 | Ar31 |
| 1(1212) ~ 2031310(1212)<br>1(1213) ~ 2031310(1213) | D713<br>D713 | Ar32<br>Ar33 |
| 1(1214) ~ 2031310(1214) | D713 | Ar34 |
| 1(1215) ~ 2031310(1215)<br>1(1216) ~ 2031310(1216) | D713<br>D713 | Ar35<br>Ar36 |
| 1(1217) ~ 2031310(1217) | D713 | Ar37 |
| 1(1218) ~ 2031310(1218) | D713 | Ar38 |
| 1(1219) ~ 2031310(1219) | D713 | Ar39 |
| 1(1220) ~ 2031310(1220) | D713 | Ar40 |
| 1(1221) ~ 2031310(1221) | D713 | Ar41 |
| 1(1222) ~ 2031310(1222) | D713 | Ar42 |
| 1(1223) ~ 2031310(1223) | D713 | Ar43 |
| 1(1224) ~ 2031310(1224) | D713 | Ar44 |
| 1(1225) ~ 2031310(1225) | D713 | Ar45 |
| 1(1226) ~ 2031310(1226) | D713 | Ar46 |
| 1(1227) ~ 2031310(1227) | D713 | Ar47 |
| 1(1228) ~ 2031310(1228) | D713 | Ar48 |
| 1(1229) ~ 2031310(1229) | D713 | Ar49 |
| 1(1230) ~ 2031310(1230) | D713 | Ar50 |
| 1(1231) ~ 2031310(1231) | D713 | Ar51 |
| 1(1232) ~ 2031310(1232)<br>1(1233) ~ 2031310(1233) | D713<br>D713 | Ar52<br>Ar53 |
| 1(1234) ~ 2031310(1234) | D713 | Ar54 |
| 1(1235) ~ 2031310(1235) | D713 | Ar55 |
| 1(1236) ~ 2031310(1236) | D713 | Ar56 |
| 1(1237) ~ 2031310(1237) | D713 | Ar57 |
| 1(1238) ~ 2031310(1238) | D713 | Ar58 |
| 1(1239) ~ 2031310(1239) | D713 | Ar59 |
| 1(1240) ~ 2031310(1240) | D714 | Ar1 |
| 1(1241) ~ 2031310(1241) | D714 | Ar2 |
| 1(1242) ~ 2031310(1242) | D714 | Ar3 |
| 1(1243) ~ 2031310(1243) | D714 | Ar4 |
| 1(1244) ~ 2031310(1244)<br>1(1245) ~ 2031310(1245) | D714<br>D714 | Ar5<br>Ar6 |

(continued)

| No. | Ar1 | Ar2 |
|---|---|---|
| 1(1246) ~ 2031310(1246) | D714 | Ar7 |
| 1(1247) ~ 2031310(1247) | D714 | Ar8 |
| 1(1248) ~ 2031310(1248) | D714 | Ar9 |
| 1(1249) ~ 2031310(1249) | D714 | Ar10 |
| 1(1250) ~ 2031310(1250) | D714 | Ar11 |
| 1(1251) ~ 2031310(1251) | D714 | Ar12 |
| 1(1252) ~ 2031310(1252) | D714 | Ar13 |
| 1(1253) ~ 2031310(1253) | D714 | Ar14 |
| 1(1254) ~ 2031310(1254) | D714 | Ar15 |
| 1(1255) ~ 2031310(1255) | D714 | Ar16 |
| 1(1256) ~ 2031310(1256) | D714 | Ar17 |
| 1(1257) ~ 2031310(1257) | D714 | Ar18 |
| 1(1258) ~ 2031310(1258) | D714 | Ar19 |
| 1(1259) ~ 2031310(1259) | D714 | Ar20 |
| 1(1260) ~ 2031310(1260) | D714 | Ar21 |
| 1(1261) ~ 2031310(1261) | D714 | Ar22 |
| 1(1262) ~ 2031310(1262) | D714 | Ar23 |
| 1(1263) ~ 2031310(1263) | D714 | Ar24 |
| 1(1264) ~ 2031310(1264)<br>1(1265) ~ 2031310(1265) | D714<br>D714 | Ar25<br>Ar26 |
| 1(1266) ~ 2031310(1266) | D714 | Ar27 |
| 1(1267) ~ 2031310(1267) | D714 | Ar28 |
| 1(1268) ~ 2031310(1268) | D714 | Ar29 |
| 1(1269) ~ 2031310(1269)<br>1(1270) ~ 2031310(1270)<br>1(1271) ~ 2031310(1271) | D714<br>D714<br>D714 | Ar30<br>Ar31<br>Ar32 |
| 1(1272) ~ 2031310(1272) | D714 | Ar33 |
| 1(1273) ~ 2031310(1273) | D714 | Ar34 |
| 1(1274) ~ 2031310(1274) | D714 | Ar35 |
| 1(1275) ~ 2031310(1275)<br>1(1276) ~ 2031310(1276) | D714<br>D714 | Ar36<br>Ar37 |
| 1(1277) ~ 2031310(1277) | D714 | Ar38 |
| 1(1278) ~ 2031310(1278) | D714 | Ar39 |
| 1(1279) ~ 2031310(1279) | D714 | Ar40 |
| 1(1280) ~ 2031310(1280)<br>1(1281) ~ 2031310(1281) | D714<br>D714 | Ar41<br>Ar42 |
| 1(1282) ~ 2031310(1282) | D714 | Ar43 |
| 1(1283) ~ 2031310(1283) | D714 | Ar44 |
| 1(1284) ~ 2031310(1284) | D714 | Ar45 |
| 1(1285) ~ 2031310(1285) | D714 | Ar46 |

(continued)

| No. | $Ar^1$ | $Ar^2$ |
|---|---|---|
| 1(1286) ~ 2031310(1286) | D714 | Ar47 |
| 1(1287) ~ 2031310(1287) | D714 | Ar48 |
| 1(1288) ~ 2031310(1288) | D714 | Ar49 |
| 1(1289) ~ 2031310(1289) | D714 | Ar50 |
| 1(1290) ~ 2031310(1290) | D714 | Ar51 |
| 1(1291) ~ 2031310(1291) | D714 | Ar52 |
| 1(1292) ~ 2031310(1292) | D714 | Ar53 |
| 1(1293) ~ 2031310(1293) | D714 | Ar54 |
| 1(1294) ~ 2031310(1294) | D714 | Ar55 |
| 1(1295) ~ 2031310(1295) | D714 | Ar56 |
| 1(1296) ~ 2031310(1296) | D714 | Ar57 |
| 1(1297) ~ 2031310(1297) | D714 | Ar58 |
| 1(1298) ~ 2031310(1298) | D714 | Ar59 |
| 1(1299) ~ 2031310(1299) | D715 | Ar1 |
| 1(1300) ~ 2031310(1300) | D715 | Ar2 |
| 1(1301) ~ 2031310(1301) | D715 | Ar3 |
| 1(1302) ~ 2031310(1302) | D715 | Ar4 |
| 1(1303) ~ 2031310(1303) | D715 | Ar5 |
| 1(1304) ~ 2031310(1304) | D715 | Ar6 |
| 1(1305) ~ 2031310(1305) | D715 | Ar7 |
| 1(1306) ~ 2031310(1306) | D715 | Ar8 |
| 1(1307) ~ 2031310(1307) | D715 | Ar9 |
| 1(1308) ~ 2031310(1308) | D715 | Ar10 |
| 1(1309) ~ 2031310(1309) | D715 | Ar11 |
| 1(1310) ~ 2031310(1310) | D715 | Ar12 |
| 1(1311) ~ 2031310(1311) | D715 | Ar13 |
| 1(1312) ~ 2031310(1312) | D715 | Ar14 |
| 1(1313) ~ 2031310(1313) | D715 | Ar15 |
| 1(1314) ~ 2031310(1314) | D715 | Ar16 |
| 1(1315) ~ 2031310(1315) | D715 | Ar17 |
| 1(1316) ~ 2031310(1316) | D715 | Ar18 |
| 1(1317) ~ 2031310(1317) | D715 | Ar19 |
| 1(1318) ~ 2031310(1318) | D715 | Ar20 |
| 1(1319) ~ 2031310(1319) | D715 | Ar21 |
| 1(1320) ~ 2031310(1320) | D715 | Ar22 |
| 1(1321) ~ 2031310(1321) | D715 | Ar23 |
| 1(1322) ~ 2031310(1322) | D715 | Ar24 |
| 1(1323) ~ 2031310(1323) | D715 | Ar25 |
| 1(1324) ~ 2031310(1324) | D715 | Ar26 |
| 1(1325) ~ 2031310(1325) | D715 | Ar27 |

(continued)

| No. | Ar1 | Ar2 |
|---|---|---|
| 1(1326) ~ 2031310(1326) | D715 | Ar28 |
| 1(1327) ~ 2031310(1327) | D715 | Ar29 |
| 1(1328) ~ 2031310(1328) | D715 | Ar30 |
| 1(1329) ~ 2031310(1329) | D715 | Ar31 |
| 1(1330) ~ 2031310(1330) | D715 | Ar32 |
| 1(1331) ~ 2031310(1331) | D715 | Ar33 |
| 1(1332) ~ 2031310(1332) | D715 | Ar34 |
| 1(1333) ~ 2031310(1333) | D715 | Ar35 |
| 1(1334) ~ 2031310(1334) | D715 | Ar36 |
| 1(1335) ~ 2031310(1335) | D715 | Ar37 |
| 1(1336) ~ 2031310(1336) | D715 | Ar38 |
| 1(1337) ~ 2031310(1337) | D715 | Ar39 |
| 1(1338) ~ 2031310(1338) | D715 | Ar40 |
| 1(1339) ~ 2031310(1339) | D715 | Ar41 |
| 1(1340) ~ 2031310(1340) | D715 | Ar42 |
| 1(1341) ~ 2031310(1341) | D715 | Ar43 |
| 1(1342) ~ 2031310(1342) | D715 | Ar44 |
| 1(1343) ~ 2031310(1343) | D715 | Ar45 |
| 1(1344) ~ 2031310(1344) | D715 | Ar46 |
| 1(1345) ~ 2031310(1345) | D715 | Ar47 |
| 1(1346) ~ 2031310(1346) | D715 | Ar48 |
| 1(1347) ~ 2031310(1347) | D715 | Ar49 |
| 1(1348) ~ 2031310(1348) | D715 | Ar50 |
| 1(1349) ~ 2031310(1349) | D715 | Ar51 |
| 1(1350) ~ 2031310(1350) | D715 | Ar52 |
| 1(1351) ~ 2031310(1351) | D715 | Ar53 |
| 1(1352) ~ 2031310(1352) | D715 | Ar54 |
| 1(1353) ~ 2031310(1353) | D715 | Ar55 |
| 1(1354) ~ 2031310(1354) | D715 | Ar56 |
| 1(1355) ~ 2031310(1355) | D715 | Ar57 |
| 1(1356) ~ 2031310(1356) | D715 | Ar58 |
| 1(1357) ~ 2031310(1357) | D715 | Ar59 |
| 1(1358) ~ 2031310(1358) | D716 | Ar1 |
| 1(1359) ~ 2031310(1359) | D716 | Ar2 |
| 1(1360) ~ 2031310(1360) | D716 | Ar3 |
| 1(1361) ~ 2031310(1361) | D716 | Ar4 |
| 1(1362) ~ 2031310(1362) | D716 | Ar5 |
| 1(1363) ~ 2031310(1363) | D716 | Ar6 |
| 1(1364) ~ 2031310(1364) | D716 | Ar7 |
| 1(1365) ~ 2031310(1365) | D716 | Ar8 |
| 1(1366) ~ 2031310(1366) | D716 | Ar9 |

(continued)

| No. | Ar1 | Ar2 |
|---|---|---|
| 1(1367) ~ 2031310(1367) | D716 | Ar10 |
| 1(1368) ~ 2031310(1368) | D716 | Ar11 |
| 1(1369) ~ 2031310(1369) | D716 | Ar12 |
| 1(1370) ~ 2031310(1370) | D716 | Ar13 |
| 1(1371) ~ 2031310(1371) | D716 | Ar14 |
| 1(1372) ~ 2031310(1372) | D716 | Ar15 |
| 1(1373) ~ 2031310(1373) | D716 | Ar16 |
| 1(1374) ~ 2031310(1374) | D716 | Ar17 |
| 1(1375) ~ 2031310(1375) | D716 | Ar18 |
| 1(1376) ~ 2031310(1376) | D716 | Ar19 |
| 1(1377) ~ 2031310(1377) | D716 | Ar20 |
| 1(1378) ~ 2031310(1378) | D716 | Ar21 |
| 1(1379) ~ 2031310(1379) | D716 | Ar22 |
| 1(1380) ~ 2031310(1380) | D716 | Ar23 |
| 1(1381) ~ 2031310(1381) | D716 | Ar24 |
| 1(1382) ~ 2031310(1382) | D716 | Ar25 |
| 1(1383) ~ 2031310(1383) | D716 | Ar26 |
| 1(1384) ~ 2031310(1384) | D716 | Ar27 |
| 1(1385) ~ 2031310(1385) | D716 | Ar28 |
| 1(1386) ~ 2031310(1386) | D716 | Ar29 |
| 1(1387) ~ 2031310(1387) | D716 | Ar30 |
| 1(1388) ~ 2031310(1388) | D716 | Ar31 |
| 1(1389) ~ 2031310(1389) | D716 | Ar32 |
| 1(1390) ~ 2031310(1390) | D716 | Ar33 |
| 1(1391) ~ 2031310(1391) | D716 | Ar34 |
| 1(1392) ~ 2031310(1392) | D716 | Ar35 |
| 1(1393) ~ 2031310(1393) | D716 | Ar36 |
| 1(1394) ~ 2031310(1394) | D716 | Ar37 |
| 1(1395) ~ 2031310(1395) | D716 | Ar38 |
| 1(1396) ~ 2031310(1396) | D716 | Ar39 |
| 1(1397) ~ 2031310(1397) | D716 | Ar40 |
| 1(1398) ~ 2031310(1398) | D716 | Ar41 |
| 1(1399) ~ 2031310(1399) | D716 | Ar42 |
| 1(1400) ~ 2031310(1400) | D716 | Ar43 |
| 1(1401) ~ 2031310(1401) | D716 | Ar44 |
| 1(1402) ~ 2031310(1402) | D716 | Ar45 |
| 1(1403) ~ 2031310(1403) | D716 | Ar46 |
| 1(1404) ~ 2031310(1404) | D716 | Ar47 |
| 1(1405) ~ 2031310(1405) | D716 | Ar48 |
| 1(1406) ~ 2031310(1406) | D716 | Ar49 |

(continued)

| No. | Ar1 | Ar2 |
|---|---|---|
| 1(1407) ~ 2031310(1407) | D716 | Ar50 |
| 1(1408) ~ 2031310(1408) | D716 | Ar51 |
| 1(1409) ~ 2031310(1409) | D716 | Ar52 |
| 1(1410) ~ 2031310(1410) | D716 | Ar53 |
| 1(1411) ~ 2031310(1411) | D716 | Ar54 |
| 1(1412) ~ 2031310(1412) | D716 | Ar55 |
| 1(1413) ~ 2031310(1413) | D716 | Ar56 |
| 1(1414) ~ 2031310(1414) | D716 | Ar57 |
| 1(1415) ~ 2031310(1415) | D716 | Ar58 |
| 1(1416) ~ 2031310(1416) | D716 | Ar59 |
| 1(1417) ~ 2031310(1417) | D724 | Ar1 |
| 1(1418) ~ 2031310(1418) | D724 | Ar2 |
| 1(1419) ~ 2031310(1419) | D724 | Ar3 |
| 1(1420) ~ 2031310(1420) | D724 | Ar4 |
| 1(1421) ~ 2031310(1421) | D724 | Ar5 |
| 1(1422) ~ 2031310(1422) | D724 | Ar6 |
| 1(1423) ~ 2031310(1423) | D724 | Ar7 |
| 1(1424) ~ 2031310(1424) | D724 | Ar8 |
| 1(1425) ~ 2031310(1425) | D724 | Ar9 |
| 1(1426) ~ 2031310(1426) | D724 | Ar10 |
| 1(1427) ~ 2031310(1427) | D724 | Ar11 |
| 1(1428) ~ 2031310(1428) | D724 | Ar12 |
| 1(1429) ~ 2031310(1429) | D724 | Ar13 |
| 1(1430) ~ 2031310(1430) | D724 | Ar14 |
| 1(1431) ~ 2031310(1431) | D724 | Ar15 |
| 1(1432) ~ 2031310(1432) | D724 | Ar16 |
| 1(1433) ~ 2031310(1433) | D724 | Ar17 |
| 1(1434) ~ 2031310(1434) | D724 | Ar18 |
| 1(1435) ~ 2031310(1435) | D724 | Ar19 |
| 1(1436) ~ 2031310(1436) | D724 | Ar20 |
| 1(1437) ~ 2031310(1437) | D724 | Ar21 |
| 1(1438) ~ 2031310(1438) | D724 | Ar22 |
| 1(1439) ~ 2031310(1439) | D724 | Ar23 |
| 1(1440) ~ 2031310(1440) | D724 | Ar24 |
| 1(1441) ~ 2031310(1441) | D724 | Ar25 |
| 1(1442) ~ 2031310(1442) | D724 | Ar26 |
| 1(1443) ~ 2031310(1443) | D724 | Ar27 |
| 1(1444) ~ 2031310(1444) | D724 | Ar28 |
| 1(1445) ~ 2031310(1445) | D724 | Ar29 |
| 1(1446) ~ 2031310(1446) | D724 | Ar30 |

(continued)

| No. | Ar1 | Ar2 |
|---|---|---|
| 1(1447) ~ 2031310(1447) | D724 | Ar31 |
| 1(1448) ~ 2031310(1448) | D724 | Ar32 |
| 1(1449) ~ 2031310(1449) | D724 | Ar33 |
| 1(1450) ~ 2031310(1450) | D724 | Ar34 |
| 1(1451) ~ 2031310(1451) | D724 | Ar35 |
| 1(1452) ~ 2031310(1452) | D724 | Ar36 |
| 1(1453) ~ 2031310(1453) | D724 | Ar37 |
| 1(1454) ~ 2031310(1454) | D724 | Ar38 |
| 1(1455) ~ 2031310(1455) | D724 | Ar39 |
| 1(1456) ~ 2031310(1456) | D724 | Ar40 |
| 1(1457) ~ 2031310(1457) | D724 | Ar41 |
| 1(1458) ~ 2031310(1458) | D724 | Ar42 |
| 1(1459) ~ 2031310(1459) | D724 | Ar43 |
| 1(1460) ~ 2031310(1460) | D724 | Ar44 |
| 1(1461) ~ 2031310(1461) | D724 | Ar45 |
| 1(1462) ~ 2031310(1462) | D724 | Ar46 |
| 1(1463) ~ 2031310(1463) | D724 | Ar47 |
| 1(1464) ~ 2031310(1464) | D724 | Ar48 |
| 1(1465) ~ 2031310(1465) | D724 | Ar49 |
| 1(1466) ~ 2031310(1466) | D724 | Ar50 |
| 1(1467) ~ 2031310(1467) | D724 | Ar51 |
| 1(1468) ~ 2031310(1468) | D724 | Ar52 |
| 1(1469) ~ 2031310(1469) | D724 | Ar53 |
| 1(1470) ~ 2031310(1470) | D724 | Ar54 |
| 1(1471) ~ 2031310(1471) | D724 | Ar55 |
| 1(1472) ~ 2031310(1472) | D724 | Ar56 |
| 1(1473) ~ 2031310(1473) | D724 | Ar57 |
| 1(1474) ~ 2031310(1474) | D724 | Ar58 |
| 1(1475) ~ 2031310(1475) | D724 | Ar59 |
| 1(1476) ~ 2031310(1476) | D725 | Ar1 |
| 1(1477) ~ 2031310(1477) | D725 | Ar2 |
| 1(1478) ~ 2031310(1478) | D725 | Ar3 |
| 1(1479) ~ 2031310(1479) | D725 | Ar4 |
| 1(1480) ~ 2031310(1480) | D725 | Ar5 |
| 1(1481) ~ 2031310(1481) | D725 | Ar6 |
| 1(1482) ~ 2031310(1482) | D725 | Ar7 |
| 1(1483) ~ 2031310(1483) | D725 | Ar8 |
| 1(1484) ~ 2031310(1484) | D725 | Ar9 |
| 1(1485) ~ 2031310(1485) | D725 | Ar10 |
| 1(1486) ~ 2031310(1486) | D725 | Ar11 |

(continued)

| No. | $Ar^1$ | $Ar^2$ |
|---|---|---|
| 1(1487) ~ 2031310(1487) | D725 | Ar12 |
| 1(1488) ~ 2031310(1488) | D725 | Ar13 |
| 1(1489) ~ 2031310(1489) | D725 | Ar14 |
| 1(1490) ~ 2031310(1490) | D725 | Ar15 |
| 1(1491) ~ 2031310(1491) | D725 | Ar16 |
| 1(1492) ~ 2031310(1492) | D725 | Ar17 |
| 1(1493) ~ 2031310(1493) | D725 | Ar18 |
| 1(1494) ~ 2031310(1494) | D725 | Ar19 |
| 1(1495) ~ 2031310(1495) | D725 | Ar20 |
| 1(1496) ~ 2031310(1496) | D725 | Ar21 |
| 1(1497) ~ 2031310(1497) | D725 | Ar22 |
| 1(1498) ~ 2031310(1498) | D725 | Ar23 |
| 1(1499) ~ 2031310(1499) | D725 | Ar24 |
| 1(1500) ~ 2031310(1500) | D725 | Ar25 |
| 1(1501) ~ 2031310(1501) | D725 | Ar26 |
| 1(1502) ~ 2031310(1502) | D725 | Ar27 |
| 1(1503) ~ 2031310(1503) | D725 | Ar28 |
| 1(1504) ~ 2031310(1504) | D725 | Ar29 |
| 1(1505) ~ 2031310(1505) | D725 | Ar30 |
| 1(1506) ~ 2031310(1506) | D725 | Ar31 |
| 1(1507) ~ 2031310(1507) | D725 | Ar32 |
| 1(1508) ~ 2031310(1508) | D725 | Ar33 |
| 1(1509) ~ 2031310(1509) | D725 | Ar34 |
| 1(1510) ~ 2031310(1510) | D725 | Ar35 |
| 1(1511) ~ 2031310(1511) | D725 | Ar36 |
| 1(1512) ~ 2031310(1512) | D725 | Ar37 |
| 1(1513) ~ 2031310(1513) | D725 | Ar38 |
| 1(1514) ~ 2031310(1514) | D725 | Ar39 |
| 1(1515) ~ 2031310(1515) | D725 | Ar40 |
| 1(1516) ~ 2031310(1516) | D725 | Ar41 |
| 1(1517) ~ 2031310(1517) | D725 | Ar42 |
| 1(1518) ~ 2031310(1518) | D725 | Ar43 |
| 1(1519) ~ 2031310(1519) | D725 | Ar44 |
| 1(1520) ~ 2031310(1520) | D725 | Ar45 |
| 1(1521) ~ 2031310(1521) | D725 | Ar46 |
| 1(1522) ~ 2031310(1522) | D725 | Ar47 |
| 1(1523) ~ 2031310(1523) | D725 | Ar48 |
| 1(1524) ~ 2031310(1524) | D725 | Ar49 |
| 1(1525) ~ 2031310(1525) | D725 | Ar50 |
| 1(1526) ~ 2031310(1526) | D725 | Ar51 |

(continued)

| No. | $Ar^1$ | $Ar^2$ |
|---|---|---|
| 1(1527) ~ 2031310(1527) | D725 | Ar52 |
| 1(1528) ~ 2031310(1528) | D725 | Ar53 |
| 1(1529) ~ 2031310(1529) | D725 | Ar54 |
| 1(1530) ~ 2031310(1530) | D725 | Ar55 |
| 1(1531) ~ 2031310(1531) | D725 | Ar56 |
| 1(1532) ~ 2031310(1532) | D725 | Ar57 |
| 1(1533) ~ 2031310(1533) | D725 | Ar58 |
| 1(1534) ~ 2031310(1534) | D725 | Ar59 |
| 1(1535) ~ 2031310(1535) | D735 | Ar1 |
| 1(1536) ~ 2031310(1536) | D735 | Ar2 |
| 1(1537) ~ 2031310(1537) | D735 | Ar3 |
| 1(1538) ~ 2031310(1538) | D735 | Ar4 |
| 1(1539) ~ 2031310(1539) | D735 | Ar5 |
| 1(1540) ~ 2031310(1540) | D735 | Ar6 |
| 1(1541) ~ 2031310(1541) | D735 | Ar7 |
| 1(1542) ~ 2031310(1542) | D735 | Ar8 |
| 1(1543) ~ 2031310(1543) | D735 | Ar9 |
| 1(1544) ~ 2031310(1544) | D735 | Ar10 |
| 1(1545) ~ 2031310(1545) | D735 | Ar11 |
| 1(1546) ~ 2031310(1546) | D735 | Ar12 |
| 1(1547) ~ 2031310(1547) | D735 | Ar13 |
| 1(1548) ~ 2031310(1548) | D735 | Ar14 |
| 1(1549) ~ 2031310(1549) | D735 | Ar15 |
| 1(1550) ~ 2031310(1550) | D735 | Ar16 |
| 1(1551) ~ 2031310(1551) | D735 | Ar17 |
| 1(1552) ~ 2031310(1552) | D735 | Ar18 |
| 1(1553) ~ 2031310(1553) | D735 | Ar19 |
| 1(1554) ~ 2031310(1554) | D735 | Ar20 |
| 1(1555) ~ 2031310(1555) | D735 | Ar21 |
| 1(1556) ~ 2031310(1556) | D735 | Ar22 |
| 1(1557) ~ 2031310(1557)<br>1(1558) ~ 2031310(1558) | D735<br>D735 | Ar23<br>Ar24 |
| 1(1559) ~ 2031310(1559) | D735 | Ar25 |
| 1(1560) ~ 2031310(1560) | D735 | Ar26 |
| 1(1561) ~ 2031310(1561)<br>1(1562) ~ 2031310(1562) | D735<br>D735 | Ar27<br>Ar28 |
| 1(1563) ~ 2031310(1563) | D735 | Ar29 |
| 1(1564) ~ 2031310(1564) | D735 | Ar30 |
| 1(1565) ~ 2031310(1565) | D735 | Ar31 |
| 1(1566) ~ 2031310(1566) | D735 | Ar32 |

(continued)

| No. | Ar1 | Ar2 |
|---|---|---|
| 1(1567) ~ 2031310(1567) | D735 | Ar33 |
| 1(1568) ~ 2031310(1568) | D735 | Ar34 |
| 1(1569) ~ 2031310(1569) | D735 | Ar35 |
| 1(1570) ~ 2031310(1570) | D735 | Ar36 |
| 1(1571) ~ 2031310(1571) | D735 | Ar37 |
| 1(1572) ~ 2031310(1572)<br>1(1573) ~ 2031310(1573) | D735<br>D735 | Ar38<br>Ar39 |
| 1(1574) ~ 2031310(1574) | D735 | Ar40 |
| 1(1575) ~ 2031310(1575) | D735 | Ar41 |
| 1(1576) ~ 2031310(1576) | D735 | Ar42 |
| 1(1577) ~ 2031310(1577) | D735 | Ar43 |
| 1(1578) ~ 2031310(1578) | D735 | Ar44 |
| 1(1579) ~ 2031310(1579) | D735 | Ar45 |
| 1(1580) ~ 2031310(1580) | D735 | Ar46 |
| 1(1581) ~ 2031310(1581) | D735 | Ar47 |
| 1(1582) ~ 2031310(1582) | D735 | Ar48 |
| 1(1583) ~ 2031310(1583)<br>1(1584) ~ 2031310(1584) | D735<br>D735 | Ar49<br>Ar50 |
| 1(1585) ~ 2031310(1585) | D735 | Ar51 |
| 1(1586) ~ 2031310(1586) | D735 | Ar52 |
| 1(1587) ~ 2031310(1587) | D735 | Ar53 |
| 1(1588) ~ 2031310(1588) | D735 | Ar54 |
| 1(1589) ~ 2031310(1589) | D735 | Ar55 |
| 1(1590) ~ 2031310(1590) | D735 | Ar56 |
| 1(1591) ~ 2031310(1591) | D735 | Ar57 |
| 1(1592) ~ 2031310(1592) | D735 | Ar58 |
| 1(1593) ~ 2031310(1593) | D735 | Ar59 |
| 1(1594) ~ 2031310(1594) | D843 | Ar1 |
| 1(1595) ~ 2031310(1595) | D843 | Ar2 |
| 1(1596) ~ 2031310(1596) | D843 | Ar3 |
| 1(1597) ~ 2031310(1597) | D843 | Ar4 |
| 1(1598) ~ 2031310(1598) | D843 | Ar5 |
| 1(1599) ~ 2031310(1599) | D843 | Ar6 |
| 1(1600) ~ 2031310(1600) | D843 | Ar7 |
| 1(1601) ~ 2031310(1601) | D843 | Ar8 |
| 1(1602) ~ 2031310(1602) | D843 | Ar9 |
| 1(1603) ~ 2031310(1603) | D843 | Ar10 |
| 1(1604) ~ 2031310(1604) | D843 | Ar11 |
| 1(1605) ~ 2031310(1605) | D843 | Ar12 |
| 1(1606) ~ 2031310(1606) | D843 | Ar13 |

(continued)

| No. | Ar1 | Ar2 |
|---|---|---|
| 1(1607) ~ 2031310(1607) | D843 | Ar14 |
| 1(1608) ~ 2031310(1608)<br>1(1609) ~ 2031310(1609) | D843<br>D843 | Ar15<br>Ar16 |
| 1(1610) ~ 2031310(1610)<br>1(1611) ~ 2031310(1611) | D843<br>D843 | Ar17<br>Ar18 |
| 1(1612) ~ 2031310(1612) | D843 | Ar19 |
| 1(1613) ~ 2031310(1613) | D843 | Ar20 |
| 1(1614) ~ 2031310(1614) | D843 | Ar21 |
| 1(1615) ~ 2031310(1615) | D843 | Ar22 |
| 1(1616) ~ 2031310(1616) | D843 | Ar23 |
| 1(1617) ~ 2031310(1617) | D843 | Ar24 |
| 1(1618) ~ 2031310(1618) | D843 | Ar25 |
| 1(1619) ~ 2031310(1619) | D843 | Ar26 |
| 1(1620) ~ 2031310(1620) | D843 | Ar27 |
| 1(1621) ~ 2031310(1621) | D843 | Ar28 |
| 1(1622) ~ 2031310(1622) | D843 | Ar29 |
| 1(1623) ~ 2031310(1623) | D843 | Ar30 |
| 1(1624) ~ 2031310(1624) | D843 | Ar31 |
| 1(1625) ~ 2031310(1625)<br>1(1626) ~ 2031310(1626) | D843<br>D843 | Ar32<br>Ar33 |
| 1(1627) ~ 2031310(1627) | D843 | Ar34 |
| 1(1628) ~ 2031310(1628) | D843 | Ar35 |
| 1(1629) ~ 2031310(1629)<br>1(1630) ~ 2031310(1630) | D843<br>D843 | Ar36<br>Ar37 |
| 1(1631) ~ 2031310(1631) | D843 | Ar38 |
| 1(1632) ~ 2031310(1632) | D843 | Ar39 |
| 1(1633) ~ 2031310(1633) | D843 | Ar40 |
| 1(1634) ~ 2031310(1634) | D843 | Ar41 |
| 1(1635) ~ 2031310(1635) | D843 | Ar42 |
| 1(1636) ~ 2031310(1636) | D843 | Ar43 |
| 1(1637) ~ 2031310(1637) | D843 | Ar44 |
| 1(1638) ~ 2031310(1638) | D843 | Ar45 |
| 1(1639) ~ 2031310(1639) | D843 | Ar46 |
| 1(1640) ~ 2031310(1640) | D843 | Ar47 |
| 1(1641) ~ 2031310(1641) | D843 | Ar48 |
| 1(1642) ~ 2031310(1642) | D843 | Ar49 |
| 1(1643) ~ 2031310(1643) | D843 | Ar50 |
| 1(1644) ~ 2031310(1644) | D843 | Ar51 |
| 1(1645) ~ 2031310(1645) | D843 | Ar52 |
| 1(1646) ~ 2031310(1646) | D843 | Ar53 |

(continued)

| No. | Ar1 | Ar2 |
|---|---|---|
| 1(1647) ~ 2031310(1647) | D843 | Ar54 |
| 1(1648) ~ 2031310(1648) | D843 | Ar55 |
| 1(1649) ~ 2031310(1649) | D843 | Ar56 |
| 1(1650) ~ 2031310(1650) | D843 | Ar57 |
| 1(1651) ~ 2031310(1651) | D843 | Ar58 |
| 1(1652) ~ 2031310(1652) | D843 | Ar59 |
| 1(1653) ~ 2031310(1653) | D717 | D1 |
| 1(1654) ~ 2031310(1654) | D717 | D7 |
| 1(1655) ~ 2031310(1655) | D717 | D8 |
| 1(1656) ~ 2031310(1656) | D717 | D9 |
| 1(1657) ~ 2031310(1657) | D717 | D19 |
| 1(1658) ~ 2031310(1658) | D717 | D37 |
| 1(1659) ~ 2031310(1659) | D717 | D50 |
| 1(1660) ~ 2031310(1660) | D717 | D77 |
| 1(1661) ~ 2031310(1661) | D717 | D281 |
| 1(1662) ~ 2031310(1662) | D717 | D459 |
| 1(1663) ~ 2031310(1663) | D717 | D465 |
| 1(1664) ~ 2031310(1664) | D717 | D466 |
| 1(1665) ~ 2031310(1665) | D717 | D467 |
| 1(1666) ~ 2031310(1666) | D717 | D471 |
| 1(1667) ~ 2031310(1667) | D717 | D486 |
| 1(1668) ~ 2031310(1668) | D717 | D520 |
| 1(1669) ~ 2031310(1669) | D717 | D621 |
| 1(1670) ~ 2031310(1670) | D717 | D711 |
| 1(1671) ~ 2031310(1671) | D717 | D712 |
| 1(1672) ~ 2031310(1672) | D717 | D713 |
| 1(1673) ~ 2031310(1673) | D717 | D714 |
| 1(1674) ~ 2031310(1674) | D717 | D715 |
| 1(1675) ~ 2031310(1675) | D717 | D716 |
| 1(1676) ~ 2031310(1676) | D717 | D725 |
| 1(1677) ~ 2031310(1677) | D717 | D735 |
| 1(1678) ~ 2031310(1678) | D717 | D843 |
| 1(1679) ~ 2031310(1679) | D1 | D7 |
| 1(1680) ~ 2031310(1680) | D1 | D8 |
| 1(1681) ~ 2031310(1681) | D1 | D9 |
| 1(1682) ~ 2031310(1682) | D1 | D19 |
| 1(1683) ~ 2031310(1683) | D1 | D37 |
| 1(1684) ~ 2031310(1684) | D1 | D50 |
| 1(1685) ~ 2031310(1685) | D1 | D77 |
| 1(1686) ~ 2031310(1686) | D1 | D281 |

(continued)

| No. | $Ar^1$ | $Ar^2$ |
|---|---|---|
| 1(1687) ~ 2031310(1687) | D1 | D459 |
| 1(1688) ~ 2031310(1688) | D1 | D465 |
| 1(1689) ~ 2031310(1689) | D1 | D466 |
| 1(1690) ~ 2031310(1690) | D1 | D467 |
| 1(1691) ~ 2031310(1691) | D1 | D471 |
| 1(1692) ~ 2031310(1692) | D1 | D486 |
| 1(1693) ~ 2031310(1693) | D1 | D520 |
| 1(1694) ~ 2031310(1694) | D1 | D621 |
| 1(1695) ~ 2031310(1695) | D1 | D711 |
| 1(1696) ~ 2031310(1696) | D1 | D712 |
| 1(1697) ~ 2031310(1697) | D1 | D713 |
| 1(1698) ~ 2031310(1698) | D1 | D714 |
| 1(1699) ~ 2031310(1699) | D1 | D715 |
| 1(1700) ~ 2031310(1700) | D1 | D716 |
| 1(1701) ~ 2031310(1701) | D1 | D725 |
| 1(1702) ~ 2031310(1702) | D1 | D735 |
| 1(1703) ~ 2031310(1703) | D1 | D843 |
| 1(1704) ~ 2031310(1704) | D7 | D8 |
| 1(1705) ~ 2031310(1705) | D7 | D9 |
| 1(1706) ~ 2031310(1706) | D7 | D19 |
| 1(1707) ~ 2031310(1707) | D7 | D37 |
| 1(1708) ~ 2031310(1708) | D7 | D50 |
| 1(1709) ~ 2031310(1709) | D7 | D77 |
| 1(1710) ~ 2031310(1710) | D7 | D281 |
| 1(1711) ~ 2031310(1711) | D7 | D459 |
| 1(1712) ~ 2031310(1712) | D7 | D465 |
| 1(1713) ~ 2031310(1713) | D7 | D466 |
| 1(1714) ~ 2031310(1714) | D7 | D467 |
| 1(1715) ~ 2031310(1715) | D7 | D471 |
| 1(1716) ~ 2031310(1716) | D7 | D486 |
| 1(1717) ~ 2031310(1717) | D7 | D520 |
| 1(1718) ~ 2031310(1718) | D7 | D621 |
| 1(1719) ~ 2031310(1719) | D7 | D711 |
| 1(1720) ~ 2031310(1720) | D7 | D712 |
| 1(1721) ~ 2031310(1721) | D7 | D713 |
| 1(1722) ~ 2031310(1722) | D7 | D714 |
| 1(1723) ~ 2031310(1723) | D7 | D715 |
| 1(1724) ~ 2031310(1724) | D7 | D716 |
| 1(1725) ~ 2031310(1725) | D7 | D725 |
| 1(1726) ~ 2031310(1726) | D7 | D735 |

(continued)

| No. | Ar1 | Ar2 |
|---|---|---|
| 1(1727) ~ 2031310(1727) | D7 | D843 |
| 1(1728) ~ 2031310(1728) | D8 | D9 |
| 1(1729) ~ 2031310(1729) | D8 | D19 |
| 1(1730) ~ 2031310(1730) | D8 | D37 |
| 1(1731) ~ 2031310(1731) | D8 | D50 |
| 1(1732) ~ 2031310(1732) | D8 | D77 |
| 1(1733) ~ 2031310(1733) | D8 | D281 |
| 1(1734) ~ 2031310(1734) | D8 | D459 |
| 1(1735) ~ 2031310(1735) | D8 | D465 |
| 1(1736) ~ 2031310(1736) | D8 | D466 |
| 1(1737) ~ 2031310(1737) | D8 | D467 |
| 1(1738) ~ 2031310(1738) | D8 | D471 |
| 1(1739) ~ 2031310(1739) | D8 | D486 |
| 1(1740) ~ 2031310(1740) | D8 | D520 |
| 1(1741) ~ 2031310(1741) | D8 | D621 |
| 1(1742) ~ 2031310(1742) | D8 | D711 |
| 1(1743) ~ 2031310(1743) | D8 | D712 |
| 1(1744) ~ 2031310(1744) | D8 | D713 |
| 1(1745) ~ 2031310(1745) | D8 | D714 |
| 1(1746) ~ 2031310(1746) | D8 | D715 |
| 1(1747) ~ 2031310(1747) | D8 | D716 |
| 1(1748) ~ 2031310(1748) | D8 | D725 |
| 1(1749) ~ 2031310(1749) | D8 | D735 |
| 1(1750) ~ 2031310(1750) | D8 | D843 |
| 1(1751) ~ 2031310(1751) | D9 | D19 |
| 1(1752) ~ 2031310(1752) | D9 | D37 |
| 1(1753) ~ 2031310(1753) | D9 | D50 |
| 1(1754) ~ 2031310(1754) | D9 | D77 |
| 1(1755) ~ 2031310(1755) | D9 | D281 |
| 1(1756) ~ 2031310(1756) | D9 | D459 |
| 1(1757) ~ 2031310(1757) | D9 | D465 |
| 1(1758) ~ 2031310(1758) | D9 | D466 |
| 1(1759) ~ 2031310(1759) | D9 | D467 |
| 1(1760) ~ 2031310(1760) | D9 | D471 |
| 1(1761) ~ 2031310(1761) | D9 | D486 |
| 1(1762) ~ 2031310(1762) | D9 | D520 |
| 1(1763) ~ 2031310(1763) | D9 | D621 |
| 1(1764) ~ 2031310(1764) | D9 | D711 |
| 1(1765) ~ 2031310(1765) | D9 | D712 |
| 1(1766) ~ 2031310(1766) | D9 | D713 |

(continued)

| No. | Ar1 | Ar2 |
|---|---|---|
| 1(1767) ~ 2031310(1767) | D9 | D714 |
| 1(1768) ~ 2031310(1768) | D9 | D715 |
| 1(1769) ~ 2031310(1769) | D9 | D716 |
| 1(1770) ~ 2031310(1770) | D9 | D725 |
| 1(1771) ~ 2031310(1771) | D9 | D735 |
| 1(1772) ~ 2031310(1772) | D9 | D843 |
| 1(1773) ~ 2031310(1773)<br>1(1774) ~ 2031310(1774) | D19<br>D19 | D37<br>D50 |
| 1(1775) ~ 2031310(1775) | D19 | D77 |
| 1(1776) ~ 2031310(1776) | D19 | D281 |
| 1(1777) ~ 2031310(1777) | D19 | D459 |
| 1(1778) ~ 2031310(1778)<br>1(1779) ~ 2031310(1779) | D19<br>D19 | D465<br>D466 |
| 1(1780) ~ 2031310(1780) | D19 | D467 |
| 1(1781) ~ 2031310(1781) | D19 | D471 |
| 1(1782) ~ 2031310(1782) | D19 | D486 |
| 1(1783) ~ 2031310(1783) | D19 | D520 |
| 1(1784) ~ 2031310(1784) | D19 | D621 |
| 1(1785) ~ 2031310(1785) | D19 | D711 |
| 1(1786) ~ 2031310(1786) | D19 | D712 |
| 1(1787) ~ 2031310(1787) | D19 | D713 |
| 1(1788) ~ 2031310(1788) | D19 | D714 |
| 1(1789) ~ 2031310(1789) | D19 | D715 |
| 1(1790) ~ 2031310(1790) | D19 | D716 |
| 1(1791) ~ 2031310(1791) | D19 | D725 |
| 1(1792) ~ 2031310(1792) | D19 | D735 |
| 1(1793) ~ 2031310(1793) | D19 | D843 |
| 1(1794) ~ 2031310(1794) | D37 | D50 |
| 1(1795) ~ 2031310(1795) | D37 | D77 |
| 1(1796) ~ 2031310(1796) | D37 | D281 |
| 1(1797) ~ 2031310(1797) | D37 | D459 |
| 1(1798) ~ 2031310(1798)<br>1(1799) ~ 2031310(1799) | D37<br>D37 | D465<br>D466 |
| 1(1800) ~ 2031310(1800)<br>1(1801) ~ 2031310(1801) | D37<br>D37 | D467<br>D471 |
| 1(1802) ~ 2031310(1802) | D37 | D486 |
| 1(1803) ~ 2031310(1803)<br>1(1804) ~ 2031310(1804) | D37<br>D37 | D520<br>D621 |
| 1(1805) ~ 2031310(1805) | D37 | D711 |
| 1(1806) ~ 2031310(1806) | D37 | D712 |

(continued)

| No. | $Ar^1$ | $Ar^2$ |
|---|---|---|
| 1(1807) ~ 2031310(1807) | D37 | D713 |
| 1(1808) ~ 2031310(1808) | D37 | D714 |
| 1(1809) ~ 2031310(1809) | D37 | D715 |
| 1(1810) ~ 2031310(1810) | D37 | D716 |
| 1(1811) ~ 2031310(1811) | D37 | D725 |
| 1(1812) ~ 2031310(1812) | D37 | D735 |
| 1(1813) ~ 2031310(1813) | D37 | D843 |

The compounds specified by the above numbers are all individually disclosed. In addition, among the specific examples of the compounds, in the case where a rotamer is present, a mixture of rotamers and each separated rotamer are also disclosed in the description herein.

In one aspect of the present invention, compounds are selected from Compound Group α consisting of Compounds 1 to 2031310 and Compounds 1(n) to 2031310(n), where n is 1 to 1813. In one aspect of the present invention, compounds are selected from those of Compound Group α satisfying the above [4]. In one aspect of the present invention, compounds are selected from those of Compound Group α satisfying the above [4]. In one aspect of the present invention, compounds are selected from those of Compound Group α satisfying the above [5]. In one aspect of the present invention, compounds are selected from those of Compound Group α satisfying the above [6]. In one aspect of the present invention, compounds are selected from those of Compound Group α satisfying the above [7]. In one aspect of the present invention, compounds are selected from those of Compound Group α satisfying the above [8]. In one aspect of the present invention, compounds are selected from those of Compound Group α satisfying the above [9]. In one aspect of the present invention, compounds are selected from those of Compound Group α satisfying the above [10]. In one aspect of the present invention, compounds are selected from those of Compound Group α satisfying the above [11]. In one aspect of the present invention, compounds are selected from those of Compound Group α satisfying the above [15].

In one aspect of the present invention, compounds are selected from Compound Group β consisting of Compounds 1 to 1015655 and Compounds 1(n) to 1015655(n), where n is 1 to 1813. In one aspect of the present invention, compounds are selected from those of Compound Group β satisfying the above [4]. In one aspect of the present invention, compounds are selected from those of Compound Group β satisfying the above [5]. In one aspect of the present invention, compounds are selected from those of Compound Group β satisfying the above [6]. In one aspect of the present invention, compounds are selected from those of Compound Group β satisfying the above [7]. In one aspect of the present invention, compounds are selected from those of Compound Group β satisfying the above [8]. In one aspect of the present invention, compounds are selected from those of Compound Group β satisfying the above [9]. In one aspect of the present invention, compounds are selected from those of Compound Group β satisfying the above [10]. In one aspect of the present invention, compounds are selected from those of Compound Group β satisfying the above [11]. In one aspect of the present invention, compounds are selected from those of Compound Group β satisfying the above [15]. In one aspect of the present invention, compounds are selected from Compound Group γ consisting of Compounds 1015656 to 2031310 and Compounds 1015656(n) to 2031310(n), where n is 1 to 1813. In one aspect of the present invention, compounds are selected from those of Compound Group γ satisfying the above [4]. In one aspect of the present invention, compounds are selected from those of Compound Group γ satisfying the above [5]. In one aspect of the present invention, compounds are selected from those of Compound Group γ satisfying the above [6]. In one aspect of the present invention, compounds are selected from those of Compound Group γ satisfying the above [7]. In one aspect of the present invention, compounds are selected from those of Compound Group γ satisfying the above [8]. In one aspect of the present invention, compounds are selected from those of Compound Group γ satisfying the above [10]. In one aspect of the present invention, compounds are selected from those of Compound Group γ satisfying the above [11]. In one aspect of the present invention, compounds are selected from those of Compound Group γ satisfying the above [15].

In one aspect of the present invention, the compound represented by the general formula (1) is selected from the following group of compounds.

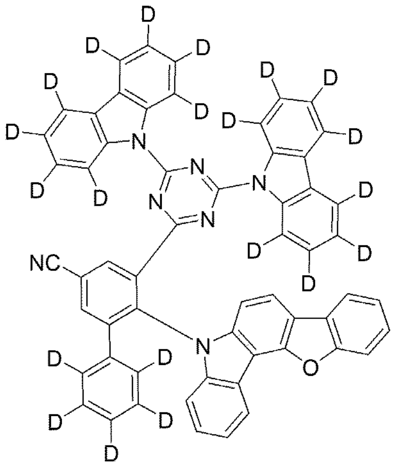

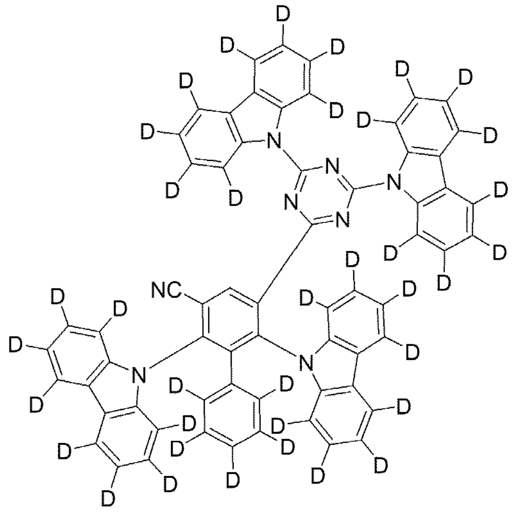

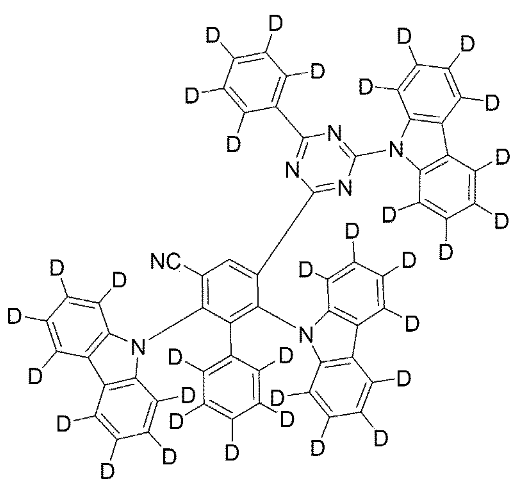

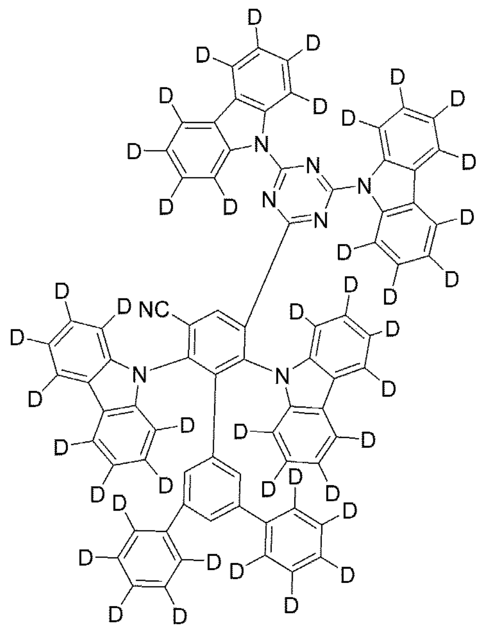

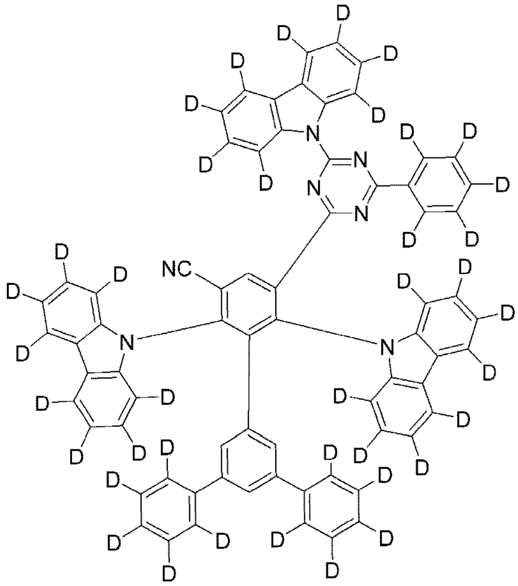

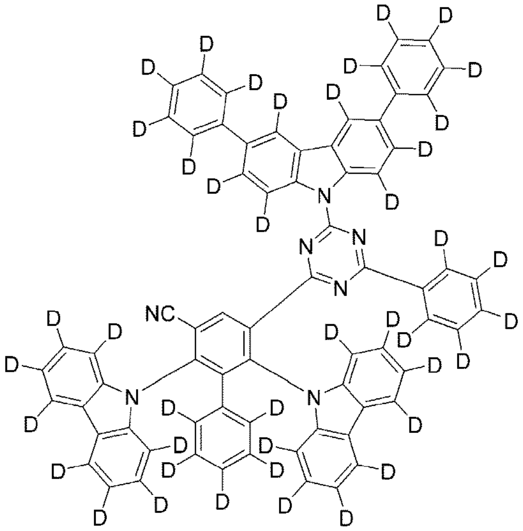

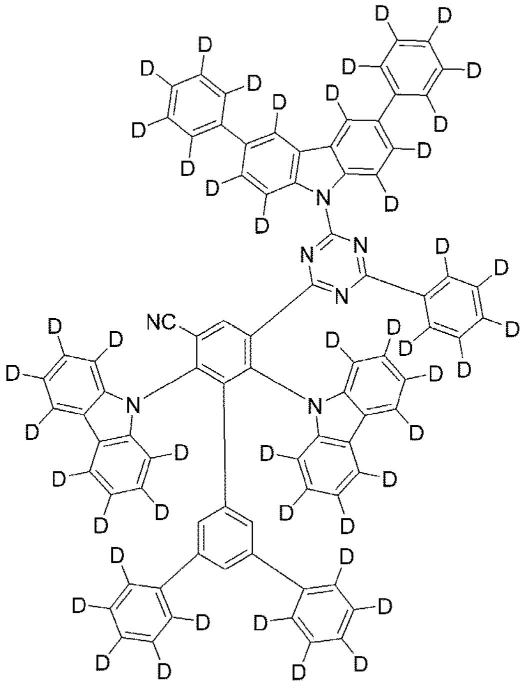

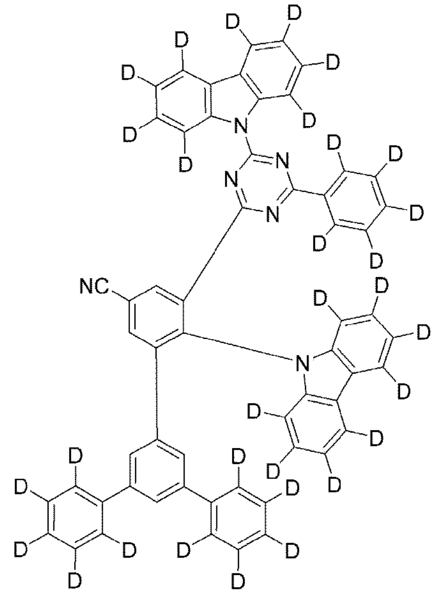

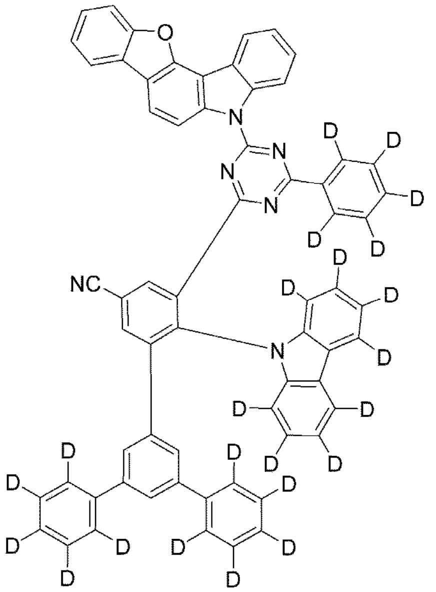

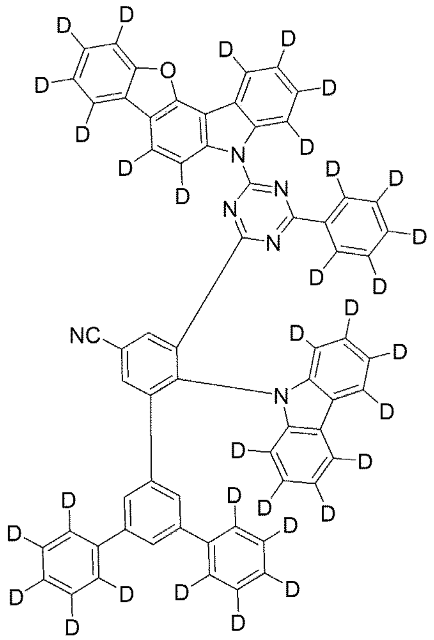

In one aspect of the present invention, the compound represented by the general formula (1) is selected from the following group of compounds.

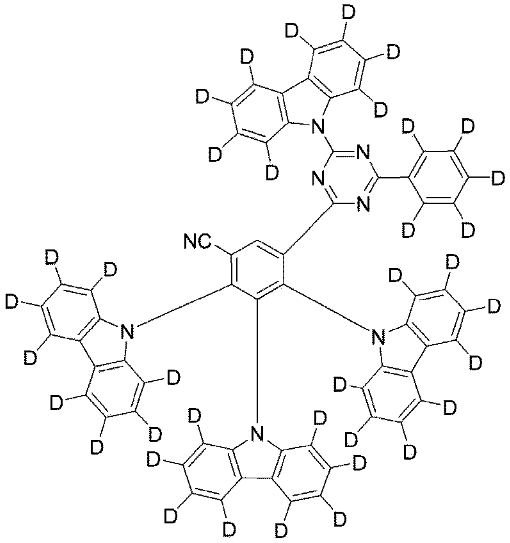

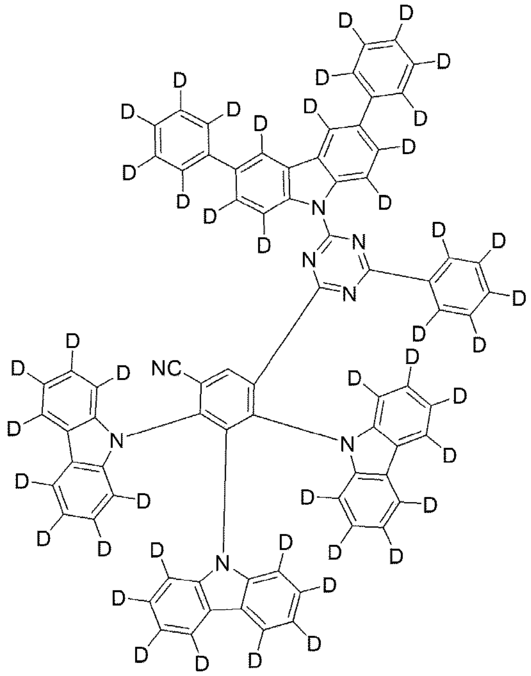

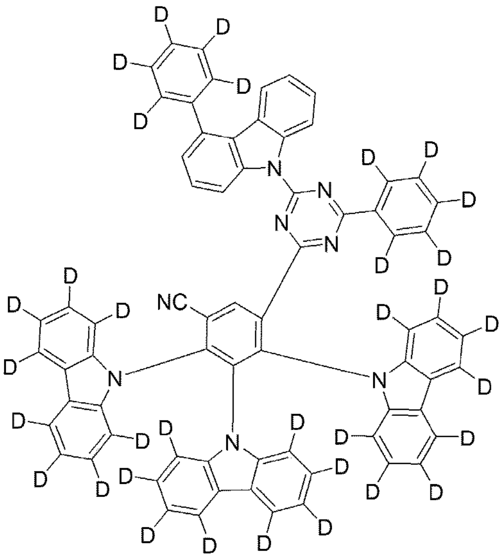

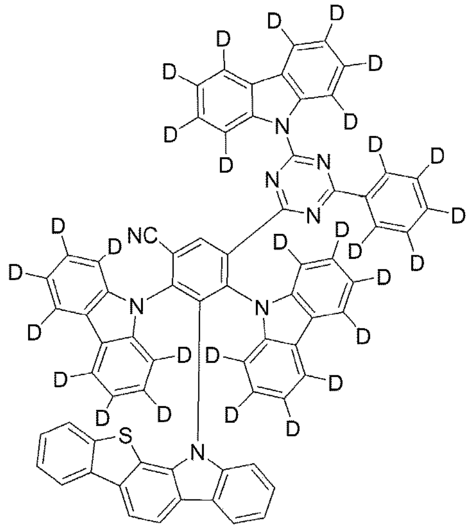

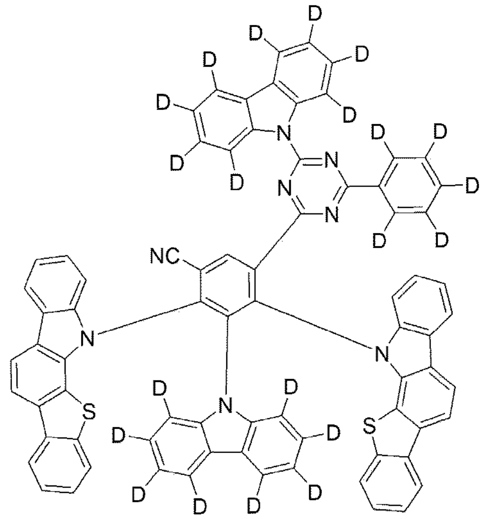

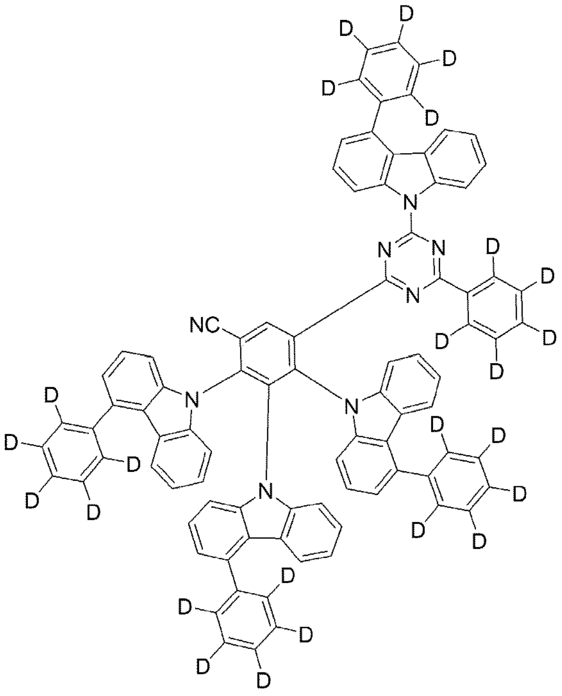

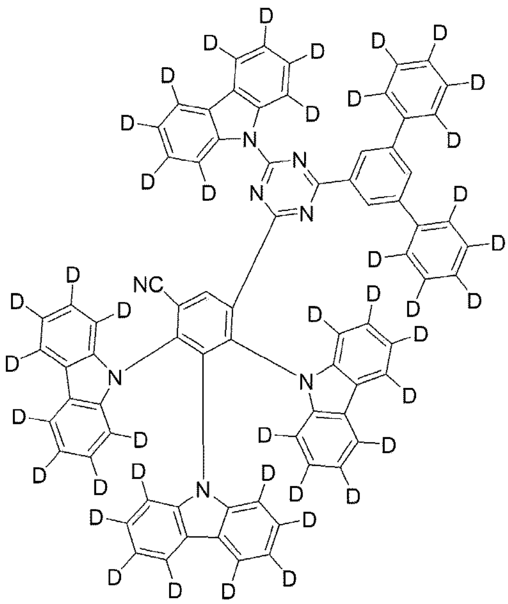

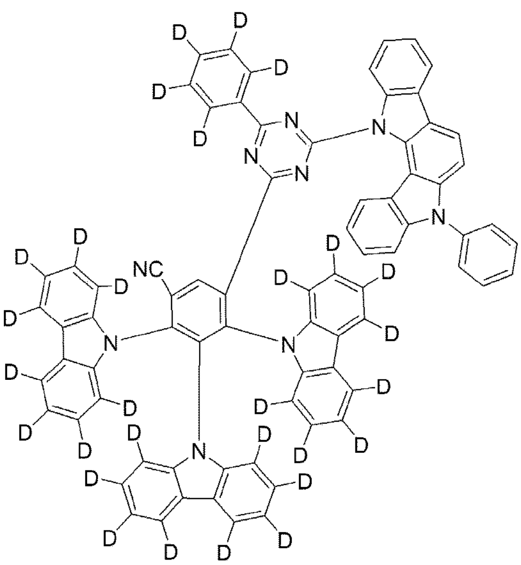

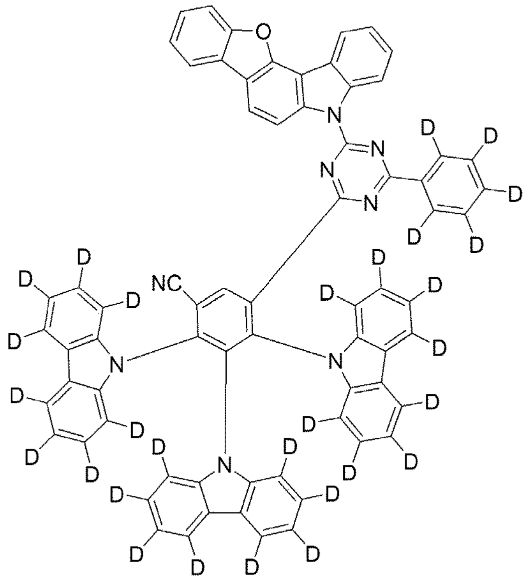

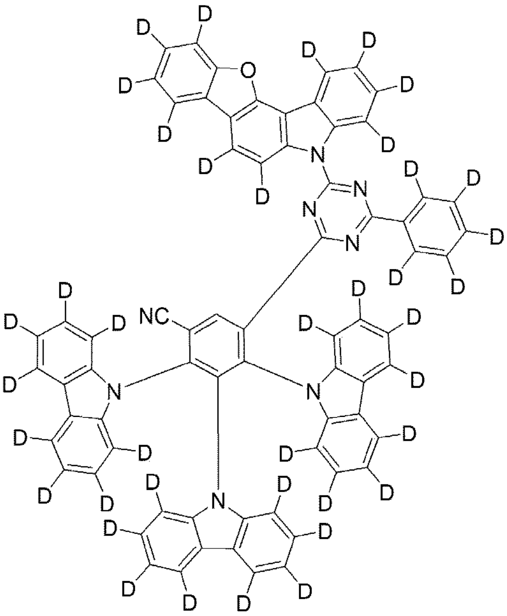

The molecular weight of the compound represented by the general formula (1) is preferably 1500 or less, more preferably 1200 or less, still more preferably 1000 or less, and even more preferably 900 or less, for example, in the case where an organic layer containing the compound represented by the general formula (1) is intended to be film-formed and used by a vapor deposition method. The lower limit of the molecular weight is the molecular weight of the minimum compound represented by the general formula (1).

The compound represented by the general formula (1) can be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable to a coating method and is easily purified to increase its purity.

It is also conceivable to use a compound containing a plurality of structures represented by the general formula (1) in a molecule as a light emitting material by applying the present invention.

For example, it is conceivable that a polymer obtained by allowing a polymerizable group to be present in the structure represented by the general formula (1) in advance and polymerizing the polymerizable group is used as the light emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light emitting material. Alternatively, it is also conceivable to obtain a dimer or a trimer by coupling compounds having a structure represented by the general formula (1) to each other and to use the dimer or the trimer as a light emitting material.

Examples of the polymer having a repeating unit containing a structure represented by the general formula (1) include polymers containing a structure represented by any one of the following two general formulae.

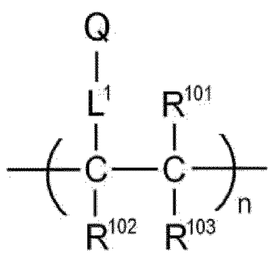

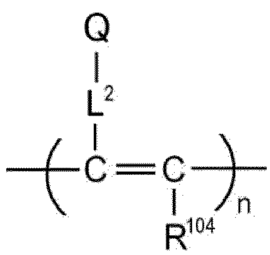

In the above general formulae, Q represents a group containing the structure represented by the general formula (1), and $L^1$ and $L^2$ represent a linking group. The linking group preferably has 0 to 20 carbon atoms, more preferably 1 to 15 carbon atoms, and still more preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or

unsubstituted phenylene group.

In the above general formulae, $R^{101}$, $R^{102}$, $R^{103}$ and $R^{104}$ each independently represent a substituent. It is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and still more preferably an unsubstituted alkyl group having 1 to 3 carbon atoms or an unsubstituted alkoxy group having 1 to 3 carbon atoms.

The linking group represented by $L^1$ and $L^2$ can bond to any site of the general formula (1) constituting Q. Two or more linking groups can be linked to one Q to form a cross-linked structure or a network structure.

Specific structural examples of the repeating unit include structures represented by the following formulae.

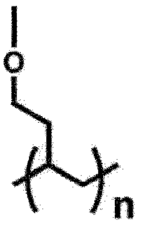
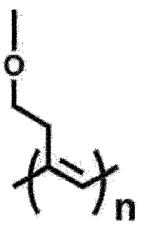
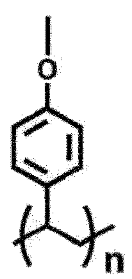
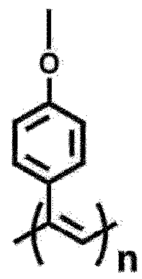

The polymer having a repeating unit including these formulae can be synthesized by introducing a hydroxy group into any site of the general formula (1), reacting the following compound using the hydroxy group as a linker to introduce a polymerizable group, and polymerizing the polymerizable group.

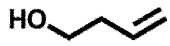
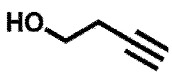
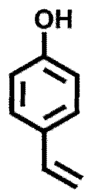
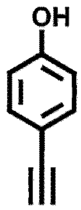

The polymer having a structure represented by the general formula (1) in the molecule can be a polymer having only a repeating unit that has the structure represented by the general formula (1), or can be a polymer containing a repeating unit that has any other structure. The repeating unit having the structure represented by the general formula (1) to be contained in the polymer can be a single kind or two or more kinds. The repeating unit not having the structure represented by the general formula (1) includes those derived from monomers used in general copolymerization. For example, it includes repeating units derived from monomers having an ethylenically unsaturated bond, such as ethylene or styrene.

In some embodiments, the compound represented by the general formula (1) is a light emitting material.

In some embodiments, the compound represented by the general formula (1) is a compound capable of emitting delayed fluorescence.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm) or emit light in a near IR region.

In some embodiments of the present disclosure, the compound represented by the general formula (1) is, when excited thermally or by an electronic means, able to emit light of red or orange in a visible spectral region (e.g., about 620 nm to about 780 nm, about 650 nm).

In some embodiments of the present disclosure, the compound represented by the general formula (1) is, when excited thermally or by an electronic means, able to emit light of orange or yellow in a visible spectral region (e.g., about 570 nm to about 620 nm, about 590 nm, about 570 nm).

In some embodiments of the present disclosure, the compound represented by the general formula (1) is, when excited thermally or by an electronic means, able to emit light of green in a visible spectral region (e.g., about 490 nm to about 575 nm, about 510 nm).

In some embodiments of the present disclosure, the compound represented by the general formula (1) is, when excited thermally or by an electronic means, able to emit light of blue in a visible spectral region (e.g., about 400 nm to about

490 nm, about 475 nm).

In some embodiments of the present disclosure, the compound represented by the general formula (1) is, when excited thermally or by an electronic means, able to emit light in a UV spectral region (e.g., about 280 to 400 nm).

In some embodiments of the present disclosure, the compound represented by the general formula (1) is, when excited thermally or by an electronic means, able to emit light in an IR spectral region (e.g., about 780 nm to 2 μm).

In some embodiments of the present disclosure, an organic semiconductor device using the compound represented by the general formula (1) can be produced. The organic semiconductor device referred to herein can be an organic optical device in which light is interposed or an organic device in which light is not interposed. The organic optical device can be an organic light emitting device in which the device emits light, an organic light receiving device in which the device receives light, or a device in which energy transfer by light occurs in the device. In some embodiments of the present disclosure, an organic optical device such as an organic electroluminescent device or a solid-state imaging device (for example, a CMOS image sensor) can be produced by using the compound represented by the general formula (1). In some embodiments of the present disclosure, a CMOS (complementary metal-oxide film semiconductor) or the like using the compound represented by the general formula (1) can be produced.

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFT/B3LYP/6-31G*) is analyzed and molecular fractions (parts) having HOMO not lower than a specific threshold value and LUMO not higher than a specific threshold value can be screened.

With that, for example, in the presence of a HOMO energy (for example, ionizing potential) of -6.5 eV or more, a donor part ("D") can be selected. On the other hand, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the π-conjugated system.

In some embodiments, a compound library is screened using one or more of the following characteristics.

1. Light emission around a specific wavelength
2. A triplet state over a calculated specific energy level
3. $\Delta E_{ST}$ value lower than a specific value
4. Quantum yield more than a specific value
5. HOMO level
6. LUMO level

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV. In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV.

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Method for Synthesizing Compound Represented by General Formula (1)]

The compound represented by the general formula (1) includes a novel compound.

The compound represented by the general formula (1) can be synthesized by combining known reactions. For example, by reacting a cyanobenzene having a substituted or unsubstituted aryl group (e.g., a phenyl group) and a halogen atom with a substituted ring-fused carbazole, the compound represented by the general formula (1) substituted with a substituted ring-fused carbazol-9-yl group can be synthesized. For details of the reaction conditions, Synthesis Examples described later can be referred to.

[Structure Using Compound Represented by General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound

represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In the case where a vacuum deposition method is employed, compounds to constitute a film can be co-deposited from individual vapor deposition sources, or can be co-deposited from a single vapor deposition source formed by mixing the compounds. In the case where a single vapor deposition source is used, a mixed powder prepared by mixing compound powders can be used, or a compression molded body prepared by compression-molding the mixed powder can be used, or a mixture prepared by heating and melting the compounds and cooling the resulting melt can be used. In some embodiments, by co-deposition under the condition where the vapor deposition rate (weight reduction rate) of the plural compounds contained in a single vapor deposition source is the same or is nearly the same, a film having a compositional ratio corresponding to the compositional ratio of the plural compounds contained in the vapor deposition source can be formed. When plural compounds are mixed in the same compositional ratio as the compositional ratio of the film to be formed to prepare a vapor deposition source, a film having a desired compositional ratio can be formed in a simplified manner. In some embodiments, the temperature at which the compounds to be co-deposited have the same weight reduction ratio is specifically defined, and the temperature can be employed as the temperature of co-deposition.

[Use Examples of Compound Represented by General Formula (1)]

The compound represented by the general formula (1) is useful as a material for an organic light emitting device. In particular, the compound is preferably used for an organic light emitting diode or the like.

Organic Light Emitting Diode:

One embodiment of the present invention relates to use of the compound represented by the general formula (1) of the present invention as a light emitting material for organic light emitting devices. In some embodiments, the compound represented by the general formula (1) of the present invention can be effectively used as a light emitting material in a light emitting layer in an organic light emitting device. In some embodiments, the compound represented by the general formula (1) includes a delayed fluorescent material that emits delayed fluorescence. In some embodiments, the present invention provides a delayed fluorescent material having a structure represented by the general formula (1). In some embodiments, the present invention relates to use of the compound represented by the general formula (1) as a delayed fluorescent material. In some embodiments, the compound represented by the general formula (1) of the present invention can be used as a host material, and can be used along with one or more light emitting materials, and the light emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light emitting device containing the compound as a light emitting material emits delayed fluorescence and shows a high light emission efficiency.

In some embodiments, the light emitting layer contains the compound represented by the general formula (1), and the compound represented by the general formula (1) is aligned in parallel to the substrate. In some embodiments, the substrate is a film-forming surface. In some embodiment, the alignment of the compound represented by the general

formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light emitting layer can be improved.

One aspect of the present invention relates to an organic light emitting device. In some embodiments, the organic light emitting device includes a light emitting layer. In some embodiments, the light emitting layer contains, as a light emitting material, the compound represented by the general formula (1). In some embodiments, the organic light emitting device is an organic photoluminescent device (organic PL device). In some embodiments, the organic light emitting device is an organic electroluminescent device (organic EL device). In some embodiments, the compound represented by the general formula (1) assists light irradiation from the other light emitting materials contained in the light emitting layer (as a so-called assist dopant). In some embodiments, the compound represented by the general formula (1) contained in the light emitting layer is in a lowest excited singlet energy level, and is contained between the lowest excited single energy level of the host material contained in the light emitting layer and the lowest excited singlet energy level of the other light emitting materials contained in the light emitting layer.

In some embodiments, the organic photoluminescent device comprises at least one light emitting layer. In some embodiments, the organic electroluminescent device includes at least an anode, a cathode, and an organic layer between the anode and the cathode. In some embodiments, the organic layer includes at least a light emitting layer. In some embodiments, the organic layer includes only a light emitting layer. In some embodiments, the organic layer includes one or more organic layers in addition to the light emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer can be a hole injection and transporting layer having a hole injection function, and the electron transporting layer can be an electron injection and transporting layer having an electron injection function.

Light Emitting Layer:

In some embodiments, the light emitting layer is a layer where holes and electrons injected from the anode and the cathode, respectively, are recombined to form excitons. In some embodiments, the layer emits light.

In some embodiments, only a light emitting material is used as the light emitting layer. In some embodiments, the light emitting layer contains a light emitting material and a host material. In some embodiments, the light emitting material is one or more compounds represented by the general formula (1). In some embodiments, for improving light emission efficiencies of an organic electroluminescent device and an organic photoluminescent device, the singlet exciton and the triplet exciton generated in a light emitting material are confined inside the light emitting material. In some embodiments, a host material is used in the light emitting layer in addition to a light emitting material. In some embodiments, the host material is an organic compound. In some embodiments, the organic compound has an excited singlet energy and an excited triplet energy, and at least one of them is higher than those in the light emitting material of the present invention. In some embodiments, the singlet exciton and the triplet exciton generated in the light emitting material of the present invention are confined in the molecules of the light emitting material of the present invention. In some embodiments, the singlet and triplet excitons are fully confined for improving light emission efficiency. In some embodiments, although high luminous radiation efficiency is still attained, singlet excitons and triplet excitons are not fully confined, that is, a host material capable of attaining high light emission efficiency can be used in the present invention with no specific limitation. In some embodiments, in the light emitting material in the light emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant and has a lower excited singlet energy than the host material in the light emitting layer and a higher excited singlet energy than the light emitting material in the light emitting layer.

In the case where the compound represented by the general formula (1) is used as an assist dopant, various compounds can be employed as a light emitting material (preferably a fluorescent material). As such light emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a carbazole derivative, a julolidine derivative, a thiazole derivative, and a derivative having a metal (Al, Zn). These exemplified skeletons can have a substituent, or may not have a substituent. These exemplified skeletons can be combined.

Light emitting materials that can be used in combination with the assist dopant having a structure represented by the general formula (1) are shown below.

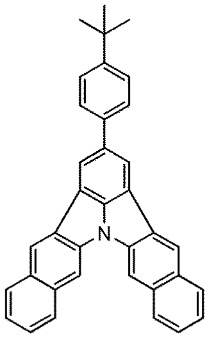

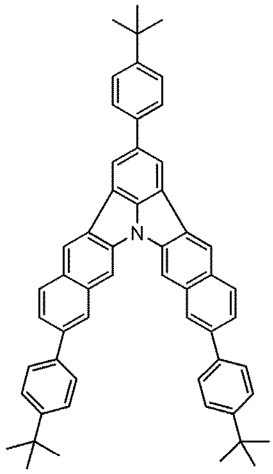

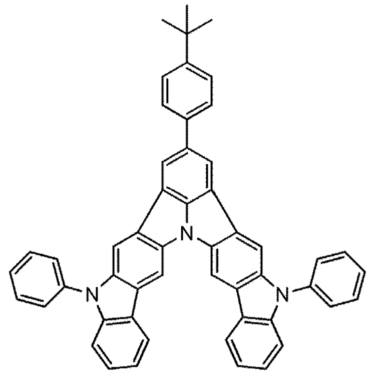

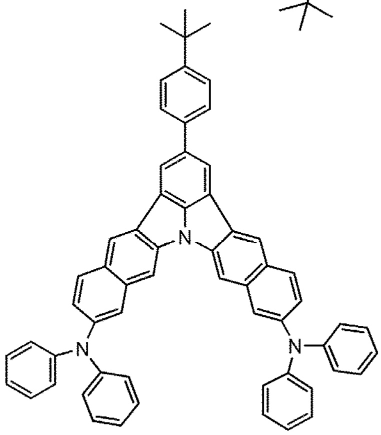

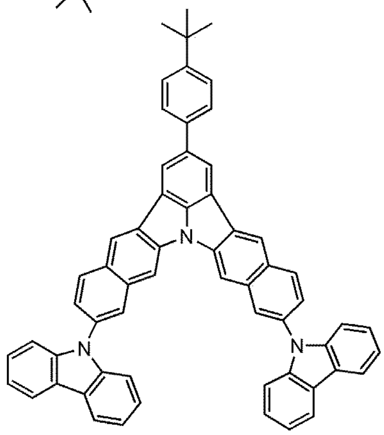

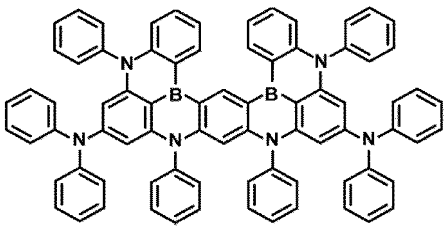

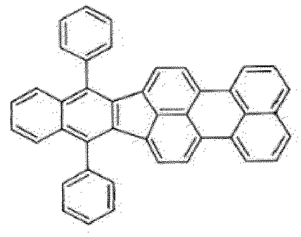

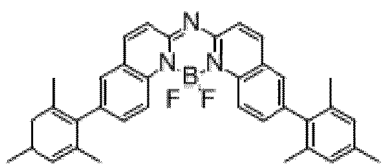

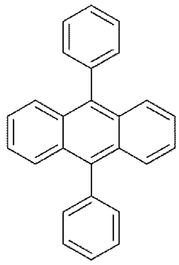
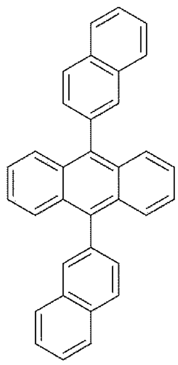
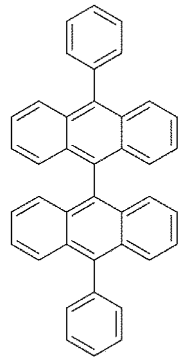
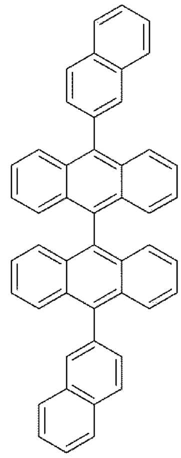

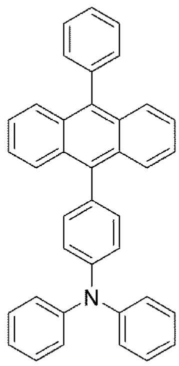
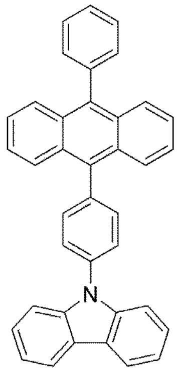
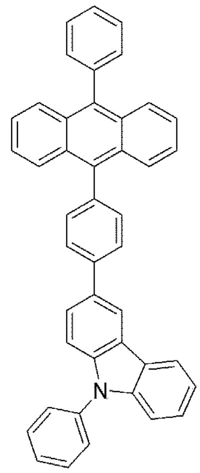
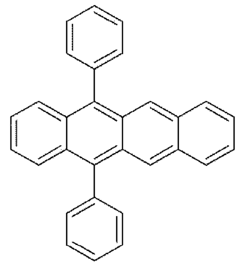

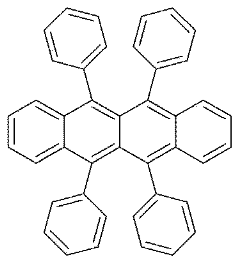
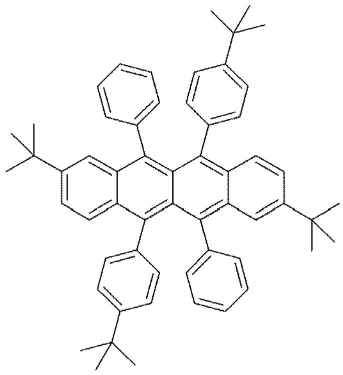
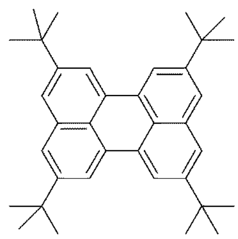

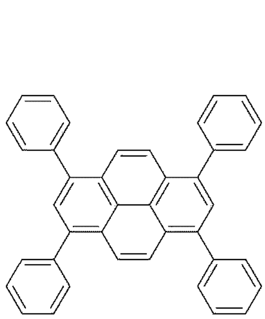
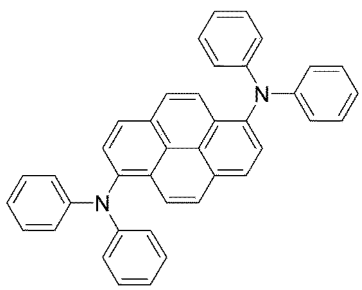
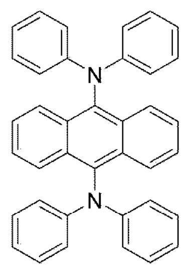

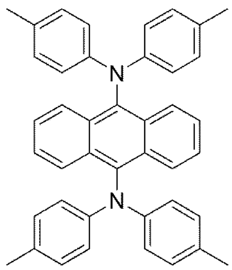
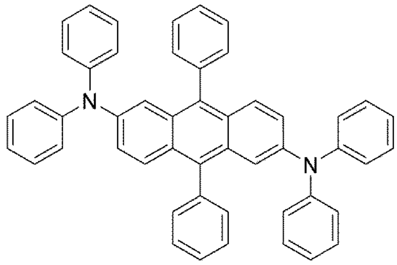
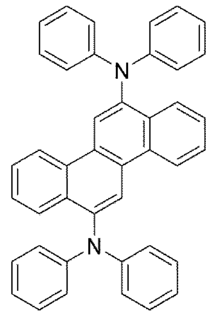

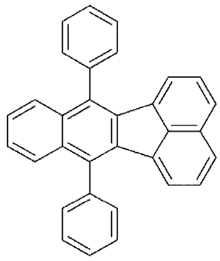
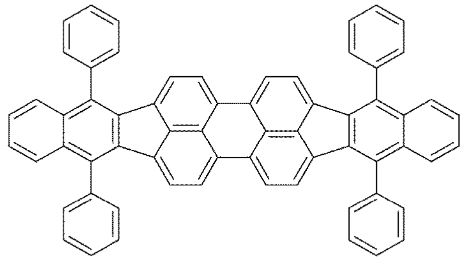
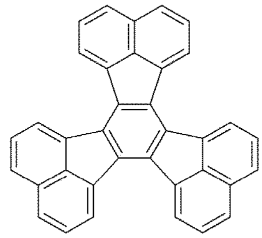

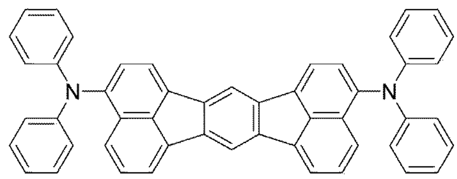
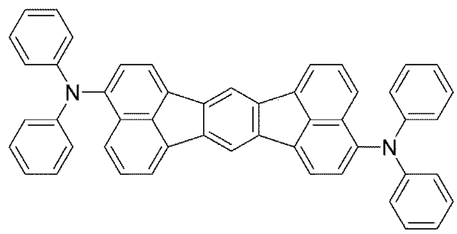

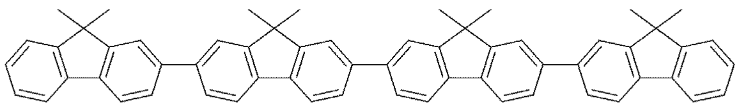

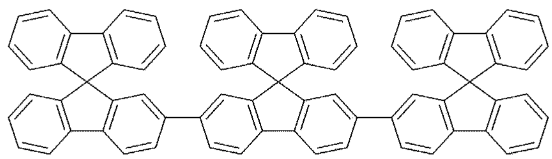

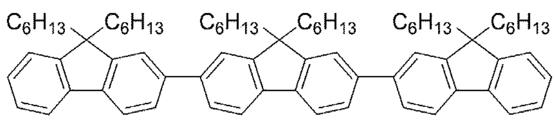

In addition, the compounds described in WO2015/022974, paragraphs 0220 to 0239 are also especially favorably employable as a light emitting material for use along with the assist dopant having a structure represented by the general formula (1).

Compounds represented by the following general formula (2) are further preferred light emitting materials.

General Formula (2)

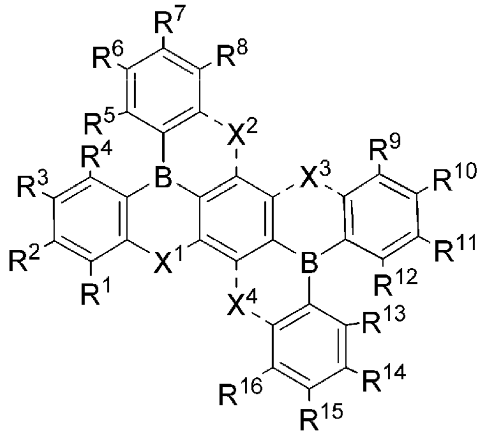

In the general formula (2), $R^1$ and $R^3$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group. $R^3$ and $R^4$, $R^4$ and $R^5$, $R^5$ and $R^6$, $R^6$ and $R^7$, $R^7$ and $R^8$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ is O or NR, and the remainder can be O or NR, or unlinked. When not linked, both ends each independently represent a hydrogen atom, a deuterium atom or a substituent. In the general formula (2), C-$R^1$, C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, when $X^2$ is O or NR, $R^7$ is an acceptor group, $R^6$ and $R^7$ bond to each other to form an acceptor group, or $R^7$ and $R^8$ bond to each other to form an acceptor group. In one aspect of the present invention, when $X^3$ is O or NR, $R^{10}$ is an acceptor group, $R^9$ and $R^{10}$ bond to each other to form an acceptor group, or $R^{10}$ and $R^{11}$ bond to each other to form an acceptor group. In one aspect of the present invention, when $X^4$ is O or NR, $R^{15}$ is an acceptor group, $R^{14}$ and $R^{15}$ bond to each other to form an acceptor group, or $R^{15}$ and $R^{16}$ bond to each other to form an acceptor group. In one aspect of the present invention, when $X^2$ is NR and when R is a substituted or unsubstituted phenyl group and forms a carbazole ring by directly bonding to the carbon atom to which $R^8$ bonds, at least one of the 3-position and the 6-position of the carbazole ring is substituted with an acceptor group. In one aspect of the present invention, when $X^3$ is NR and when R is a substituted or unsubstituted phenyl group and forms a carbazole ring by directly bonding to the carbon atom to which $R^9$ bonds, at least one of the 3-position and the 6-position of the carbazole ring is substituted with an acceptor group. In one aspect of the present invention, when $X^4$ is NR and when R is a substituted or unsubstituted phenyl group and forms a carbazole ring by directly bonding to the carbon atom to which $R^{16}$ bonds, at least one of the 3-position and the 6-position of the carbazole ring is substituted with an acceptor group. In one aspect of the present invention, when $X^1$ is NR and when R is a substituted or unsubstituted phenyl group and forms a carbazole ring by directly bonding to the carbon atom to which $R^1$ bonds, the 3-position of the carbazole ring is substituted with an acceptor group (here, the 3-position is on the phenyl group). One aspect of the present invention is a compound represented by the following general formula (2a).

General Formula (2a)

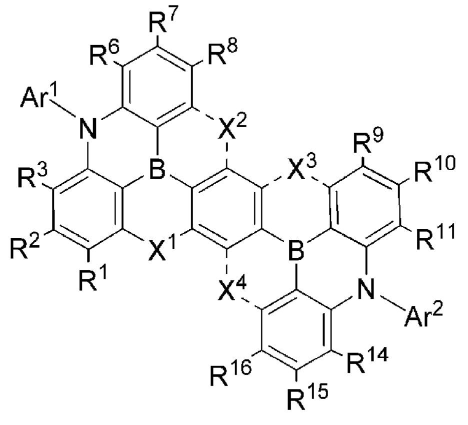

In the general formula (2a), $R^1$, $R^3$, $R^6$ to $R^{11}$, and $R^{14}$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group.

$R^6$ and $R^7$, $R^7$ and $R^8$, $R^9$ and $R^{10}$, $R^{10}$ and $R^{11}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ is O or NR, and the remainder can be O or NR, or unlinked. When not linked, both ends each independently represent a hydrogen atom, a deuterium atom or a substituent. $Ar^1$ and $Ar^2$ each independently represent a substituted or unsubstituted aryl group, or a substituted or unsubstituted heteroaryl group. In the general formula (2a), C-$R^1$, C-$R^3$, C-$R^6$, C-$R^7$, C-$R^8$, C-$R^9$, C-$R^{10}$, C-$R^{11}$, C-$R^{14}$, C-$R^{15}$, and C-$R^{16}$ can be substituted with N.

Compounds represented by the following general formula (3) are further preferred light emitting materials.

General Formula (3)

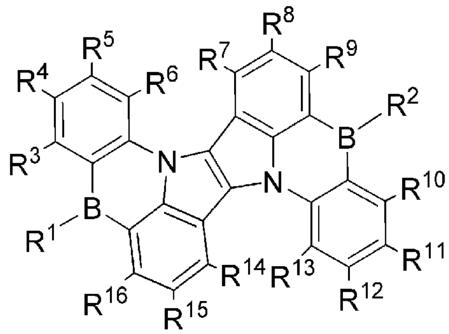

In the general formula (3), $R^1$ and $R^2$ each independently represent a substituted or unsubstituted alkyl group, a substituted or unsubstituted aryl group, or a substituted or unsubstituted heteroaryl group, and $R^3$ to $R^{16}$ each independently represent a hydrogen atom, a deuterium atom or a substituent. $R^1$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^2$, $R^2$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, $R^{15}$ and $R^{16}$, and $R^{16}$ and $R^1$ each can bond to each other to form a cyclic structure. In the general formula (3), C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, $R^1$ and $R^2$ are each independently a substituted or unsubstituted phenyl group optionally fused with any other ring. In one aspect of the present invention, $R^3$ and $R^{10}$ are each independently a substituted amino group. In one aspect of the present invention, at least one pair of $R^1$ and $R^3$, and $R^2$ and $R^{10}$ bonds to each other to form a cyclic structure. In one aspect of the present invention, the cyclic structure includes a benzazaborine ring.

Compounds represented by the following general formula (4) are further preferred light emitting materials.

General Formula (4)

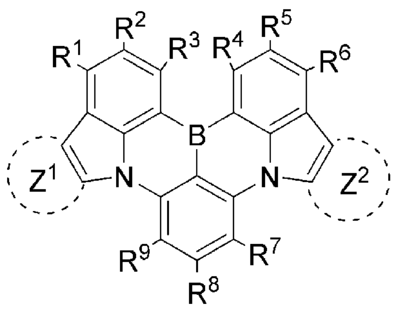

In the general formula (4), $Z^1$ and $Z^2$ each independently represent a substituted or unsubstituted aromatic ring, or a substituted or unsubstituted heteroaromatic ring, $R^1$ to $R^9$ each independently represent a hydrogen atom, a deuterium atom or a substituent. $R^1$ and $R^2$ , $R^2$ and $R^3$, $R^3$ and $R^4$, $R^4$ and $R^5$, $R^5$ and $R^6$, $R^7$ and $R^8$, and $R^8$ and $R^9$ each can bond to each other to form a cyclic structure. However, at least one of the ring formed by $Z^1$, $Z^2$, $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring of a substituted or unsubstituted benzofuran, a thiophene ring of a substituted or unsubstituted benzothiophene, or a pyrrole ring of a substituted or unsubstituted indole, and at least one of $R^1$ to $R^9$ is a substituted or unsubstituted aryl group or an acceptor group, or at least one of $Z^1$ and $Z^2$ is a ring having an aryl group or an acceptor group as a substituent. Of the benzene ring skeleton-constituting carbon atoms to constitute the

benzofuran ring, the benzothiophene ring, and the indole ring, a substitutable carbon atom can be substituted with a nitrogen atom. In the general formula (4), C-$R^1$, C-$R^2$, C-$R^3$, C-$R^4$, C-$R^5$, C-$R^6$, C-$R^7$, C-$R^8$, and C-$R^9$ can be substituted with N.

In one aspect of the present invention, $Z^1$ and $Z^2$ are each independently a substituted or unsubstituted non-fused benzene ring, a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^1$ to $R^9$ are each independently substituted or unsubstituted aryl group or an acceptor group, or one or more rings selected from the group consisting of the ring formed by $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^8$ is a substituted or unsubstituted aryl group, or an acceptor group. One aspect of the present invention contains two or more rings selected from the group consisting of the benzofuran ring, the benzothiophene ring, and the indole ring.

Further more preferred light emitting materials include compounds having a ring-fused structure A, in which the carbon-carbon bond a in the following structure $\alpha$ is fused with a furan ring constituting a substituted or unsubstituted benzofuran ring, a thiophene ring constituting a substituted or unsubstituted benzothiophene ring, or a pyrrole ring constituting a substituted or unsubstituted indole ring, or the carbon-carbon bond b is fused with a benzene ring constituting a substituted or unsubstituted dibenzofuran ring, a benzene ring constituting a substituted or unsubstituted dibenzothiophene ring, a benzene ring constituting a substituted or unsubstituted carbazole ring, or a benzene ring constituting a substituted or unsubstituted dibenzodioxane ring (the hydrogen atom in the structure can be substituted with a deuterium atom or a substituent).

Structure α

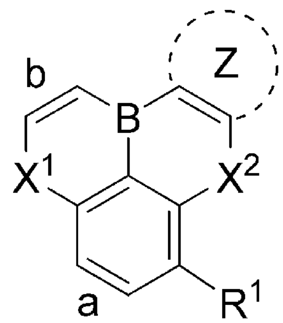

In the structure $\alpha$, $X^1$ and $X^2$ each independently represent a nitrogen atom to which a substituted or unsubstituted aryl group or a substituted or unsubstituted aryl group bonds, or an oxygen atom, Z represents a substituted or unsubstituted aromatic ring, or a substituted or unsubstituted heteroaromatic ring, $R^1$ represents a hydrogen atom, a deuterium atom or a substituent, and Z and $X^2$ can bond to each other to form a cyclic structure.

In the ring-fused structure A, the structure fused to b and $X^1$, the structure fused to b and Z, and Z and $X^2$ each can bond to each other to form a cyclic structure.

Compounds represented by the following general formula (5) are further preferred light emitting materials.

General Formula (5)

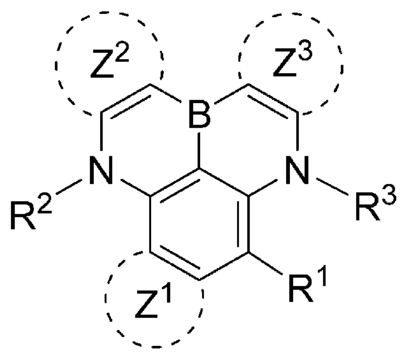

In the general formula (5), $Z^1$ represents a furan ring fused with a substituted or unsubstituted benzene ring, a

thiophene ring fused with a substituted or unsubstituted benzene ring, or an N-substituted pyrrole ring fused with a substituted or unsubstituted benzene ring, $Z^2$ and $Z^3$ each independently represent a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^2$, $Z^2$ and $Z^3$, and $Z^3$ and $R^3$ each can bond to each other to form a cyclic structure. However, at least one pair of $R^2$ and $Z^2$, $Z^2$ and $Z^3$, and $Z^3$ and $R^3$ bonds to each other to form a cyclic structure.

Compounds represented by the following general formula (6) are further preferred light emitting materials.

General Formula (6)

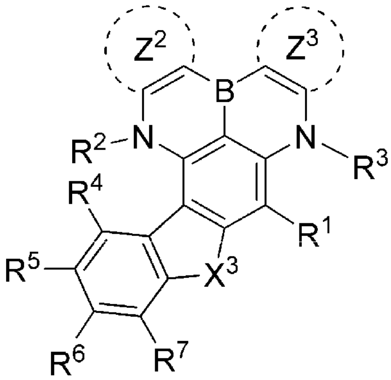

In the general formula (6), $X^3$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^4$ to $R^7$ each represent a hydrogen atom, a deuterium atom or a substituent, $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^4$ and $R^5$, $R^5$ and $R^6$, and $R^6$ and $R^7$ each can bond to each other to form a cyclic structure. However, at least one pair of $R^2$ and $Z^2$, $Z^2$ and $Z^3$, and $Z^3$ and $R^3$ bonds to each other to form a cyclic structure.

Compounds represented by the following general formula (7) are further preferred light emitting materials.

General Formula (7)

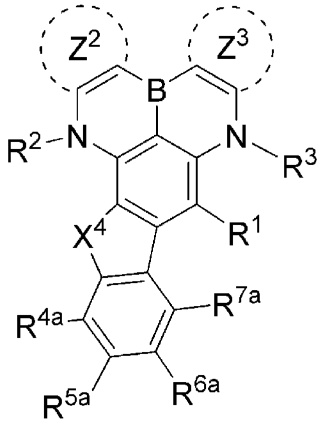

In the general formula (7), $X^4$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^{4a}$ to $R^{7a}$ each represent a hydrogen atom, a deuterium atom or a substituent, and $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^{4a}$ and $R^{5a}$, $R^{5a}$ and $R^{6a}$, $R^{6a}$ and $R^{7a}$, and $R^{7a}$ and $R^1$ each can bond to each other to form a cyclic structure. However, at least one pair of $R^2$ and $Z^2$, $Z^2$ and $Z^3$, and $Z^3$ and $R^3$ bonds to each other to form a cyclic structure.

Compounds represented by the following general formula (8) are further preferred light emitting materials.

General Formula (8)

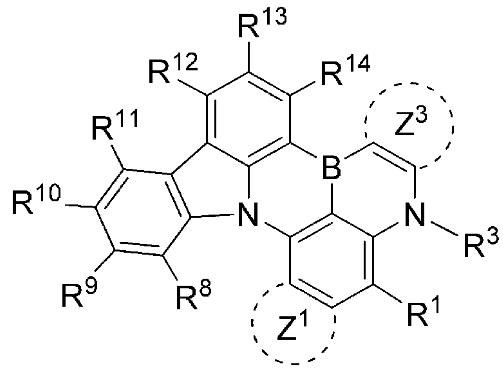

**In** the general formula (8), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^8$ to $R^{14}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $R^8$ and $R^9$ , $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $Z^3$, and $Z^3$ and $R^3$ each can bond to each other to form a cyclic structure.

Compounds represented by the following general formula (9) are further preferred light emitting materials.

General Formula (9)

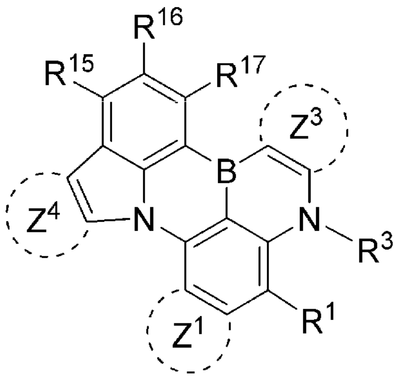

**In** the general formula (9), $Z^1$ and $Z^4$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^{15}$ to $R^{17}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $Z^4$ and $R^{15}$, $R^{15}$ and $R^{16}$, $R^{16}$ and $R^{17}$, $R^{17}$ and $Z^3$, and $Z^3$ and $R^3$ each can bond to each other to form a cyclic structure.

Compounds represented by the following general formula (10) are further preferred light emitting materials.

General Formula (10)

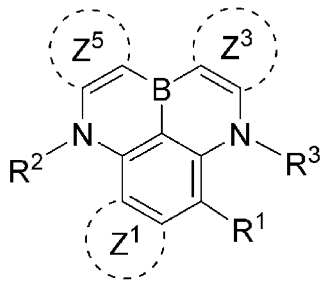

In the general formula (10), $Z^1$ and $Z^5$ each independently represent a furan ring fused with a substituted or

unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^5$, $Z^5$ and $Z^3$, and $Z^3$ and $R^3$ each can bond to each other to form a cyclic structure. However, at least one pair of $R^2$ and $Z^2$, $Z^2$ and $Z^3$, and $Z^3$ and $R^3$ bonds to each other to form a cyclic structure.

Compounds represented by the following general formula (11) are further preferred light emitting materials.

General Formula (11)

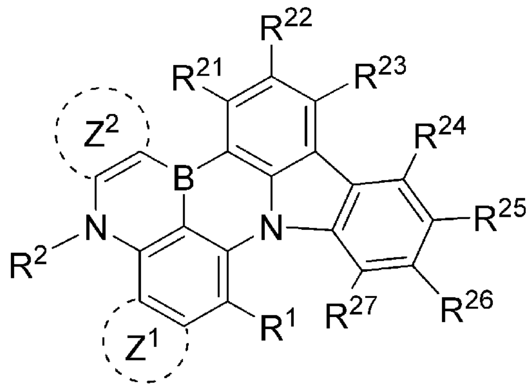

In the general formula (11), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{21}$ to $R^{27}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{21}$, $R^{21}$ and $R^{22}$, $R^{22}$ and $R^{23}$, $R^{23}$ and $R^{24}$, $R^{24}$ and $R^{25}$, $R^{25}$ and $R^{26}$, and $R^{26}$ and $R^{27}$ each can bond to each other to form a cyclic structure.

Compounds represented by the following general formula (12) are further preferred light emitting materials.

General Formula (12)

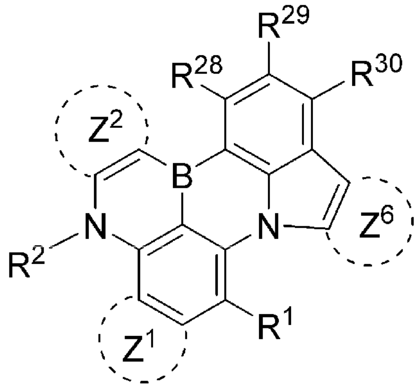

In the general formula (12), $Z^1$ and $Z^6$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{28}$ to $R^{30}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{28}$, $R^{28}$ and $R^{29}$, $R^{29}$ and $R^{30}$, and $R^{30}$ and $Z^6$ each can bond to each other to form a cyclic structure.

Compounds represented by the following general formula (13) are further preferred light emitting materials.

General Formula (13)

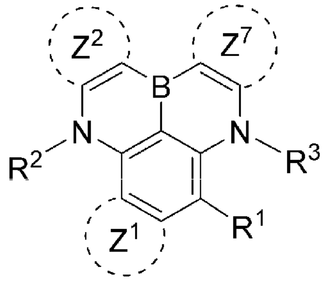

In the general formula (13), $Z^1$ and $Z^7$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group; $R^1$ and $Z^1$, $R^2$ and $Z^2$, $Z^2$ and $Z^7$. and $Z^7$ and $R^3$ each can bond to each other to form a cyclic structure; however, at least one pair of $R^2$ and $Z^2$, $Z^2$ and $Z^7$, and $Z^7$ and $R^3$ bonds to each other to form a cyclic structure.

Compounds represented by the following general formula (14) are further preferred light emitting materials.

General Formula (14)

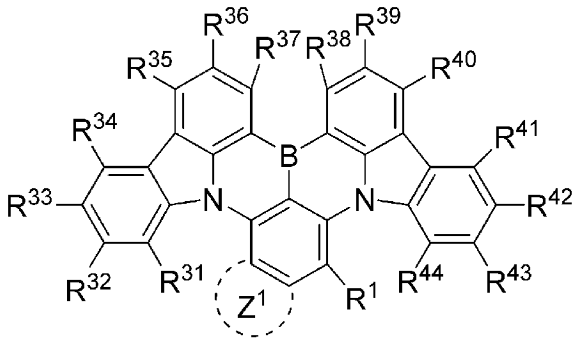

In the general formula (14), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{31}$ to $R^{44}$ each independently represent a substituted or unsubstituted aryl group, or a substituted or unsubstituted heteroaryl group; $R^1$ and $Z^1$, $R^{31}$ and $R^{32}$, $R^{32}$ and $R^{33}$, $R^{33}$ and $R^{34}$, $R^{34}$ and $R^{35}$, $R^{35}$ and $R^{36}$, $R^{36}$ and $R^{37}$, $R^{37}$ and $R^{38}$, $R^{38}$ and $R^{39}$, $R^{39}$ and $R^{40}$, $R^{40}$ and $R^{41}$, $R^{41}$ and $R^{42}$, $R^{42}$ and $R^{43}$, and $R^{43}$ and $R^{44}$ each can bond to each other to form a cyclic structure.

Compounds represented by the following general formula (15) are further preferred light emitting materials.

General Formula (15)

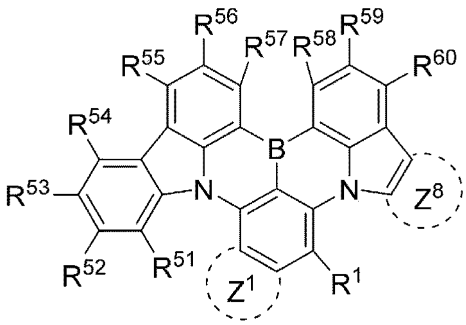

In the general formula (15), $Z^1$ and $Z^8$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{51}$ to $R^{60}$ each independently represent a hydrogen atom, a deuterium atom, or a substituent. $R^1$ and $Z^1$, $R^{51}$ and $R^{52}$, $R^{52}$ and $R^{53}$, $R^{53}$ and $R^{54}$, $R^{54}$ and $R^{55}$, $R^{55}$

and $R^{56}$, $R^{56}$ and $R^{57}$, $R^{57}$ and $R^{58}$, $R^{58}$ and $R^{59}$, $R^{59}$ and $R^{60}$, and $R^{60}$ and $Z^8$ each can bond to each other to form a cyclic structure.

Compounds represented by the following general formula (16) are further preferred light emitting materials.

General Formula (16)

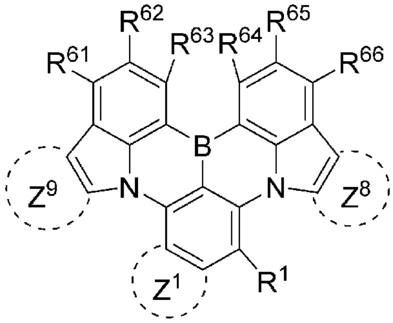

In the general formula (16), $Z^1$, $Z^8$ and $Z^9$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, and $R^1$ and $R^{61}$ to $R^{66}$ each independently represent a hydrogen atom, a deuterium atom, or a substituent. $R^1$ and $Z^1$, $Z^9$ and $R^{61}$, $R^{61}$ and $R^{62}$, $R^{62}$ and $R^{63}$, $R^{63}$ and $R^{64}$, $R^{64}$ and $R^{65}$, $R^{65}$ and $R^{66}$, and $R^{66}$ and $Z^8$ each can bond to each other to form a cyclic structure.

Compounds represented by the following general formula (17) are further preferred light emitting materials.

General Formula (17)

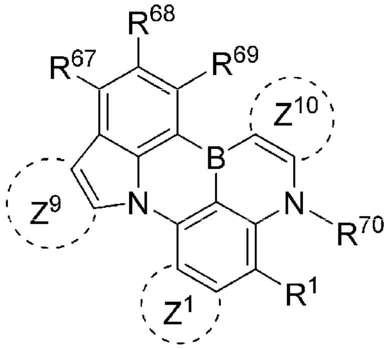

In the general formula (17), $Z^1$, $Z^9$ and $Z^{10}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{67}$ to $R^{69}$ each independently represent a hydrogen atom, a deuterium atom, or a substituent, and $R^{70}$ represents a substituted or unsubstituted aryl group, or a substituted or unsubstituted heteroaryl group. $R^1$ and $Z^1$, $Z^9$ and $R^{67}$, $R^{67}$ and $R^{68}$, $R^{68}$ and $R^{69}$, $R^{69}$ and $Z^{10}$, and $Z^{10}$ and $R^{70}$ each can bond to each other to form a cyclic structure.

Compounds represented by the following general formula (18) are further preferred light emitting materials.

General Formula (18)

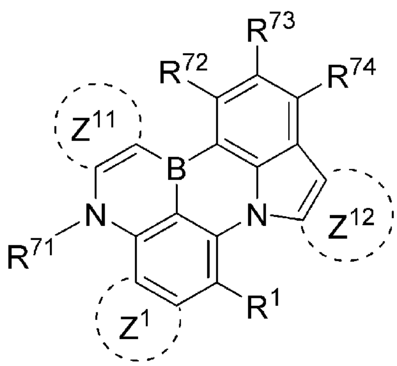

In the general formula (18), $Z^1$, $Z^{11}$ and $Z^{12}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{72}$ to $R^{74}$ each independently represent a hydrogen atom, a deuterium atom, or a substituent, and $R^{71}$ represents a substituted or unsubstituted aryl group, or a substituted or unsubstituted heteroaryl group. $R^1$ and $Z^1$, $R^{71}$ and $Z^{11}$, $Z^{11}$ and $R^{72}$, $R^{72}$ and $R^{73}$, $R^{73}$ and $Z^{74}$, and $R^{74}$ and $Z^{12}$ each can bond to each other to form a cyclic structure.

Compounds represented by the following general formula (19) are further preferred light emitting materials.

General Formula (19)

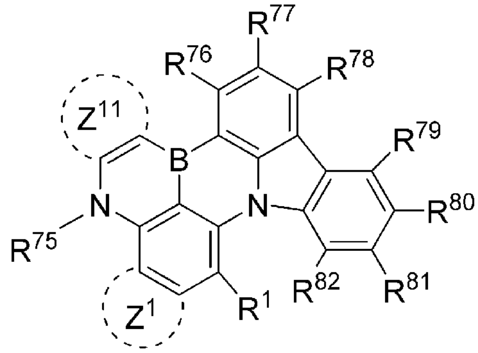

**In** the general formula (19), $Z^1$ and $Z^{11}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{76}$ to $R^{82}$ each independently represent a substituted or unsubstituted aryl group, or a substituted or unsubstituted heteroaryl group, and $R^{75}$ represents a substituted or unsubstituted aryl group, or a substituted or unsubstituted heteroaryl group. $R^1$ and $Z^1$, $R^{75}$ and $Z^{11}$, $Z^{11}$ and $R^{76}$, $R^{76}$ and $R^{77}$, $R^{77}$ and $R^{78}$, $R^{78}$ and $R^{79}$, $R^{79}$ and $R^{80}$, $R^{80}$ and $R^{81}$, and $R^{81}$ and $R^{82}$ each can bond to each other to form a cyclic structure.

Compounds represented by the following general formula (20) are further preferred light emitting materials.

General Formula (20)

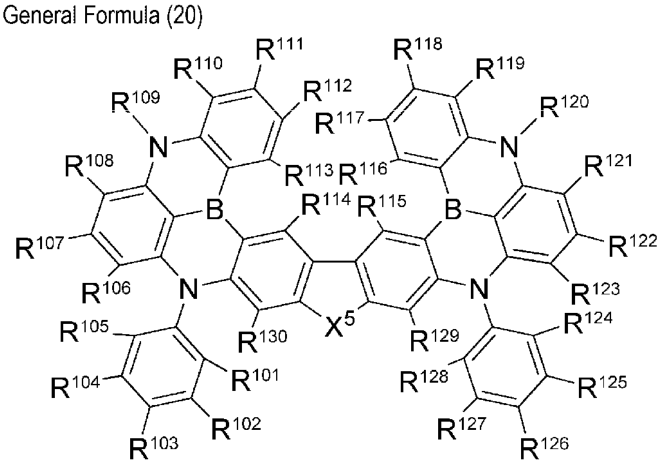

In the general formula (20), $X^5$ represents an oxygen atom, a sulfur atom, or a nitrogen atom to which a substituted or unsubstituted aryl group or a substituted or unsubstituted heteroaryl group bonds, $R^{101}$ to $R^{130}$ each independently represents a hydrogen atom, a deuterium atom or a substituent, and $R^{101}$ and $R^{102}$, $R^{102}$ and $R^{103}$, $R^{103}$ and $R^{104}$, $R^{104}$ and $R^{105}$, $R^{105}$ and $R^{106}$, $R^{106}$ and $R^{107}$, $R^{107}$ and $R^{108}$, $R^{108}$ and $R^{109}$, $R^{109}$ and $R^{110}$, $R^{110}$ and $R^{111}$, $R^{111}$ and $R^{112}$, $R^{112}$ and $R^{113}$, $R^{113}$ and $R^{114}$, $R^{114}$ and $R^{115}$, $R^{115}$ and $R^{116}$, $R^{116}$ and $R^{117}$, $R^{117}$ and $R^{118}$, $R^{118}$ and $R^{119}$, $R^{119}$ and $R^{120}$, $R^{120}$ and $R^{121}$, $R^{121}$ and $R^{122}$, $R^{122}$ and $R^{123}$, $R^{123}$ and $R^{124}$, $R^{124}$ and $R^{125}$, $R^{125}$ and $R^{126}$, $R^{126}$ and $R^{127}$, $R^{127}$ and $R^{128}$, $R^{128}$ and $R^{129}$, $R^{129}$ and $R^{130}$, and $R^{130}$ and $R^{101}$ each can bond to each other to form a cyclic structure.

Compounds represented by the following general formula (21) are further preferred light emitting materials.

General Formula (21)

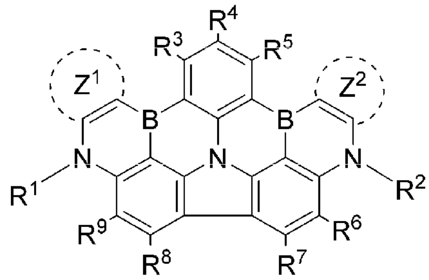

In the general formula (21), $R^1$ and $R^2$ each independently represent a substituted or unsubstituted alkyl group, a substituted or unsubstituted aryl group, or a substituted or unsubstituted heteroaryl group, $Z^1$ and $Z^2$ each independently represent a substituted or unsubstituted aromatic ring, or a substituted or unsubstituted heteroaromatic ring, and $R^3$ to $R^9$ each independently represent a hydrogen atom, a deuterium atom or a substituent. However, at least one of $R^1$, $R^2$, $Z^1$ and $Z^2$ includes a substituted or unsubstituted benzofuran ring, a substituted or unsubstituted benzothiophene ring, or a substituted or unsubstituted indole ring. $R^1$ and $Z^1$, $Z^1$ and $R^3$, $R^3$ and $R^4$, $R^4$ and $R^5$, $R^5$ and $Z^2$, $Z^2$ and $R^2$, $R^2$ and $R^6$, $R^6$ and $R^7$, $R^7$ and $R^8$, $R^8$ and $R^9$, and $R^9$ and $R^1$ each can bond to each other to form a cyclic structure. Of the benzene ring skeleton-constituting carbon atoms to constitute the benzofuran ring, the benzothiophene ring, and the indole ring, a substitutable carbon atom can be substituted with a nitrogen atom. In the general formula (21), C-$R^3$, C-$R^4$, C-$R^5$, C-$R^6$, C-$R^7$, C-$R^8$, and C-$R^9$ can be substituted with N.

In one aspect of the present invention, $R^1$ and $R^2$ are each independently a substituted or unsubstituted alkyl group, a substituted or unsubstituted phenyl group, or a group containing one or more ring structures selected from the group consisting of a substituted or unsubstituted benzofuran ring, a substituted or unsubstituted benzothiophene ring and a substituted or unsubstituted indole ring. In one aspect of the present invention, $Z^1$ and $Z^2$ are each independently a substituted or unsubstituted nonfused benzene ring, a furan ring fused with a substituted or unsubstituted benzene ring, a thiophene ring fused with a substituted or unsubstituted benzene ring, a pyrrole ring fused with a substituted or unsubstituted benzene ring, a benzene ring fused with a substituted or unsubstituted benzofuran ring, a benzene ring fused with a substituted or unsubstituted benzothiophene ring, or a benzene ring fused with a substituted or unsubstituted indole ring. In one aspect of the present invention, $R^1$ and $Z^1$ bond to each other to form a cyclic structure. In one aspect of the present invention, $R^1$ and $Z^1$ bond to each other to form a pyrrole ring.

Compounds represented by the following general formula (22) are further preferred light emitting materials.

General Formula (22)

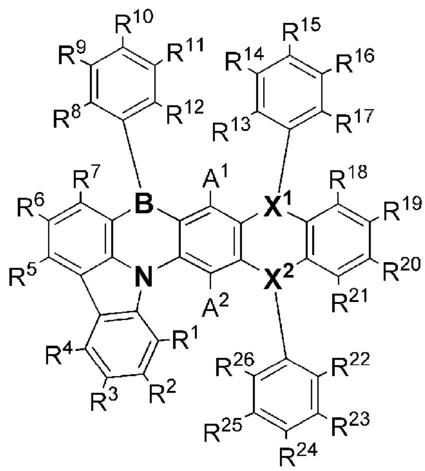

In the general formula (22), one of $X^1$ and $X^2$ is a nitrogen atom, and the other is a boron atom. $R^1$ to $R^{26}$, $A^1$ and $A^2$ each independently represent a hydrogen atom, a deuterium atom, or a substituent. $R^1$ and $R^2$, $R^2$ and $R^3$, $R^3$ and $R^4$, $R^4$ and $R^5$, $R^5$ and $R^6$ , $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ each can bond to each other to form a cyclic structure. However, when $X^1$ is a nitrogen atom, $R^{17}$ and $R^{18}$ bond to each other to be a single bond to form a pyrrole ring, and when $X^2$ is a nitrogen atom, $R^{21}$ and $R^{22}$ bond to each

other to be a single bond to form a pyrrole ring. However, in the case where $X^1$ is a nitrogen atom, and where $R^7$ and $R^8$ and $R^{21}$ and $R^{22}$ each bond to each other via a nitrogen atom to form a 6-membered ring, and $R^{17}$ and $R^{18}$ bond to each other to form a single bond, at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ bond to each other to form an aromatic ring or a heteroaromatic ring.

In one aspect of the present invention, at least one of $R^3$ and $R^6$ is a substituent. In one aspect of the present invention, both $R^3$ and $R^6$ are substituents. In one aspect of the present invention, the substituent represented by $R^3$ and $R^6$ is one group selected from the group consisting of an alkyl group and an aryl group, or a group obtained by combining two or more of the groups. In one aspect of the present invention, both $R^8$ and $R^{12}$ are substituents. In one aspect of the present invention, the compounds are represented by the following general formula (1a).

General Formula (22a)

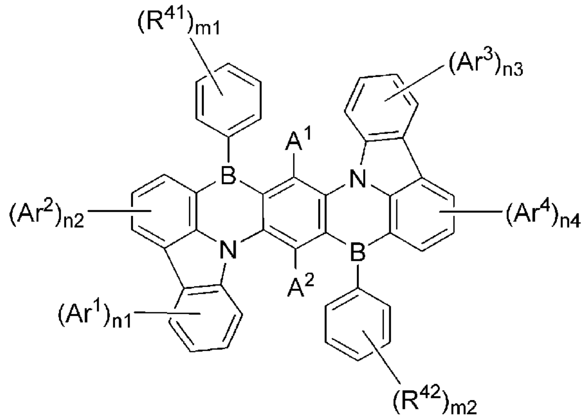

In the general formula (22a), $Ar^1$ to $Ar^4$ each independently represent a substituted or unsubstituted aryl group, or a substituted or unsubstituted heteroaryl group. Each of $R^{41}$ and $R^{42}$ independently represents a substituted or unsubstituted alkyl group. m1 and m2 each independently represent an integer of 0 to 5, n1 and n3 each independently represent an integer of 0 to 4, and n2 and n4 each independently represent an integer of 0 to 3. Each of $A^1$ and $A^2$ independently represents a hydrogen atom, a deuterium atom, or a substituent.

In one aspect of the present invention, $A^1$ and $A^2$ each are independently a group having a Hammett' σp value of more than 0.2. In one aspect of the present invention, both $A^1$ and $A^2$ are cyano groups. In one aspect of the present invention, both $A^1$ and $A^2$ are halogen atoms. One aspect of the present invention has a rotationally symmetrical structure.

Preferred specific examples of compounds having the above-mentioned ringfused structure A, and compounds represented by any of the general formulae (5) to (22) are shown below.

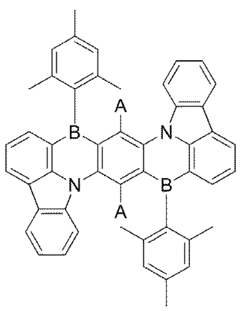

1a : A=A1

1b : A=A2
1c : A=A3
1d : A=A4
1e : A=A7
1f : A=A10
1g : A=A11
1h : A=A16
1i : A=A35

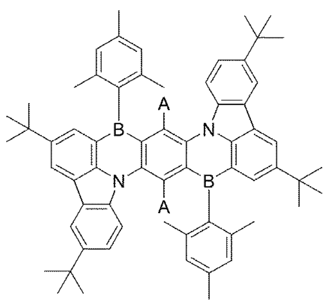

2a : A=A1

2b : A=A2
2c : A=A3
2d : A=A4
2e : A=A7
2f : A=A10
2g : A=A11
2h : A=A16
2i : A=A35

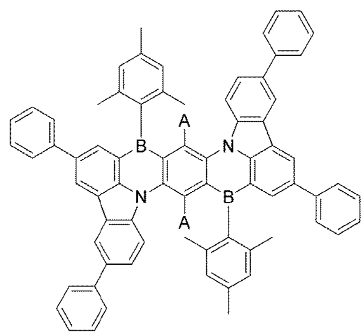

3a : A=A1

3b : A=A2
3c : A=A3
3d : A=A4
3e : A=A7
3f : A=A10
3g : A=A11
3h : A=A16
3i : A=A35

(continued)

1j : A=A39
1k : A=A40
1l : A=A41
1m : A=A42

2j : A=A39
2k : A=A40
2l : A=A41
2m : A=A42

3j : A=A39
3k : A=A40
3l : A=A41
3m : A=A42

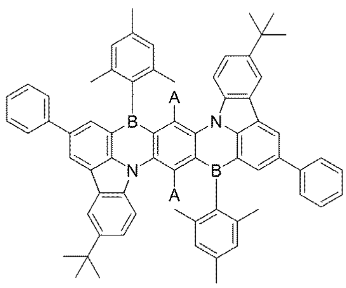

4a : A=A1
4b : A=A2
4c : A=A3
4d : A=A4
4e : A=A7
4f : A=A10
4g : A=A11
4h : A=A16
4i : A=A35
4j : A=A39
4k : A=A40
41:A=A41
4m:A=A42

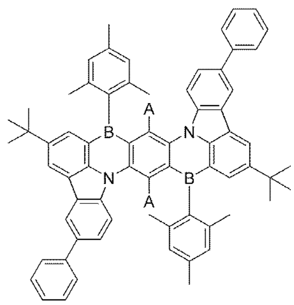

5a : A=A1
5b : A=A2
5c : A=A3
5d : A=A4
5e : A=A7
5f : A=A10
5g : A=A11
5h : A=A16
5i : A=A35
5j : A=A39
5k : A=A40
5l : A=A41
5m: A=A42

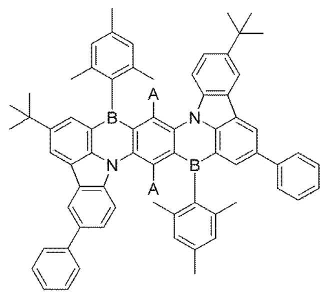

6a : A=A1
6b : A=A2
6c : A=A3
6d : A=A4
6e : A=A7
6f : A=A10
6g : A=A11
6h : A=A16
6i : A=A35
6j : A=A39
6k : A=A40
6l : A=A41
6m : A=A42

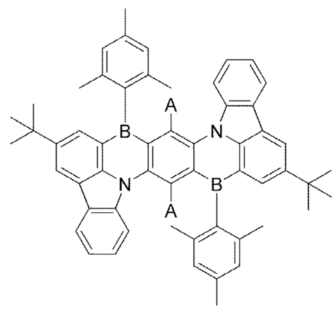

7a : A=A1
7b : A=A2
7c : A=A3
7d : A=A4
7e : A=A7
7f : A=A10
7g : A=A11
7h : A=A16
7i : A=A35
7j : A=A39
7k : A=A40
7l : A=A41
7m : A=A42

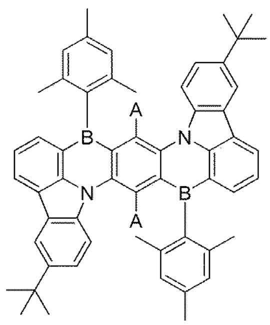

8a : A=A1
8b:A=A2
8c:A=A3
8d:A=A4
8e:A=A7
8f : A=A10
8g : A=A11
8h : A=A16
8i : A=A35
8j : A=A39
8k : A=A40
8l : A=A41
8m : A=A42

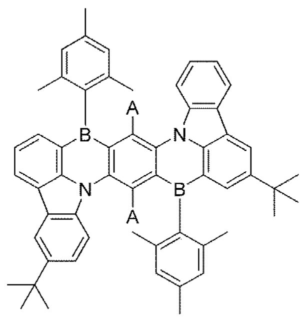

9a: A=A1
9b : A=A2
9c : A=A3
9d : A=A4
9e : A=A7
9f : A=A10
9g : A=A11
9h : A=A16
9i : A=A35
9j : A=A39
9k : A=A40
9l : A=A41
9m : A=A42

(continued)

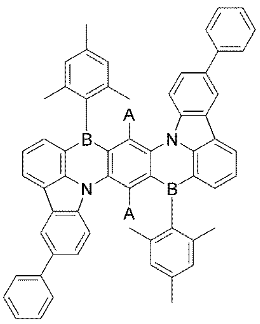

10a : A=A1
10b : A=A2
10c : A=A3
10d : A=A4
10e : A=A7
10f : A=A10
10g : A=A11
10h : A=A16
10i : A=A35
10j : A=A39
10k : A=A40
10l : A=A41
10m : A=A42

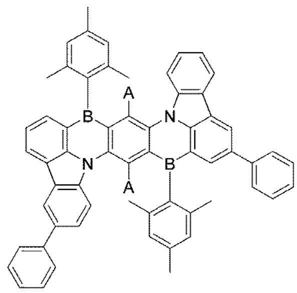

11a : A=A1
11b : A=A2
11c : A=A3
11d : A=A4
11e : A=A7
11f : A=A10
11g : A=A11
11h : A=A16
11i : A=A35
11j : A=A39
11k : A=A40
11l : A=A41
11m : A=A42

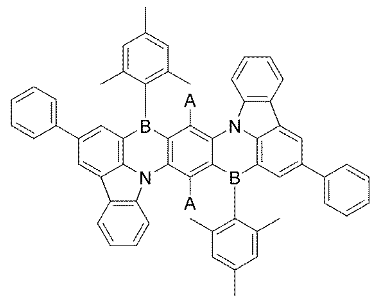

12a : A=A1
12b : A=A2
12c : A=A3
12d : A=A4
12e : A=A7
12f : A=A10
12g : A=A11
12h : A=A16
12i : A=A35
12j : A=A39
12k : A=A40
12l : A=A41
12m: A=A42

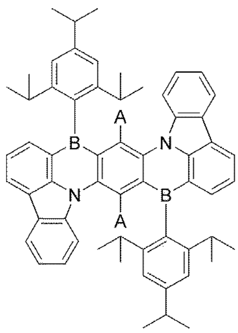

13a : A=A1
13b : A=A2
13c : A=A3
13d : A=A4
13e : A=A7
13f : A=A10
13g : A=A11
13h : A=A16
13i : A=A35
13j : A=A39
13k : A=A40
13l : A=A41
13m : A=A42

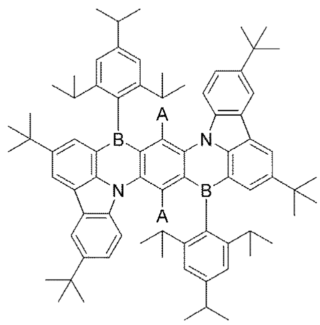

14a : A=A1
14b : A=A2
14c : A=A3
14d : A=A4
14e : A=A7
14f : A=A10
14g:A=A11
14h : A=A16
14i : A=A35
14j : A=A39
14k : A=A40
14l : A=A41
14m : A=A42

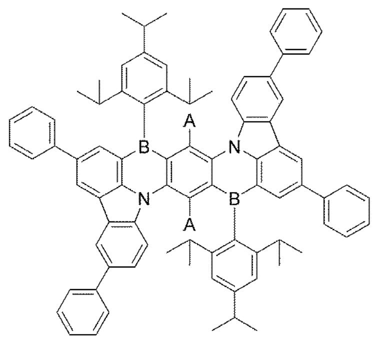

15a : A=A1
15b : A=A2
15c : A=A3
15d : A=A4
15e : A=A7
15f : A=A10
15g : A=A11
15h : A=A16
15i : A=A35
15j : A=A39
15k : A=A40
15l : A=A41
15m : A=A42

(continued)

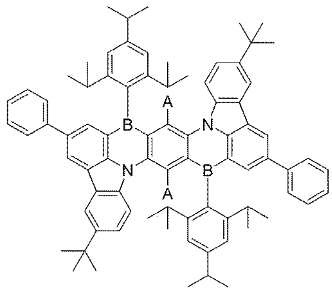

16a : A=A1
16b : A=A2
16c : A=A3
16d : A=A4
16e : A=A7
16f : A=A10
16g : A=A11
16h : A=A16
16i : A=A35
16j : A=A39
16k : A=A40
16l : A=A41
16m : A=A42

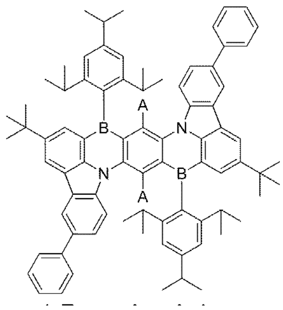

17a : A=A1
17b : A=A2
17c : A=A3
17d : A=A4
17e : A=A7
17f : A=A10
17g : A=A11
17h : A=A16
17i : A=A35
17j:A=A39
17k:A=A40
17l : A=A41
17m : A=A42

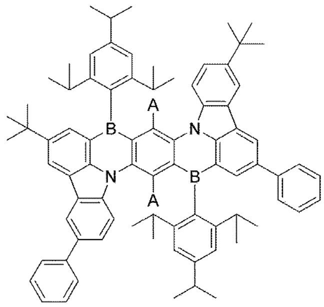

18a : A=A1
18b : A=A2
18c : A=A3
18d : A=A4
18e : A=A7
18f : A=A10
18g : A=A11
18h : A=A16
18i : A=A35
18j : A=A39
18k : A=A40
18l : A=A41
18m : A=A42

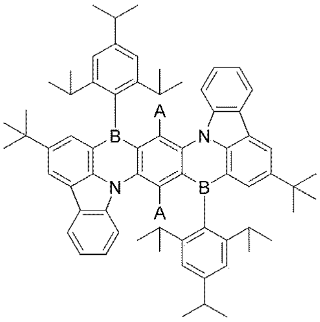

19a : A=A1
19b : A=A2
19c : A=A3
19d : A=A4
19e : A=A7
19f : A=A10
19g : A=A11
19h : A=A16
19i : A=A35
19j : A=A39
19k : A=A40
19l : A=A41
19m : A=A42

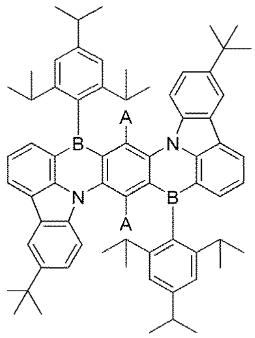

20a : A=A1
20b : A=A2
20c : A=A3
20d : A=A4
20e : A=A7
20f : A=A10
20g : A=A11
20h : A=A16
20i : A=A35
20j : A=A39
20k : A=A40
20l : A=A41
20m : A=A42

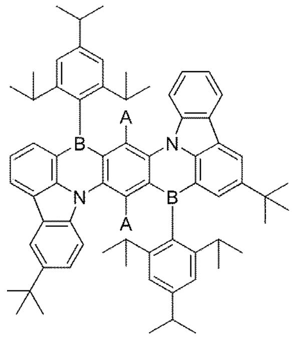

21a : A=A1
21b : A=A2
21c : A=A3
21d : A=A4
21e : A=A7
21f : A=A10
21g : A=A11
21h : A=A16
21i : A=A35
21j : A=A39
21k : A=A40
21l : A=A41
21m : A=A42

(continued)

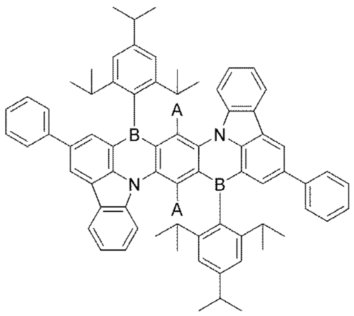

22a : A=A1
22b : A=A2
22c : A=A3
22d : A=A4
22e : A=A7
22f : A=A10
22g : A=A11
22h:A=A16
22i : A=A35
22j : A=A39
22k : A=A40
22l : A=A41
22m : A=A42

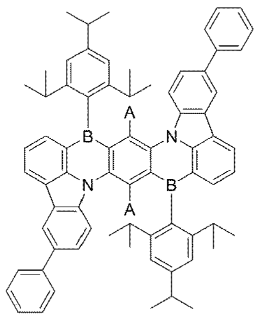

23a : A=A1
23b : A=A2
23c : A=A3
23d : A=A4
23e:A=A7
23f : A=A10
23g : A=A11
23h : A=A16
23i : A=A35
23j : A=A39
23k : A=A40
23l : A=A41
23m : A=A42

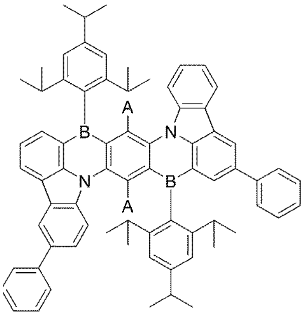

24a : A=A1
24b : A=A2
24c : A=A3
24d:A=A4
24e : A=A7
24f : A=A10
24g : A=A11
24h:A=A16
24i : A=A35
24j : A=A39
24k : A=A40
24l : A=A41
24m : A=A42

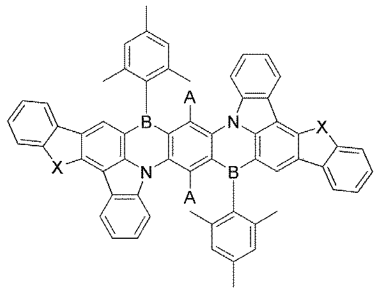

25a : A=A1
25b : A=A2
25c : A=A3
25d : A=A4
25e : A=A7
25f : A=A10
25g : A=A11
25h : A=A16
25i : A=A35
25j : A=A39 25k : A=A40
25l : A=A41
25m : A=A42

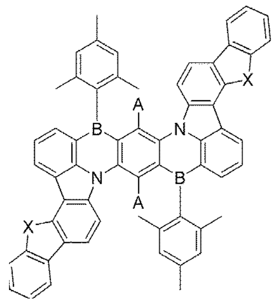

26a : A=A1
26b : A=A2
26c : A=A3
26d : A=A4
26e : A=A7
26f : A=A10
26g : A=A11
26h : A=A16
26i : A=A35
26j : A=A39 26k : A=A40
26l : A=A41
26m : A=A42

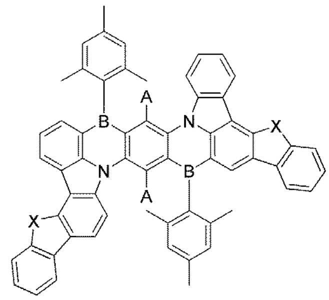

27a : A=A1
27b : A=A2
27c : A=A3
27d : A=A4
27e : A=A7
27f : A=A10
27g : A=A11
27h : A=A16
27i : A=A35
27j : A=A39 27k : A=A40
27l : A=A41
27m : A=A42

(continued)

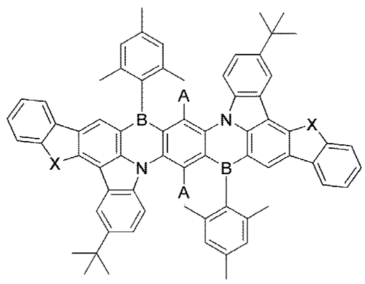

28a : A=A1
28b : A=A2
28c : A=A3
28d : A=A4
28e : A=A7
28f : A=A10
28g : A=A11
28h: A=A16
28i : A=A35
28j : A=A39
28k : A=A40
28l : A=A41
28m : A=A42

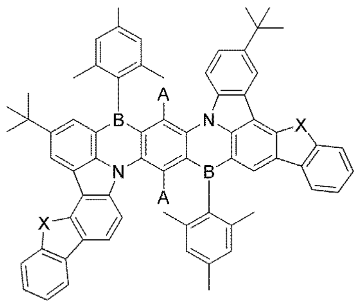

29a : A=A1
29b : A=A2
29c : A=A3
29d : A=A4
29e : A=A7
29f : A=A10
29g : A=A11
29h : A=A16
29i : A=A35
29j : A=A39
29k : A=A40
29l : A=A41
29m: A=A42

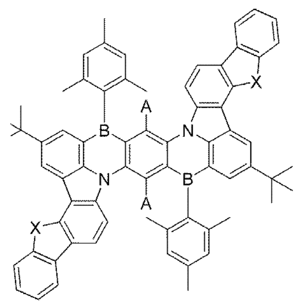

30a : A=A1
30b : A=A2
30c : A=A3
30d : A=A4
30e : A=A7
30f : A=A10
30g : A=A11
30h : A=A16
30i : A=A35
30j : A=A39
30k : A=A40
30l : A=A41
30m : A=A42

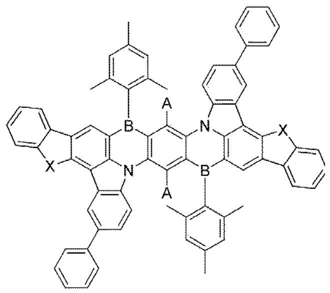

31a : A=A1
31b : A=A2
31c : A=A3
31d : A=A4
31e : A=A7
31f : A=A10
31g : A=A11
31h : A=A16
31i : A=A35
31j : A=A39 31k : A=A40
31l: A=A41
31m : A=A42

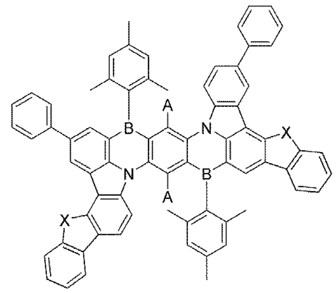

32a : A=A1
32b : A=A2
32c : A=A3
32d:A=A4
32e : A=A7
32f : A=A10
32g : A=A11
32h:A=A16
32i : A=A35
32j : A=A39 32k : A=A40
32l : A=A41
32m : A=A42

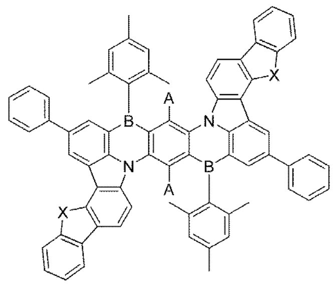

33a : A=A1
33b:A=A2
33c : A=A3
33d : A=A4
33e : A=A7
33f : A=A10
33g : A=A11
33h:A=A16
33i : A=A35
33j : A=A39 33k : A=A40
33l:A=A41
33m : A=A42

(continued)

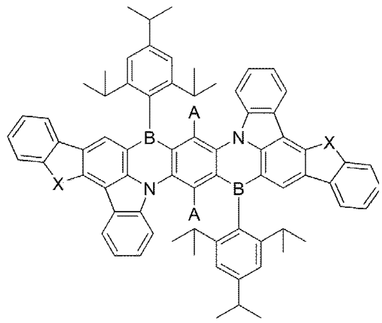

34a : A=A1
34b : A=A2
34c : A=A3
34d : A=A4
34e : A=A7
34f : A=A10
34g : A=A11
34h:A=A16
34i : A=A35
34j : A=A39
34k : A=A40
34l : A=A41
34m : A=A42

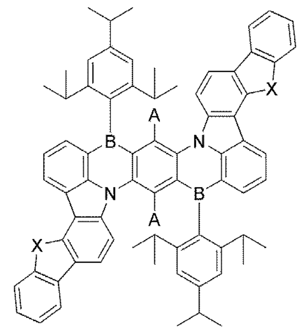

35a : A=A1
35b : A=A2
35c : A=A3
35d : A=A4
35e: A=A7
35f : A=A10
35g : A=A11
35h : A=A16
35i : A=A35
35j : A=A39
35k : A=A40
35l : A=A41
35m : A=A42

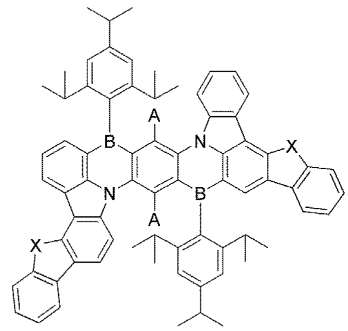

36a : A=A1
36b : A=A2
36c : A=A3
36d : A=A4
36e : A=A7
36f : A=A10
36g : A=A11
36h:A=A16
36i : A=A35
36j : A=A39
36k : A=A40
36l : A=A41
36m : A=A42

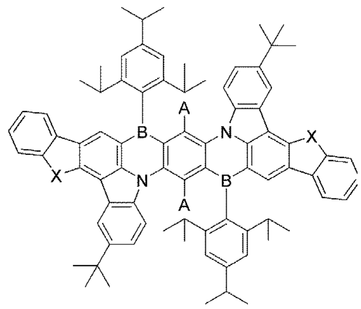

37a:A=A1
37b:A=A2
37c:A=A3
37d:A=A4
37e:A=A7
37f:A=A10
37g:A=A11
37h:A=A16
37i:A=A35
37j:A=A39
37k:A=A40
37l:A=A41
37m:A=A42

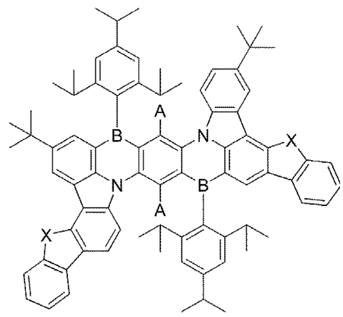

38a:A=A1
38b:A=A2
38c:A=A3
38d:A=A4
38e:A=A7
38f:A=A10
38g:A=A11
38h:A=A16
38i:A=A35
38j:A=A39
38k:A=A40
38l:A=A41
38m:A=A42

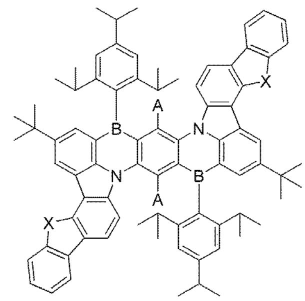

39a:A=A1
39b:A=A2
39c:A=A3
39d:A=A4
39e:A=A7
39f:A=A10
39g:A=A11
39h:A=A16
39i:A=A35
39j:A=A39
39k:A=A40
39l:A=A41
39m:A=A42

(continued)

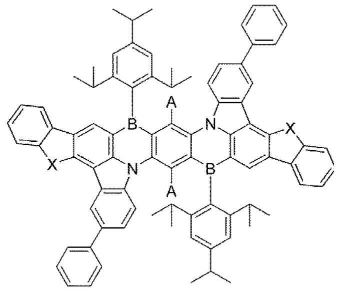

40a:A=A1
40b:A=A2
40c:A=A3
40d:A=A4
40e:A=A7
40f:A=A10
40g:A=A11
40h:A=A16
40i:A=A35
40j:A=A39
40k:A=A40
40l:A=A41
40m:A=A42

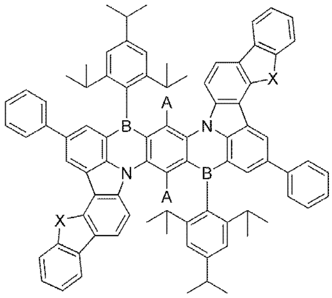

41a:A=A1
41b:A=A2
41c:A=A3
41d:A=A4
41e:A=A7
41f:A=A10
41g:A=A11
41h:A=A16
41i:A=A35
41i:A=A39
41k:A=A40
41l:A=A41
41m:A=A42

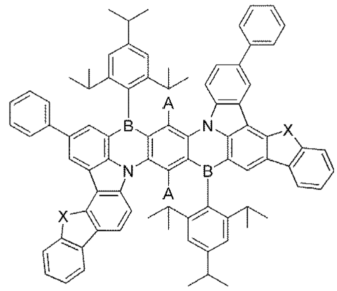

42a:A=A1
42b:A=A2
42c:A=A3
42d:A=A4
42e:A=A7
42f:A=A10
42g:A=A11
42h:A=A16
42 i : A=A35
42j:A=A39
42k:A=A40
42l:A=A41
42m:A=A42

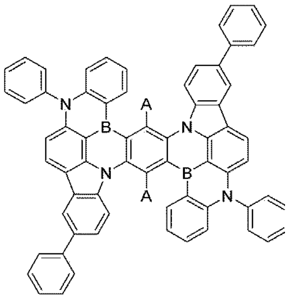

43a:A=A1
43b:A=A2
43c:A=A3
43d:A=A4
43e:A=A7
43f:A=A10
43g:A=A11
43h:A=A16
43i:A=A35
43j:A=A39 43k:A=A40
43l:A=A41
43m:A=A42

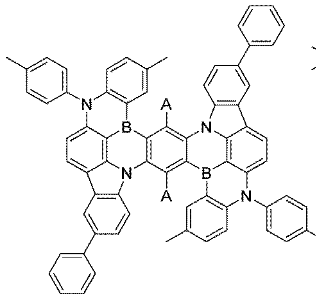

44a:A=A1
44b:A=A2
44c:A=A3
44d:A=A4
44e:A=A7
44f:A=A10
44g:A=A11
44h:A=A16
44i:A=A35
44j:A=A39 44k:A=A40
44l:A=A41
44m:A=A42

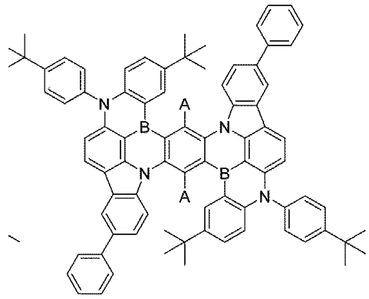

45a:A=A1
45b:A=A2
45c:A=A3
45d:A=A4
45e:A=A7
45f:A=A10
45g:A=A11
45h:A=A16
45i:A=A35
45j:A=A39 45k:A=A40
45l:A=A41
45m:A=A42

(continued)

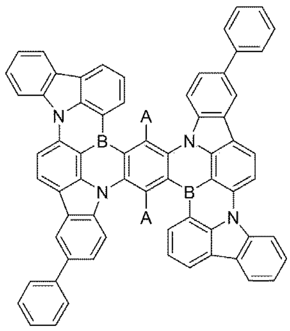

46a:A=A1

46b:A=A2
46c:A=A3
46d:A=A4
46e:A=A7
46f:A=A10
46g:A=A11
46h:A=A16
46i:A=A35
46j:A=A39
46k:A=A40
46l:A=A41
46m:A=A42

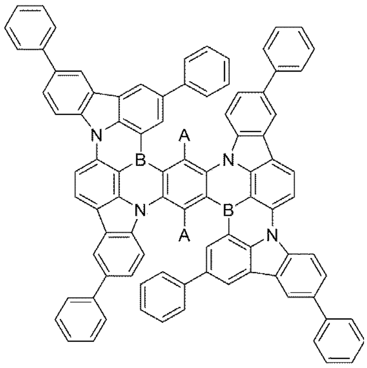

47a:A=A1
47b:A=A2
47c:A=A3
47d:A=A4
47e:A=A7
47f:A=A10
47g:A=A11
47h:A=A16
47i:A=A35
47j:A=A39
47k:A=A40
47l:A=A41
47m: A=A42

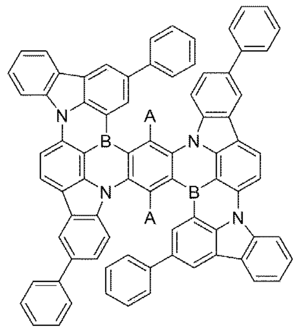

48a:A=A1

48b:A=A2
48c:A=A3
48d:A=A4
48e:A=A7
48f:A=A10
48g:A=A11
48h:A=A16
48 i A=A35
48j:A=A39
48k:A=A40
48l:A=A41
48m:A=A42

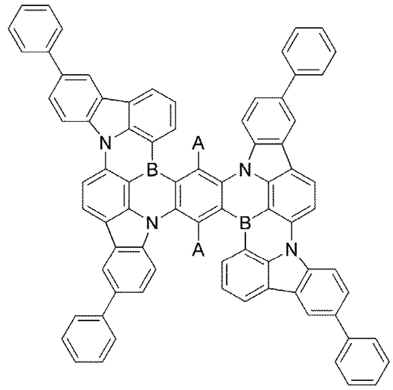

49a:A=A1
49b:A=A2
49c:A=A3
49d:A=A4
49e:A=A7
49f:A=A10
49g:A=A11
49h:A=A16
49i:A=A35
49j:A=A39
49k:A=A40
49l:A=A41
49m:A=A42

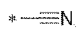
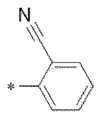
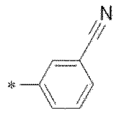
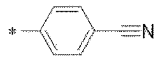
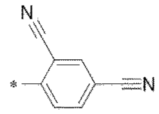
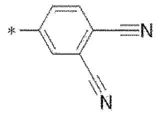

A1 A2 A3 A4 A5 A6

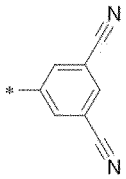
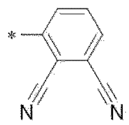
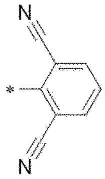
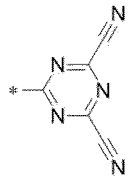
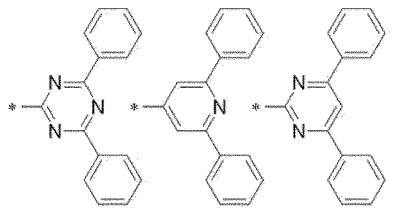

A7 A8 A9 A10 A11 A12 A13

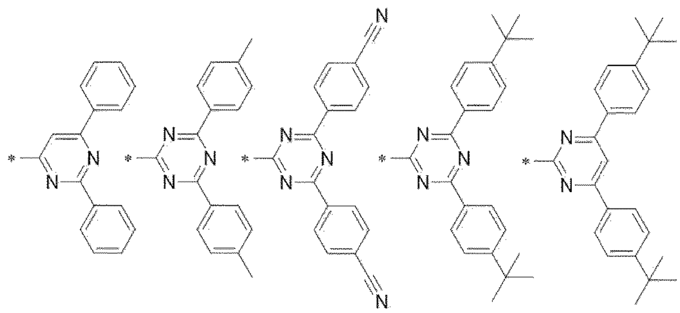

A14 A15 A16 A17 A18

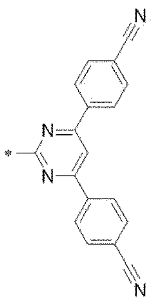
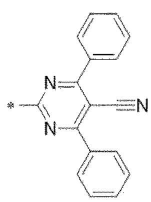
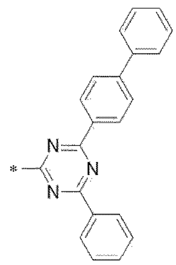
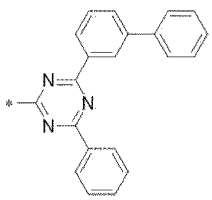

A19 A20 A21 A22

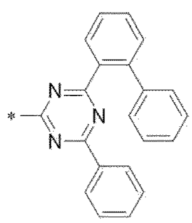
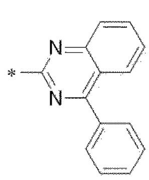
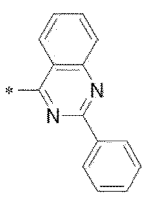

A23 A24 A25

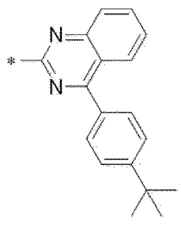 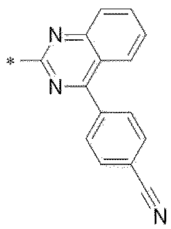 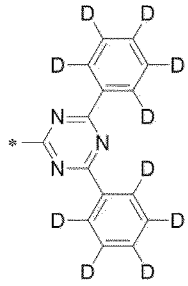 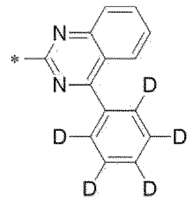

A26 A27 A28 A29

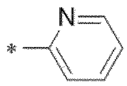 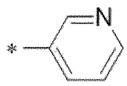 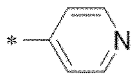 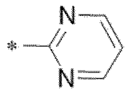 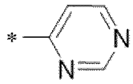

A30 A31 A32 A33 A34

$*-CF_3$ $*-C_2F_5$ 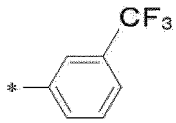 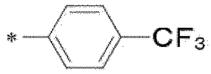

A35 A36 A37 A38

$*-F$ $*-Cl$ $*-Br$ $*-I$

A39 A40 A41 A42

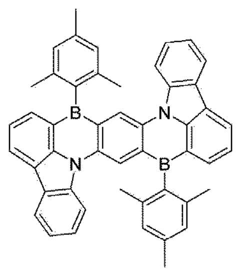 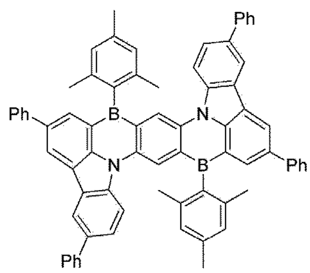 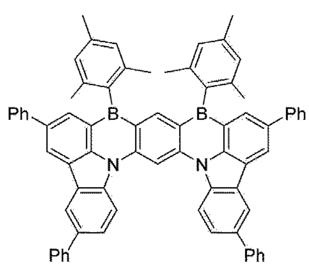

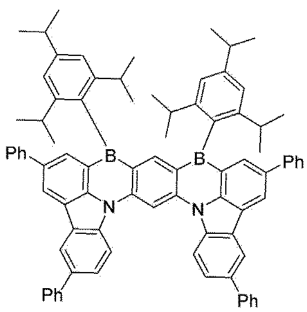 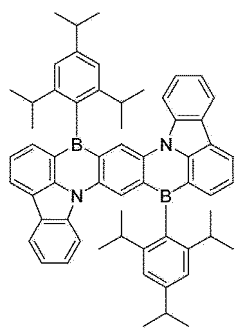 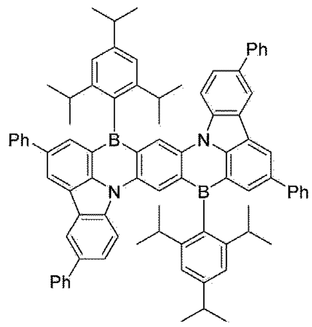

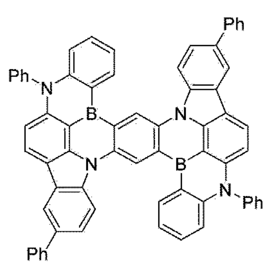
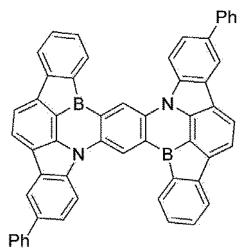
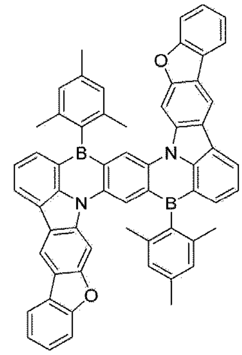
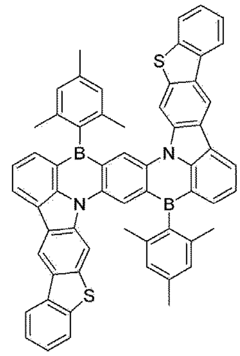

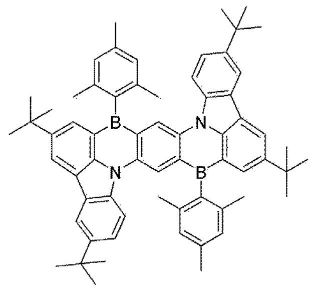
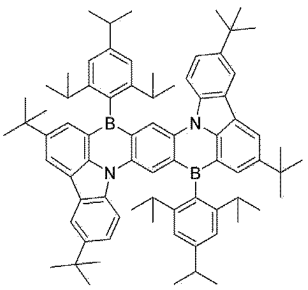
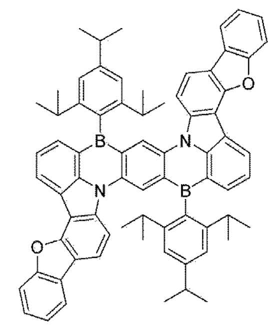

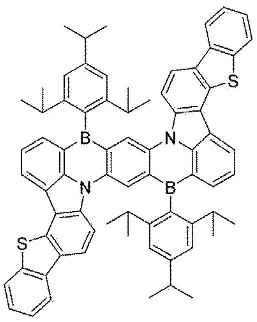
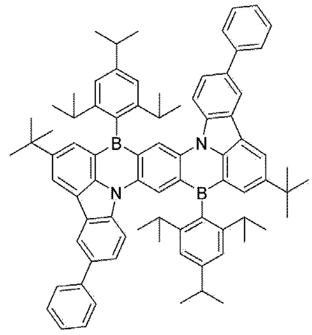
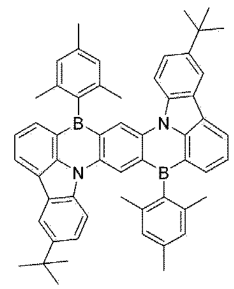

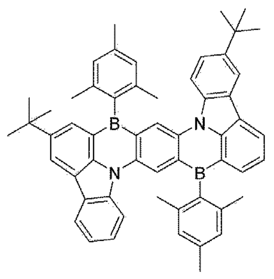
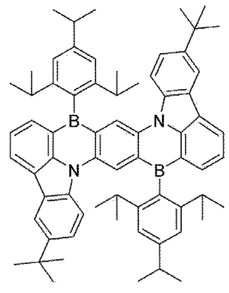
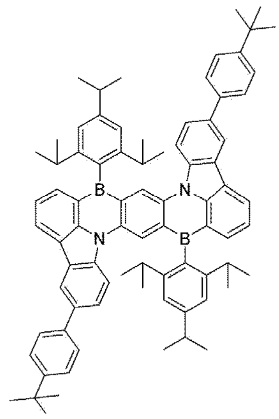

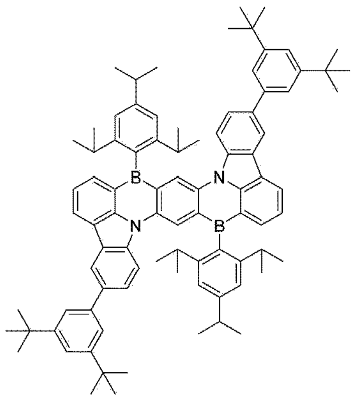 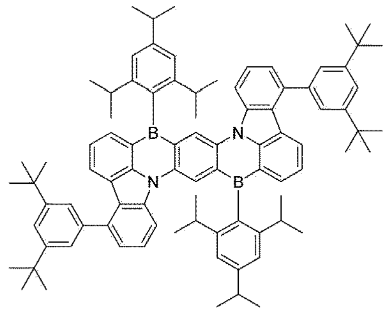

In some embodiments where a host material is used, the amount of the compound of the present invention contained in a light emitting layer as a light emitting material is 0.1% by weight or more. In some embodiments where a host material is used, the amount of the compound of the present invention contained in a light emitting layer as a light emitting material is 1% by weight or more. In some embodiments where a host material is used, the amount of the compound of the present invention contained in a light emitting layer as a light emitting material is 50% by weight or less. In some embodiments where a host material is used, the amount of the compound of the present invention contained in a light emitting layer as a light emitting material is 20% by weight or less. In some embodiments where a host material is used, the amount of the compound of the present invention contained in a light emitting layer as a light emitting material is 10% by weight or less.

In some embodiments, the host material in a light emitting layer is an organic compound having a hole transporting capability and an electron transporting capability. In some embodiments, the host material in a light emitting layer is an organic compound that prevents increase in the wavelength of emitted light. In some embodiments, the host material in a light emitting layer is an organic compound having a high glass transition temperature.

In some embodiments, the host material is selected from the group consisting of the followings:

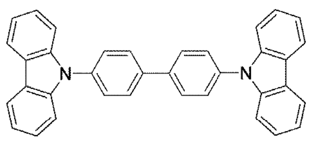 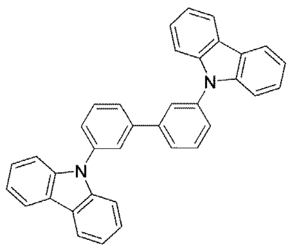 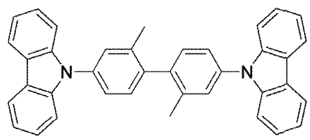

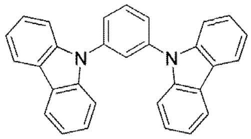 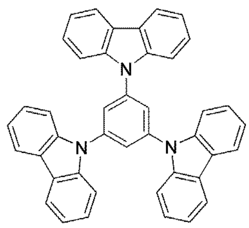 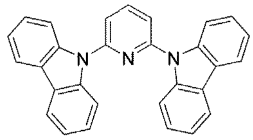

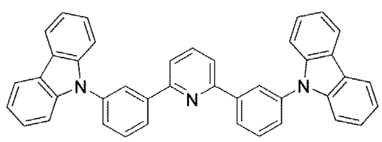 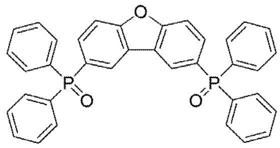

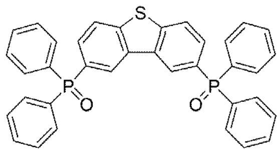
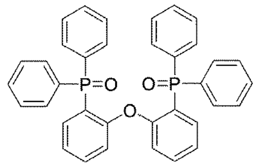
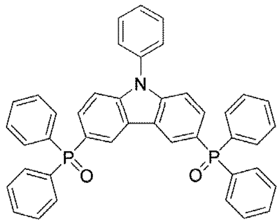

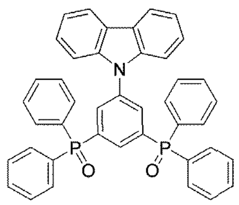
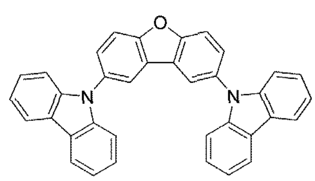
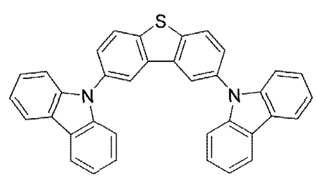

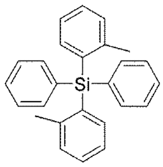
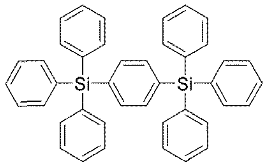
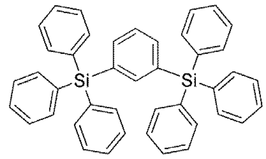
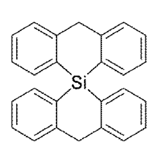

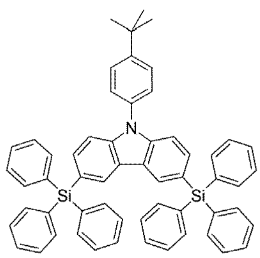
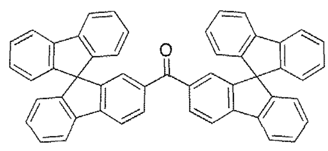
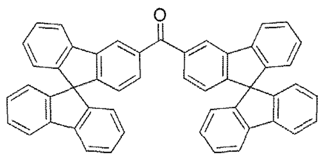

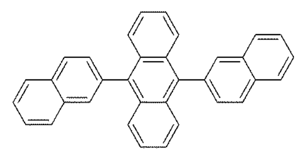
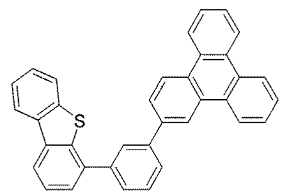
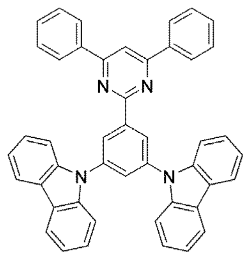

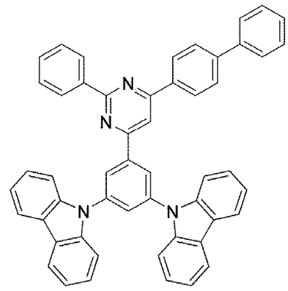
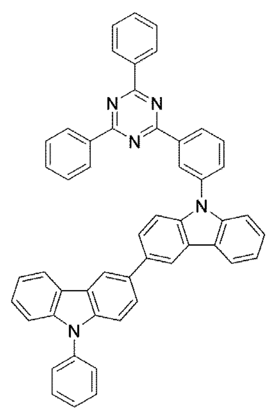
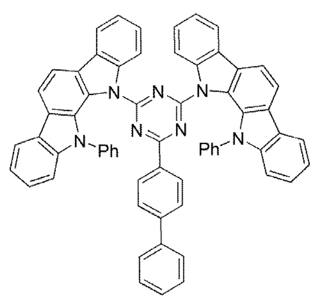

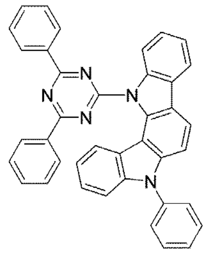 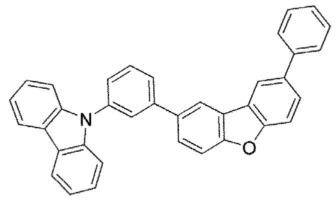 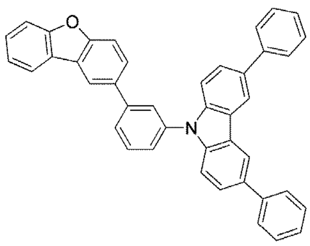

In some embodiments, the light emitting layer contains two or more kinds of TADF molecules differing in the structure. For example, the light emitting layer can contain three kinds of materials of a host material, a first TADF molecule and a second TADF molecule whose excited singlet energy level is higher in that order. In that case, both the first TADF molecule and the second TADF molecule are preferably such that the difference $\Delta E_{ST}$ between the lowest excited singlet energy level and the lowest excited triplet energy level at 77 K is 0.3 eV or less, more preferably 0.25 eV or less, even more preferably 0.2 eV or less, further more preferably 0.15 eV or less, further more preferably 0.1 eV or less, further more preferably 0.07 eV or less, further more preferably 0.05 eV or less, further more preferably 0.03 eV or less, and particularly preferably 0.01 eV or less. The content of the first TADF molecule in the light emitting layer is preferably larger than the content of the second TADF molecule therein. The content of the host material in the light emitting layer is preferably larger than the content of the second TADF molecule therein. The content of the first TADF molecule in the light emitting layer can be larger than or can be smaller than or can be the same as the content of the host material therein. In some embodiments, the composition in the light emitting layer can be 10 to 70% by weight of a host material, 10 to 80% by weight of a first TADF molecule, and 0.1 to 30% by weighty of a second TADF molecule. In some embodiments, the composition in the light emitting layer can be 20 to 45% by weight of a host material, 50 to 75% by weight of a first TADF molecule, and 5 to 20% by weighty of a second TADF molecule. In some embodiments, the photoluminescence quantum yield φPL1(A) by photo-excitation of a co-deposited film of a first TADF molecule and a host material (the content of the first TADF molecule in the co-deposited film = A% by weight) and the photoluminescence quantum yield φPL2(A) by photo-excitation of a co-deposited film of a second TADF molecule and a host material (the content of the second TADF molecule in the co-deposited film = A% by weight) satisfy a relational formula φPL1(A) > φPL2(A). In some embodiments, the photoluminescence quantum yield φPL2(B) by photo-excitation of a co-deposited film of a second TADF molecule and a host material (the content of the second TADF molecule in the co-deposited film = B% by weight) and the photoluminescence quantum yield φPL2(100) by photo-excitation of a single film of a second TADF molecule satisfy a relational formula φPL2(B) > φPL2(100). In some embodiments, the light emitting layer can contain three kinds of TADF molecules differing in the structure. The compound of the present invention can be any of the plural TADF compounds contained in the light emitting layer.

In some embodiments, the light emitting layer can be composed of materials selected from the group consisting of a host material, an assist dopant and a light emitting material. In some embodiments, the light emitting layer does not contain a metal element. In some embodiments, the light emitting layer can be formed of a material composed of atoms alone selected from the group consisting of a carbon atom, a hydrogen atom, a deuterium atom, a nitrogen atom, an oxygen atom and a sulfur atom. Or the light emitting layer can be formed of a material composed of atoms alone selected from the group consisting of a carbon atom, a hydrogen atom, a deuterium atom, a nitrogen atom and an oxygen atom. Or the light emitting layer can be formed of a material composed of atoms alone selected from the group consisting of a carbon atom, a hydrogen atom, a nitrogen atom and an oxygen atom.

In the case where the light emitting layer contains any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP

2015-129240 A, WO2015/129714, WO2015/129715, WO2015/133501, WO2015/136880, WO2015/137244, WO2015/137202, WO2015/137136, WO2015/146541, and WO2015/159541. These patent publications described in this paragraph are hereby incorporated as a part of this description by reference.

In the following, the constituent members and the other layers than the light emitting layer of the organic electroluminescent device are described.

Substrate:

In some embodiments, the organic electroluminescent device of the present invention is supported by a substrate, wherein the substrate is not particularly limited and can be any of those that have been commonly used in an organic electroluminescent device, for example those formed of glass, transparent plastics, quartz and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, where the pattern may not require high accuracy (for example, approximately 100 $\mu$m or more), the pattern can be formed with a mask having a desired shape on vapor deposition or sputtering of the electrode material. In some embodiments, when a material can be applied as a coating, such as an organic electroconductive compound, a wet film forming method, such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is from 10 to 1,000 nm. In some embodiments, the thickness of the anode is from 10 to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, and a lithium-aluminum mixture and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode ranges from 10 nm to 5 $\mu$m. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light emitting layer or the hole transporting layer, and between the cathode and the light emitting layer or the electron transporting layer. In some embodiments, an injection layer is present. In some embodiments, no injection layer is present.

Preferred compound examples for use as a hole injection material are shown below.

$MoO_3$,

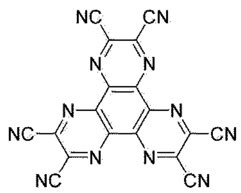
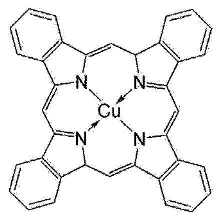
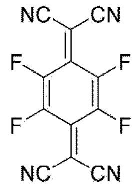
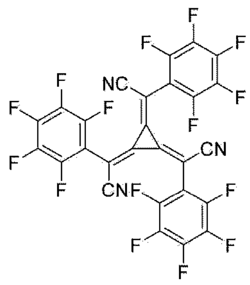

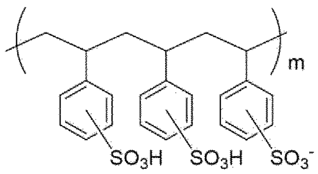
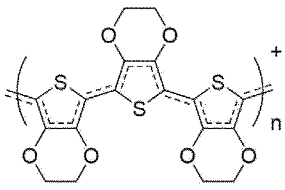

Next, preferred compound examples for use as an electron injection material are shown below.
LiF, CsF,

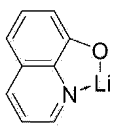

Barrier Layer:

A barrier layer is a layer capable of inhibiting charges (electrons or holes) and/or excitons present in the light emitting layer from being diffused outside the light emitting layer. In some embodiments, the electron barrier layer is between the light emitting layer and the hole transporting layer, and inhibits electrons from passing through the light emitting layer toward the hole transporting layer. In some embodiments, the hole barrier layer is between the light emitting layer and the electron transporting layer, and inhibits holes from passing through the light emitting layer toward the electron transporting layer. In some embodiments, the barrier layer inhibits excitons from being diffused outside the light emitting layer. In some embodiments, the electron barrier layer and the hole barrier layer are exciton barrier layers. As used herein, the term "electron barrier layer" or "exciton barrier layer" includes a layer that has both the function of an electron barrier layer and the function of an exciton barrier layer.

Hole Barrier Layer:

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light emitting layer. The material used for the hole barrier layer can be the same materials as the ones described for the electron transporting layer.
Preferred compound examples for use for the hole barrier layer are shown below.

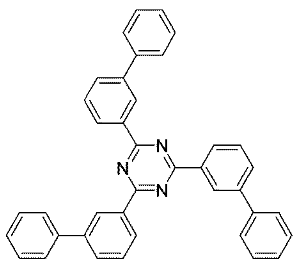
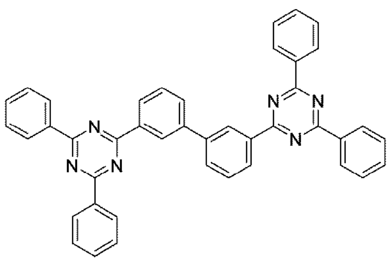
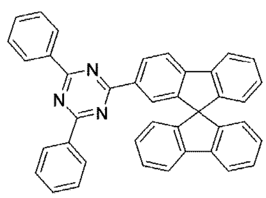

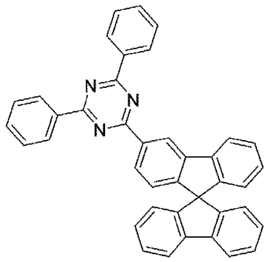
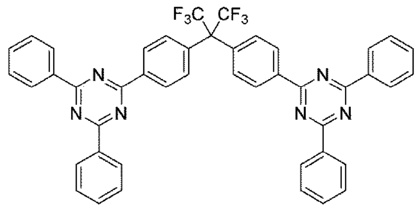
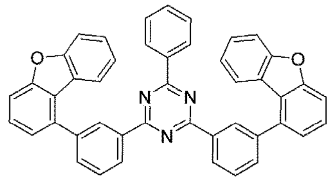

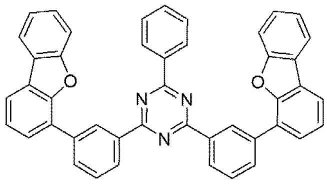
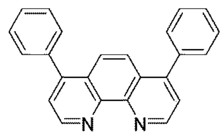
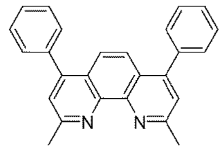

Electron Barrier Layer:

An electron barrier layer transports holes. In some embodiments, the electron barrier layer inhibits electrons from reaching the hole transporting layer while transporting holes. In some embodiments, the electron barrier layer enhances the recombination probability of electrons and holes in the light emitting layer. The material used for the electron barrier layer can be the same material as the ones described above for the hole transporting layer.

Preferred compound examples for use as the electron barrier material are shown below.

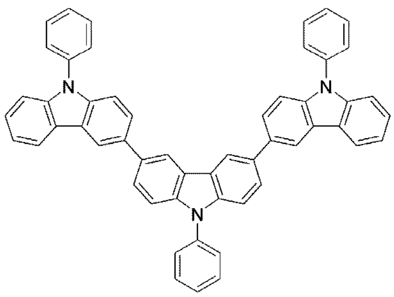
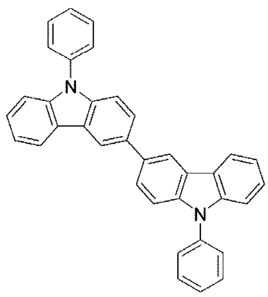
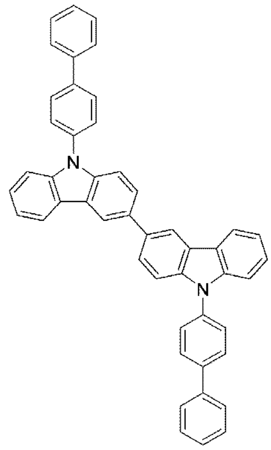

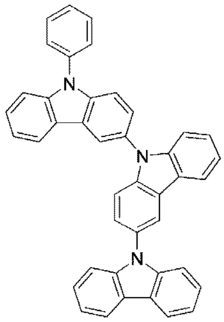
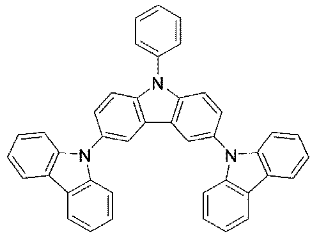
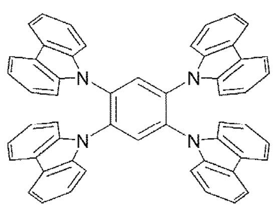

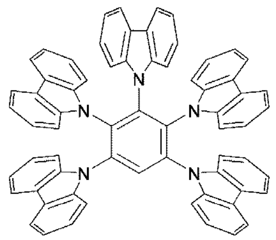 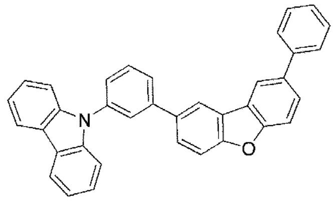 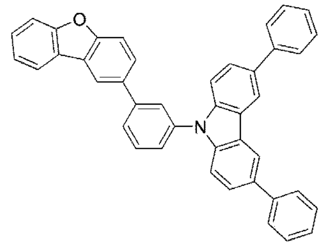

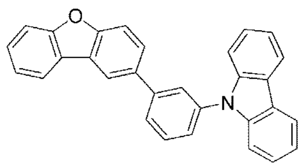

Exciton Barrier Layer:

An exciton barrier layer inhibits excitons generated through recombination of holes and electrons in the light emitting layer from being diffused to the charge transporting layer. In some embodiments, the exciton barrier layer enables effective confinement of excitons in the light emitting layer. In some embodiments, the light emission efficiency of the device is enhanced. In some embodiments, the exciton barrier layer is adjacent to the light emitting layer on any of the side of the anode and the side of the cathode, and on both the sides. In some embodiments, where the exciton barrier layer is on the side of the anode, the layer can be between the hole transporting layer and the light emitting layer and adjacent to the light emitting layer. In some embodiments, where the exciton barrier layer is on the side of the cathode, the layer can be between the light emitting layer and the cathode and adjacent to the light emitting layer. In some embodiments, a hole injection layer, an electron barrier layer, or a similar layer is between the anode and the exciton barrier layer that is adjacent to the light emitting layer on the side of the anode. In some embodiments, a hole injection layer, an electron barrier layer, a hole barrier layer, or a similar layer is between the cathode and the exciton barrier layer that is adjacent to the light emitting layer on the side of the cathode. In some embodiments, the exciton barrier layer comprises excited singlet energy and excited triplet energy, at least one of which is higher than the excited singlet energy and the excited triplet energy of the light emitting material, respectively.

Hole Transporting Layer:

The hole transporting layer comprises a hole transporting material. In some embodiments, the hole transporting layer is a single layer. In some embodiments, the hole transporting layer comprises a plurality of layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that can be used in the present invention include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer (particularly, a thiophene oligomer), or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

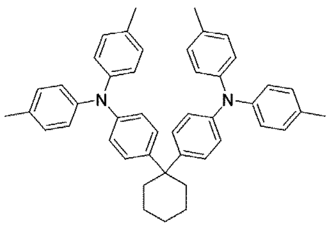
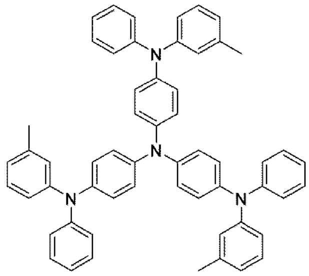
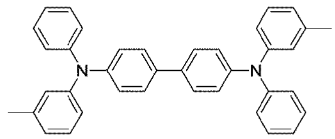

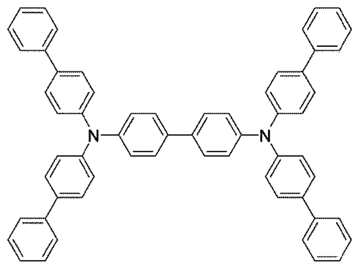
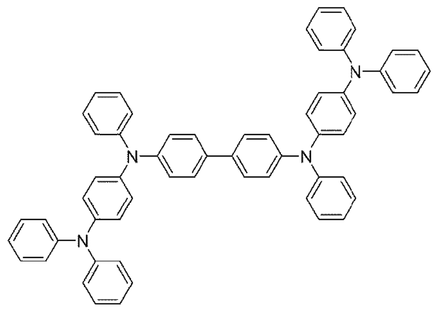

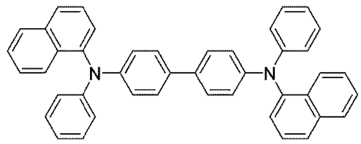
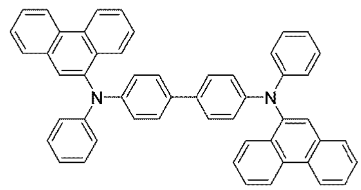

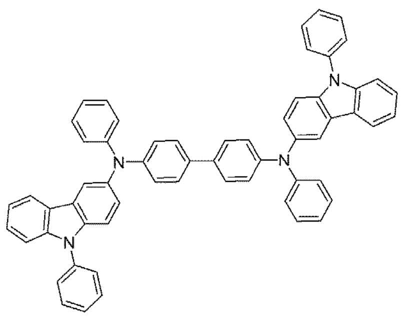
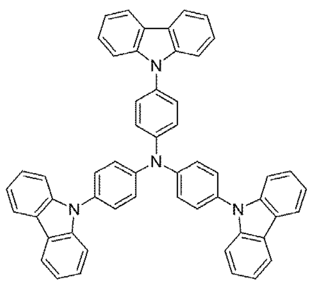
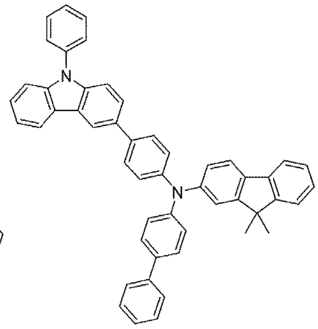

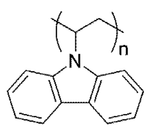
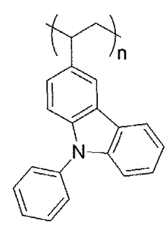
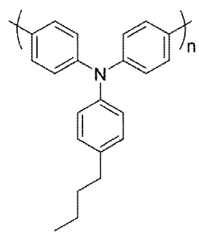

Electron Transporting Layer:

The electron transporting layer comprises an electron transporting material. In some embodiments, the electron transporting layer is a single layer. In some embodiments, the electron transporting layer comprises a plurality of layers.

In some embodiments, the electron transporting material needs only to have a function of transporting electrons, which are injected from the cathode, to the light emitting layer. In some embodiments, the electron transporting material also function as a hole barrier material. Examples of the electron transporting layer that can be used in the present invention include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an

oxadiazole derivative, an azole derivative, an azine derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

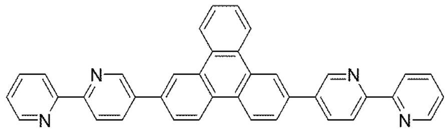
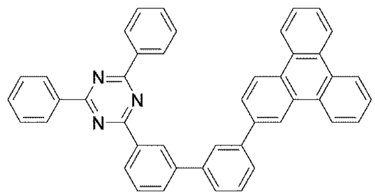
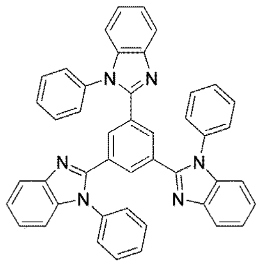
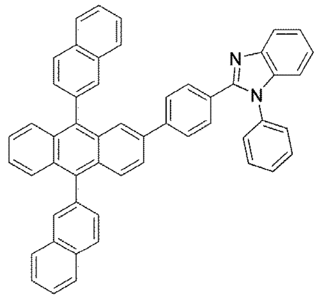
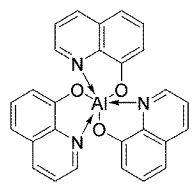
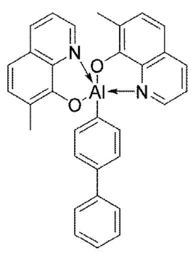
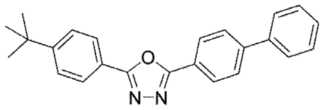
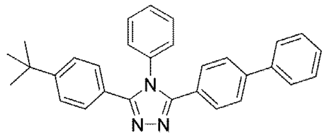

Hereinunder, compound examples preferred as a material that can be added to the organic layers are shown. For example, it is conceivable to add these as a stabilization material.

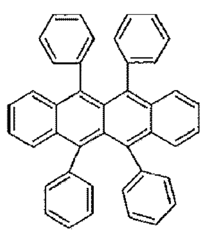
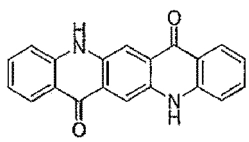
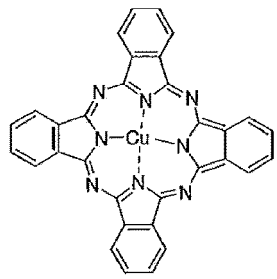
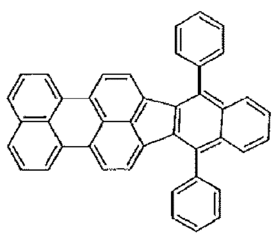
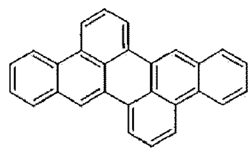

Preferred materials for use in the organic electroluminescent device are specifically shown. However, the materials usable in the present invention should not be limitatively interpreted by the following exemplary compounds. Compounds that are exemplified as materials having a specific function can also be used as materials having any other function.

Devices:

In some embodiments, an light emitting layer is incorporated into a device. For example, the device includes, but is not limited to an OLED bulb, an OLED lamp, a television screen, a computer monitor, a mobile phone, and a tablet.
In some embodiments, an electronic device includes an OLED comprising an anode, a cathode, and at least one organic layer comprising a light emitting layer between the anode and the cathode.
In some embodiments, compositions described herein can be incorporated into various light-sensitive or light-activated devices, such as OLEDs or opto-electronic devices. In some embodiments, the composition can be useful in facilitating charge transfer or energy transfer within a device and/or as a hole transport material. The device can be, for example, an organic light emitting diode (OLED), an organic integrated circuit (O-IC), an organic field-effect transistor (O-FET), an organic thin-film transistor (O-TFT), an organic light emitting transistor (O-LET), an organic solar cell (O-SC), an organic optical detector, an organic photoreceptor, an organic field-quench device (O-FQD), a light emitting electrochemical cell (LEC) or an organic laser diode (O-laser).

Bulbs or Lamps:

In some embodiments, an electronic device includes an OLED comprising an anode, a cathode, and at least one organic layer comprising a light emitting layer between the anode and the cathode.
In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.
In some embodiments, a device is an OLED light comprising:

a circuit board having a first surface with a mounting surface and an opposing second surface, and defining at least one opening;
at least one OLED on the mounting surface, the at least one OLED configured to emanate light, the OLED comprising: an anode, a cathode, and at least one organic layer comprising a light emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens:

In some embodiments, the light emitting layer of the present invention can be used in a screen or a display. In some embodiments, the compounds of the present invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen (which can also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the fine patterning of pixels needed for high resolution displays while optimizing the chemical vapor deposition onto a TFT backplane.
The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etching rate but the depths are varying depending on the halftone pattern. Varying the size and spacing of the halftone pattern allows etching to be inhibited at different rates within the pixel and allow a localized deeper etch needed to create steep vertical bevels.
A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the opening areas in the mask used for deposition is through a wet chemical etching.
In some embodiments, a screen or display pattern is a pixel matrix on a substrate. In some embodiments, a

screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

In another aspect of the present invention, provided herein is a method of manufacturing an organic light emitting diode (OLED) display, the method comprising:

forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light emitting layer, a gate electrode, and a source/drain electrode. Each of the plurality of display units may include a thin film transistor (TFT) layer, a planarization film formed on the TFT layer, and a light emitting unit formed on the planarization film, wherein the organic film applied to the interface portion is formed of a same material as a material of the planarization film and is formed at a same time as the planarization film is formed. In some embodiments, a light emitting unit is connected to the TFT layer with a passivation layer and a planarization film therebetween and an encapsulation layer that covers and protects the light emitting unit. In some embodiments of the method of manufacturing, the organic film contacts neither the display units nor the encapsulation layer.

Each of the organic film and the planarization film can include any one of polyimide and acryl. In some embodiments, the barrier layer can be an inorganic film. In some embodiments, the base substrate can be formed of polyimide. The method can further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film can be formed on the edge portion of the barrier layer such that a portion of the organic film directly contacts the base substrate and a remaining portion of the organic film contacts the barrier layer while surrounding the edge portion of the barrier layer.

In some embodiments, the light emitting layer includes a pixel electrode, a counter electrode, and an organic light emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light emitting layer emits light, thereby forming an image. Hereinafter, an image forming unit including the TFT layer and the light emitting unit is referred to as a display unit.

In some embodiments, the encapsulation layer that covers the display unit and prevents penetration of external moisture can be formed to have a thin film encapsulation structure in which an organic film and an inorganic film are alternately stacked. In some embodiments, the encapsulation layer has a thin film encapsulation structure in which a

plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels can be softly cut and cracks can be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below can be appropriately modified unless they deviate from the substance of the present invention. Accordingly, the scope of the present invention is not construed as being limited to the specific examples shown below. Hereunder, the light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from Hamamatsu Photonics K.K., Model C4334). The energies of HOMO and LUMO were measured by photoelectron spectroscopy in air (such as AC-3 manufactured by Riken Keiki Co., Ltd.).

In the following Synthesis Examples, compounds included in the general formula (1) were synthesized.

(Synthesis Example 1) Synthesis of Compound 46542

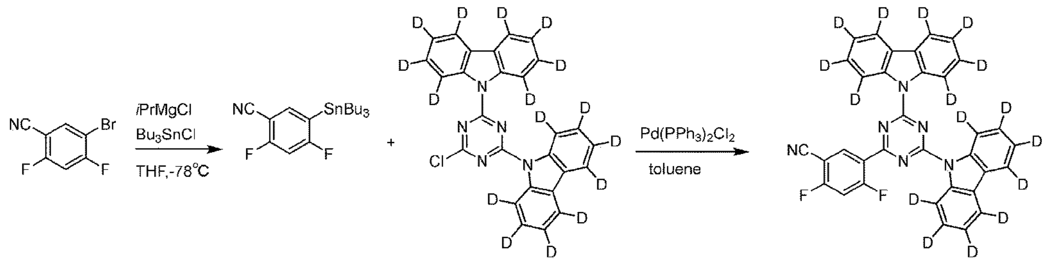


a

Compound a

Under a nitrogen stream, a tetrahydrofuran (THF) solution of 2.0 M isopropyl magnesium chloride (8.2 mL, 16.4 mmol) was gradually dropwise added to a tetrahydrofuran (150 mL) solution of 5-bromo-2,4-difluorobenzonitrile (3.35 g, 15.3 mmol) at -78°C. After dropwise addition, this was stirred for 1 hour, then tributyltin chloride (5.92 g, 18.2 mmol) was added, heated up to room temperature, and stirred for 3 hours. The reaction vessel was cooled to 0°C, and a saturated ammonium chloride solution was added. The resultant reaction solution was extracted with toluene, the organic layer was washed with saturated saline water, and dried with anhydrous magnesium. The solvent was removed, the resultant yellow liquid was dissolved in dewatered toluene (150 mL), and bis(triphenylphosphine)palladium(II) dichloride (1.05 g, 1.50 mmol) and 9,9'-(6-chloro-1,3,5-triazine-2,4-diyl)bis(9H-carbazole-1,2,3,4,5,6,7,8-$d_8$) (6.3 g, 13.6 mmol) were added, and heated at 120°C for 18 hours. The reaction solution was restored to room temperature, and the resultant gray solid was filtered out. The solid was washed with toluene, tetrahydrofuran and hexane to give a gray solid compound a in 5.61 g (9.93 mmol, 73% yield).
ASAP MS Spectrometry: $C_{34}H_2D_{16}F_2N_6$: theoretical value 564.26, observed value 565.50 [M+$H^+$]

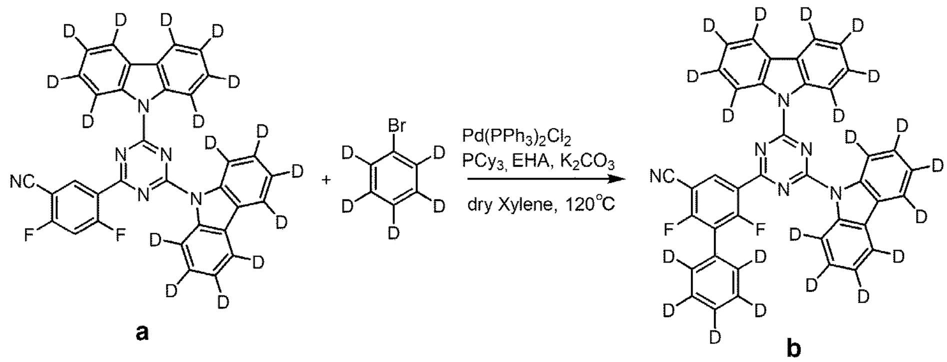


a b

Compound b

Under a nitrogen stream, 2-ethylhexanoic acid (0.30 g, 2.00 mmol) and bromobenzene-$d_5$ (1.77 g, 10.9 mmol) were added to a dewatered xylene (100 mL) solution of the compound a (5.61 g, 9.93 mmol), bis(triphenylphosphine) palladium(II) dichloride (0.33 g, 0.50 mmol), tricyclohexylphosphine (0.28 g, 1.00 mmol) and potassium carbonate (2.77 g, 20 mmol), and stirred at 120°C for 18 hours. The reaction solution was restored to room temperature, and the resultant gray solid was filtered out. The solid was washed with toluene, ion-exchanged water and methanol, and then the solid was dissolved in hot toluene. The toluene solution was led to pass through a silica pad, and the filtrate was concentrated to give a white compound b in 2.80 g (4.33 mmol, 44% yield).
ASAP MS Spectrometry: $C_{40}HD_{21}F_2N_6$: theoretical value 645.32, observed value 646.49 [M+$H^+$]

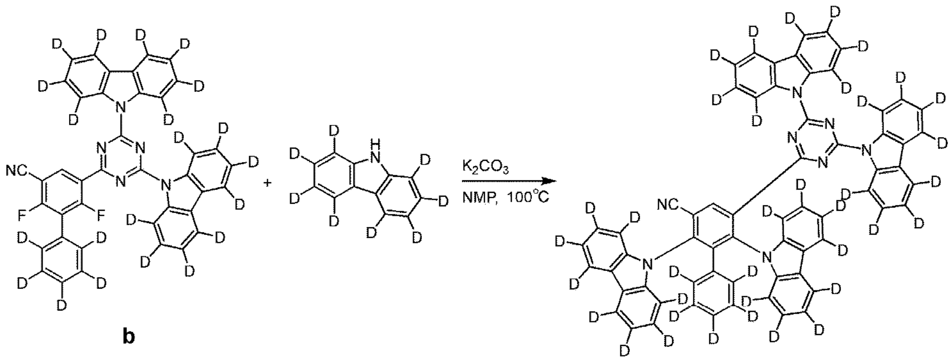


b

46542

Compound 46542

Potassium carbonate (0.87 g, 6.29 mmol) was added to an N-methyl-2-pyrrolidone (50 mL) solution of Compound b (1.62 g, 2.50 mmol) and carbazole-1,2,3,4,5,6,7,8-$d_8$ (0.96 g, 5.50 mmol), and stirred at 100°C for 4 hours. The reaction solution was cooled to 0°C, ion-exchanged water was added, and this was extracted with ethyl acetate. The combined organic layer was washed with a saturated saline solution, dried with anhydrous magnesium sulfate, and the solvent was removed. The resultant reaction mixture was purified by silica gel chromatography (toluene/hexane = 2/1). The resultant solid was reprecipitated with ethyl acetate/hexane to give a pale green compound 46542 in 0.90 g (0.94 mmol, 38% yield).

$^1$H-NMR (400 MHz, DMSO-d6): δ 9.03 (s, 1H).
ASAP MS Spectrometry: $C_{64}HD_{37}N_8$: theoretical value 955.55, observed value 956.99 [M+H$^+$]

(Synthesis Example 2) Synthesis of Compound 46542(40)

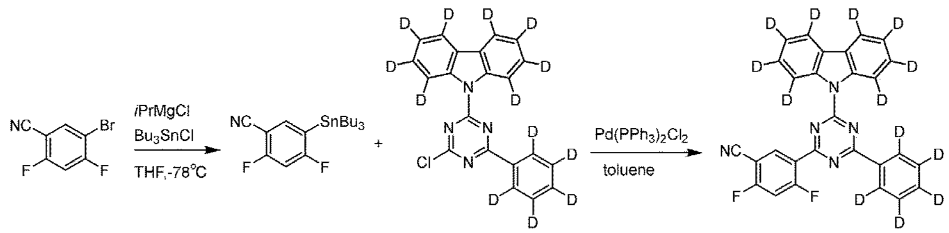


c

Compound c

Under a nitrogen stream, a tetrahydrofuran solution of 2.0 M isopropyl magnesium chloride (16.5 mL, 33.0 mmol) was gradually dropwise added to a tetrahydrofuran (300 mL) solution of 5-bromo-2,4-difluorobenzonitrile (6.54 g, 30.0 mmol) at -78°C. After dropwise addition, this was stirred for 1 hour, then tributyltin chloride (11.7 g, 35.9 mmol) was added, heated up to room temperature, and stirred for 3 hours. The reaction vessel was cooled to 0°C, and a saturated ammonium chloride solution was added. The resultant reaction solution was extracted with toluene, the organic layer was washed with saturated saline water, and dried with anhydrous magnesium. The solvent was removed, the resultant yellow liquid was dissolved in dewatered toluene (300 mL), and bis(triphenylphosphine)palladium(II) dichloride (2.11 g, 3.00 mmol) and 9-(4-chloro-6-phenyl-ds)-1,3,5-triazin-2-yl)-9H-carbazole-1,2,3,4,5,6,7,8-$d_8$ (11.0 g, 29.67 mmol) were added, and heated at 120°C for 18 hours. The reaction solution was restored to room temperature, and filtered through Celite. The filtrate was concentrated, the resultant solid was washed with hexane, toluene and tetrahydrofuran to give a pale orange solid compound c in 8.1 g (17.1 mmol, 57% yield).

$^1$H-NMR (400 MHz, DMSO-d6): δ 9.61 (t, J=9.2 Hz, 1H), 7.98 (t, J=10.0 Hz, 1H).
ASAP MS Spectrometry: $C_{28}H_2D_{13}F_2N_5$: theoretical value 472.21, observed value 473.41 [M+H$^+$]

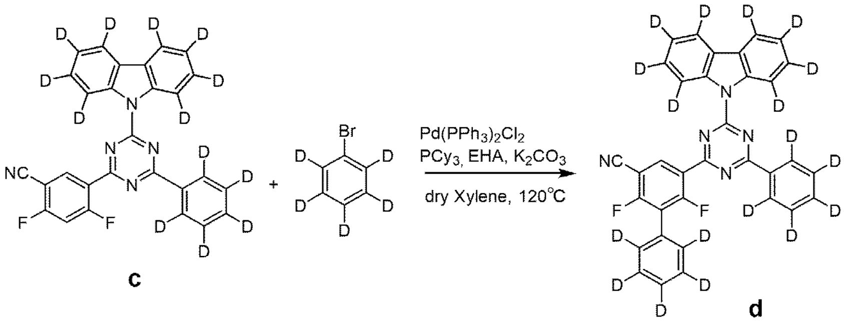


c

d

Compound d

Under a nitrogen stream, 2-ethylhexanoic acid (0.49 g, 3.40 mmol) and bromobenzene-$d_5$ (3.32 g, 20.5 mmol) were added to a dewatered xylene (340 mL) solution of Compound c (8.08 g, 17.1 mmol), bis(triphenylphosphine) palladium(II) dichloride (0.60 g, 0.85 mmol), tricyclohexylphosphine (0.49 g, 1.75 mmol) and potassium carbonate (4.75 g, 34.4 mmol), and stirred at 120°C for 20 hours. The reaction solution was restored to room temperature, and the resultant white solid was filtered out. The solid was washed with toluene, ion-exchanged water and methanol, and then the solid was dissolved in hot toluene. The toluene solution was led to pass through a silica pad, and the filtrate was concentrated to give a white compound d in 4.2 g (7.59 mmol, 44% yield).
ASAP MS Spectrometry: $C_{34}HD_{16}F_2N_5$: theoretical value 553.27, observed value 554.50 [M+H$^+$]

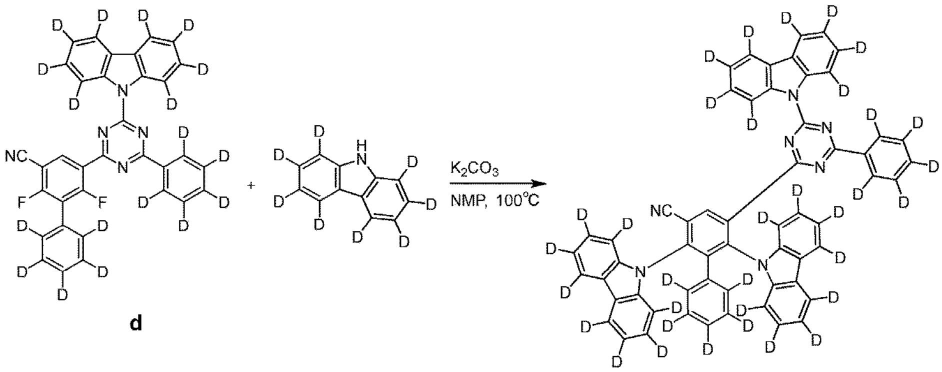


d

**46542(40)**

Compound 46542(40)

Potassium carbonate (1.38 g, 9.98 mmol) was added to an N-methyl-2-pyrrolidone (80 mL) solution of Compound d (2.21 g, 3.99 mmol) and carbazole-1,2,3,4,5,6,7,8-$d_8$ (1.47 g, 8.39 mmol), and stirred at 100°C for 6 hours. The reaction solution was cooled to 0°C, and ion-exchanged water and methanol were added to sort out a solid by filtration. The solid was washed with a mixed solution of ethyl acetate/hexane, and then purified by silica gel chromatography (toluene/hexane = 4/1). The resultant solid was reprecipitated with ethyl acetate/hexane to give a pale green compound 46542(40) in 2.77 g (3.21 mmol, 80% yield).

$^1$H-NMR (400 MHz, DMSO-d6): $\delta$ 8.85 (s, 1H).
ASAP MS Spectrometry: $C_{58}HD_{34}N_7$: theoretical value 863.50, observed value 864.87 [M+H$^+$]

(Synthesis Example 3) Synthesis of Compound 38317

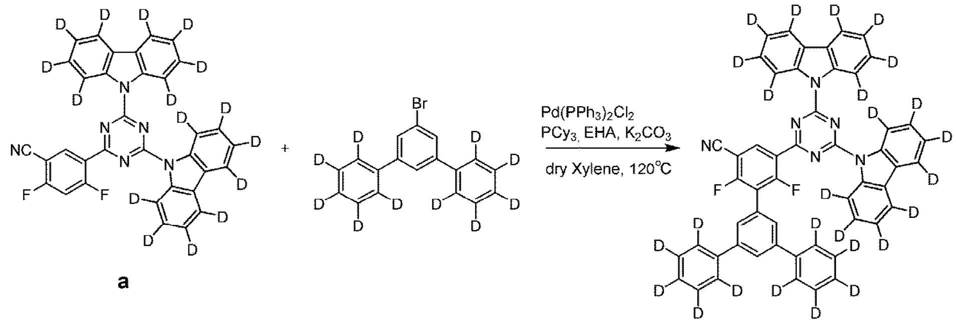


a

e

Compound e

Under a nitrogen stream, 2-ethylhexanoic acid (0.20 g, 1.42 mmol) and 5'-bromo-1,1':3',1"-terphenyl-2,2",3,3",4,4",5,5",6,6"-$d_{10}$ (2.50 g, 7.79 mmol) were added to a dewatered xylene (71 mL) solution of the compound a (4.00 g, 7.08 mmol), bis(triphenylphosphine)palladium(II) dichloride (0.24 g, 0.35 mmol), tricyclohexylphosphine (0.20 g, 0.71 mmol) and potassium carbonate (1.95 g, 34.4 mmol), and stirred at 120°C for 20 hours. The reaction solution was restored to room temperature, and the resultant white solid was filtered out. The solid was washed with toluene, ion-exchanged water and methanol, and then the solid was dissolved in hot toluene. The toluene solution was led to pass through a silica pad, and the filtrate was concentrated to give a white compound e in 1.36 g (1.69 mmol, 24% yield). ASAP MS Spectrometry: $C_{52}H_4D_{26}F_2N_6$: theoretical value 802.41, observed value 803.82 [M+H$^+$]

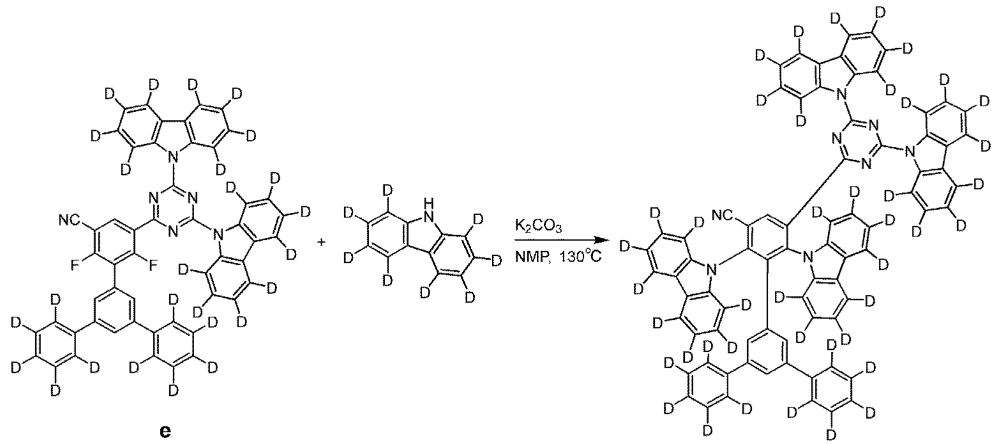


e

38317

Compound 38317

Potassium carbonate (0.46 g, 6.29 mmol) was added to an N-methyl-2-pyrrolidone (26 mL) solution of Compound e (1.06 g, 1.32 mmol) and carbazole-1,2,3,4,5,6,7,8-$d_8$ (0.49 g, 2.80 mmol), and stirred at 130°C for 4 hours. The reaction solution was cooled to room temperature, and diluted with ethyl acetate. The solution was washed with a saturated saline solution, dried with anhydrous magnesium sulfate, and the solvent was removed. The solid was reprecipitated with a mixed solvent of ethyl acetate/hexane, and the solid was filtered out. The crude product was purified by silica gel chromatography (toluene/hexane = 2/1). The resultant solid was reprecipitated with ethyl acetate/hexane to give a pale yellow compound 38317 in 0.46 g (0.41 mmol, 31% yield).

$^1$H-NMR (400 MHz, DMSO-d6): δ 9.13 (s, 1H), 7.02 (t, J=1.6 Hz, 1H), 6.90 (d, J=1.6 Hz, 2H).
ASAP MS Spectrometry: $C_{76}H_4D_{42}N_8$: theoretical value 1112.64, observed value 1113.21 [M+H$^+$]

(Synthesis Example 4) Synthesis of Compound 38317(40)

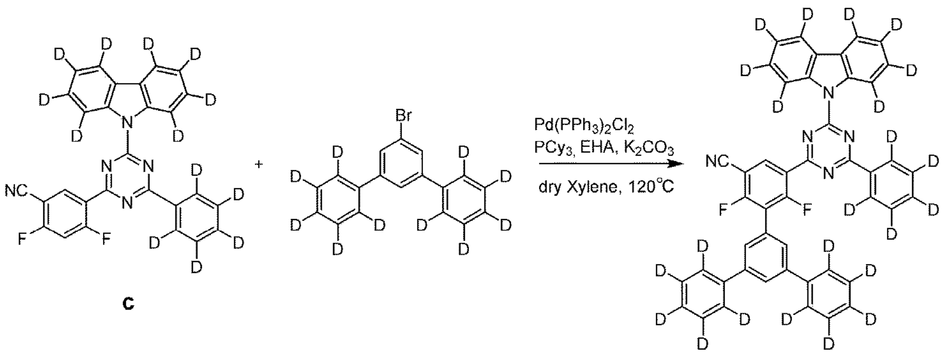


c

f

Compound f

Under a nitrogen stream, 2-ethylhexanoic acid (0.39 g, 2.70 mmol) and 5'-bromo-1,1':3',1"-terphenyl-2,2",3,3",4,4",5,5",6,6"-$d_{10}$ (3.13 g, 9.80 mmol) were added to a dewatered xylene (180 mL) solution of the compound c (4.21 g, 8.90 mmol), bis(triphenylphosphine)palladium(II) dichloride (0.48 g, 0.68 mmol), tricyclohexylphosphine (0.39 g, 1.39 mmol) and potassium carbonate (2.46 g, 34.4 mmol), and stirred at 120°C for 20 hours. The reaction solution was restored to room temperature, and the resultant white solid was filtered out. The solid was washed with toluene, ion-exchanged water and methanol, and then the solid was dissolved in hot toluene. The toluene solution was led to pass through a silica pad, and the filtrate was concentrated to give a white compound f in 2.90 g (4.08 mmol, 46% yield). ASAP MS Spectrometry: $C_{46}H_4D_{23}F_2N_5$: theoretical value 710.36, observed value 711.78 [M+$H^+$]

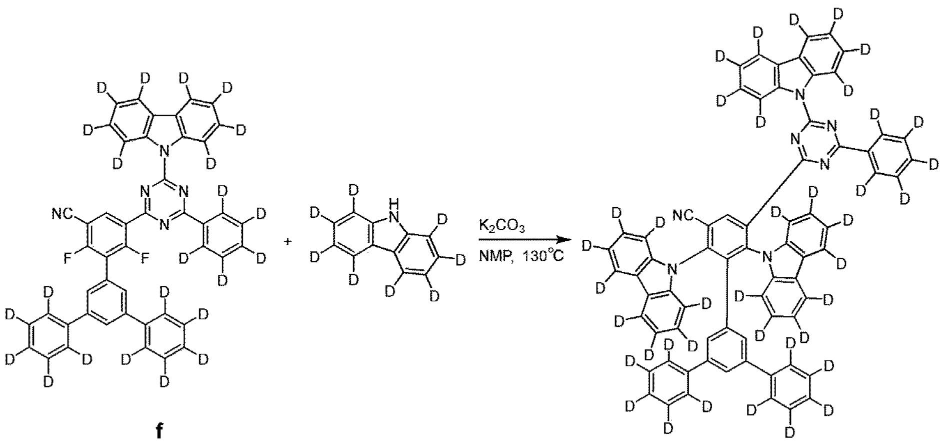


f

38317(40)

Compound 38317(40)

Potassium carbonate (1.04 g, 7.52 mmol) was added to an N-methyl-2-pyrrolidone (60 mL) solution of Compound f (2.13 g, 3.00 mmol) and carbazole-1,2,3,4,5,6,7,8-$d_8$ (1.10 g, 6.28 mmol), and stirred at 100°C for 6 hours. The reaction solution was cooled to 0°C, and ion-exchanged water and methanol were added. The solid was filtered out, washed with water, and dissolved in ethyl acetate. The solution was washed with a saturated saline solution, dried with anhydrous magnesium sulfate, the solvent was removed, the resultant solid was reprecipitated with a mixed solvent of ethyl acetate/hexane, and the solid was filtered out. The crude product was purified by silica gel chromatography (toluene/hexane = 2/1). The resultant solid was reprecipitated with toluene/hexane to give a pale yellow compound 38317(40) in 2.10 g (2.05 mmol, 68% yield).

$^1$H-NMR (400 MHz, DMSO-d6): δ 9.19 (s, 1H), 7.07 (t, J=1.6 Hz, 1H), 6.98 (d, J=1.6 Hz, 2H).
ASAP MS Spectrometry: $C_{70}H_4D_{39}N_7$: theoretical value 1020.60, observed value 1021.21 [M+$H^+$]

(Synthesis Example 5) Synthesis of Compound 937647(40)

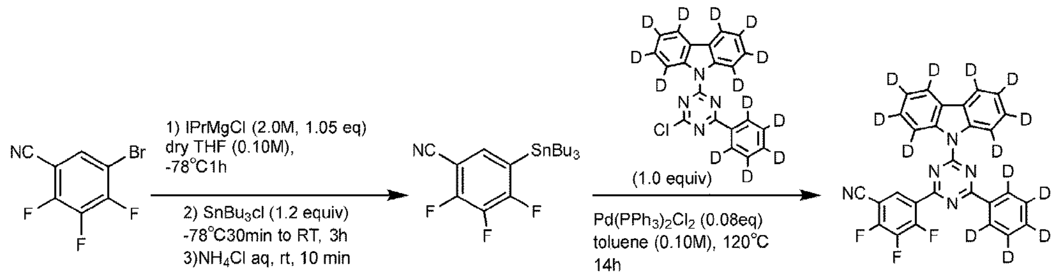


g

Compound g

Under a nitrogen stream, a tetrahydrofuran solution of 2.0 M isopropyl magnesium chloride (4.6 mL, 9.32 mmol) was gradually dropwise added to a tetrahydrofuran (85 mL) solution of 3-bromo-4,5,6-trifluorobenzonitrile (2.00 g, 8.47 mmol) at -78°C. After dropwise addition, this was stirred for 0.5 hours, then tributyltin chloride (3.31 g, 10.17 mmol) was added, heated up to room temperature, and stirred for 3 hours. The reaction vessel was cooled to 0°C, and a saturated ammonium chloride solution was added. The resultant reaction solution was extracted with toluene, the organic layer was washed with saturated saline water, and dried with anhydrous magnesium. The solvent was removed, the resultant yellow liquid was dissolved in dewatered toluene (85 mL), and bis(triphenylphosphine)palladium(II) dichloride (0.48 g, 0.68 mmol) and 9-(4-chloro-6-phenyl-$d_5$)-1,3,5-triazin-2-yl)-9H-carbazole-1,2,3,4,5,6,7,8-$d_8$ (3.91 g, 8.47 mmol) were added, and heated at 120°C for 14 hours. The reaction solution was restored to room temperature, and the resultant liquid was filtered through silica gel and Celite, and dried to solid. The resultant creamy-colored solid was recrystallized with toluene to give a white solid compound g in 2.05 g (4.18 mmol, 49% yield).

$^1$H-NMR (400 MHz, $CDCl_3$): δ 8.67 (td, J=7.2, 2.4 Hz, 1H),
ASAP MS Spectrometry: $C_{28}HD_{13}F_4N_5$: theoretical value 490.20, observed value 491.4 [M+H$^+$]

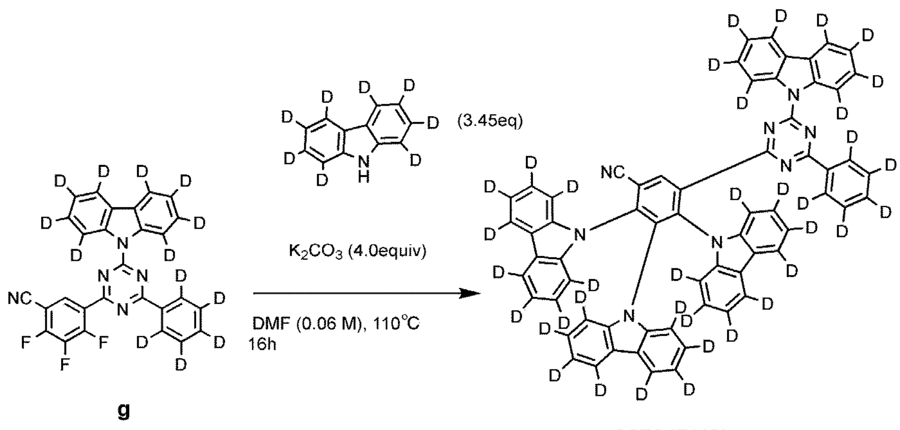


g

937647(40)

Compound 937647(40)

Potassium carbonate (1.13 g, 8.15 mmol) was added to an N,N-dimethylformamide (34 mL) solution of Compound g (1.0 g, 2.03 mmol) and carbazole-1,2,3,4,5,6,7,8-$d_8$ (1.23 g, 7.03 mmol), and stirred at 110°C for 16 hours. Ion-exchanged water and methanol were added to the reaction solution for filtration. The resultant yellow solid was purified by silica gel chromatography (toluene/hexane/chloroform =6/3.5/0.5). The resultant solid was reprecipitated with toluene/methanol to give an intensely orange compound 937647(40) in 1.40 g (1.46 mmol, 72% yield).

$^1$H-NMR (400 MHz, $CHCl_3$-d): δ 8.96 (s, 1H).
ASAP MS Spectrometry: $C_{64}HD_{37}N_8$: theoretical value 955.55, observed value 957.07 [M+H$^+$]

(Synthesis Example 6) Synthesis of Compound 935051(866)

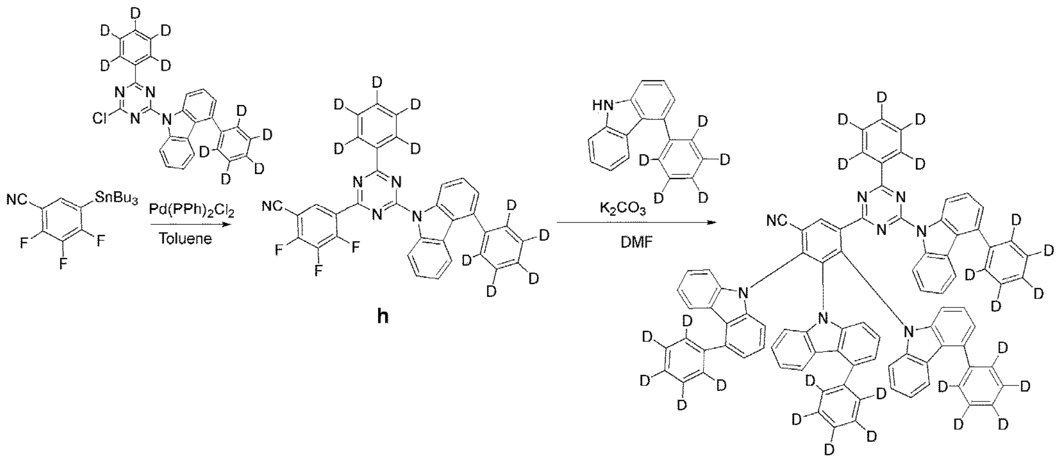


935051(866)

Using the same starting material as in Synthesis Example 5, the reactants were changed as in the reaction formula above, and the product was purified by the reaction in the same manner as in Synthesis Example 5. Compound h was obtained in 62% yield, and compound 935051(866) was obtained in 54% yield.

Compound h

$^1$H-NMR (400 MHz, $CHCl_3$-d): δ 9.11 (d, J = 8.4 Hz, 1H), 9.06 (d, J = 8.4 Hz, 1H), 8.68 (td, J =6.0 Hz and 2.4 Hz, 1H), 7.61 (t, J = 8.4 Hz, 1H), 7.50 (t, J = 8.4 Hz, 1H), 7.30 (d, J = 7.6 Hz, 1H), 7.27-7.25 (m, 1H), 7.14 (t, J = 8.4 Hz, 1H). ASAP MS Spectrometry: $C_{64}HD_{37}N_8$: theoretical value 563.21, observed value 564.43 [M+H$^+$]

Compound 935051(866)

$^1$H-NMR (400 MHz, $CHCl_3$-d): δ 9.03 (s, 1H), 8.87-8.83 (m, 2H), 7.51-7.46 (m, 1H), 7.42-7.33 (m, 3H), 7.27-7.23 (m, 2H), 7.16-6.53 (m, 20H).

(Synthesis Example 7) Synthesis of Compound 937647(866)

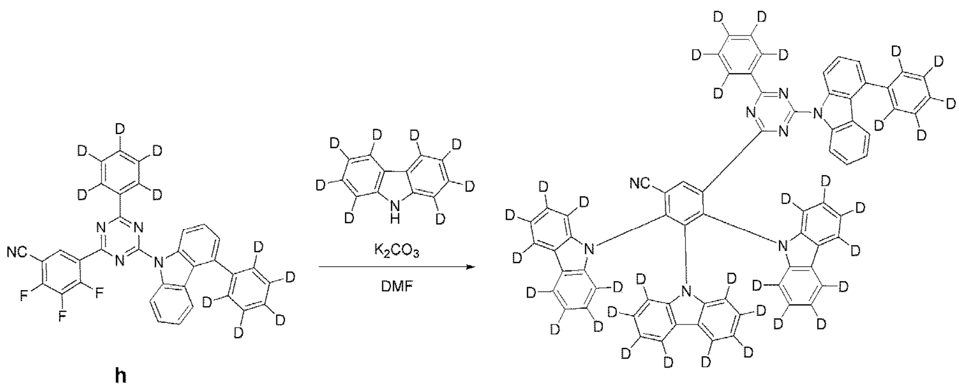


h

937647(866)

Compound 937647(866)

Compound h and carbazole-1,2,3,4,5,6,7,8-$d_8$ were reacted in the same manner as in Example 5 to give

compound 937647 (866) in 32% yield.
ASAP MS Spectrometry: $C_{70}H_8D_{34}N_8$: theoretical value 1028.57, observed value 1030.01 [M+H+]

(Synthesis Example 8) Synthesis of Compound 992174

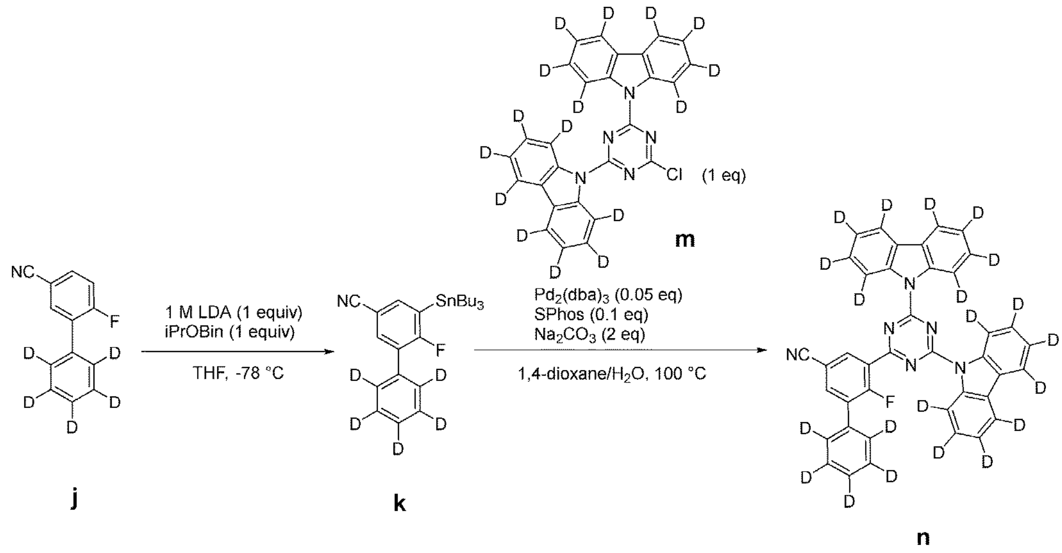

m
j
k
n

Compound n

Under a nitrogen stream, a tetrahydrofuran/hexane solution of 1.0 M lithium diisopropylamide (8.5 mL, 8.5 mmol) was gradually dropwise added to a tetrahydrofuran (THF) (35 mL) solution of Compound j (1.7 g, 8.40 mmol) at -78°C. After dropwise addition, this was stirred for 30 minutes, then pinacol isopropoxyboronate (1.5 g, 8.40 mmol) was added, and further stirred for 30 minutes. The reaction vessel was cooled to -60°C, 7 mL of ion exchanged water was added, then heated up to room temperature, and tris(dibenzylideneacetone)dipalladium(0) (0.19 g, 0.21 mmol), compound m (3.8 g, 8.4 mmol), sodium carbonate (1.7 g, 16.8 mmol), SPhos (0.35 g, 0.84 mmol), and 1.4-dioxane (35 mL) were added, and heated at 100°C for 14 hours.

The resultant gray solid was filtered out and washed with ion-exchanged water, methanol, ethyl acetate, and hexane. The solid was dissolved in THF, the THF solution was passed through a silica pad, and the filtrate was concentrated to give a gray solid compound n in 0.96 g (1.5 mmol, 18% yield).
ASAP MS Spectrometry: $C_{40}H_2D_{21}FN_6$: theoretical value 627.33, observed value 628.55 [M+H+]

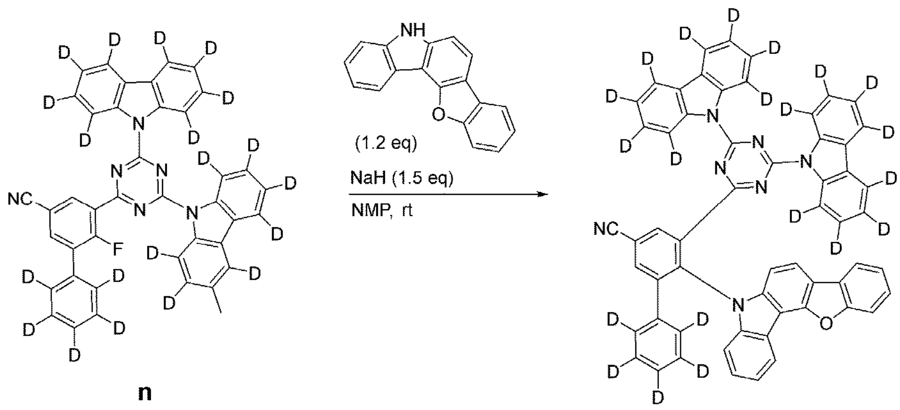

n

992174

Compound 992174

Under a nitrogen stream, a solution of 5H-benzofuro[3,2-c]carbazole (0.44 g, 1.72 mmol) and sodium hydride (60%, 0.086 g, 2.15 mmol) in N-methyl-2-pyrrolidone (5 mL) was added to a solution of Compound X4 (0.90 g, 1.43 mmol) in N-methyl-2-pyrrolidone (40 mL) at room temperature, and stirred at room temperature for 3 hours. To the reaction solution was added a saturated aqueous ammonium chloride solution, and the resultant solid was filtered out and washed with ion-exchanged water and methanol. The resultant reaction mixture was purified by silica gel chromatography (toluene/hexane = 1/1) to give a yellow solid 992174 in 0.39 g (0.45 mmol, 31% yield).
ASAP MS Spectrometry: $C_{58}H_{12}D_{21}N_7O$: theoretical value 864.41, observed value 865.72 [M+H$^+$]

(Synthesis Example 9) Synthesis of Compound 46542(1456)

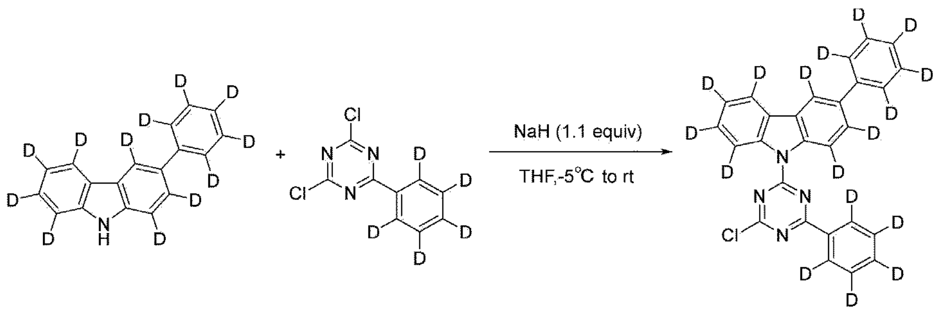


**o**

Compound o

Under a nitrogen atmosphere, a mixture of 3-(phenyl-d5)-9H-carbazole-1,2,4,5,6,7,8-d7 (5.36 g, 21.8 g) and 60 wt% sodium hydride (0.98 g, 24.5 mmol) and THF (100 mL) was stirred at room temperature for 1 hour. The resultant light brown solution was gradually dropwise added to a THF solution (100 mL) of 2,4-dichloro-6-(phenyl-2,3,4,5,6-d5) -1,3,5-triazine (5.04 g, 21.8 mmol) cooled at -5°C. This was heated up to room temperature, then stirred for 1 hour, thereafter cooled to 0°C, and ion-exchanged water was added thereto. After filtration of the white solid, washing with ion exchanged water, methanol, ethyl acetate and hexane in that order gave a white solid compound o in 8.9 g (19.8 mmol, 91% yield).
ASAP MS Spectrometry: $C_{27}D_{17}ClN_4$: theoretical value 449.22, observed value 450.31 [M+H$^+$]

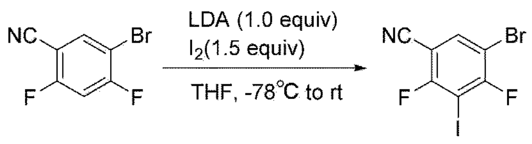


**p**

Compound p

Under a nitrogen stream, a THF solution of 1.0 M lithium diisopropylamide (LDA) (30 mL, 30.0 mmol) was gradually dropwise added to a THF solution (100 mL) of 5-bromo-2,4-difluorobenzonitrile (6.58 g, 30.1 mmol) at -78°C. After stirring for 1 hour, iodine (11.4 g, 45.0 mmol) was added and heated up to room temperature. After stirring for 17 hours, the mixture was cooled to 0°C, and ion-exchanged water and a saturated saline solution were added. After returning to room temperature, this was separated into an organic phase and an aqueous phase, the organic phase was washed with a saturated saline solution, dried with anhydrous magnesium sulfate, and filtered. The filtrate was concentrated and the resultant crude product was purified by silica gel chromatography (hexane/ethyl acetate = 5/1) to give a brown solid compound p in 9.81 g (28.5 mmol, 95% yield).

$^1$H NMR (400 MHz, $CDCl_3$): $\delta$ 7.86 (t, J = 6.8 Hz, 1H).

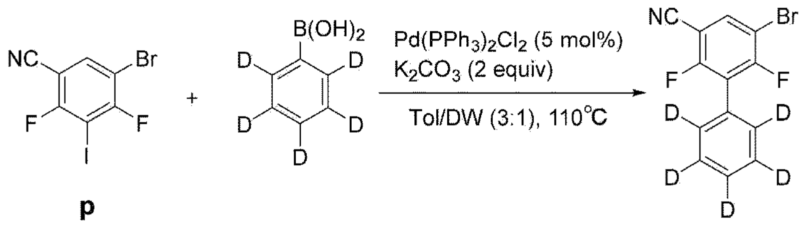


p q

Compound q

To a solution of Compound p (9.80 g, 28.5 mmol) in a mixture of toluene (75 mL) and ion-exchanged water (30 mL), phenyl-d5-boronic acid (3.8 g, 29.9 mmol), bis(triphenylphosphine)palladium (II) dichloride (1.0 g, 1.42 mmol) and potassium carbonate (7.88 g, 57.2 mmol) were added and the mixture was stirred at 110°C for 18 hours under a nitrogen atmosphere. The reaction solution was cooled to room temperature, and a saturated saline solution was added thereto to separate the solution into an organic phase and an aqueous phase. After extracting the aqueous phase with toluene, the combined organic phase was dried with anhydrous magnesium sulfate, filtered, and the filtrate was concentrated. The crude product was purified by silica gel chromatography (toluene/hexane = 1/1) to give a white solid compound q in 6.1 g (20.4 mmol, 71% yield).

$^1H$ NMR (400 MHz, $CDCl_3$): δ 7.84 (t, J=6.8 Hz, 1H).

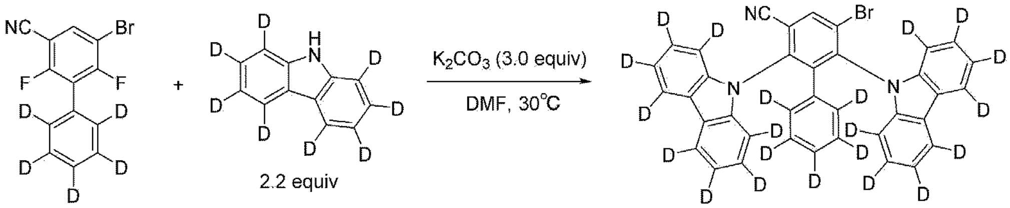


q r

Compound r

Potassium carbonate (8.46 g, 61.2 mmol) was added to an N,N-dimethylformamide (DMF, 200 mL) solution of Compound q (6.10 g, 20.4 mmol) and carbazole-1,2,3,4,5,6,7,8-d8 (7.88 g, 45.0 mmol), and stirred at 30 °C under a nitrogen atmosphere for 25 hours. Ion-exchanged water was added, and the resultant solid was filtered and washed with ion-exchanged water, methanol, and hexane. The resultant solid was purified by silica gel chromatography (toluene/hexane = 2/1). The resultant solid was washed with methanol to give a white solid compound r in 10.3 g (16.1 mmol, 83% yield).

$^1H$ NMR (400 MHz, DMSO-d6): δ 8.98 (s, 1H).

ASAP MS Spectrometry: $C_{37}HD_{21}BrN_3$: theoretical value 608.23, observed value 609.35 [M+H+]

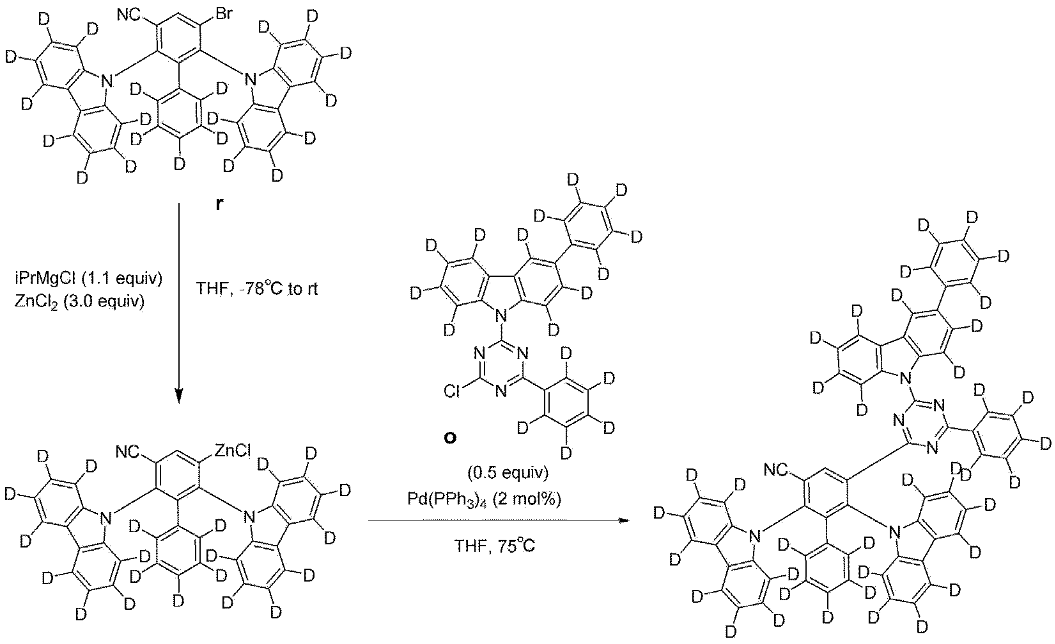


46542(1456)

Compound 46542(1456)

Under a nitrogen stream, a THF solution of 2.0 M isopropylmagnesium chloride (5.5 mL, 11.0 mmol) was gradually dropwise added to a THF (65 mL) solution of Compound r (6.11 g, 10.0 mmol) at -78°C. After stirring for 90 minutes, a THF solution of 1.0 M zinc chloride (30.0 ml, 30.0 mmol) was added and stirred for 30 minutes. After returning to room temperature, the mixture was further stirred for 1 hour. Compound o (3.0 g, 6.60 mmol) and tetrakistriphenylphosphine palladium(0) (0.38 g, 0.19 mmol) were added and stirred at 80°C for 20 hours. The reaction vessel was returned to room temperature, and a saturated saline solution and ethyl acetate were added to separate it into an organic phase and an aqueous phase. The resultant crude product was reprecipitated with ethyl acetate/hexane, and the resultant solid was filtered out. The resultant solid was purified by flash column chromatography (toluene/hexane = 2/1), then reprecipitated with ethyl acetate/hexane, and the solid was filtered out to give a pale green solid compound 46542 (1456) in 1.93 g (2.04 mmol, 31% yield).

$^1$H NMR (400 MHz, DMSO-d6): $\delta$ 9.08 (s, 1H).
ASAP MS Spectrometry: $C_{64}HD_{38}N_7$: theoretical value 943.56, observed value 944.66 [$M+H^+$]

(Synthesis Example 10) Synthesis of Compound 984647(40)

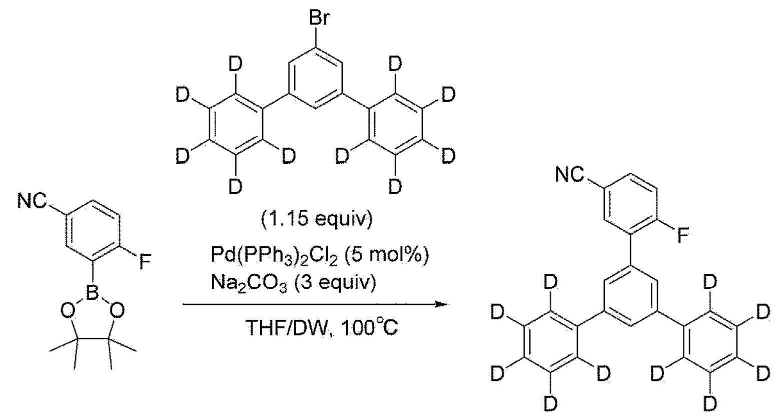


s

Compound s

4-Fluoro-3-(4,4,5,5-tetramethyl-1,3,2-dioxaborolan-2-yl)benzonitrile (1.85 g, 7.49 mmol) was dissolved in 50 mL of a mixed solvent of THF and ion-exchanged water, and 5'-bromo-1,1':3',1"-tert-phenyl-2,2",3,3",4,4",5,5",6,6"-d10 (2.8 g, 8.61 mmol), bis(triphenylphosphine)palladium(II) dichloride (0.26 g, 0.37 mmol), and sodium carbonate (2.38 g, 22.5 mmol) were added and stirred at 100°C overnight. The reaction solution was cooled to room temperature, and a saturated saline solution was added thereto to separate the solution into an organic phase and an aqueous phase. The organic phase was dried with anhydrous magnesium sulfate, filtered, and the filtrate was concentrated. The crude product was purified by silica gel chromatography to give a compound s in 2.2 g (6.2 mmol), 82% yield).
ASAP MS Spectrometry: $C_{25}H_6D_{10}FN$: theoretical value 359.19, observed value 359.24 [M]

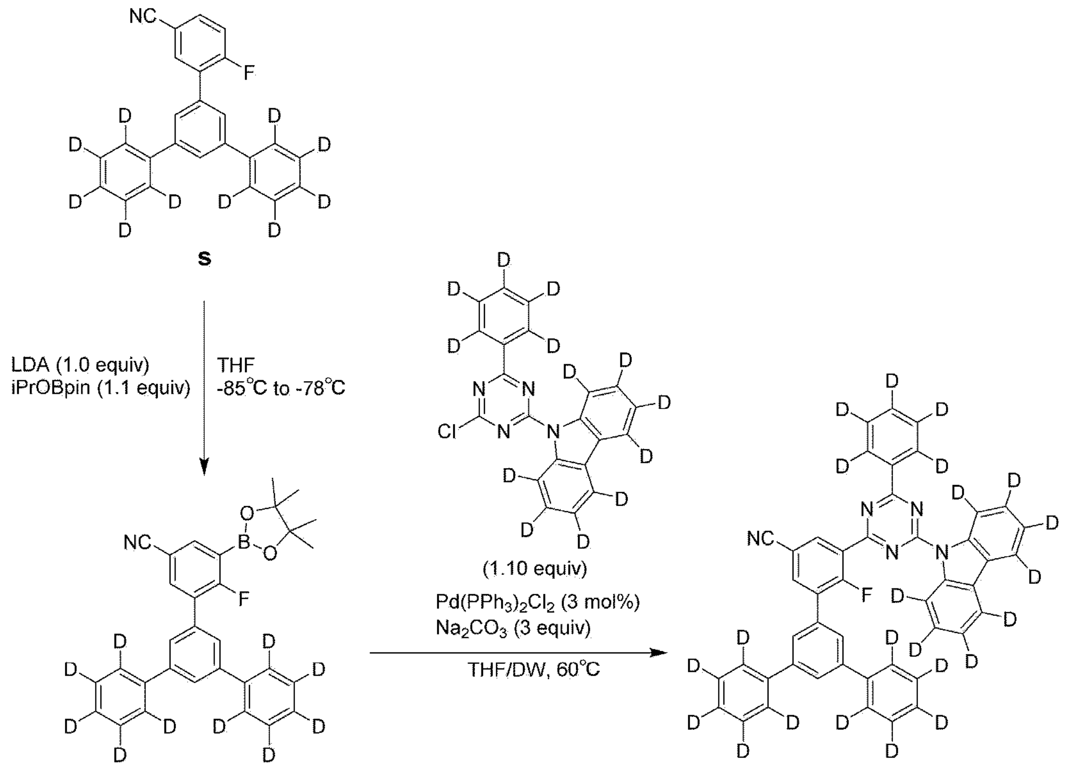

s

t

Compound t

Under a nitrogen atmosphere, a THF solution of 1.0 MLDA (2.8 mL, 2.80 mmol) was gradually dropwise added to a THF solution (20 mL)of Compound s (1.0 g, 2.78 mmol) at -85°C. After stirring for 1 hour, 2-isopropoxy-4,4,5,5-tetramethyl-1,3,2-dioxaborolane (0.57 g, 3.06 mmol) was added at -78°C, and stirred for 1 hour. After adding ion-exchanged water, this was heated up to room temperature, and 9-(4-chloro-6-(phenyl-d5)-1,3,5-triazin-2-yl)-9H-carbazole-1,2,3,4,5,6,7,8-d8 (1.13 g, 3.06 mmol), bis(triphenylphosphine)palladium(II) dichloride (0.05 g, 0.08 mmol), and sodium carbonate (0.44 g, 4.17 mmol) were added and stirred at 60°C overnight. The reaction solution was cooled to room temperature, the solid was filtered out, and washed with ion-exchanged water to give a compound t in 1.4 g (2.02 mmol, 73% yield).
ASAP MS Spectrometry: $C_{46}H_5D_{23}FN_5$: theoretical value 692.38, observed value 692.47 [M]

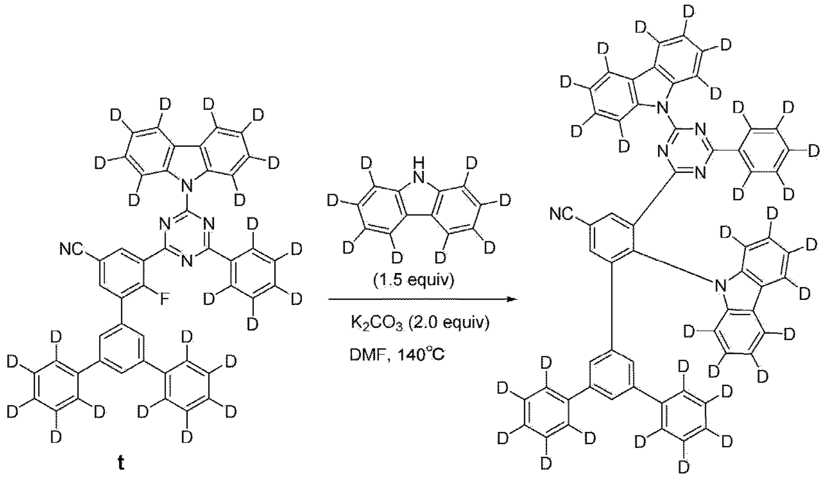


t

984647(40)

Compound 984647(40)

Potassium carbonate (0.32 g, 2.31 mmol) was added to a mixture of Compound t (0.80 g, 1.15 mmol), carbazole-1,2,3,4,5,6,7,8-d8 (0.30 g, 1.73 mmol) and DMF (70 mL), and stirred at 140 °C under a nitrogen atmosphere. After confirming the disappearance of the raw material, the reaction vessel was cooled to room temperature and ion-exchanged water was added. The resultant solid was filtered out and washed with ion-exchanged water and methanol. The resultant solid was purified by silica gel chromatography to give Compound 984647(40) in 0.60 g (0.71 mmol, 62% yield).

$^1$H NMR (400 MHz, $CDCl_3$): δ 8.67 (d, J=2.0 Hz, 1H), 8.28 (d, J=1.6 Hz, 1H), 7.46 (t, J=1.6 Hz, 1H), 7.23 (d, J=1.2 Hz, 1H).
ASAP MS Spectrometry: $C_{58}H_6D_{30}N_6$: theoretical value 846.49, observed value 847.65 [M+H$^+$]

(Synthesis Example 11) Synthesis of Compound 937647(33)

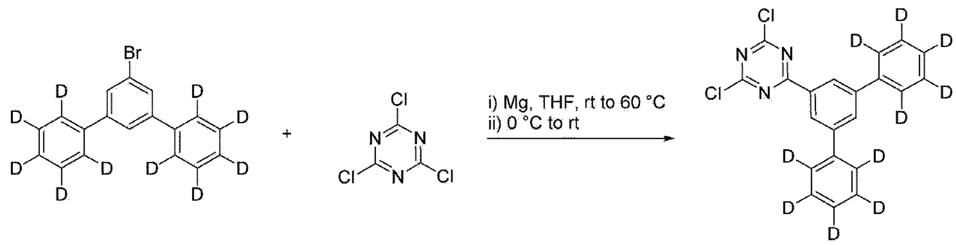


u

Compound u

Under a nitrogen atmosphere, a THF 10 mL solution of 5'-bromo-1,1':3',1"-tert-phenyl-2,2",3,3",4,4",5,5",6,6"-d10 (1.01 g, 3.16 mmol) was added dropwise to magnesium (0.0962 g, 3.95 mmol) at room temperature, and then heated up to 60°C and stirred for 3 hours. The resultant solution was gradually dropwise added to a THF solution (10 mL) of 2,4,6-trichloro-1,3,5-triazine (0.596 g, 3.23 mmol) cooled to 0°C. After stirring at 0°C for 30 minutes, this was heated up to room temperature and stirred for 23 hours. This was cooled to 0°C, then 100 mL of a saturated aqueous ammonium chloride solution was added, and extracted with ethyl acetate. The combined organic layer was washed with a saturated saline solution, dried with anhydrous magnesium sulfate, filtered, and the filtrate was concentrated. The resultant solid was purified by silica gel chromatography (hexane/methylene chloride = 5/1). The resultant solid was washed with hexane to give a white solid compound u in 0.443 g (1.14 mmol, 36% yield).

$^1H$ NMR (400 MHz, $CDCl_3$): δ 8.72 (d, J = 1.6 Hz, 2H), 8.09 (t, J = 1.6 Hz, 1H).
ASAP MS Spectrometry: $C_{21}H_3D_{10}Cl_2N_3$: theoretical value 387.11, observed value 388.05 [M+H$^+$]

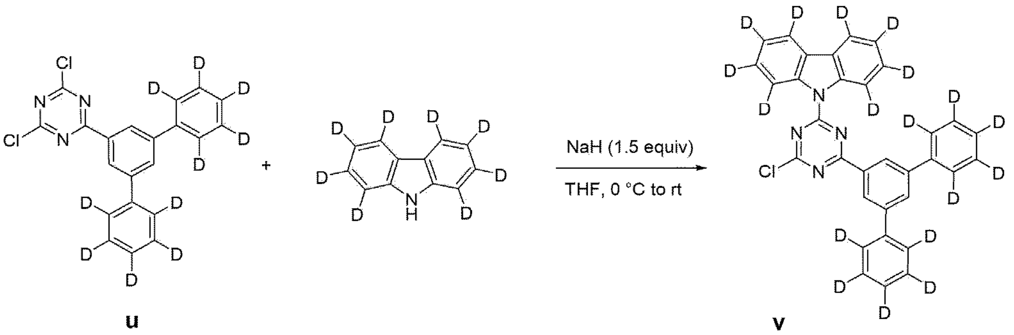


u

v

Compound v

Under a nitrogen atmosphere, a mixture of carbazole-1,2,3,4,5,6,7,8-d8 (0.201 g, 0.507 mmol), 60 wt% sodium hydride (0.0303 g, 0.757 mmol) and THF (2.5 mL) was stirred at room temperature for 1 hour. The resultant solution was gradually dropwise added to a THF solution (2.5 mL) of Compound u (0.201 g, 0.517 mmol) cooled to 0°C. This was heated up to room temperature, then stirred for 1 hour, thereafter cooled to 0°C, and an aqueous saturated ammonium chloride solution was added thereto. A white solid was filtered out, and washed with ion-exchanged water and methanol to give a white solid compound v in 0.212 g (0.402 mmol, 79.3% yield).

$^1H$ NMR (400 MHz, $CDCl_3$): δ 8.84 (d, J = 2.0 Hz, 2H), 8.12 (t, J = 2.0 Hz, 1H).
ASAP MS Spectrometry: $C_{33}H_3D_{18}ClN_4$: theoretical value 526.26, observed value 527.30 [M+H$^+$]

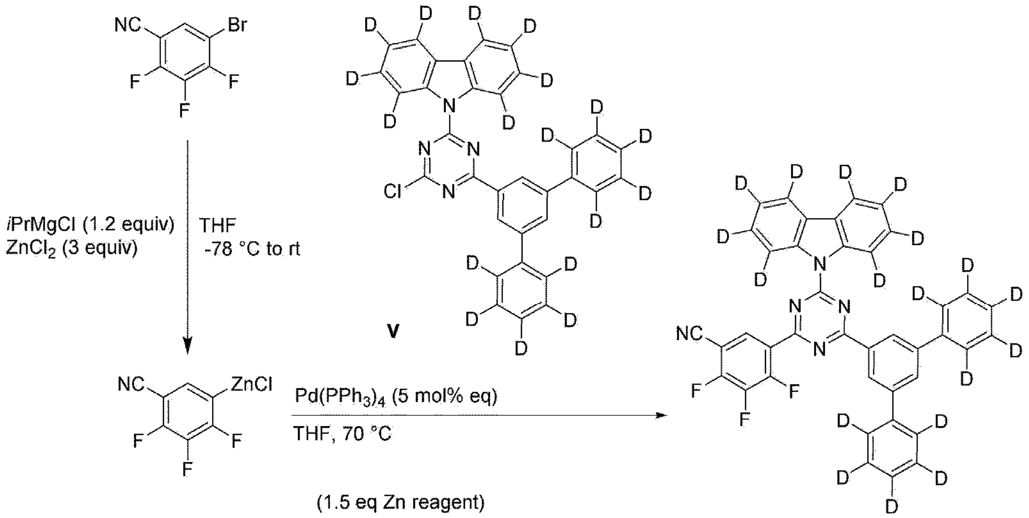


w

Compound w

Under a nitrogen stream, a THF solution of 2.0 M isopropylmagnesium chloride (0.4 mL, 0.8 mmol) was slowly dropwise added to a THF (2.4 mL) solution of 5-bromo-2,3,4-trifluorobenzonitrile (0.141 g, 0.600 mmol) at -78°C. After stirring for 30 minutes, a THF solution of 1.0 M zinc chloride (1.8 mL, 1.8 mmol) was added and stirred for 1 hour. After restoring to room temperature, the mixture was further stirred for 30 minutes. Compound v (0.201 g, 0.381 mmol) and tetrakistriphenylphosphine palladium(0) (0.0216 g, 0.0186 mmol) were added and stirred at 70°C for 16 hours. The reaction vessel was restored to room temperature, and the precipitated solid was filtered out, and washed with ion-exchanged water, methanol, ethyl acetate and hexane to give a white solid compound w in 0.168 g (0.259 mmol, 67.9% yield).
ASAP MS Spectrometry: $C_{40}H_4D_{18}F_3N_5$: theoretical value 647.30, observed value 648.35 [M+H$^+$]

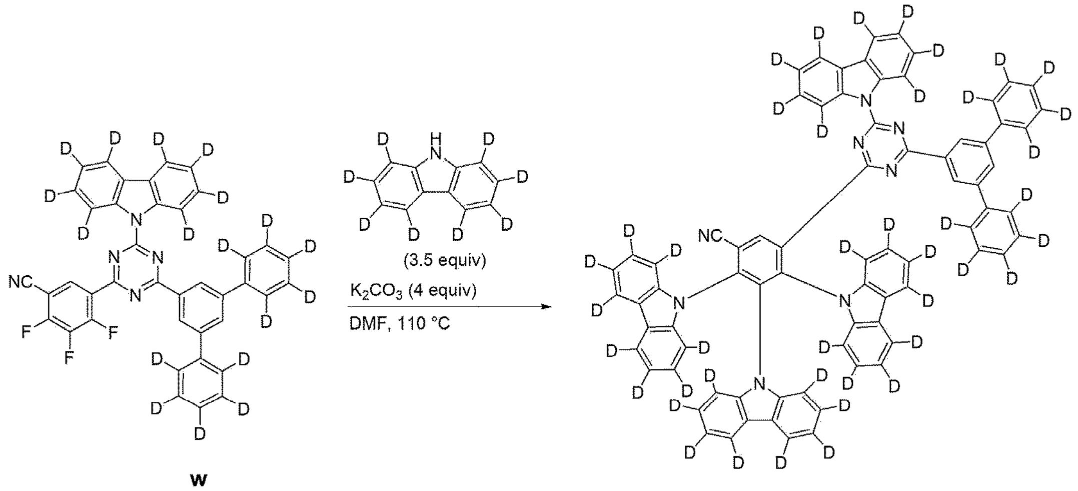


w

**937647(33)**

Compound 937647(33)

Potassium carbonate (0.646 g, 4.67 mmol) was added to a DMF (20 mL) solution of Compound w (0.763 g, 1.17 mmol) and carbazole-1,2,3,4,5,6,7,8-d8 (0.719 g, 4.10 mmol), and stirred at 110°C for 17 hours under a nitrogen atmosphere. After adding ion-exchanged water and extracting with methylene chloride, the organic phase was washed with a saturated saline solution, dried with magnesium sulfate, and filtered out. Concentration and purification of the resulting solid by silica gel chromatography (hexane/methylene chloride = 2/1) gave a yellow solid compound 937647(33) in 0.63 g (0.565 mmol, 48.3% yield).

$^1$H NMR (400 MHz, $CDCl_3$): $\delta$ 8.94 (s, 1H), 8.17 (d, J = 2.0 Hz, 1H), 7.93 (t, J = 2.0 Hz, 1H) .
ASAP MS Spectrometry: $C_{76}H_4D_{42}N_8$: theoretical value 1112.65, observed value 1113.76 [M+H$^+$]

(Synthesis Example 11) Synthesis of Compound 937647(512)

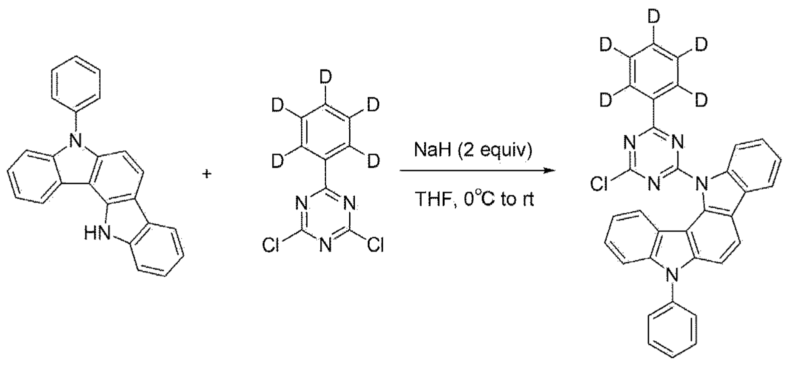


**x**

Compound x

Under a nitrogen atmosphere, a mixture of 5,12-dihydro-5-phenylindole [3,2-a] (8.6 g, 25.8 mmol), 60 wt% sodium hydride (1.2 g, 51.7 mmol) and THF (100 mL) was stirred at room temperature for 1 hour. The resultant light brown solution was gradually dropwise added to a THF solution (100 mL) of 2,4-dichloro-6-(phenyl-2,3,4,5,6-d5)-1,3,5-triazine (6.0 g, 25.9 mmol) cooled to 0°C. This was heated up to room temperature, then stirred for 1 hour, thereafter cooled to 0°C, and ion-exchanged water was added thereto. After filtration of the resultant white solid, washing with ion-exchanged water, methanol, ethyl acetate and hexane in that order gave a white solid compound x in 11.18 g (21.2 mmol, 82% yield).

$^1H$ NMR (400 MHz, $CDCl_3$) : δ 8.83 (dd, J = 7.2, 0.6 Hz, 1H). 8.03-8.06 (m, 2H), 7.67 (m, 4H), 7.40 -7.57 (m, 5H), 7.27-7.31 (m, 1H), 6.98 -7.04 (m, 2H)

ASAP MS Spectrometry: $C_{33}H_{15}D_5ClN_5$: theoretical value 526.17, observed value 527.62 [M+H$^+$]

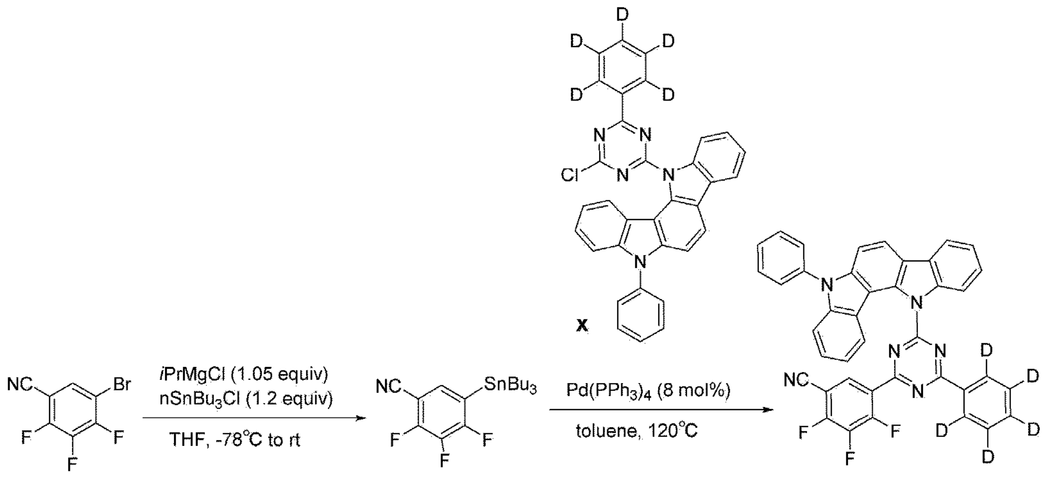


x

y

Compound y

Under a nitrogen atmosphere, a THF solution of 2.0 M isopropylmagnesium chloride (11.1 mL, 22.2 mmol) was slowly dropwise added to a THF (423 mL) solution of 5-bromo-2,3,4-trifluorobenzonitrile (5.0 g, 21.9 mmol) at -78°C. After stirring for 1 hour, tributyltin chloride (6.8 ml, 25.42 mmol) was added and stirred for 30 minutes. After restoring to room temperature, the mixture was further stirred for 3 hours. A saturated aqueous ammonium chloride solution (25 mL) was added, extracted with toluene, dried with a saturated saline solution, dewatered with magnesium sulfate, filtered, and the solvent was evaporated away under reduced pressure. The resultant crude product was dissolved in toluene (211 mL), Compound x (11.18 g, 21.18 mmol) and bis(triphenylphosphine)palladium(II) dichloride (1.19 g, 1.69 mmol) were added and stirred at 120°C for 14 hours. The reaction vessel was returned to room temperature, and a saturated saline solution and ethyl acetate were added to separate it into an organic phase and an aqueous phase. The resultant crude product was dissolved in toluene and led to pass through a silica pad. The silica was washed with toluene, and the filtrate was concentrated. The resultant solid was washed with ethyl acetate and reprecipitated with chloroform/methanol. The solid was filtered out to give a yellow solid compound y in 1.96 g (3.0 mmol, 14.2% yield).

$^1H$ NMR (400 MHz, $CDCl_3$): δ 9.92 (d, J =7.6 Hz, 1H), 7.65-7.73 (m, 5H), 7.45-7.57 (m, 6H), 7.27-7.31(m, 2H), 6.99-7.00(m, 1H),6.80 (t, J =7.6 Hz, 1H)

ASAP MS Spectrometry: $C_{40}H_{16}D_5F_3N_6$: theoretical value 647.21, observed value 647.38 [M+H$^+$]

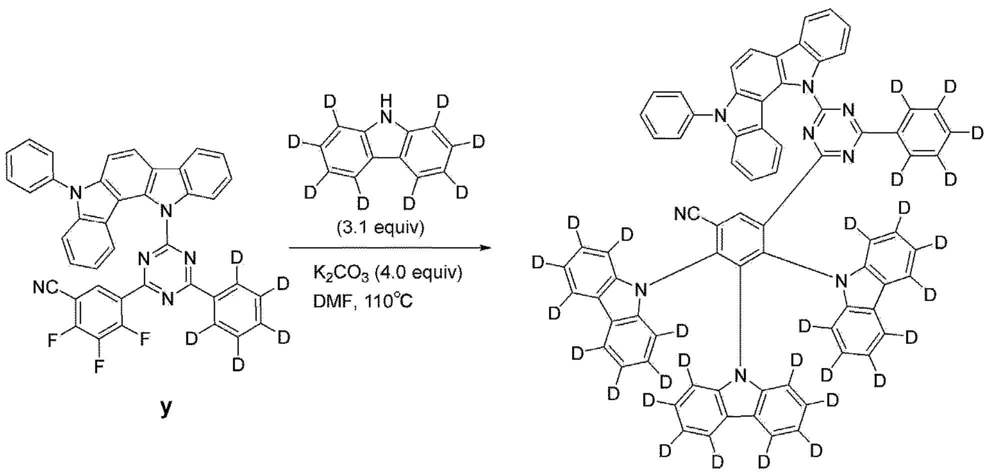


y

937647(512)

Compound 937647(512)

Potassium carbonate (0.93 g, 6.79 mmol) was added to a DMF (34 mL) solution of Compound y (1.1 g, 1.69 mmol) and carbazole-1,2,3,4,5,6,7,8-d8 (0.92 g, 5.26 mmol), and stirred at 110°C for 21 hours. Ion-exchanged water and methanol were added, and the resultant solid was filtered out and washed with methanol and hexane. The resultant solid was purified by silica gel chromatography (hexane/toluene/chloroform =6/3.5/0.5). The resultant solid was washed with methanol to give a yellow solid compound 937647(512) in 1.07 g (0.96 mmol, 53% yield).

$^1$H NMR (400 MHz, $CDCl_3$): δ 8.47 (d, J =7.2 Hz, 1H), 8.06-8.10 (m, 3H),7.61-7.70 (m, 4H),7.39-7.56 (m, 6H), 6.98-7.02 (m, 1H), 6.72 (d, J =8.4 Hz, 1H).
ASAP MS Spectrometry: $C_{76}H_{16}D_{29}N_9$: theoretical value 1112.56, observed value 1112.95 [M+H$^+$]

(Example 1) Preparation and Evaluation of Thin Film

Compound 46542 was vapor-deposited on a quartz substrate by a vacuum deposition method under conditions of a vacuum degree of less than $1 \times 10^{-3}$ Pa to form a neat thin film of Compound 46542 having a thickness of 100 nm.

Separately, Compound 46542 and H1 having the following structure were vapor-deposited from different vapor deposition sources on a quartz substrate by a vacuum deposition method under conditions of a vacuum degree of less than $1 \times 10^{-3}$ Pa to form a doped thin film having a content of Compound 46542 of 30% by weight and a thickness of 100 nm.

In the same manner but using Compound 46542(40), Compound 38317, Compound 38317(40), Compound 46542(1456), Compound 984647(40), Compound 937647(40), Compound 935051(866), Compound 937647(512), Compound 937647(33), and Comparative Compound A, respectively, in place of Compound 46542, neat thin films and doped thin films were formed. However, the concentration of Compound 937647(512) and Compound 937647(33) in the doped thin films was 35% by weight.

The percentage of the delayed fluorescent component and the lifetime (τ2) of the delayed fluorescent component were measured by analyzing the photoluminescence of each doped thin film formed when irradiated with 300 nm excitation light. Also, using the formed neat thin films, the HOMO energy and the LUMO energy were measured. The results are shown in the following Table.

[Table 5]

| | Percentage of Delayed Fluorescent Component (%) | τ2 (nanosecond) | HOMO (eV) | LUMO (eV) |
|---|---|---|---|---|
| Compound 46542 | 66.6 | 3534 | 6.07 | 3.40 |
| Compound 46542(40) | 83.3 | 3125 | 6.05 | 3.35 |
| Compound 38317 | 71.0 | 3312 | 6.06 | 3.35 |
| Compound 38317(40) | 85.0 | 2889 | 6.08 | 3.42 |
| Compound 46542(1456) | 83.7 | 2837 | 6.03 | 3.36 |
| Compound 984647(40) | 77.6 | 4375 | 6.06 | 3.32 |
| Compound 937647(40) | 72.2 | 2194 | 5.98 | 3.37 |
| Compound 935051(866) | 64.3 | 1599 | 6.03 | 3.50 |
| Compound 937647(512) | - | 1252 | 5.94 | 3.42 |
| Compound 937647(33) | 73.6 | 2553 | 6.02 | 3.44 |
| Comparative Compound A | 62.8 | 8873 | 5.99 | 3.26 |

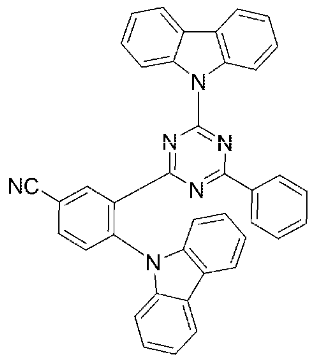

Comparative Compound A

(Example 2) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of $5.0 \times 10^{-5}$ Pa. First, HAT-CN was formed to a thickness of 10 nm on the ITO, NPD was formed to a thickness of 30 nm thereon, further TrisPCz was formed to a thickness of 10 nm, and further H1 was formed to a thickness of 5 nm. Next, H1 and Compound 46542 were co-deposited from different vapor deposition sources to form a layer with a thickness of 40 nm as a light emitting layer. The content of Compound 46542 in the light emitting layer was 30% by weight. Next, after SF3-TRZ was formed at a thickness of 10 nm, Liq and SF3-TRZ were co-deposited from different vapor deposition sources to form a layer with a thickness of 30 nm. The contents of Liq and SF3-TRZ in this layer were 30% by weight and 70% by weight, respectively. Furthermore, Liq was formed to a thickness of 2 nm, and aluminum (Al) was vapor-deposited to a thickness of 100 nm to form a cathode, thereby obtaining an organic electroluminescent device.

Organic electroluminescent devices were produced in the same manner except that Compound 46542(40), Compound 38317(40), Compound 46542(1456) and Comparative Compound A were used instead of Compound 46542.

The lapse time (LT95) until the luminescence intensity reached 95% of that at the start of the test when each organic electroluminescent device was driven at 2.0 $mA/cm^2$ was measured. The results are shown in the following Table. LT95 in the Table is expressed as a relative value, when LT95 of Comparative Compound A is defined as a standard (1). LT95 of each organic electroluminescent device using the compound represented by the general formula (1) was confirmed to be long and the device lifetime was improved.

[Table 6]

| | LT95 |
|---|---|
| Compound 46542 | 6.7 |
| Compound 46542(40) | 7.0 |
| Compound 38317(40) | 8.0 |
| Compound 46542(1456) | 12.2 |
| Comparative Compound A | 1 |

(Example 3) Production and Evaluation of Organic Electroluminescent Device Using Assist Dopant

An organic electroluminescent device was produced in the same manner as in Example 2 only except that a light emitting layer having a thickness of 40 nm was formed by depositing H1, Compound 937647(40) and EM1 as a light emitting material in order of 69.5 % by weight, 30.0 % by weight and 0.5 % by weight from different evaporation sources in place of the light emitting layer in Example 2.

Organic electroluminescent devices were produced in the same manner except that Compound 935051(866) and Comparative Compound A were used instead of Compound 937647(40).

Device lifetime is also improved when the compound represented by the general formula (1) is used as an assist dopant.

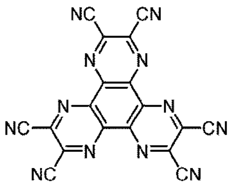
HAT-CN

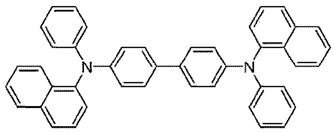
NPD

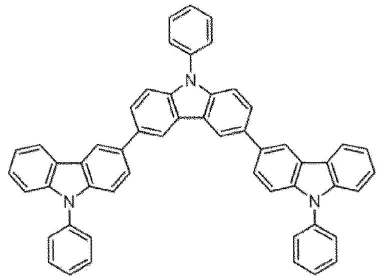
TrisPCz

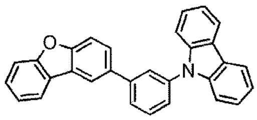
H1

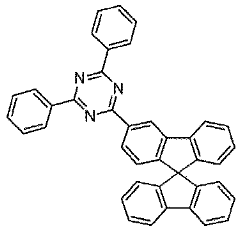
SF3-TRZ

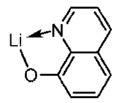
Liq

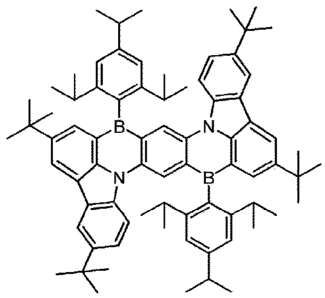
EM1

Industrial Applicability

By using a compound represented by the general formula (1), there can be provided an organic light emitting device having good light emission characteristics. Accordingly, the industrial applicability of the present invention is great.

## Claims

**1.** A compound represented by the following general formula (1):

General Formula (1)

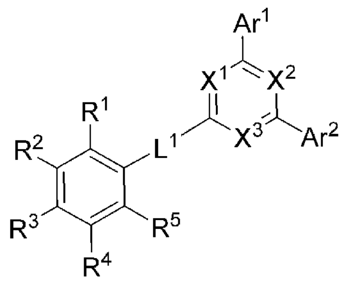

wherein in the general formula (1), $R^1$ to $R^5$ each independently represent a hydrogen atom, a deuterium atom, a cyano group, a substituted or unsubstituted alkyl group, a substituted or unsubstituted aryl group, or a donor group;

one or more of $R^1$ to $R^5$ is a cyano group, one or more of $R^1$ to $R^5$ is a donor group, 0 to 2 of $R^1$ to $R^5$ is a hydrogen atom or a deuterium atom, and 0 to 1 of $R^1$ to $R^5$ is a substituted or unsubstituted aryl group;

$X^1$ to $X^3$ each independently represent N or C(R), and at least one of $X^1$ to $X^3$ is N;

R represents a hydrogen atom, a deuterium atom or a substituent;

$Ar^1$ and $Ar^2$ each independently represent a substituted or unsubstituted aryl group, or a substituted or unsubstituted heteroaryl group containing a nitrogen atom as a ring skeleton-constituting atom, and at least one of $Ar^1$ and $Ar^2$ is a substituted or unsubstituted heteroaryl group bonding via the nitrogen atom; and

$L^1$ represents a single bond or a divalent linking group.

**2.** The compound according to claim 1, wherein only one of $R^1$ to $R^5$ is a cyano group.

3. The compound according to claim 2, wherein $R^2$ is a cyano group.

4. The compound according to claim 1, wherein only one of $R^1$ to $R^5$ is a substituted or unsubstituted aryl group.

5. The compound according to claim 4, wherein $R^4$ is a substituted or unsubstituted aryl group.

6. The compound according to claim 1, wherein two of $R^1$ to $R^5$ are donor groups.

7. The compound according to claim 1, wherein three of $R^1$ to $R^5$ are donor groups.

8. The compound according to claim 1, wherein the donor group is a substituted or unsubstituted carbazol-9-yl group.

9. The compound according to claim 1, wherein $R^3$ to $R^5$ each are independently a substituted or unsubstituted aryl group, or a donor group.

10. The compound according to claim 1, wherein $X^1$ to $X^3$ are N.

11. The compound according to claim 1, wherein $Ar^1$ is a substituted or unsubstituted carbazol-9-yl group, and $Ar^2$ is a substituted or unsubstituted aryl group.

12. The compound according to claim 1, wherein $Ar^1$ and $Ar^2$ each independently represent a substituted or unsubstituted carbazol-9-yl group.

13. The compound according to claim 1, wherein $L^1$ is a single bond.

14. The compound according to claim 1, wherein $R^1$ is a hydrogen atom.

15. The compound according to claim 1, wherein the compound has at least one deuterium atom.

16. A light emitting material comprising the compound according to any one of claims 1 to 15.

17. A delayed fluorescent material comprising the compound according to any one of claims 1 to 15.

18. A film comprising the compound according to any one of claims 1 to 15.

19. An organic semiconductor device comprising the compound according to any one of claims 1 to 15.

20. An organic light emitting device comprising the compound according to any one of claims 1 to 15.

21. The organic light emitting device according to claim 20, wherein the device has a layer containing the compound, and the layer also contains a host material.

22. The organic light emitting device according to claim 21, wherein the layer containing the compound further contains a delayed fluorescent material in addition to the compound and the host material, and the delayed fluorescent material has a lowest excited singlet energy lower than that of the host material and higher than that of the compound.

23. The organic light emitting device according to claim 21, wherein the device has a layer containing the compound, and the layer also contains a light emitting material having a structure different from that of the compound.

24. The organic light emitting device according to claim 21, wherein the amount of light emitted from the compound is the largest among materials contained in the device.

25. The organic light emitting device according to claim 23, wherein the amount of light emitted from the light emitting material is larger than the amount of light emitted from the compound.

26. The organic light emitting device according to claim 20, which is an organic electroluminescent device.

27. The organic light emitting device according to claim 20, which emits delayed fluorescence.

## INTERNATIONAL SEARCH REPORT

International application No.
**PCT/JP2023/033219**

### A. CLASSIFICATION OF SUBJECT MATTER

***C07D 403/14***(2006.01)i; ***C07D 487/04***(2006.01)i; ***H10K 85/60***(2023.01)i; *H10K 101/20*(2023.01)n; *H10K 101/30*(2023.01)n; *H10K 101/40*(2023.01)n; ***H10K 50/12***(2023.01)i; ***H10K 59/10***(2023.01)i
FI: C07D403/14 CSP; C07D487/04 137; H10K50/12; H10K59/10; H10K85/60; H10K101:20; H10K101:40; H10K101:30

According to International Patent Classification (IPC) or to both national classification and IPC

### B. FIELDS SEARCHED

Minimum documentation searched (classification system followed by classification symbols)

C07D403/14; C07D487/04; H10K85/60; H10K50/12; H10K59/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | CN 115340544 A (JIANGSU SUNERA TECHNOLOGY CO., LTD.) 15 November 2022 (2022-11-15) particularly, claims, examples, compounds in page 14 | 1-3, 8, 10-14, 16-27 |
| Y | | 1-27 |
| X | WO 2022/074122 A2 (CYNORA GMBH) 14 April 2022 (2022-04-14) particularly, claims, examples, lower central compounds in page 115 | 1-3, 8, 10-14, 16-27 |
| Y | | 1-27 |
| X | US 2021/0163427 A1 (LG DISPLAY CO., LTD.) 03 June 2021 (2021-06-03) particularly, claims, examples, compounds 202-226 in pages 71-78 | 1-3, 10-11, 13-14, 16-27 |
| Y | | 1-27 |
| X | WO 2020/091521 A1 (LG CHEM, LTD.) 07 May 2020 (2020-05-07) particularly, claims, examples, second compound from the left at the bottom in page 78 of claim 7 | 1-3, 8, 10-11, 13-27 |
| Y | | 1-27 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2023** | **21 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/033219**

**C. DOCUMENTS CONSIDERED TO BE RELEVANT**

| Category* | Citation of document, with indication, where appropriate, of the relevant passages | Relevant to claim No. |
|---|---|---|
| X | EP 3543230 A1 (SAMSUNG DISPLAY CO., LTD.) 25 September 2019 (2019-09-25) particularly, claims, examples, compounds 85, 86, 89, 90 in page 46 and compounds 194, 195, 201 in page 49 of claim 8 | 1-3, 11, 13-14, 16-27 |
| Y | | 1-27 |
| X | US 2019/0058130 A1 (KYULUX, INC.) 21 February 2019 (2019-02-21) particularly, claims, examples, first and fifth compounds in page 85, first compound in page 86 to 88, first and third compounds in page 90, first, third and fourth compounds in pages 91, 92 of claim 15 | 1, 2, 6, 8-10, 12, 13, 16-27 |
| Y | | 1-27 |

Form PCT/ISA/210 (second sheet) (January 2015)

**INTERNATIONAL SEARCH REPORT**
**Information on patent family members**

International application No.
**PCT/JP2023/033219**

| Patent document cited in search report | Publication date (day/month/year) | Patent family member(s) | Publication date (day/month/year) |
|---|---|---|---|
| CN 115340544 A | 15 November 2022 | (Family: none) | |
| WO 2022/074122 A2 | 14 April 2022 | CN 116323615 A<br>particularly, claims, examples<br>KR 10-2023-0085911 A | |
| US 2021/0163427 A1 | 03 June 2021 | KR 10-2021-0066708 A<br>CN 112851687 A | |
| WO 2020/091521 A1 | 07 May 2020 | CN 112334463 A<br>KR 10-2020-0050893 A | |
| EP 3543230 A1 | 25 September 2019 | US 2019/0296247 A1<br>KR 10-2019-0112231 A<br>CN 110294743 A | |
| US 2019/0058130 A1 | 21 February 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022074122 A1
- WO 2015022974 A
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A

### Non-patent literature cited in the description

- **HANSCH, C.** *Chem. Rev.*, 1991, vol. 91, 165-195